# EUROPEAN PATENT SPECIFICATION

(11) **EP 2 331 709 B1**
(45) Date of publication and mention of the grant of the patent: **29.05.2013**
(21) Application number: 09816956.8
(22) Date of filing: 25.09.2009
(51) Int. Cl.: C12Q 1/68

(54) **GENETIC MARKERS FOR ASSESSING RISK OF DEVELOPING SCHIZOPHRENIA**
GENETISCHE MARKER ZUM TESTEN DES RISIKOS DER ENTWICKLUNG EINER SCHIZOPHRENIE
MARQUEURS GÉNÉTIQUES POUR ÉVALUER LE RISQUE D'APPARITION DE LA SCHIZOPHRÉNIE

(30) Priority: 25.09.2008 US 100176 P
(43) Date of publication of application: 15.06.2011
(73) Proprietor: SureGene LLC, Louisville, KY 40299 (US)
(72) Inventor: BRENNAN, Mark, David, Jeffersonville, IN 47130 (US); RAMSEY, Timothy, Lynn, Shelbyville Kentucky 40065 (US)
(74) Representative: Peterreins, Frank
(86) International application number: PCT/US2009/058487
(87) International publication number: WO 2010/036969

(56) References cited:
- RILEY BRIEN ET AL: "Molecular genetic studies of schizophrenia", EUROPEAN JOURNAL OF HUMAN GENETICS, KARGER, BASEL, CH, vol. 14, no. 6, 1 June 2006 (2006-06-01), pages 669-680, XP002519427, ISSN: 1018-4813, DOI: 10.1038/SJ.EJHG.5201571
- BERTOLINO ET AL: "COMT Val<158>Met polymorphism predicts negative symptoms response to treatment with olanzapine in schizophrenia", SCHIZOPHRENIA RESEARCH, ELSEVIER, NETHERLANDS, vol. 95, no. 1-3, 29 August 2007 (2007-08-29), pages 253-255, XP022217143, ISSN: 0920-9964, DOI: 10.1016/J.SCHRES.2007.06.014
- BRAFF ET AL.: 'Advances in endophenotyping schizophrenia' WORLD PSYCHIATRY vol. 7, no. 1, February 2008, page LL-18, XP008144731
- O'DONOVAN ET AL.: 'Identification of loci associated with schizophrenia by genome-wide association and follow-up' NAT. GENET. vol. 40, no. 9, 30 July 2008, pages 1053 - 1055, XP008144669
- LIU ET AL.: 'HTF9C gene of 22qll.21 region associates with schizophrenia hav ing deficit-sustained attention' PSYCHIATR. GENET. vol. 17, no. 6, December 2007, pages 333 - 338, XP008144671
- PINHEIRO ET AL.: 'AKT1 and neurocognition in schizophrenia' AUST. N. Z. J. PSYCHIATRY. vol. 41, no. 2, February 2007, pages 169 - 177, XP008144668

## Description

### TECHNICAL FIELD

This invention relates to genetic markers of schizophrenia (SZ). For example, methods of using such genetic markers for assessing risk of developing SZ are provided.

### BACKGROUND

The schizophrenia spectrum disorders include schizophrenia (SZ), schizotypal personality disorder (SPD), and/or schizoaffective disorder (SD). Schizophrenia (SZ) is considered a clinical syndrome, and is probably a constellation of several pathologies. Substantial heterogeneity is seen between cases, which is thought to reflect multiple overlapping etiologic factors, including both genetic and environmental contributions. SD is characterized by the presence of affective (depressive or manic) symptoms and schizophrenic symptoms within the same, uninterrupted episode of illness. SPD is characterized by a pervasive pattern of social and interpersonal deficits marked by acute discomfort with, and reduced capacity for, close relationships as well as by cognitive or perceptual distortions and eccentricities of behavior, beginning by early adulthood and present in a variety of contexts.

Various genes and chromosomes have been implicated in etiology of SZ. Many studies have suggested the presence of one or more important genes relating to SZ on most or all of the autosomes (Williams et al., Hum. Mol. Genet. 8:1729-1739 (1999); Fallin et al., Am. J. Hum. Genet. 77:918-936 (2005); ; Badner et al., Mol. Psychiatry 7:405-411 (2002); Cooper-Casey et al., Mol. Psychiatry 10:651-656 (2005); Devlin et al., Mol. Psychiatry 7:689-694 (2002); Fallin et al., Am. J. Hum. Genet. 73:601-611 (2003); Jablensky, Mol. Psychiatry 11: 815-836 (2006); Kirov et al., J. Clin. Invest. 115:1440-1448 (2005); Norton et al., Curr. Opin. Psychiatry 19:158-164 (2006); Owen et al., Mol. Psychiatry 9:14-27 (2004)). However, none of these prior studies have used high resolution genetic association methods to systematically compare genes involved in SZ. Neither have any of these studies demonstrated that genetic polymorphisms in the genes defined herein are important, in particular in the genetic etiology of SZ.

Due to the severity of these disorders, especially the negative impact of a psychotic episode on a patient, and the diminishing recovery after each psychotic episode, there is a need to more conclusively identify individuals who have or are at risk of developing schizophrenia (SZ), schizotypal personality disorder (SPD), or schizoaffective disorder (SD), for example, to confirm clinical diagnoses, to allow for prophylactic therapies, to determine appropriate therapies based on their genotypic subtype, and to provide genetic counseling for prospective parents with a history of the disorder.

### SUMMARY

The present invention relates to a method of determining risk of developing schizophrenia (SZ) as set forth in the claims. The disclosure provides methods for determining a subject's risk of developing
schizophrenia based on detecting variations in genes involved in a number of pathways including: glutamate signaling and metabolism, cell adhesion, cytoskeletal architecture, vesicle formation, and trafficking, G-protein coupled receptors, carrier proteins and transporters, cell cycle modulators, neuronal development, calcium/calmodulin signaling, neuropeptide signaling, and several additional genes identified by virtue of their interaction with genes in high impact pathways and their expression in the central nervous system. This disclosure also provides methods and materials relating to determining the genetic risk of developing an SSD (e.g., SZ).
For example, the allelic and genotypic variants identified as described herein can be used for assessing genetic risk. Specifically, the disclosure includes methods for assessing genetic risk based on evaluation of 1025 exemplary SNPs relating to SZ risk. Additionally, the specific SNPs can be used to capture copy number variation related to SZ risk. The allelic and genotypic variants thus identified can be used for assessing genetic risk.

This document features methods for determining risk of developing schizophrenia (SZ) in a human subject. Methods can include determining the identity of at least one allele of a single nucleotide polymorphism (SNP) listed in Table 1 (e.g., at nucleotide 31 of SEQ ID 656, 665, 248, 639, 25, 494, 700, or 691); and comparing the identity of the allele in the subject with a reference allele, wherein the reference allele is associated with a known risk of developing SZ; and wherein the presence of an allele in the subject that is the same as the reference allele that is associated with the known risk of developing SZ indicates the risk that the subject will develop SZ.

The invention features a method for determining risk of developing SZ in a human subject. Methods can include: determining the copy number of at least one single nucleotide polymorphism (SNP), wherein the SNP is rs2180782 in BMP7 or a polymorphism that is in linkage disequilibrium (LD) with rs2180782 in BMP7: and comparing the copy number of the SNP in the subject with a reference copy number, wherein the reference copy number is associated with a known risk of developing SZ; and wherein the presence of a copy number of the SNP in the subject that is the same as a reference copy number that is associated with SZ indicates that the risk that the subject will develop SZ.

Determining the identity or copy number of an allele can include obtaining a sample comprising DNA from the subject, and determining identity or copy number of the nucleotide at the polymorphic site. Determining the identity of the nucleotide can include contacting the sample with a probe specific for a selected allele of the polymorphism, and detecting the formation of complexes between the probe and the selected allele of the polymorphism, wherein 20 the formation of complexes between the probe and the test marker indicates the presence of the selected allele in the sample. Determining the identity of an allele can include determining the identity of the nucleotide at position 31 of SEQ ID 1003. Determining the copy number of an allele can include contacting the sample with a probe specific for a selected allele, detecting the formation of complexes between the probe and the selected allele of the polymorphism, and quantifying the complexes, wherein the quantification of the complexes indicates the copy number of the selected allele in the sample. A probe can include a fluorescent label, and quantifying the complexes comprises detecting intensity of emission from the label. Determining the copy number of an allele can include detecting the absence or duplication of the allele in the subject. A subject can be a patient having or suspected of having SZ. A subject can 30 have one or more risk factors associated with SZ. Risk factors associated with SZ can include one or more of: a relative afflicted with SZ; and a genetically based phenotypic trait associated with risk for a SZ. A subject can have exhibited or exhibits symptoms of psychosis. Methods can further include selecting or excluding a subject for enrollment in a clinical trial based on the identity of the allele. Methods can further include stratifying a subject population for analysis of a clinical trial based on the identity of the allele in the subjects. Methods can further include confirming a diagnosis of a SZ using psychometric instruments. Methods can further include selecting a treatment for SZ if an allele in the subject is the same as a reference allele associated with SZ. Methods can further include selecting a treatment for SZ if the copy number of the allele in the subject is the same as a reference copy number associated with SZ. Methods can further include administering the selected treatment to the subject. A treatment can include one or more of an anti-psychotic drug, an anti-depressant drug, anti-anxiety drug, mood stabilizer, selective serotonin reuptake inhibitor (SSRI), psychotherapy, or a stimulant. Methods can further include recording the identity of the allele in a tangible medium. A tangible medium can include a computer-readable disk, a solid state memory device, or an optical storage device.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials arc described below.

The details of one or more embodiments of the invention are set forth in the accompanying drawings and the description below. Other features, objects, and advantages of the invention will be apparent from the description and drawings, and from the claims.

### DETAILED DESCRIPTION

This document provides methods for assessing genetic risk for developing SSDs based on evaluation of single nucleotide polymorphisms (SNPs) for genes relating to SSDs including schizophrenia (SZ), schizotypal personality disorder (SPD), and schizoaffective disorder (SD). As described herein, bioinformatic and genetic analyses provided evidence of association of the disclosed SNPs and haplotypes SZ. Specific allelic and copy number variants identified herein can be used to assess genetic risk.

### Definitions

As used herein, an "allele" is one of a pair or series of genetic variants of a polymorphism at a specific genomic location. An "SZ risk allele" is an allele that is associated with increased risk of developing SZ. An "SZ allele" is an allele that is statistically associated with a diagnosis of SZ.

As used herein, "genotype" refers to the diploid combination of alleles for a given genetic polymorphism. A homozygous subject carries two copies of the same allele and a heterozygous subject carries two different alleles.

As used herein, "genetic model" refers to the manner in which an allele influences risk or differential diagnosis. In a "dominant model" the allele impacts the clinical state to the same extent whether present in one copy or two copies, i.e., whether homozygous or heterozygous. In a "recessive model" the allele impacts the clinical state only when homozygous. In an "additive model" the allele impacts the clinical state in proportion to the number of copies present, i.e., the homozygous state has twice the impact of the heterozygous state.

As used herein, a "haplotype" is one or a set of signature genetic changes (polymorphisms) that are normally grouped closely together on the DNA strand, and are usually inherited as a group; the polymorphisms are also referred to herein as "markers." A "haplotype" as used herein is information regarding the presence or absence of one or more genetic markers in a given chromosomal region in a subject. A haplotype can consist of a variety of genetic markers, including indels (insertions or deletions of the DNA at particular locations on the chromosome); single nucleotide polymorphisms (SNPs) in which a particular nucleotide is changed; microsatellites; and minisatellites.

Microsatellites (sometimes referred to as a variable number of tandem repeats or VNTRs) are short segments of DNA that have a repeated sequence, usually about 2 to 5 nucleotides long (e.g., CACACA), that tend to occur in non-coding DNA. Changes in the microsatellites sometimes occur during the genetic recombination of sexual reproduction, increasing or decreasing the number of repeats found at an allele, changing the length of the allele. Microsatellite markers are stable, polymorphic, easily analyzed and occur regularly throughout the genome, making them especially suitable for genetic analysis.

"Copy number variation" (CNV), as used herein, refers to variation from the normal diploid condition for a gene or polymorphism. Individual segments of human chromosomes can be deleted or duplicated such that the subject's two chromosome carry fewer than two copies of the gene or polymorphism (a deletion or deficiency) or two or more copies (a duplication).

"Linkage disequilibrium" refers to when the observed frequencies of haplotypes in a population does not agree with haplotype frequencies predicted by multiplying together the frequency of individual genetic markers in each haplotype.

The term "chromosome" as used herein refers to a gene carrier of a cell that is derived from chromatin and comprises DNA and protein components (e.g., histones). The conventional internationally recognized individual human genome chromosome numbering identification system is employed herein. The size of an individual chromosome can vary from one type to another with a given multi-chromosomal genome and from one genome to another. In the case of the human genome, the entire DNA mass of a given chromosome is usually greater than about 100,000,000 base pairs. For example, the size of the entire human genome is about 3 X 10⁹ base pairs.

The term "gene" refers to a DNA sequence in a chromosome that codes for a product (either RNA or its translation product, a polypeptide). A gene contains a coding region and includes regions preceding and following the coding region (termed respectively "leader" and "trailer"). The coding region is comprised of a plurality of coding segments ("exons") and intervening sequences ("introns") between individual coding segments.

The term "probe" refers to an oligonucleotide. A probe can be single stranded at the time of hybridization to a target. As used herein, probes include primers, i.e., oligonucleotides that can be used to prime a reaction, e.g., a PCR reaction.

The term "label" or "label containing moiety" refers in a moiety capable of detection, such as a radioactive isotope or group containing same, and nonisotopic labels, such as enzymes, biotin, avidin, streptavidin, digoxygenin, luminescent agents, dyes, haptens, and the like. Luminescent agents, depending upon the source of exciting energy, can be classified as radioluminescent, chemiluminescent, bioluminescent, and photoluminescent (including fluorescent and phosphorescent). A probe described herein can be bound, e.g., chemically bound to label-containing moieties or can be suitable to be so bound. The probe can be directly or indirectly labeled.

The term "direct label probe" (or "directly labeled probe") refers to a nucleic acid probe whose label after hybrid formation with a target is detectable without further reactive processing of hybrid. The term "indirect label probe" (or "indirectly labeled probe") refers to a nucleic acid probe whose label after hybrid formation with a target is further reacted in subsequent processing with one or more reagents to associate therewith one or more moieties that finally result in a detectable entity.

The terms "target," "DNA target," or "DNA target region" refers to a nucleotide sequence that occurs at a specific chromosomal location. Each such sequence or portion is preferably at least partially, single stranded (e.g., denatured) at the time of hybridization. When the target nucleotide sequences are located only in a single region or fraction of a given chromosome, the term "target region" is sometimes used. Targets for hybridization can be derived from specimens which include, but are not limited to, chromosomes or regions of chromosomes in normal, diseased or malignant human cells, either interphase or at any state of meiosis or mitosis, and either extracted or derived from living or postmortem tissues, organs or fluids; germinal cells including sperm and egg cells, or cells from zygotes, fetuses, or embryos, or chorionic or amniotic cells, or cells from any other germinating body; cells grown in vitro, from either long-term or short-term culture, and either normal, immortalized or transformed; inter- or intraspecific hybrids of different types of cells or differentiation states of these cells; individual chromosomes or portions of chromosomes, or translocated, deleted or other damaged chromosomes, isolated by any of a number of means known to those with skill in the art, including libraries of such chromosomes cloned and propagated in prokaryotic or other cloning vectors, or amplified in vitro by means well known to those with skill; or any forensic material, including but not limited to blood, or other samples.

The term "hybrid" refers to the product of a hybridization procedure between a probe and a target.

The term "hybridizing conditions" has general reference to the combinations of conditions that are employable in a given hybridization procedure to produce hybrids, such conditions typically involving controlled temperature, liquid phase, and contact between a probe (or probe composition) and a target. Conveniently and preferably, at least one denaturation step precedes a step wherein a probe or probe composition is contacted with a target. Guidance for performing hybridization reactions can be found in Ausubel et al., Current Protocols in Molecular Biology, John Wiley & Sons, N.Y. (2003), 6.3.1-6.3.6. Aqueous and nonaqueous methods are described in that reference and either can be used. Hybridization conditions referred to herein are a 50% formamide, 2X SSC wash for 10 minutes at 45°C followed by a 2X SSC wash for 10 minutes at 37°C.

Calculations of "identity" between two sequences can be performed as follows. The sequences are aligned for optimal comparison purposes (e.g., gaps can be introduced in one or both of a first and a second nucleic acid sequence for optimal alignment and non-identical sequences can be disregarded for comparison purposes). The length of a sequence aligned for comparison purposes is at least 30% (e.g., at least 40%, 50%, 60%, 70%, 80%, 90% or 100%) of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In some embodiments, the percent identity between two nucleotide sequences is determined using the GAP program in the GCG software package, using a Blossum 62 scoring matrix with a gap penalty of 12, a gap extend penalty of 4, and a frameshift gap penalty of 5.

As used herein, the term "substantially identical" is used to refer to a first nucleotide sequence that contains a sufficient number of identical nucleotides to a second nucleotide sequence such that the first and second nucleotide sequences have similar activities. Nucleotide sequences that are substantially identical are at least 80% (e.g., 85%, 90%, 95%, 97% or more) identical.

The term "nonspecific binding DNA" refers to DNA which is complementary to DNA segments of a probe, which DNA occurs in at least one other position in a genome, outside of a selected chromosomal target region within that genome. An example of nonspecific binding DNA comprises a class of DNA repeated segments whose members commonly occur in more than one chromosome or chromosome region. Such common repetitive segments tend to hybridize to a greater extent than other DNA segments that are present in probe composition.

As used herein, the term "stratification" refers to the creation of a distinction between subjects on the basis of a characteristic or characteristics of the subjects. Generally, in the context of clinical trials, the distinction is used to distinguish responses or effects in different sets of patients distinguished according to the stratification parameters. In some embodiments, stratification includes distinction of subject groups based on the presence or absence of particular markers or haplotypes described herein. The stratification can be performed, e.g., in the course of analysis, or can be used in creation of distinct groups or in other ways.

### Methods of Diagnoses and Evaluation of Risk

Described herein are a variety of methods for determining susceptibility to an SSD (e.g., SZ). "Susceptibility" does not necessarily mean that the subject will develop an SSD (e.g., SZ), but rather that the subject is, in a statistical sense, more likely to develop SZ than an average member of the population, i.e., has an increased risk of developing an SSD (e.g., SZ). As used herein, susceptibility to an SSD exists if the subject has a genetic variation, e.g., an allele or CNV, associated with an increased risk of an SSD (e.g., SZ) as described herein. Ascertaining whether the subject has such an allele or CNV is included in the concept of diagnosing susceptibility to an SSD (e.g., SZ) as used herein. Such determination is useful, for example, for purposes of diagnosis, treatment selection, and genetic counseling. Thus, the methods described herein can include obtaining a haplotype associated with an increased risk of an SSD (e.g., SZ) as described herein for the subject.

As used herein, "determining the identity of an allele" or "determining copy number" includes obtaining information regarding the identity, number, presence or absence of one or more specific alleles or SNPs in a subject. Determining the identity of an allele or determining copy number can, but need not, include obtaining a sample comprising DNA from a subject, and/or assessing the identity, copy number, presence or absence of one or more genetic markers in the sample. The individual or organization who performs the determination need not actually carry out the physical analysis of a sample from a subject; the methods can include using information obtained by analysis of the sample by a third party. Thus the methods can include steps that occur at more than one site. For example, a sample can be obtained from a subject at a first site, such as at a health care provider, or at the subject's home in the case of a self-testing kit. The sample can be analyzed at the same or a second site, e.g., at a laboratory or other testing facility.

Determining the identity of an allele or copy number of a SNP can also include or consist of reviewing a subject's medical history, where the medical history includes information regarding the identity, copy number, presence or absence of one or more alleles or SNPs in the subject, e.g., results of a genetic test.

In some embodiments, to determine the identity of an allele or copy number/presence/absence of an allele or genotype described herein, a biological sample that includes nucleated cells (such as blood, a cheek swab or mouthwash) is prepared and analyzed for the copy number, presence or absence of preselected markers. Such diagnoses may be performed by diagnostic laboratories, or, alternatively, diagnostic kits can be manufactured and sold to health care providers or to private individuals for self-diagnosis. Diagnostic or prognostic tests can be performed as described herein or using well known techniques, such as described in U.S. Pat. No. 5,800,998.

Results of these tests, and optionally interpretive information, can be returned to the subject, the health care provider or to a third party payor. The results can be used in a number of ways. The information can be, e.g., communicated to the tested subject, e.g., with a prognosis and optionally interpretive materials that help the subject understand the test results and prognosis. The information can be used, e.g., by a health care provider, to determine whether to administer a specific drug, or whether a subject should be assigned to a specific category, e.g., a category associated with a specific disease endophenotype, or with drug response or non-response. The information can be used, e.g., by a third party payor such as a healthcare payer (e.g., insurance company or HMO) or other agency, to determine whether or not to reimburse a health care provider for services to the subject, or whether to approve the provision of services to the subject. For example, the healthcare payer may decide to reimburse a health care provider for treatments for SZ if the subject has SZ or has an increased risk of developing SZ. The presence or absence of the allele or genotype in a patient may be ascertained by using any of the methods described herein.

### Alleles and Genoypes Associated with SSDs

This document provides methods for diagnosing and assessing genetic risk based on evaluation of single nucleotide polymorphisms (SNPs) for genes relating to SZ-spectrum disorders including schizophrenia (SZ), schizotypal personality disorder (SPD) and schizoaffective disorder (SD). Tables 1-3 and Table A list specific exemplary SNPs that can be used to capture significant haplotype variation in these genes. One of skill in the art will appreciate that additional variants can be identified via TDT using families with multiple affected individuals (such as those collected CCGS) and verified by Case/Control comparisons, e.g., using the methods and markers presented herein. Using the SNP markers described herein, one can determine the alleles, copy number, genotypes or haplotypes in these genes relating to diagnosis or genetic risk of developing SZ. This information can then be used to detennine risk of developing SZ, or for making a diagnosis ofSZ. The allelic and genotypic variants thus identified can be used for diagnosis and for assessing genetic risk.

In some embodiments, the methods can include determining the presence of a haplotype that includes one or more polymorphisms near D22S526 and/or the polymorphisms in the *Sult4a1* gene and/or polymorphisms within 1 linkage disequilibrium unit (LDU) of these markers, e.g., as described in U.S. Pat. Pub. No. US2006/0177851.

### Linkage Disequilibrium Analysis

Linkage disequilibrium (LD) is a measure of the degree of association between alleles in a population. One of skill in the art will appreciate that haplotypes involving markers in LD with the polymorphisms described herein can also be used in a similar manner to those described herein. Methods of calculating LD are known in the art (see, e.g., Morton et al., Proc. Natl. Acad. Sci. USA 98(9):5217-21 (2001); Tapper et al., Proc. Natl. Acad. Sci. USA 102(33):11835-11839 (2005); Maniatis et al., Proc. Natl. Acad. Sci. USA 99:2228--2233 (2002)). Thus, in some cases, the methods can include analysis of polymorphisms that are in LD with a polymorphism described herein. Methods are known in the art for identifying such polymorphisms; for example, the International HapMap Project provides a public database that can be used, see hapmap.org, as well as The International HapMap Consortium, Nature 426:789-796 (2003), and The International HapMap Consortium, Nature 437:1299-1320 (2005). Generally, it will be desirable to use a HapMap constructed using data from individuals who share ethnicity with the subject. For example, a HapMap for African Americans would ideally be used to identify markers in LD with an exemplary marker described herein for use in genotyping a subject of African American descent.

Alternatively, methods described herein can include analysis of polymorphisms that show a correlation coefficient (r²) of value ≥ 0.5 with the markers described herein. Results can be obtained from on line public resources such as HapMap.org on the World Wide Web. The correlation coefficient is a measure of LD, and reflects the degree to which alleles at two loci (for example, two SNPs) occur together, such that an allele at one SNP position can predict the correlated allele at a second SNP position, in the case where r² is > 0.5.

### Identifying Additional Genetic Markers

In general, genetic markers can be identified using any of a number of methods well known in the art. For example, numerous polymorphisms in the regions described herein are known to exist and are available in public databases, which can be searched using methods and algorithms known in the art. Alternately, polymorphisms can be identified by sequencing either genomic DNA or cDNA in the region in which it is desired to find a polymorphism. According to one approach, primers are designed to amplify such a region, and DNA from a subject is obtained and amplified. The DNA is sequenced, and the sequence (referred to as a "subject sequence" or "test sequence") is compared with a reference sequence, which can represent the "normal" or "wild type" sequence, or the "affected" sequence. In some embodiments, a reference sequence can be from, for example, the human draft genome sequence, publicly available in various databases, or a sequence deposited in a database such as GenBank. In some embodiments, the reference sequence is a composite of ethnically diverse individuals.

In general, if sequencing reveals a difference between the sequenced region and the reference sequence, a polymorphism has been identified. The fact that a difference in nucleotide sequence is identified at a particular site that determines that a polymorphism exists at that site. In most instances, particularly in the case of SNPs, only two polymorphic variants will exist at any location. However, in the case of SNPs, up to four variants may exist since there are four naturally occurring nucleotides in DNA. Other polymorphisms, such as insertions and deletions, may have more than four alleles.

The methods described herein can also include determining the presence or absence of other markers known or suspected to be associated with risk of bipolar disorder (BD) and/or SZ, or differentially inherited in BD versus SZ. In some embodiments, the methods include determining the presence or absence of one or more other markers that are or may be associated with BD or SZ, e.g., in one or more genes, e.g., e.g., as described in WO 2009/092032, WO 2009/089120, WO 2009/082743, US2006/0177851, and US2009/0012371 See also, e.g., OMIM entry no. 181500 (SCZD).

### Methods of Determining the Identity of an Allele or Obtaining a Genotype

The methods described herein include determining the identity, copy number, presence, or absence of alleles or genotypes associated with diagnosis or risk of developing SZ. In some embodiments, an association with SZ is determined by the statistical likelihood of the presence of an allele or genotype in an individual with SZ, e.g., an unrelated individual or a first or second-degree relation of the subject, and optionally the statistical likelihood of the absence of the same allele, copy number, or genotype in an unaffected reference individual, e.g., an unrelated individual or a first or second-degree relation of the subject. Thus the methods can include obtaining and analyzing a sample from one or more suitable reference individuals.

Samples that are suitable for use in the methods described herein contain genetic material, e.g., genomic DNA (gDNA). Genomic DNA is typically extracted from biological samples such as blood or mucosal scrapings of the lining of the mouth, but can be extracted from other biological samples including urine or expectorant. The sample itself will typically consist of nucleated cells (e.g., blood or buccal cells) or tissue removed from the subject. The subject can be an adult, child, fetus, or embryo. In some embodiments, the sample is obtained prenatally, either from a fetus or embryo or from the mother (e.g., from fetal or embryonic cells in the maternal circulation). Methods and reagents are known in the art for obtaining, processing, and analyzing samples. In some embodiments, the sample is obtained with the assistance of a health care provider, e.g., to draw blood. In some embodiments, the sample is obtained without the assistance of a health care provider, e.g., where the sample is obtained non-invasively, such as a sample comprising buccal cells that is obtained using a buccal swab or brush, or a mouthwash sample.

In some cases, a biological sample may be processed for DNA isolation. For example, DNA in a cell or tissue sample can be separated from other components of the sample. Cells can be harvested from a biological sample using standard techniques known in the art. For example, cells can be harvested by centrifuging a cell sample and resuspending the pelleted cells. The cells can be resuspended in a buffered solution such as phosphate-buffered saline (PBS). After centrifuging the cell suspension to obtain a cell pellet, the cells can be lysed to extract DNA, e.g., gDNA. See, e.g., Ausubel et al., 2003, supra. The sample can be concentrated and/or purified to isolate DNA. All samples obtained from a subject, including those subjected to any sort of further processing, are considered to be obtained from the subject. Routine methods can be used to extract genomic DNA from a biological sample, including, for example, phenol extraction. Alternatively, genomic DNA can be extracted with kits such as the QIAamp® Tissue Kit (Qiagen, Chatsworth, CA) and the Wizard® Genomic DNA purification kit (Promega). Nonlimiting examples of sources of samples include urine, blood, and tissue.

The copy number, absence or presence of an allele or genotype associated SZ as described herein can be determined using methods known in the art. For example, gel electrophoresis, capillary electrophoresis, size exclusion chromatography, sequencing, and/or arrays can be used to detect the presence or absence of the allele or genotype. Amplification of nucleic acids, where desirable, can be accomplished using methods known in the art, e.g., PCR. In one example, a sample (e.g., a sample comprising genomic DNA), is obtained from a subject. The DNA in the sample is then examined to identify or detect the presence of an allele or genotype as described herein. The allele or genotype can be identified or determined by any method described herein, e.g., by sequencing or by hybridization of the gene in the genomic DNA, RNA, or cDNA to a nucleic acid probe, e.g., a DNA probe (which includes cDNA and oligonucleotide probes) or an RNA probe. The nucleic acid probe can be designed to specifically or preferentially hybridize with a particular polymorphic variant.

Other methods of nucleic acid analysis can include direct manual sequencing (Church and Gilbert, Proc. Natl. Acad. Sci. USA 81:1991-1995 (1988); Sanger et al., Proc. Natl. Acad. Sci. USA 74:5463-5467 (1977); Beavis et al., U.S. Pat. No. 5,288,644); automated fluorescent sequencing; single-stranded conformation polymorphism assays (SSCP) (Schafer et al., Nat. Biotechnol. 15:33-39 (1995)); clamped denaturing gel electrophoresis (CDGE); two-dimensional gel electrophoresis (2DGE or TDGE); conformational sensitive gel electrophoresis (CSGE); denaturing gradient gel electrophoresis (DGGE) (Sheffield et al., Proc. Natl. Acad. Sci. USA 86:232-236 (1989)); denaturing high performance liquid chromatography (DHPLC, Underhill et al., Genome Res. 7:996-1005 (1997)); infrared matrix-assisted laser desorption/ionization (IR-MALDI) mass spectrometry (WO 99/57318); mobility shift analysis (Orita et al., Proc. Natl. Acad. Sci. USA 86:2766-2770 (1989)); restriction enzyme analysis (Flavell et al., Cell 15:25 (1978); Geever et al., Proc. Natl. Acad. Sci. USA 78:5081 (1981)); quantitative real-time PCR (Raca et al., Genet Test 8(4):387-94 (2004)); heteroduplex analysis; chemical mismatch cleavage (CMC) (Cotton et al., Proc. Natl. Acad. Sci. USA 85:4397-4401 (1985)); RNase protection assays (Myers et al., Science 230:1242 (1985)); use of polypeptides that recognize nucleotide mismatches, e.g., *E*. *coli* mutS protein; allele-specific PCR, and combinations of such methods. See, e.g., Gerber et al., U.S. Patent Publication No. 2004/0014095.

Sequence analysis can also be used to detect specific polymorphic variants. For example, polymorphic variants can be detected by sequencing exons, introns, 5' untranslated sequences, or 3' untranslated sequences. A sample comprising DNA or RNA is obtained from the subject. PCR or other appropriate methods can be used to amplify a portion encompassing the polymorphic site, if desired. The sequence is then ascertained, using any standard method, and the presence of a polymorphic variant is determined. Real-time pyrophosphate DNA sequencing is yet another approach to detection of polymorphisms and polymorphic variants (Alderborn et al., Genome Research 10(8):1249-1258 (2000)). Additional methods include, for example, PCR amplification in combination with denaturing high performance liquid chromatography (dHPLC) (Underhill et al., Genome Research 7(10):996-1005 (1997)).

In order to detect polymorphisms and/or polymorphic variants, it will frequently be desirable to amplify a portion of genomic DNA (gDNA) encompassing the polymorphic site. Such regions can be amplified and isolated by PCR using oligonucleotide primers designed based on genomic and/or cDNA sequences that flank the site. PCR refers to procedures in which target nucleic acid (e.g., genomic DNA) is amplified in a manner similar to that described in U.S. Patent No. 4,683,195, and subsequent modifications of the procedure described therein. Generally, sequence information from the ends of the region of interest or beyond are used to design oligonucleotide primers that are identical or similar in sequence to opposite strands of a potential template to be amplified. See e.g., PCR Primer: A Laboratory Manual, Dieffenbach and Dveksler, (Eds.); McPherson et al., PCR Basics: From Background to Bench (Springer Verlag, 2000); Mattila et al., Nucleic Acids Res., 19:4967 (1991); Eckert et al., PCR Methods and Applications, 1:17 (1991); PCR (eds. McPherson et al., IRL Press, Oxford); and U.S. Pat. No. 4,683,202. Other amplification methods that may be employed include the ligase chain reaction (LCR) (Wu and Wallace, Genomics 4:560 (1989), Landegren et al., Science 241:1077 (1988), transcription amplification (Kwoh et al., Proc. Natl. Acad. Sci. USA 86:1173 (1989)), self-sustained sequence replication (Guatelli et al., Proc. Nat. Acad. Sci. USA 87:1874 (1990)), and nucleic acid based sequence amplification (NASBA). Guidelines for selecting primers for PCR amplification are well known in the art. See, e.g., McPherson et al., PCR Basics: From Background to Blench, Springer-Verlag, 2000. A variety of computer programs for designing primers are available, e.g., 'Oligo' (National Biosciences, Inc, Plymouth Minn.), MacVector (Kodak/IBI), and the GCG suite of sequence analysis programs (Genetics Computer Group, Madison, Wis. 53711).

In some cases, PCR conditions and primers can be developed that amplify a product only when the variant allele is present or only when the wild type allele is present (MSPCR or allele-specific PCR). For example, patient DNA and a control can be amplified separately using either a wild type primer or a primer specific for the variant allele. Each set of reactions is then examined for the presence of amplification products using standard methods to visualize the DNA. For example, the reactions can be electrophoresed through an agarose gel and the DNA visualized by staining with ethidium bromide or other DNA intercalating dye. In DNA samples from heterozygous patients, reaction products would be detected in each reaction.

Real-time quantitative PCR can also be used to determine copy number. Quantitative PCR permits both detection and quantification of specific DNA sequence in a sample as an absolute number of copies or as a relative amount when normalized to DNA input or other normalizing genes. A key feature of quantitative PCR is that the amplified DNA product is quantified in real-time as it accumulates in the reaction after each amplification cycle. Methods of quantification can include the use of fluorescent dyes that intercalate with double-stranded DNA, and modified DNA oligonucleotide probes that fluoresce when hybridized with a complementary DNA.

The first report of extensive copy number variation (CNV) in the human genome used intensity analysis of microarray data to document numerous examples of genes that vary in copy number (Redon et al., Nature 444(7118):444-54 (2006)). Subsequent studies have shown that certain copy number variants are associated with complex genetic diseases such as SZ (Walsh et al., Science 320(5875):539-43 (2008); and Stone et al., Nature 455(7210):237-41 (2008)).

In some embodiments, a peptide nucleic acid (PNA) probe can be used instead of a nucleic acid probe in the hybridization methods described above. PNA is a DNA mimetic with a peptide-like, inorganic backbone, e.g., N-(2-aminoethyl)glycine units, with an organic base (A, G, C, T or U) attached to the glycine nitrogen via a methylene carbonyl linker (see, e.g., Nielsen et al., Bioconjugate Chemistry, The American Chemical Society, 5:1 (1994)). The PNA probe can be designed to specifically hybridize to a nucleic acid comprising a polymorphic variant conferring susceptibility to or indicative of the presence of, for example, SZ.

In some cases, allele-specific oligonucleotides can also be used to detect the presence of a polymorphic variant. For example, polymorphic variants can be detected by performing allele-specific hybridization or allele-specific restriction digests. Allele specific hybridization is an example of a method that can be used to detect sequence variants, including complete haplotypes of a subject (e.g., a mammal such as a human). See Stoneking et al., Am. J. Hum. Genet. 48:370-382 (1991); and Prince et al., Genome Res. 11:152-162 (2001). An "allele-specific oligonucleotide" (also referred to herein as an "allele-specific oligonucleotide probe") is an oligonucleotide that is specific for particular a polymorphism can be prepared using standard methods (see Ausubel et al., Current Protocols in Molecular Biology*,* supra). Allele-specific oligonucleotide probes typically can be approximately 10-50 base pairs, preferably approximately 15-30 base pairs, that specifically hybridizes to a nucleic acid region that contains a polymorphism. Hybridization conditions are selected such that a nucleic acid probe can specifically bind to the sequence of interest, e.g., the variant nucleic acid sequence. Such hybridizations typically are performed under high stringency as some sequence variants include only a single nucleotide difference. In some cases, dot-blot hybridization of amplified oligonucleotides with allele-specific oligonucleotide (ASO) probes can be performed. See, for example, Saiki et al., Nature (London) 324:163-166 (1986).

In some embodiments, allele-specific restriction digest analysis can be used to detect the existence of a polymorphic variant of a polymorphism, if alternate polymorphic variants of the polymorphism result in the creation or elimination of a restriction site. Allele-specific restriction digests can be performed in the following manner. A sample containing genomic DNA is obtained from the individual and genomic DNA is isolated for analysis. For nucleotide sequence variants that introduce a restriction site, restriction digest with the particular restriction enzyme can differentiate the alleles. In some cases, polymerase chain reaction (PCR) can be used to amplify a region comprising the polymorphic site, and restriction fragment length polymorphism analysis is conducted (see Ausubel et al., Current Protocols in Molecular Biology*,* supra). The digestion pattern of the relevant DNA fragment indicates the presence or absence of a particular polymorphic variant of the polymorphism and is therefore indicative of the presence or absence of susceptibility to SZ. For sequence variants that do not alter a common restriction site, mutagenic primers can be designed that introduce a restriction site when the variant allele is present or when the wild type allele is present. For example, a portion of a nucleic acid can be amplified using the mutagenic primer and a wild type primer, followed by digest with the appropriate restriction endonuclease.

In some embodiments, fluorescence polarization template-directed dye-terminator incorporation (FP-TDI) is used to determine which of multiple polymorphic variants of a polymorphism is present in a subject (Chen et al., Genome Research 9(5):492-498 (1999)). Rather than involving use of allele-specific probes or primers, this method employs primers that terminate adjacent to a polymorphic site, so that extension of the primer by a single nucleotide results in incorporation of a nucleotide complementary to the polymorphic variant at the polymorphic site.

In some cases, DNA containing an amplified portion may be dot-blotted, using standard methods (see Ausubel et al., Current Protocols in Molecular Biology, supra), and the blot contacted with the oligonucleotide probe. The presence of specific hybridization of the probe to the DNA is then detected. Specific hybridization of an allele-specific oligonucleotide probe (specific for a polymorphic variant indicative of susceptibility to an SSD (e.g., SZ)) to DNA from the subject is indicative of susceptibility to an SSD (e.g., SZ).

The methods can include determining the genotype of a subject with respect to both copies of the polymorphic site present in the genome. For example, the complete genotype may be characterized as -/-, as -/+, or as +/+, where a minus sign indicates the presence of the reference or wild type sequence at the polymorphic site, and the plus sign indicates the presence of a polymorphic variant other than the reference sequence. If multiple polymorphic variants exist at a site, this can be appropriately indicated by specifying which ones are present in the subject. Any of the detection means described herein can be used to determine the genotype of a subject with respect to one or both copies of the polymorphism present in the subject's genome.

Methods of nucleic acid analysis to detect polymorphisms and/or polymorphic variants can include, e.g., microarray analysis. Hybridization methods, such as Southern analysis, Northern analysis, or *in situ* hybridizations, can also be used (see Ausubel et al., Current Protocols in Molecular Biology, eds., John Wiley & Sons (2003)). To detect microdeletions, fluorescence in situ hybridization (FISH) using DNA probes that are directed to a putatively deleted region in a chromosome can be used. For example, probes that detect all or a part of a microsatellite marker can be used to detect microdeletions in the region that contains that marker.

In some embodiments, it is desirable to employ methods that can detect the presence of multiple polymorphisms (e.g., polymorphic variants at a plurality of polymorphic sites) in parallel or substantially simultaneously. Oligonucleotide arrays represent one suitable means for doing so. Other methods, including methods in which reactions (e.g., amplification, hybridization) are performed in individual vessels, e.g., within individual wells of a multi-well plate or other vessel may also be performed so as to detect the presence of multiple polymorphic variants (e.g., polymorphic variants at a plurality of polymorphic sites) in parallel or substantially simultaneously according to certain embodiments.

Nucleic acid probes can be used to detect and/or quantify the presence of a particular target nucleic acid sequence within a sample of nucleic acid sequences, e.g., as hybridization probes, or to amplify a particular target sequence within a sample, e.g., as a primer. Probes have a complimentary nucleic acid sequence that selectively hybridizes to the target nucleic acid sequence. In order for a probe to hybridize to a target sequence, the hybridization probe must have sufficient identity with the target sequence, i.e., at least 70% (e.g., 80%, 90%, 95%, 98% or more) identity to the target sequence. The probe sequence must also be sufficiently long so that the probe exhibits selectivity for the target sequence over non-target sequences. For example, the probe will be at least 20 (e.g., 25, 30, 35, 50, 100, 200, 300, 400, 500, 600, 700, 800, 900 or more) nucleotides in length. In some embodiments, the probes are not more than 30, 50, 100, 200, 300, 500, 750, or 1000 nucleotides in length. Probes are typically about 20 to about 1 x 10⁶ nucleotides in length. Probes include primers, which generally refers to a single-stranded oligonucleotide probe that can act as a point of initiation of template-directed DNA synthesis using methods such as PCR (polymerase chain reaction), LCR (ligase chain reaction), etc., for amplification of a target sequence.

The probe can be a test probe such as a probe that can be used to detect polymorphisms in a region described herein (e.g., polymorphisms as described herein). For example, the probe can hybridize to an allele described herein. In some embodiments, the probe can bind to another marker sequence associated with SZ, SPD, SD or BD as described herein.

Control probes can also be used. For example, a probe that binds a less variable sequence, e.g., repetitive DNA associated with a centromere of a chromosome, can be used as a control. Probes that hybridize with various centromeric DNA and locus-specific DNA are available commercially, for example, from Vysis, Inc. (Downers Grove, III.), Molecular Probes, Inc. (Eugene, Oreg.), or from Cytocell (Oxfordshire, UK). Probe sets are available commercially such from Applied Biosystems, e.g., the Assays-on-Demand SNP kits Alternatively, probes can be synthesized, e.g., chemically or in vitro, or made from chromosomal or genomic DNA through standard techniques. For example, sources of DNA that can be used include genomic DNA, cloned DNA sequences, somatic cell hybrids that contain one, or a part of one, human chromosome along with the normal chromosome complement of the host, and chromosomes purified by flow cytometry or microdissection. The region of interest can be isolated through cloning, or by site-specific amplification via the polymerase chain reaction (PCR). See, for example, Nath and Johnson, Biotechnic. Histochem. 73(1):6-22 (1998); Wheeless et al., Cytometry 17:319-326 (1994); and U.S. Pat. No. 5,491,224.

In some embodiments, the probes are labeled, e.g., by direct labeling, with a fluorophore, an organic molecule that fluoresces after absorbing light of lower wavelength/higher energy. A directly labeled fluorophore allows the probe to be visualized without a secondary detection molecule. After covalently attaching a fluorophore to a nucleotide, the nucleotide can be directly incorporated into the probe with standard techniques such as nick translation, random priming, and PCR labeling. Alternatively, deoxycytidine nucleotides within the probe can be transaminated with a linker. The fluorophore then is covalently attached to the transaminated deoxycytidine nucleotides. See, e.g., U.S. Pat. No. 5,491,224.

Fluorophores of different colors can be chosen such that each probe in a set can be distinctly visualized. For example, a combination of the following fluorophores can be used: 7-amino-4-methylcoumarin-3-acetic acid (AMCA), TEXAS RED™ (Molecular Probes, Inc., Eugene, OR), 5-(and-6)-carboxy-X-rhodamine, lissamine rhodamine B, 5-(and-6)-carboxyfluorescein, fluorescein-5-isothiocyanate (FITC), 7-diethylaminocoumarin-3-carboxylic acid, tetramethylrhodamine-5-(and-6)-isothiocyanate, 5-(and-6)-carboxytetramethylrhodamine, 7-hydroxycoumarin-3-carboxylic acid, 6-[fluorescein 5-(and-6)-carboxamido]hexanoic acid, N-(4,4-difluoro-5,7-dimethyl-4-bora-3a,4a diaza-3-indacenepropionic acid, eosin-5-isothiocyanate, erythrosin-5-isothiocyanate, and CASCADE™ blue acetylazide (Molecular Probes, Inc., Eugene, OR). Fluorescently labeled probes can be viewed with a fluorescence microscope and an appropriate filter for each fluorophore, or by using dual or triple band-pass filter sets to observe multiple fluorophores. See, for example, U.S. Pat. No. 5,776,688. Alternatively, techniques such as flow cytometry can be used to examine the hybridization pattern of the probes. Fluorescence-based arrays are also known in the art.

In other embodiments, the probes can be indirectly labeled with, e.g., biotin or digoxygenin, or labeled with radioactive isotopes such as ³²P and ³H. For example, a probe indirectly labeled with biotin can be detected by avidin conjugated to a detectable marker. For example, avidin can be conjugated to an enzymatic marker such as alkaline phosphatase or horseradish peroxidase. Enzymatic markers can be detected in standard colorimetric reactions using a substrate and/or a catalyst for the enzyme. Catalysts for alkaline phosphatase include 5-bromo-4-chloro-3-indolylphosphate and nitro blue tetrazolium. Diaminobenzoate can be used as a catalyst for horseradish peroxidase.

In another aspect, this document features arrays that include a substrate having a plurality of addressable areas, and methods of using them. At least one area of the plurality includes a nucleic acid probe that binds specifically to a sequence comprising a polymorphism listed in Tables 1-3 or Table A, and can be used to detect the absence or presence of said polymorphism, e.g., one or more SNPs, microsatellites, minisatellites, or indels, as described herein, to determine or identify an allele or genotype. For example, the array can include one or more nucleic acid probes that can be used to detect a polymorphism listed in Tables 1-3 or Table A. In some embodiments, the array further includes at least one area that includes a nucleic acid probe that can be used to specifically detect another marker associated with SZ or one associated with BD as described herein or known in the art. In some embodiments, the probes are nucleic acid capture probes.

Generally, microarray hybridization is performed by hybridizing a nucleic acid of interest (e.g., a nucleic acid encompassing a polymorphic site) with the array and detecting hybridization using nucleic acid probes. In some cases, the nucleic acid of interest is amplified prior to hybridization. Hybridization and detecting are generally carried out according to standard methods. See, e.g., Published PCT Application Nos. WO 92/10092 and WO 95/11995, and U.S. Pat. No. 5,424,186. For example, the array can be scanned to determine the position on the array to which the nucleic acid hybridizes. The hybridization data obtained from the scan is typically in the form of fluorescence intensities as a function of location on the array.

Arrays can be formed on substrates fabricated with materials such as paper, glass, plastic (e.g., polypropylene, nylon, or polystyrene), polyacrylamide, nitrocellulose, silicon, optical fiber, or any other suitable solid or semisolid support, and can be configured in a planar (e.g., glass plates, silicon chips) or three dimensional (e.g., pins, fibers, beads, particles, microtiter wells, capillaries) configuration. Methods for generating arrays are known in the art and include, e.g., photolithographic methods (see, e.g., U.S. Patent Nos. 5,143,854; 5,510,270; and 5,527,681), mechanical methods (e.g., directed-flow methods as described in U.S. Patent No. 5,384,261), pin-based methods (e.g., as described in U.S. Pat. No. 5,288,514), and bead-based techniques (e.g., as described in PCT US/93/04145). The array typically includes oligonucleotide hybridization probes capable of specifically hybridizing to different polymorphic variants. Oligonucleotide probes that exhibit differential or selective binding to polymorphic sites may readily be designed by one of ordinary skill in the art. For example, an oligonucleotide that is perfectly complementary to a sequence that encompasses a polymorphic site (i.e., a sequence that includes the polymorphic site, within it or at one end) will generally hybridize preferentially to a nucleic acid comprising that sequence, as opposed to a nucleic acid comprising an alternate polymorphic variant.

Oligonucleotide probes forming an array may be attached to a substrate by any number of techniques, including, without limitation, (i) *in situ* synthesis (e.g., high-density oligonucleotide arrays) using photolithographic techniques; (ii) spotting/printing at medium to low density on glass, nylon or nitrocellulose; (iii) by masking, and (iv) by dot-blotting on a nylon or nitrocellulose hybridization membrane. Oligonucleotides can be immobilized via a linker, including by covalent, ionic, or physical linkage. Linkers for immobilizing nucleic acids and polypeptides, including reversible or cleavable linkers, are known in the art. See, for example, U.S. Patent No. 5,451,683 and WO98/20019. Alternatively, oligonucleotides can be non-covalently immobilized on a substrate by hybridization to anchors, by means of magnetic beads, or in a fluid phase such as in microtiter wells or capillaries. Immobilized oligonucleotide probes are typically about 20 nucleotides in length, but can vary from about 10 nucleotides to about 1000 nucleotides in length.

Arrays can include multiple detection blocks (i.e., multiple groups of probes designed for detection of particular polymorphisms). Such arrays can be used to analyze multiple different polymorphisms. Detection blocks may be grouped within a single array or in multiple, separate arrays so that varying conditions (e.g., conditions optimized for particular polymorphisms) may be used during the hybridization. For example, it may be desirable to provide for the detection of those polymorphisms that fall within G-C rich stretches of a genomic sequence, separately from those falling in A-T rich segments. General descriptions of using oligonucleotide arrays for detection of polymorphisms can be found, for example, in U.S. Pat. Nos. 5,858,659 and 5,837,832. In addition to oligonucleotide arrays, cDNA arrays may be used similarly in certain embodiments.

The methods described herein can include providing an array as described herein; contacting the array with a sample (e.g., a portion of genomic DNA that includes at least a portion of a human chromosome) and/or optionally, a different portion of genomic DNA (e.g., a portion that includes a different portion of a human chromosome, e.g., including another region associated with SZ), and detecting binding of a nucleic acid from the sample to the array. Optionally, the method includes amplifying nucleic acid from the sample, e.g., genomic DNA that includes a portion of a human chromosome described herein, and, optionally, a region that includes another region associated with SZ, prior to or during contact with the array.

In some aspects, the methods described herein can include using an array that can ascertain differential expression patterns or copy numbers of one or more genes in samples from normal and affected individuals (see, e.g., Redon et al., Nature 444(7118):444-54 (2006)). For example, arrays of probes to a marker described herein can be used to measure polymorphisms between DNA from a subject having an SSD (e.g., SZ), and control DNA, e.g., DNA obtained from an individual that does not have SZ, SPD, or SD, and has no risk factors for an SSD (e.g., SZ). Since the clones on the array contain sequence tags, their positions on the array are accurately known relative to the genomic sequence. Different hybridization patterns between DNA from an individual afflicted with an SSD (e.g., SZ) and DNA from a normal individual at areas in the array corresponding to markers in human chromosome 4p and/or 22q as described herein, and, optionally, one or more other regions associated with an SSD (e.g., SZ), are indicative of a risk of SZ. Methods for array production, hybridization, and analysis are described, e.g., in Snijders et al., Nat. Genetics 29:263-264 (2001); Klein et al., Proc. Natl Acad. Sci. USA 96:4494-4499 (1999); Albertson et al., Breast Cancer Research and Treatment 78:289-298 (2003); and Snijders et al., "BAC microarray based comparative genomic hybridization," in: Zhao et al. (eds), Bacterial Artificial Chromosomes: Methods and Protocols, Methods in Molecular Biology, Humana Press, 2002.

In another aspect, this document provides methods of determining the absence or presence of a haplotype associated with SZ as described herein, using an array described above. The methods can include providing a two dimensional array having a plurality of addresses, each address of the plurality being positionally distinguishable from each other address of the plurality having a unique nucleic acid capture probe, contacting the array with a first sample from a test subject who is suspected of having or being at risk for SZ, and comparing the binding of the first sample with one or more references, e.g., binding of a sample from a subject who is known to have an SSD (e.g., SZ), and/or binding of a sample from a subject who is unaffected, e.g., a control sample from a subject who neither has, nor has any risk factors for an SSD (e.g., SZ). In some embodiments, the methods can include contacting the array with a second sample from a subject who has an SSD (e.g., SZ); and comparing the binding of the first sample with the binding of the second sample. In some embodiments, the methods can include contacting the array with a third sample from a cell or subject that does not have SZ and is not at risk for SZ; and comparing the binding of the first sample with the binding of the third sample. In some embodiments, the second and third samples are from first or second-degree relatives of the test subject. In the case of a nucleic acid hybridization, binding with a capture probe at an address of the plurality, can be detected by any method known in the art, e.g., by detection of a signal generated from a label attached to the nucleic acid.

### Communicating a Diagnosis or Risk Assessment

This document also provides methods and materials to assist medical or research professionals in determining whether or not a subject has or is at risk for developing an SSD (e.g., SZ). Medical professionals can be, for example, doctors, nurses, medical laboratory technologists, and pharmacists. Research professionals can be, for example, principle investigators, research technicians, postdoctoral trainees, and graduate students. A professional can be assisted by (1) determining whether specific polymorphic variants are present in a biological sample from a subject, and (2) communicating information about polymorphic variants to that professional.

After information about specific polymorphic variants is reported, a medical professional can take one or more actions that can affect patient care. For example, a medical professional can record information in the patient's medical record regarding the patient's risk of developing an SSD (e.g., SZ). In some cases, a medical professional can record information regarding risk assessment, or otherwise transform the patient's medical record, to reflect the patient's current medical condition. In some cases, a medical professional can review and evaluate a patient's entire medical record and assess multiple treatment strategies for clinical intervention of a patient's condition.

A medical professional can initiate or modify treatment after receiving information regarding a patient's diagnosis of or risk of developing an SSD (e.g., SZ), for example. In some cases, a medical professional can recommend a change in therapy. In some cases, a medical professional can enroll a patient in a clinical trial for, by way of example, detecting correlations between a haplotype as described herein and any measurable or quantifiable parameter relating to the outcome of the treatment as described above.

A medical professional can communicate information regarding a patient's diagnosis of risk of developing an SSD (e.g., SZ) to a patient or a patient's family. In some cases, a medical professional can provide a patient and/or a patient's family with information regarding an SSD (e.g., SZ) and diagnosis or risk assessment information, including treatment options, prognosis, and referrals to specialists. In some cases, a medical professional can provide a copy of a patient's medical records to a specialist.

A research professional can apply information regarding a subject's diagnosis of or risk of developing an SSD (e.g., SZ) to advance scientific research. For example, a researcher can compile data on wild specific polymorphic variants. In some cases, a research professional can obtain a subject's haplotype as described herein to evaluate a subject's enrollment, or continued participation, in a research study or clinical trial. In some cases, a research professional can communicate information regarding a subject's diagnosis of or risk of developing an SSD (e.g., SZ) to a medical professional. In some cases, a research professional can refer a subject to a medical professional.

Any appropriate method can be used to communicate information to another person (e.g., a professional). For example, information can be given directly or indirectly to a professional. For example, a laboratory technician can input a patient's polymorphic variant haplotype as described herein into a computer-based record. In some cases, information is communicated by making an physical alteration to medical or research records. For example, a medical professional can make a permanent notation or flag a medical record for communicating the risk assessment to other medical professionals reviewing the record. In addition, any type of communication can be used to communicate the risk assessment information. For example, mail, e-mail, telephone, and face-to-face interactions can be used. The information also can be communicated to a professional by making that information electronically available to the professional. For example, the information can be communicated to a professional by placing the information on a computer database such that the professional can access the information. In addition, the information can be communicated to a hospital, clinic, or research facility serving as an agent for the professional.

### Articles of Manufacture

Also provided herein are articles of manufacture comprising a probe that hybridizes with a region of human chromosome as described herein and can be used to detect a polymorphism described herein. For example, any of the probes for detecting polymorphisms described herein can be combined with packaging material to generate articles of manufacture or kits. The kit can include one or more other elements including: instructions for use; and other reagents such as a label or an agent useful for attaching a label to the probe. Instructions for use can include instructions for diagnostic applications of the probe for making a diagnosis of or assessing risk of an SSD (e.g., SZ) in a method described herein. Other instructions can include instructions for attaching a label to the probe, instructions for performing *in situ* analysis with the probe, and/or instructions for obtaining a sample to be analyzed from a subject. In some cases, the kit can include a labeled probe that hybridizes to a region of human chromosome as described herein.

The kit can also include one or more additional reference or control probes that hybridize to the same chromosome or another chromosome or portion thereof that can have an abnormality associated with a particular endophenotype. A kit that includes additional probes can further include labels, e.g., one or more of the same or different labels for the probes. In other embodiments, the additional probe or probes provided with the kit can be a labeled probe or probes. When the kit further includes one or more additional probe or probes, the kit can further provide instructions for the use of the additional probe or probes. Kits for use in self-testing can also be provided. Such test kits can include devices and instructions that a subject can use to obtain a biological sample (e.g., buccal cells, blood) without the aid of a health care provider. For example, buccal cells can be obtained using a buccal swab or brush, or using mouthwash.

Kits as provided herein can also include a mailer (e.g., a postage paid envelope or mailing pack) that can be used to return the sample for analysis, e.g., to a laboratory. The kit can include one or more containers for the sample, or the sample can be in a standard blood collection vial. The kit can also include one or more of an informed consent form, a test requisition form, and instructions on how to use the kit in a method described herein. Methods for using such kits are also included herein. One or more of the forms (e.g., the test requisition form) and the container holding the sample can be coded, for example, with a bar code for identifying the subject who provided the sample.

### Databases and Reports

Also provided herein are databases that include a list of polymorphisms as described herein, and wherein the list is largely or entirely limited to polymorphisms identified as useful for determining a diagnosis or susceptibility to an SSD (e.g., SZ) as described herein. The list is stored, e.g., on a flat file or computer-readable medium. The databases can further include information regarding one or more subjects, e.g., whether a subject is affected or unaffected, clinical information such as endophenotype, age of onset of symptoms, any treatments administered and outcomes (e.g., data relevant to pharmacogenomics, diagnostics or theranostics), and other details, e.g., about the disorder in the subject, or environmental or other genetic factors. The databases can be used to detect correlations between a particular haplotype and the information regarding the subject.

The methods described herein can also include the generation of reports, e.g., for use by a patient, care giver, payor, or researcher, that include information regarding a subject's response allele(s), and optionally further information such as treatments administered, treatment history, medical history, predicted response, and actual response. The reports can be recorded in a tangible medium, e.g., a computer-readable disk, a solid state memory device, or an optical storage device.

### Engineered Cells

Also provided herein are engineered cells that harbor one or more polymorphism described herein, e.g., one or more polymorphisms associated with an SSD (e.g., SZ), e.g., a SNP or CNV. Such cells are useful for studying the effect of a polymorphism on physiological function, and for identifying and/or evaluating potential therapeutic agents such as antipsychotics for the treatment of an SSD (e.g., SZ).

As one example, included herein are cells in which one of the various alleles of the genes described herein has been re-created that is associated with an increased risk of an SSD (e.g., SZ). Methods are known in the art for generating cells, e.g., by homologous recombination between the endogenous gene and an exogenous DNA molecule introduced into a cell, e.g., a cell of an animal. In some cases, the cells can be used to generate transgenic animals using methods known in the art.

The cells are preferably mammalian cells (e.g., neuronal type cells) in which an endogenous gene has been altered to include a polymorphism as described herein. Techniques such as targeted homologous recombinations, can be used to insert the heterologous DNA as described in, e.g., Chappel, US 5,272,071; WO 91/06667, published in May 16, 1991.

### EXAMPLES

### Example 1. Alleles Associated with Risk of Developing Schizophrenia

The Clinical Antipsychotic Trials of Intervention Effectiveness (CATIE), a large federally funded clinical trial designed to assess the efficacy of antipsychotics in a real world setting, is a valuable resource for determining the role of genes in drug response (Stroup et al., Schizophr. Bull. 29:15-31 (2003); Lieberman et al., N. Engl. J. Med. 353:1209-1223 (2005)). As part of the CATIE trial, SNP genotyping was performed for roughly half of the trial participants (Sullivan et al., Mol. Psychiatry 13:570-584 (2008)). When combined with disease status, PANSS scores, and clinical drug response data, the genotyping data allows the identification of genetic variants (e.g., SNPs) that are statistically associated with specific responses to selected treatments.

The design of the CATIE study has been described in detail by others (see, e.g., Stroup et al., Schizophr. Bull. 29:15-31 (2003); Lieberman et al., N. Engl. J. Med. 353:1209-1223 (2005)). Briefly, 1460 subjects were randomly assigned one of several antipsychotics and those who did not respond or chose to quit their current medication were re-randomized to another drug. Details regarding SNP genotyping and quality control have been recently published (Sullivan et al., Mol. Psychiatry 13:570-584 (2008)).

Genotype and phenotype data for the CATIE trial were made available to qualified researchers through the NIMH Center for Collaborative Genetic Studies on Mental Disorders. Data for 417 patients with schizophrenia and 419 unaffected controls self reported as having exclusively European ancestry were evaluated. This same patient population was described in a recent study by Sullivan and coworkers, which confirmed that there is no hidden stratification in the sample (Sullivan et al., Mol. Psychiatry 13:570-584 (2008)).

For the CATIE study, individual cases were diagnosed as having SZ based on DSM-III/IV criteria.

To determine the influence of specific alleles on likelihood of having schizophrenia, case/control association using the PLINK 1.03 whole genome analysis toolset developed by Purcell and coworkers (Purcell et al., Am. J. Hum. Genet. 81:559-575 (2007)). For dichotomous comparisons of affected vs. unaffected individuals, PLINK calculates P values for the allele-specific chi-squared test and the odds ratio (OR; or relative risk) associated with the minor allele.

Table 1 lists alleles and genotypes influencing a diagnosis of SZ. 1170 SZ cases, and 1378 neurologically normal controls were used to identify these alleles. Test 1 lists gene, SEQ ID, NCBI RS#, allele, frequency of allele in cases, p-value, and Odds ratio for cases versus controls.

### Example 2: Copy Number Variants (CNVs) that Contribute to Risk of SZ

Genotyping and phenotype data were obtained from the Genetic Analysis Information Network (GAIN) Database found at ncbi.nlm.nih.gov through dbGaP, at accession number PHS000017.v1.p1. Genotypes and associated phenotype data for the GAIN Genome-Wide Association Study of Schizophrenia were provided by P. Gejman, and genotyping of these samples was provided through the Genetic Association Information Network (GAIN). Golden Helix's SVS 7.0 was used to determine the Copy Number Variation at SNP for the genes of interest. Briefly, GAIN determine SNP and CNV variation for the samples using the Affymetrix 6.0 SNP and CNV array. Probes were hybridized to the array and the intensity of the signal recorded. These raw intensity values were imported into the SVS using the import function of the program. Then CNV status was determined using the following protocol. For the present example, 1170 SZ patients and 1378 neurologically normal controls were used to identify SNPS that can detect CNV regions influencing SZ risk.

Table 2 lists SNPs and associated genes, deletions of which alter the likelihood of SZ diagnosis. P-values were calculated from Chi-square values at each SNP. Odds Ratios were calculated by comparing the frequency of a CNV in cases versus controls using the formula (Case_{freq}*(1-Control_{freq}))/(Control_{freq}*(1-Case_{freq})).

Table 3 lists SNPs and associated genes, duplications of which event that alter the likelihood of SZ diagnosis. P-values were calculated from Chi-square values at each SNP. Odds Ratios were calculated by comparing the frequency of a CNV in cases versus controls using the formula (Case_{freq}*(1-Control_{freq}))/(Control_{freq}*(1-Case_{freq})).

| **Table 1: Alleles influencing likelihood of schizophrenia diagnosis** | | | | | | |
|---|---|---|---|---|---|---|
| **Gene** | **Seq ID** | **NCBI RS#** | **Allele** | **Frequency** | **P** | **Odds Ratio** |
| PIK3CD | 1 | 11121472 | A | 0.1456 | 0.0230000 | 1.40 |
| RP1-21O18.1 | 2 | 10803343 | G | 0.0156 | 0.0324300 | 3.20 |
| AGBL4-C1ORF165 | 3 | 2153334 | G | 0.4000 | 0.0050950 | 1.33 |
| AGBL4-C1ORF165 | 4 | 1934368 | C | 0.2900 | 0.0106600 | 1.33 |
| AGBL4-C1ORF165 | 5 | 6667780 | C | 0.3597 | 0.0203800 | 1.27 |
| AGBL4-C1ORF165 | 6 | 4285747 | C | 0.3893 | 0.0032690 | 1.35 |
| AGBL4-C1ORF165 | 7 | 3121522 | A | 0.3401 | 0.0224000 | 1.27 |
| AGBL4-C1ORF165 | 8 | 12043418 | T | 0.3878 | 0.0058610 | 1.33 |
| AGBL4-C1ORF165 | 9 | 3123291 | T | 0.4234 | 0.0175500 | 1.27 |
| SCP2 | 10 | 7546714 | T | 0.1360 | 0.0231900 | 1.41 |
| LRP8 | 11 | 5174 | A | 0.3663 | 0.0044880 | 0.75 |
| LRP8 | 12 | 2297660 | A | 0.3829 | 0.0112400 | 0.78 |
| LRP8 | 13 | 2297657 | A | 0.3521 | 0.0276000 | 0.80 |
| PRKACB | 14 | 6701486 | A | 0.2476 | 0.0157500 | 1.33 |
| PRKACB | 15 | 2642186 | G | 0.2350 | 0.0400800 | 1.28 |
| CGN | 16 | 1573129 | T | 0.1365 | 0.0079840 | 0.70 |
| OLFML2B | 17 | 12025136 | C | 0.2792 | 0.0002507 | 1.52 |
| DPT | 18 | 1018453 | C | 0.5048 | 0.0053390 | 1.32 |
| SEC16B | 19 | 943762 | A | 0.0859 | 0.0097780 | 0.66 |
| SEC16B | 20 | 4652279 | A | 0.0859 | 0.0141700 | 0.67 |
| MR1 | 21 | 3845422 | A | 0.1933 | 0.0052410 | 0.72 |
| LAMC1 | 22 | 6424888 | A | 0.4253 | 0.0024470 | 0.74 |
| LAMC1 | 23 | 2274984 | C | 0.4234 | 0.0032510 | 0.75 |
| LAMC1 | 24 | 2027085 | A | 0.4246 | 0.0044230 | 0.76 |
| KCNK2 | 25 | 2841598 | G | 0.5188 | 0.0000422 | 1.51 |
| KCNK2 | 26 | 10779634 | T | 0.3484 | 0.0032330 | 1.36 |
| KCNK2 | 27 | 2123331 | G | 0.3814 | 0.0351800 | 1.25 |
| KCNK2 | 28 | 17024179 | C | 0.1859 | 0.0330100 | 1.33 |
| KCNK2 | 29 | 4655272 | A | 0.5203 | 0.0144700 | 1.27 |
| KCNK2 | 30 | 10779647 | T | 0.3804 | 0.0095630 | 1.31 |
| KCNK2 | 31 | 6684084 | G | 0.3123 | 0.0404400 | 1.25 |
| KCNK2 | 32 | 10779651 | C | 0.3140 | 0.0453500 | 1.25 |
| KCNK2 | 33 | 10494994 | A | 0.2530 | 0.0196600 | 1.31 |
| ESRRG | 34 | 1984137 | C | 0.4332 | 0.0188000 | 0.79 |
| ESRRG | 35 | 12037068 | C | 0.1745 | 0.0264100 | 1.36 |
| ESRRG | 36 | 12033378 | A | 0.1711 | 0.0477700 | 1.31 |
| GNG4 | 37 | 508208 | G | 0.3864 | 0.0403200 | 1.23 |
| RYR2 | 38 | 2169902 | A | 0.0735 | 0.0102100 | 0.64 |
| RYR2 | 39 | 515474 | C | 0.2200 | 0.0237200 | 1.32 |
| RYR2 | 40 | 4659819 | T | 0.4569 | 0.0094780 | 0.78 |
| FMN2 | 41 | 1542400 | G | 0.4709 | 0.0387700 | 0.82 |
| RGS7 | 42 | 191735 | A | 0.4294 | 0.0496000 | 1.22 |
| PLD5 | 43 | 2036407 | C | 0.3254 | 0.0340200 | 1.26 |
| C2ORF46 | 44 | 182971 | C | 0.2746 | 0.0213700 | 1.30 |
| C2ORF46 | 45 | 3102945 | C | 0.1723 | 0.0056010 | 1.47 |
| C2ORF46 | 46 | 2562011 | C | 0.1819 | 0.0160100 | 1.38 |
| KCNF1 | 47 | 1317761 | A | 0.3623 | 0.0031120 | 1.36 |
| KCNF1 | 48 | 4669647 | T | 0.2265 | 0.0378100 | 1.29 |
| KCNF1 | 49 | 4669651 | T | 0.1850 | 0.0388100 | 1.32 |
| ASXL2 | 50 | 9309437 | T | 0.2033 | 0.0009717 | 1.54 |
| CRIM1 | 51 | 4670549 | T | 0.4606 | 0.0418900 | 1.22 |
| CRIM1 | 52 | 2049367 | A | 0.3065 | 0.0301000 | 1.27 |
| CRIM1 | 53 | 3770876 | C | 0.4318 | 0.0374900 | 1.23 |
| CRIM1 | 54 | 3755197 | A | 0.3038 | 0.0037720 | 0.74 |
| CR1M1 | 55 | 11681392 | T | 0.2995 | 0.0125200 | 0.77 |
| HAAO | 56 | 3816182 | A | 0.2745 | 0.0054010 | 1.37 |
| PLEKHH2 | 57 | 17414362 | A | 0.2307 | 0.0038410 | 1.43 |
| PRKCE | 58 | 6732900 | T | 0.4522 | 0.0136500 | 1.28 |
| PRKCE | 59 | 4953370 | G | 0.4677 | 0.0307800 | 1.24 |
| FBXO11 | 60 | 874869 | G | 0.5024 | 0.0275500 | 1.24 |
| PSME4 | 61 | 805404 | C | 0.2680 | 0.0059070 | 0.74 |
| PSME4 | 62 | 10183655 | T | 0.3862 | 0.0364200 | 0.81 |
| PSME4 | 63 | 17045488 | G | 0.2339 | 0.0055770 | 0.73 |
| ACYP2 | 64 | 6731970 | A | 0.2138 | 0.0007330 | 0.68 |
| CCDC85A | 65 | 10460558 | A | 0.2709 | 0.0002575 | 0.68 |
| AAK1 | 66 | 2871965 | T | 0.4320 | 0.0214300 | 1.26 |
| AAK1 | 67 | 3821277 | G | 0.4115 | 0.0156200 | 1.28 |
| AAK1 | 68 | 6546534 | T | 0.3067 | 0.0451800 | 1.24 |
| AAK1 | 69 | 6712370 | C | 0.2990 | 0.0170000 | 1.30 |
| AAK1 | 70 | 12713679 | G | 0.3777 | 0.0048500 | 1.34 |
| AAK1 | 71 | 10779953 | G | 0.3062 | 0.0317300 | 1.26 |
| AAK1 | 72 | 12471316 | G | 0.4379 | 0.0068780 | 1.31 |
| CTNNA2 | 77 | 17339556 | C | 0.3166 | 0.0012970 | 1.43 |
| CTNNA2 | 78 | 2861914 | G | 0.3270 | 0.0042960 | 1.36 |
| CTNNA2 | 79 | 2060393 | A | 0.4545 | 0.0048890 | 1.32 |
| CTNNA2 | 80 | 17018905 | C | 0.0914 | 0.0024250 | 1.80 |
| NAP5 | 85 | 10176860 | C | 0.2129 | 0.0247700 | 0.77 |
| LRP1B | 86 | 2171107 | A | 0.3043 | 0.0299400 | 1.27 |
| LRP1B | 87 | 10496906 | A | 0.3629 | 0.0014900 | 0.73 |
| LRP1B | 88 | 10183142 | T | 0.5291 | 0.0016930 | 1.36 |
| LRP1B | 89 | 1369542 | T | 0.4830 | 0.0064530 | 1.31 |
| LRP1B | 90 | 12623563 | A | 0.4149 | 0.0192400 | 0.79 |
| LRP1B | 91 | 13382235 | G | 0.0663 | 0.0081100 | 0.62 |
| KYNU | 92 | 10176334 | A | 0.3642 | 0.0281600 | 0.80 |
| KYNU | 93 | 6429997 | C | 0.3926 | 0.0293700 | 0.81 |
| KYNU | 94 | 3816193 | A | 0.1981 | 0.0485300 | 1.29 |
| KIF5C | 95 | 4667369 | C | 0.3618 | 0.0419700 | 0.81 |
| CACNB4 | 96 | 13421383 | T | 0.1885 | 0.0151300 | 0.75 |
| CACNB4 | 97 | 12693235 | C | 0.2297 | 0.0115900 | 0.75 |
| FMNL2 | 98 | 2164402 | G | 0.2530 | 0.0177800 | 0.77 |
| FMNL2 | 99 | 4664114 | T | 0.2817 | 0.0002634 | 0.68 |
| FMNL2 | 100 | 17327752 | A | 0.2380 | 0.0051350 | 0.73 |
| FMNL2 | 101 | 2346182 | G | 0.3329 | 0.0127600 | 0.78 |
| FMNL2 | 102 | 10497108 | C | 0.1158 | 0.0138200 | 0.70 |
| FMNL2 | 103 | 10497111 | A | 0.1208 | 0.0191800 | 0.72 |
| FMNL2 | 104 | 11682487 | C | 0.3341 | 0.0127800 | 0.78 |
| PKP4 | 105 | 2528593 | A | 0.3162 | 0.0288800 | 0.80 |
| PKP4 | 106 | 2711035 | C | 0.0660 | 0.0444400 | 0.69 |
| SCN7A | 107 | 11900439 | A | 0.3135 | 0.0247800 | 0.79 |
| CERKL | 108 | 10490688 | T | 0.3425 | 0.0315200 | 1.25 |
| CERKL | 109 | 895901 | A | 0.3481 | 0.0366200 | 1.24 |
| PDE1A | 110 | 3769794 | C | 0.1824 | 0.0444500 | 0.78 |
| PARD3B | 111 | 7559302 | T | 0.2990 | 0.0117400 | 0.77 |
| NRP2 | 112 | 13396083 | C | 0.4760 | 0.0440300 | 1.22 |
| PIP5K3 | 113 | 10497899 | T | 0.0323 | 0.0421200 | 1.95 |
| CNTN6 | 115 | 17038701 | G | 0.0649 | 0.0003231 | 2.43 |
| CNTN4 | 116 | 1502582 | T | 0.1993 | 0.0214500 | 1.34 |
| CNTN4 | 117 | 13092590 | G | 0.3007 | 0.0109100 | 0.77 |
| CNTN4 | 118 | 17194455 | T | 0.3659 | 0.0114200 | 0.78 |
| CNTN4 | 119 | 1551997 | C | 0.2876 | 0.0179900 | 0.78 |
| ITPR1 | 121 | 4685803 | A | 0.4639 | 0.0456300 | 1.22 |
| IRAK2 | 122 | 6804954 | A | 0.4294 | 0.0149200 | 1.28 |
| ATP2B2 | 123 | 13100027 | T | 0.0785 | 0.0023180 | 0.60 |
| ATP2B2 | 124 | 9860273 | T | 0.0704 | 0.0152900 | 0.65 |
| SLC6A6 | 125 | 2241780 | C | 0.1901 | 0.0316600 | 0.77 |
| SLC6A6 | 126 | 2304510 | G | 0.1432 | 0.0209700 | 0.74 |
| DAZL | 127 | 890640 | A | 0.1594 | 0.0006643 | 1.65 |
| DAZL | 128 | 4234537 | C | 0.1516 | 0.0071020 | 1.49 |
| ARPP-21 | 129 | 735353 | A | 0.4453 | 0.0280100 | 1.25 |
| ARPP-21 | 130 | 11920715 | T | 0.4471 | 0.0414800 | 1.23 |
| STAC | 131 | 17035045 | T | 0.2041 | 0.0146400 | 0.75 |
| STAC | 132 | 4678878 | C | 0.3556 | 0.0278600 | 0.80 |
| ULK4 | 133 | 7627972 | A | 0.4809 | 0.0397100 | 1.22 |
| ULK4 | 134 | 11129908 | G | 0.5132 | 0.0276800 | 1.24 |
| ULK4 | 135 | 7618902 | G | 0.3037 | 0.0192100 | 1.30 |
| ULK4 | 136 | 9878069 | G | 0.3723 | 0.0496100 | 1.22 |
| ULK4 | 137 | 1495696 | C | 0.4566 | 0.0449000 | 1.22 |
| ZNF167 | 138 | 13081859 | G | 0.2876 | 0.0395700 | 0.80 |
| CACNA2D3 | 139 | 17054677 | A | 0.2005 | 0.0069870 | 1.42 |
| FLNB | 140 | 12632456 | A | 0.2852 | 0.0165800 | 1.31 |
| FHIT | 142 | 13320646 | A | 0.4523 | 0.0019850 | 1.37 |
| FHIT | 143 | 7631246 | T | 0.4487 | 0.0034280 | 1.34 |
| FHIT | 144 | 9875228 | T | 0.1811 | 0.0422500 | 1.31 |
| FHIT | 145 | 1470035 | A | 0.4422 | 0.0075020 | 1.31 |
| FHIT | 146 | 9843007 | T | 0.3971 | 0.0209600 | 1.26 |
| FHIT | 147 | 4688167 | C | 0.2955 | 0.0451000 | 1.25 |
| PRICKLE2 | 148 | 17665101 | G | 0.1938 | 0.0432600 | 0.79 |
| ADAMTS9 | 149 | 9847307 | T | 0.0167 | 0.0073290 | 4.73 |
| ADAMTS9 | 150 | 6787633 | G | 0.0131 | 0.0122100 | 5.56 |
| MAGI1 | 151 | 7612634 | C | 0.5181 | 0.0053860 | 1.32 |
| MAGI1 | 152 | 7612636 | G | 0.5180 | 0.0090200 | 1.29 |
| MAGI1 | 153 | 7612644 | C | 0.5181 | 0.0104800 | 1.29 |
| FOXP1 | 154 | 2597312 | C | 0.3565 | 0.0316900 | 1.25 |
| GBE1 | 157 | 2307058 | T | 0.3108 | 0.0168800 | 1.30 |
| GBE1 | 158 | 7613144 | T | 0.3115 | 0.0203600 | 1.29 |
| GBE1 | 159 | 3772891 | A | 0.2271 | 0.0398500 | 0.79 |
| EPHA6 | 160 | 16838196 | A | 0.0503 | 0.0304900 | 0.64 |
| PLCXD2 | 163 | 13074817 | G | 0.4112 | 0.0131700 | 0.78 |
| PLCXD2 | 164 | 4284954 | C | 0.4157 | 0.0146100 | 0.78 |
| PLCXD2 | 165 | 13079085 | C | 0.4161 | 0.0207800 | 0.80 |
| PLCXD2 | 166 | 6768713 | A | 0.4163 | 0.0312900 | 0.81 |
| PLCXD2 | 167 | 12490166 | A | 0.4233 | 0.0435100 | 0.82 |
| EPHB1 | 168 | 39704 | A | 0.4638 | 0.0343900 | 0.81 |
| SPSB4 | 169 | 7620622 | A | 0.2452 | 0.0054850 | 1.40 |
| SPSB4 | 170 | 6780365 | C | 0.1783 | 0.0007646 | 1.60 |
| SPSB4 | 171 | 2086180 | G | 0.1271 | 0.0018360 | 1.66 |
| SERPINI2 | 172 | 4955673 | C | 0.5099 | 0.0096070 | 1.30 |
| SERPINI2 | 173 | 17246389 | C | 0.2715 | 0.0186500 | 1.31 |
| SERPINI2 | 174 | 13062550 | C | 0.4842 | 0.0225700 | 1.25 |
| TNIK | 175 | 9824475 | G | 0.2649 | 0.0045900 | 0.74 |
| PLD1 | 176 | 187229 | T | 0.4171 | 0.0245500 | 1.25 |
| PLD1 | 177 | 181715 | T | 0.4077 | 0.0343400 | 1.24 |
| NLGN1 | 179 | 10936780 | G | 0.0831 | 0.0015080 | 0.60 |
| NLGN1 | 180 | 12634066 | A | 0.0827 | 0.0017380 | 0.60 |
| NLGN1 | 181 | 9828801 | T | 0.0670 | 0.0140000 | 0.64 |
| LEPREL1 | 182 | 9835230 | A | 0.2226 | 0.0027880 | 0.71 |
| LEPREL1 | 183 | 1447935 | T | 0.1896 | 0.0051530 | 0.72 |
| LEPREL1 | 184 | 6444412 | T | 0.2859 | 0.0040510 | 0.74 |
| LEPREL1 | 185 | 1447936 | C | 0.2026 | 0.0104000 | 0.74 |
| LEPREL1 | 186 | 7632740 | C | 0.3050 | 0.0301500 | 0.80 |
| LEPREL1 | 187 | 1526031 | C | 0.2041 | 0.0406100 | 0.79 |
| UBXD7 | 188 | 6774540 | T | 0.4509 | 0.0324200 | 0.81 |
| UBXD7 | 189 | 9841810 | C | 0.4523 | 0.0450600 | 0.82 |
| UBXD7 | 190 | 9847223 | T | 0.4547 | 0.0450800 | 0.82 |
| SLC2A9 | 191 | 1568318 | C | 0.2339 | 0.0444200 | 1.27 |
| SLC2A9 | 192 | 4697692 | T | 0.2124 | 0.0477700 | 1.28 |
| SLC2A9 | 193 | 3733591 | A | 0.1679 | 0.0431800 | 0.78 |
| LDB2 | 194 | 157631 | G | 0.0728 | 0.0204900 | 0.67 |
| LDB2 | 195 | 284210 | T | 0.0704 | 0.0339900 | 0.69 |
| KIAA1239 | 203 | 17575883 | A | 0.1650 | 0.0236800 | 0.75 |
| KIAA1239 | 204 | 17575897 | T | 0.1699 | 0.0341100 | 0.77 |
| UBE2K | 205 | 192779 | A | 0.1835 | 0.0004961 | 0.66 |
| LIMCH1 | 206 | 6447080 | G | 0.4263 | 0.0048350 | 1.33 |
| LIMCH1 | 207 | 6447081 | G | 0.4258 | 0.0068670 | 1.31 |
| LIMCH1 | 208 | 4610372 | G | 0.4259 | 0.0082180 | 1.31 |
| LIMCH1 | 209 | 4861118 | A | 0.4195 | 0.0115200 | 1.29 |
| LOC389207 | 211 | 13105520 | T | 0.3474 | 0.0006047 | 0.71 |
| LOC389207 | 212 | 9994547 | A | 0.3507 | 0.0019500 | 0.73 |
| LOC389207 | 213 | 1561147 | G | 0.2993 | 0.0038420 | 0.74 |
| SCD5 | 214 | 3897960 | A | 0.1791 | 0.0068990 | 0.72 |
| SCD5 | 215 | 17352017 | G | 0.1325 | 0.0383600 | 0.75 |
| HERC3 | 216 | 2972021 | A | 0.2733 | 0.0220900 | 1.30 |
| HERC3 | 217 | 3017906 | G | 0.2977 | 0.0396900 | 1.26 |
| FAM13A1 | 218 | 7657817 | T | 0.1778 | 0.0187500 | 1.37 |
| FAM13A1 | 219 | 12507401 | C | 0.2175 | 0.0200500 | 1.34 |
| COL25A1 | 220 | 9996734 | A | 0.3973 | 0.0476000 | 1.22 |
| ANK2 | 221 | 11942005 | G | 0.1555 | 0.0380200 | 1.34 |
| CAMK2D | 222 | 11098193 | T | 0.2871 | 0.0442900 | 1.25 |
| NDST3 | 223 | 6534079 | A | 0.2146 | 0.0433700 | 0.79 |
| NDST3 | 224 | 4576085 | G | 0.1108 | 0.0017390 | 0.64 |
| GPR103 | 225 | 13110738 | G | 0.2069 | 0.0025070 | 1.48 |
| MAML3 | 226 | 3796633 | G | 0.0776 | 0.0462600 | 0.71 |
| IL15 | 227 | 2139383 | T | 0.5132 | 0.0274900 | 1.24 |
| IL15 | 228 | 1389099 | T | 0.3194 | 0.0056800 | 0.75 |
| IL15 | 229 | 1389098 | T | 0.5048 | 0.0315000 | 1.23 |
| INPP4B | 234 | 1497389 | T | 0.3471 | 0.0057580 | 0.76 |
| INPP4B | 236 | 17715707 | G | 0.3026 | 0.0251400 | 0.79 |
| POU4F2 | 240 | 1104532 | A | 0.1177 | 0.0004148 | 0.61 |
| POU4F2 | 241 | 1394279 | G | 0.1187 | 0.0004232 | 0.61 |
| DCLK2 | 242 | 6535725 | A | 0.4542 | 0.0234900 | 1.25 |
| FSTL5 | 244 | 2082669 | G | 0.1953 | 0.0016150 | 1.53 |
| FSTL5 | 245 | 6814301 | T | 0.3213 | 0.0236900 | 0.79 |
| TLL1 | 246 | 17633393 | T | 0.0293 | 0.0080040 | 2.73 |
| PALLD | 247 | 924511 | T | 0.4199 | 0.0087490 | 1.30 |
| PALLD | 248 | 10033898 | C | 0.4939 | 0.0455700 | 1.22 |
| CASP3 | 249 | 9685847 | G | 0.4426 | 0.0108700 | 0.78 |
| CASP3 | 250 | 2720376 | C | 0.4439 | 0.0146200 | 0.79 |
| PLEKHG4B | 251 | 6554947 | G | 0.3128 | 0.0069270 | 1.35 |
| PLEKHG4B | 252 | 17559144 | T | 0.4545 | 0.0269000 | 1.25 |
| AHRR | 253 | 3721004 | T | 0.3075 | 0.0004755 | 1.48 |
| AHRR | 254 | 2721012 | G | 0.2555 | 0.0012100 | 1.48 |
| DNAH5 | 260 | 6554811 | G | 0.4010 | 0.0186700 | 0.79 |
| DNAH5 | 261 | 2401810 | G | 0.3532 | 0.0186800 | 0.79 |
| DNAH5 | 265 | 10513155 | A | 0.3329 | 0.0023550 | 1.39 |
| DNAH5 | 266 | 7709692 | C | 0.2094 | 0.0017740 | 0.70 |
| DNAH5 | 268 | 9885366 | C | 0.4390 | 0.0002241 | 1.45 |
| DNAH5 | 269 | 1867679 | T | 0.4010 | 0.0059750 | 1.32 |
| DNAH5 | 270 | 6896894 | C | 0.3969 | 0.0060990 | 1.32 |
| DNAH5 | 271 | 16902953 | G | 0.3178 | 0.0372700 | 1.25 |
| DNAH5 | 272 | 9312854 | C | 0.3122 | 0.0468600 | 1.24 |
| DNAH5 | 273 | 339428 | T | 0.5240 | 0.0020490 | 1.35 |
| DNAH5 | 274 | 339424 | T | 0.2446 | 0.0135500 | 1.34 |
| CDH10 | 275 | 7714498 | A | 0.1843 | 0.0482000 | 0.79 |
| CDH10 | 276 | 1896890 | T | 0.2243 | 0.0499700 | 0.80 |
| SLC45A2 | 277 | 10461928 | G | 0.3950 | 0.0260300 | 0.80 |
| SLC45A2 | 278 | 35389 | G | 0.0465 | 0.0188900 | 1.89 |
| SLC1A3 | 279 | 1645651 | A | 0.3616 | 0.0172100 | 1.28 |
| SLC1A3 | 280 | 3776569 | G | 0.4747 | 0.0082700 | 1.30 |
| EGFLAM | 281 | 4869580 | T | 0.1283 | 0.0038730 | 1.59 |
| EGFLAM | 282 | 2589787 | A | 0.0837 | 0.0368600 | 1.50 |
| EGFLAM | 283 | 2434504 | A | 0.4580 | 0.0105700 | 1.29 |
| EGFLAM | 284 | 1428263 | C | 0.2888 | 0.0126300 | 1.33 |
| EGFLAM | 285 | 9292705 | A | 0.0897 | 0.0015590 | 1.86 |
| EGFLAM | 286 | 2731979 | C | 0.1607 | 0.0156600 | 1.41 |
| ITGA2 | 288 | 7719848 | A | 0.2838 | 0.0385800 | 1.26 |
| ELOVL7 | 290 | 4700394 | T | 0.5240 | 0.0082730 | 1.30 |
| ELOVL7 | 291 | 17332824 | C | 0.3961 | 0.0497500 | 0.82 |
| TNPO1 | 292 | 7709640 | C | 0.3468 | 0.0000269 | 1.58 |
| TNPO1 | 293 | 153320 | T | 0.1815 | 0.0024290 | 1.51 |
| TNPO1 | 294 | 34653 | T | 0.1790 | 0.0032120 | 1.50 |
| TNPO1 | 295 | 266444 | C | 0.1766 | 0.0045020 | 1.48 |
| TNPO1 | 296 | 2548331 | A | 0.4270 | 0.0001619 | 0.69 |
| FCHO2 | 297 | 478575 | C | 0.5024 | 0.0001957 | 1.45 |
| FCHO2 | 298 | 185435 | G | 0.1731 | 0.0116100 | 1.42 |
| FCHO2 | 299 | 2277017 | C | 0.3244 | 0.0417000 | 1.25 |
| CMYA5 | 300 | 6880680 | C | 0.0981 | 0.0160600 | 1.54 |
| CMYA5 | 301 | 3828611 | G | 0.0740 | 0.0182300 | 1.64 |
| CMYA5 | 302 | 1991483 | G | 0.3750 | 0.0384900 | 1.24 |
| THBS4 | 303 | 256444 | C | 0.1842 | 0.0279200 | 1.34 |
| THBS4 | 304 | 7736549 | A | 0.1496 | 0.0185900 | 1.42 |
| VCAN | 305 | 178024 | A | 0.3789 | 0.0423000 | 1.23 |
| MEF2C | 306 | 700592 | G | 0.4224 | 0.0215300 | 1.26 |
| GPR98 | 307 | 16868917 | G | 0.0586 | 0.0309900 | 0.66 |
| GPR98 | 309 | 2222244 | A | 0.3701 | 0.0473700 | 1.23 |
| FBXL17 | 311 | 12519388 | T | 0.1940 | 0.0205700 | 1.35 |
| FBXL17 | 312 | 12189428 | G | 0.1902 | 0.0253400 | 1.34 |
| FBXL17 | 316 | 7731562 | C | 0.1843 | 0.0122300 | 1.40 |
| FBXL17 | 318 | 10515385 | C | 0.1850 | 0.0251400 | 1.35 |
| FBXL17 | 319 | 11242664 | G | 0.1795 | 0.0312100 | 1.34 |
| PJA2 | 320 | 1045706 | A | 0.4221 | 0.0179100 | 1.27 |
| PJA2 | 321 | 2963046 | T | 0.3741 | 0.0295800 | 1.25 |
| SNX2 | 322 | 4343835 | C | 0.3942 | 0.0015170 | 0.73 |
| SNX2 | 323 | 4835894 | C | 0.2983 | 0.0055500 | 0.75 |
| SNX2 | 324 | 6872932 | T | 0.4819 | 0.0009375 | 1.39 |
| SNX2 | 325 | 3822367 | G | 0.4795 | 0.0024510 | 1.35 |
| SNX2 | 326 | 10519715 | A | 0.2998 | 0.0078400 | 0.76 |
| SNX2 | 327 | 2407403 | G | 0.3708 | 0.0228200 | 1.27 |
| SNX24 | 328 | 27740 | T | 0.4832 | 0.0016230 | 1.37 |
| SNX24 | 329 | 6888023 | T | 0.3014 | 0.0051010 | 0.75 |
| SNX24 | 330 | 246286 | T | 0.3925 | 0.0300700 | 1.25 |
| SNX24 | 331 | 246266 | C | 0.3927 | 0.0378300 | 1.24 |
| SNX24 | 332 | 1038078 | T | 0.1295 | 0.0318400 | 1.40 |
| SNX24 | 333 | 30028 | C | 0.3462 | 0.0394200 | 1.24 |
| SNX24 | 334 | 385996 | G | 0.3584 | 0.0406900 | 1.24 |
| ADAMTS19 | 335 | 30651 | G | 0.3628 | 0.0229300 | 1.27 |
| TRPC7 | 336 | 950714 | G | 0.1196 | 0.0005632 | 1.80 |
| TRPC7 | 337 | 2649691 | A | 0.1446 | 0.0067400 | 1.51 |
| CTNNA1 | 338 | 906695 | G | 0.2718 | 0.0003555 | 0.68 |
| CTNNA1 | 339 | 10038162 | A | 0.2757 | 0.0010250 | 0.71 |
| CTNNA1 | 340 | 10045605 | C | 0.2745 | 0.0012850 | 0.71 |
| ODZ2 | 341 | 1560654 | T | 0.2163 | 0.0093960 | 0.74 |
| ODZ2 | 342 | 3733988 | C | 0.5024 | 0.0169100 | 1.27 |
| ODZ2 | 343 | 17632540 | T | 0.2723 | 0.0285300 | 0.79 |
| ODZ2 | 344 | 1422421 | T | 0.4272 | 0.0087630 | 1.30 |
| SLC22A23 | 347 | 17136575 | G | 0.1651 | 0.0137700 | 0.73 |
| RNF144B | 354 | 2038745 | A | 0.1539 | 0.0207700 | 0.74 |
| RNF144B | 355 | 1408267 | G | 0.2017 | 0.0225200 | 0.77 |
| RNF144B | 356 | 1324573 | G | 0.4189 | 0.0106700 | 0.78 |
| RNF144B | 358 | 1924467 | T | 0.4077 | 0.0077010 | 1.31 |
| RNF144B | 359 | 9477749 | C | 0.4270 | 0.0189100 | 1.27 |
| RNF144B | 360 | 9396868 | T | 0.1169 | 0.0444400 | 0.75 |
| SLC17A4 | 361 | 1892248 | T | 0.2422 | 0.0008890 | 0.70 |
| SLC17A4 | 362 | 12662869 | A | 0.2566 | 0.0001516 | 0.67 |
| SLC17A1 | 363 | 1575535 | T | 0.2421 | 0.0001745 | 0.66 |
| SLC17A1 | 364 | 1165196 | C | 0.4759 | 0.0138100 | 1.28 |
| SLC17A1 | 365 | 1165177 | T | 0.5000 | 0.0196400 | 1.26 |
| SLC17A1 | 366 | 2070642 | T | 0.2398 | 0.0006451 | 0.69 |
| SLC17A1 | 367 | 4712976 | T | 0.2272 | 0.0027410 | 0.71 |
| SLC17A3 | 368 | 1165205 | T | 0.5097 | 0.0026860 | 1.35 |
| SLC17A2 | 369 | 9467652 | G | 0.3553 | 0.0061210 | 0.76 |
| BTN3A1 | 371 | 17610161 | A | 0.1310 | 0.0157600 | 1.47 |
| BTN3A1 | 372 | 10946824 | G | 0.1854 | 0.0209400 | 1.36 |
| BTN2A3 | 373 | 7751280 | T | 0.1826 | 0.0290400 | 1.34 |
| BTN2A3 | 374 | 10946829 | A | 0.1826 | 0.0290400 | 1.34 |
| BTN3A3 | 375 | 3846847 | T | 0.1818 | 0.0322200 | 1.33 |
| BTNL2 | 376 | 2076533 | A | 0.4627 | 0.0294300 | 1.24 |
| BTNL2 | 377 | 3763307 | T | 0.3062 | 0.0432400 | 1.25 |
| LRFN2 | 378 | 1347257 | T | 0.4356 | 0.0450700 | 0.82 |
| ELOVL5 | 405 | 9474507 | A | 0.1492 | 0.0003639 | 0.63 |
| RIMS1 | 408 | 2250128 | C | 0.3194 | 0.0375900 | 0.80 |
| KLHL32 | 409 | 1206084 | A | 0.2488 | 0.0006100 | 0.69 |
| KLHL32 | 410 | 1629523 | T | 0.1247 | 0.0062920 | 0.68 |
| KLHL32 | 411 | 1737646 | A | 0.1253 | 0.0115400 | 0.70 |
| KLHL32 | 412 | 1766518 | G | 0.1482 | 0.0307100 | 0.74 |
| KLHL32 | 413 | 2294763 | G | 0.0239 | 0.0161500 | 0.51 |
| KLHL32 | 414 | 1206149 | A | 0.4964 | 0.0414000 | 1.22 |
| SLC22A16 | 415 | 17071719 | A | 0.1043 | 0.0000963 | 2.09 |
| SLC22A16 | 416 | 17071722 | T | 0.1081 | 0.0002709 | 1.96 |
| SLC22A16 | 417 | 7768422 | C | 0.1074 | 0.0004135 | 1.90 |
| HS3ST5 | 418 | 1334900 | T | 0.3952 | 0.0003151 | 1.47 |
| TRDN | 419 | 1570187 | C | 0.3046 | 0.0118000 | 0.77 |
| TRDN | 420 | 6915835 | C | 0.3075 | 0.0384000 | 0.80 |
| TRDN | 421 | 2169092 | T | 0.3911 | 0.0472200 | 0.82 |
| NKAIN2 | 422 | 173067 | A | 0.3726 | 0.0432500 | 1.23 |
| EYA4 | 423 | 9321395 | A | 0.2296 | 0.0126200 | 1.36 |
| EYA4 | 424 | 9321396 | G | 0.2297 | 0.0240800 | 1.31 |
| EYA4 | 425 | 9375953 | A | 0.1942 | 0.0297400 | 1.33 |
| EYA4 | 426 | 17641157 | G | 0.1860 | 0.0135400 | 1.39 |
| PDE7B | 428 | 6906788 | T | 0.0731 | 0.0435000 | 1.52 |
| PDE7B | 429 | 6938424 | T | 0.0501 | 0.0348800 | 1.71 |
| PLAGL1 | 430 | 6916498 | A | 0.2315 | 0.0328400 | 1.29 |
| PLAGL1 | 431 | 9484836 | A | 0.2307 | 0.0390900 | 1.28 |
| UTRN | 432 | 9496982 | T | 0.2589 | 0.0330600 | 1.28 |
| SYNE1 | 433 | 2252755 | G | 0.3732 | 0.0324300 | 1.25 |
| SYNE1 | 434 | 718527 | A | 0.4163 | 0.0067790 | 0.77 |
| SYNE1 | 435 | 2763016 | C | 0.3365 | 0.0444900 | 0.81 |
| TFB1M | 436 | 170843 | T | 0.2826 | 0.0066610 | 0.75 |
| TFB1M | 437 | 7775163 | A | 0.3819 | 0.0013430 | 1.40 |
| PARK2 | 438 | 12055483 | T | 0.3878 | 0.0329400 | 0.81 |
| PARK2 | 439 | 6924602 | C | 0.3974 | 0.0432300 | 0.82 |
| PARK2 | 440 | 9355924 | A | 0.3786 | 0.0159500 | 0.79 |
| PDE10A | 441 | 525643 | A | 0.1355 | 0.0128200 | 0.71 |
| PDE10A | 442 | 484842 | G | 0.1543 | 0.0409300 | 0.77 |
| SDK1 | 443 | 663466 | A | 0.2002 | 0.0153800 | 0.75 |
| SDK1 | 444 | 649186 | C | 0.2404 | 0.0369700 | 0.79 |
| SDK1 | 445 | 10951446 | A | 0.4260 | 0.0087200 | 0.77 |
| SDK1 | 446 | 4723434 | G | 0.5060 | 0.0189100 | 1.26 |
| SCIN | 450 | 702477 | C | 0.3029 | 0.0059970 | 0.75 |
| SCIN | 451 | 849760 | C | 0.3498 | 0.0047510 | 0.75 |
| FERD3L | 458 | 11766540 | C | 0.2488 | 0.0033220 | 0.72 |
| STK31 | 459 | 4722266 | A | 0.1723 | 0.0057610 | 1.47 |
| SKAP2 | 460 | 17372780 | C | 0.3580 | 0.0096670 | 1.31 |
| SKAP2 | 461 | 3801846 | A | 0.2550 | 0.0031060 | 1.43 |
| SKAP2 | 462 | 607099 | G | 0.5120 | 0.0098110 | 1.29 |
| SKAP2 | 463 | 13438514 | A | 0.2488 | 0.0056350 | 1.39 |
| SKAP2 | 464 | 17315929 | T | 0.2458 | 0.0080410 | 1.37 |
| SKAP2 | 465 | 10486483 | A | 0.2452 | 0.0122600 | 1.35 |
| SKAP2 | 466 | 3801844 | C | 0.2518 | 0.0033500 | 1.42 |
| CREB5 | 467 | 6977628 | T | 0.2694 | 0.0174400 | 1.31 |
| CREB5 | 468 | 886750 | A | 0.2691 | 0.0180700 | 1.31 |
| CREB5 | 469 | 10225559 | C | 0.2639 | 0.0414100 | 1.26 |
| CHN2 | 470 | 245927 | A | 0.5323 | 0.0003594 | 1.42 |
| PDE1C | 471 | 30568 | C | 0.1615 | 0.0153700 | 1.41 |
| PDE1C | 472 | 30589 | G | 0.1289 | 0.0451600 | 1.36 |
| PDE1C | 473 | 30586 | A | 0.1103 | 0.0101600 | 1.56 |
| BMPER | 474 | 11768532 | A | 0.1787 | 0.0104600 | 0.73 |
| BMPER | 475 | 1946145 | T | 0.2560 | 0.0081850 | 0.75 |
| BMPER | 476 | 1420335 | C | 0.3042 | 0.0207500 | 0.78 |
| EEPD1 | 477 | 11765336 | G | 0.5237 | 0.0256800 | 1.25 |
| EEPD1 | 478 | 197352 | C | 0.2753 | 0.0306100 | 0.79 |
| VPS41 | 479 | 6462865 | G | 0.4044 | 0.0110700 | 0.78 |
| VPS41 | 480 | 17618395 | G | 0.3923 | 0.0167700 | 0.79 |
| VPS41 | 481 | 4723793 | G | 0.3840 | 0.0086410 | 1.31 |
| VPS41 | 482 | 3801127 | G | 0.5216 | 0.0392500 | 1.22 |
| VPS41 | 483 | 10085898 | C | 0.4510 | 0.0189400 | 0.79 |
| VPS41 | 484 | 12532461 | A | 0.2500 | 0.0129200 | 1.34 |
| VPS41 | 485 | 859522 | G | 0.0962 | 0.0223400 | 1.52 |
| CDC2L5 | 486 | 12536726 | G | 0.0944 | 0.0054230 | 1.69 |
| ABCA13 | 490 | 17132335 | A | 0.0716 | 0.0243200 | 0.67 |
| ABCA13 | 491 | 6958356 | A | 0.3842 | 0.0416300 | 0.82 |
| GRB10 | 492 | 6593182 | G | 0.1551 | 0.0308400 | 0.76 |
| GRB10 | 493 | 4947465 | C | 0.1277 | 0.0182900 | 0.72 |
| MAGI2 | 503 | 12531031 | T | 0.1811 | 0.0121700 | 1.40 |
| MAGI2 | 504 | 1918930 | C | 0.1630 | 0.0406500 | 1.33 |
| MAGI2 | 505 | 7782875 | C | 0.0206 | 0.0309200 | 0.52 |
| MAGI2 | 506 | 2191806 | C | 0.1763 | 0.0234700 | 1.36 |
| MAGI2 | 508 | 1558267 | C | 0.1687 | 0.0355400 | 1.34 |
| SEMA3E | 510 | 3801535 | G | 0.2404 | 0.0092120 | 1.37 |
| ABCB4 | 511 | 31671 | G | 0.2309 | 0.0022770 | 1.46 |
| GTPBP10 | 512 | 11972149 | T | 0.2258 | 0.0487400 | 1.28 |
| PPP1R9A | 521 | 1918482 | A | 0.4723 | 0.0160800 | 1.27 |
| PPP1R9A | 522 | 12690919 | T | 0.4395 | 0.0194500 | 0.79 |
| PPP1R9A | 523 | 854520 | T | 0.4303 | 0.0104200 | 0.78 |
| PPP1R9A | 524 | 854535 | C | 0.4236 | 0.0356900 | 0.81 |
| DYNC1I1 | 526 | 42066 | A | 0.1103 | 0.0002832 | 0.59 |
| ZNF3 | 527 | 4424195 | G | 0.2902 | 0.0113800 | 1.33 |
| ZNF3 | 528 | 941290 | A | 0.1509 | 0.0101000 | 0.71 |
| KCND2 | 539 | 17142782 | G | 0.0324 | 0.0090350 | 2.49 |
| CADPS2 | 543 | 1476898 | T | 0.1878 | 0.0180500 | 1.37 |
| CADPS2 | 544 | 2471194 | A | 0.1897 | 0.0268400 | 1.34 |
| CADPS2 | 545 | 17144752 | T | 0.1933 | 0.0281300 | 1.33 |
| SLC13A1 | 546 | 10500090 | C | 0.2512 | 0.0003513 | 0.68 |
| SLC13A1 | 548 | 2140516 | C | 0.2709 | 0.0005947 | 0.69 |
| EXOC4 | 569 | 12536963 | G | 0.3055 | 0.0186200 | 0.78 |
| TBXAS1 | 578 | 13534299 | G | 0.4199 | 0.0494600 | 0.82 |
| PTPRN2 | 592 | 12674047 | T | 0.2344 | 0.0493700 | 1.26 |
| CSMD1 | 598 | 7845140 | G | 0.0537 | 0.0419000 | 1.64 |
| CSMD1 | 604 | 895695 | G | 0.5167 | 0.0165200 | 1.27 |
| CSMD1 | 605 | 12543159 | T | 0.4255 | 0.0181100 | 0.79 |
| CSMD1 | 606 | 1161518 | T | 0.3046 | 0.0177000 | 0.78 |
| MCPH1 | 608 | 2515514 | A | 0.1993 | 0.0007415 | 1.56 |
| MCPH1 | 609 | 11994063 | T | 0.1957 | 0.0379400 | 0.78 |
| SGCZ | 610 | 7822045 | A | 0.2737 | 0.0261500 | 1.29 |
| SLC7A2 | 611 | 2588233 | C | 0.4658 | 0.0074110 | 1.31 |
| SLC7A2 | 612 | 2237817 | C | 0.3902 | 0.0006031 | 1.42 |
| PSD3 | 613 | 17595783 | C | 0.2086 | 0.0252700 | 1.33 |
| PSD3 | 614 | 13275817 | A | 0.2990 | 0.0031010 | 1.39 |
| PSD3 | 615 | 1873085 | C | 0.3138 | 0.0094100 | 1.33 |
| PSD3 | 616 | 1565231 | T | 0.4735 | 0.0333100 | 1.23 |
| ENTPD4 | 627 | 17089369 | C | 0.0728 | 0.0410100 | 1.53 |
| UNC5D | 628 | 2589344 | T | 0.1778 | 0.0302100 | 0.77 |
| SNTG1 | 629 | 906656 | C | 0.3589 | 0.0433900 | 1.33 |
| SNTG1 | 630 | 11986411 | G | 0.3208 | 0.0364800 | 1.26 |
| LYN | 631 | 868541 | A | 0.4366 | 0.0205200 | 0.79 |
| LYN | 632 | 17812677 | T | 0.2201 | 0.0084810 | 0.74 |
| LYN | 633 | 16922508 | C | 0.2302 | 0.0171600 | 0.76 |
| LYN | 634 | 6988853 | A | 0.2476 | 0.0208000 | 0.77 |
| LYN | 635 | 2668003 | C | 0.2464 | 0.0223500 | 0.78 |
| PLAG1 | 636 | 7830138 | C | 0.1811 | 0.0090210 | 0.73 |
| PLAG1 | 637 | 13273123 | G | 0.1627 | 0.0116500 | 0.73 |
| NKAIN3 | 642 | 1901409 | C | 0.3852 | 0.0063910 | 0.76 |
| NKAIN3 | 643 | 10504354 | A | 0.3210 | 0.0449600 | 0.81 |
| NKAIN3 | 644 | 10504356 | G | 0.0803 | 0.0076550 | 0.64 |
| NKAIN3 | 645 | 10504358 | G | 0.0800 | 0.0252100 | 0.69 |
| DEPDC2 | 646 | 3793379 | A | 0.1379 | 0.0138700 | 1.46 |
| DEPDC2 | 647 | 3793381 | A | 0.1355 | 0.0311500 | 1.39 |
| CRISPLD1 | 648 | 16939013 | A | 0.1615 | 0.0015940 | 0.67 |
| CRISPLD1 | 649 | 1455795 | T | 0.0921 | 0.0321600 | 0.71 |
| CRISPLD1 | 650 | 7826672 | T | 0.2883 | 0.0012110 | 0.71 |
| CRISPLD1 | 651 | 6472888 | T | 0.2739 | 0.0009918 | 0.71 |
| CRISPLD1 | 652 | 16939030 | C | 0.1739 | 0.0344300 | 0.77 |
| MMP16 | 653 | 2139481 | T | 0.4081 | 0.0348300 | 1.24 |
| BAALC | 654 | 13279226 | A | 0.2542 | 0.0107500 | 0.76 |
| ZFPM2 | 655 | 13280496 | C | 0.3012 | 0.0056020 | 0.75 |
| ZFPM2 | 656 | 12679854 | T | 0.3365 | 0.0253900 | 0.80 |
| ZFPM2 | 657 | 6981187 | G | 0.3393 | 0.0296600 | 0.80 |
| ZFPM2 | 658 | 6469014 | T | 0.1775 | 0.0233600 | 1.36 |
| ZFPM2 | 659 | 11986654 | G | 0.0829 | 0.0454900 | 1.47 |
| CSMD3 | 661 | 1492678 | T | 0.5025 | 0.0352900 | 1.23 |
| CSMD3 | 663 | 17640016 | A | 0.2745 | 0.0186700 | 1.31 |
| SAMD12 | 677 | 9642841 | C | 0.3627 | 0.0491000 | 1.23 |
| FER1L6 | 683 | 6981430 | G | 0.3759 | 0.0047760 | 0.75 |
| FER1L6 | 684 | 7831422 | G | 0.1154 | 0.0189700 | 0.71 |
| FERIL6 | 685 | 12375254 | A | 0.1196 | 0.0197900 | 0.72 |
| DDEF1 | 691 | 12675390 | C | 0.2512 | 0.0098780 | 0.75 |
| DDEF1 | 692 | 4733776 | T | 0.3840 | 0.0307800 | 0.80 |
| DDEF1 | 693 | 4733572 | A | 0.3973 | 0.0384400 | 0.81 |
| DDEF1 | 694 | 4733788 | G | 0.2069 | 0.0292300 | 0.78 |
| DDEF1 | 695 | 11786705 | G | 0.2105 | 0.0377300 | 0.78 |
| COL22A1 | 701 | 13271565 | T | 0.2494 | 0.0266400 | 1.30 |
| COL22A1 | 703 | 10104531 | T | 0.3726 | 0.0223300 | 1.27 |
| COL22A1 | 704 | 12674962 | C | 0.4615 | 0.0233700 | 1.25 |
| COL22A1 | 705 | 9324496 | T | 0.3906 | 0.0209500 | 1.27 |
| ZC3H3 | 706 | 4874147 | A | 0.3235 | 0.0017120 | 1.41 |
| PIP5K1B | 709 | 963707 | G | 0.3329 | 0.0294700 | 0.80 |
| PIP5K1B | 710 | 1414949 | C | 0.5195 | 0.0205100 | 1.26 |
| TRPM3 | 711 | 718447 | A | 0.2220 | 0.0377800 | 0.79 |
| TMC1 | 712 | 1417614 | T | 0.3493 | 0.0118900 | 1.30 |
| TMC1 | 713 | 2793153 | G | 0.4663 | 0.0051150 | 1.32 |
| TMC1 | 714 | 2589615 | A | 0.4672 | 0.0052340 | 1.32 |
| TMC1 | 715 | 1796991 | G | 0.1950 | 0.0420100 | 1.30 |
| TMC1 | 716 | 13286194 | C | 0.0517 | 0.0086850 | 0.59 |
| PCSK5 | 717 | 1029034 | G | 0.3489 | 0.0285400 | 0.80 |
| GNAQ | 718 | 964423 | T | 0.2907 | 0.0443700 | 1.25 |
| GNAQ | 719 | 7032206 | A | 0.2476 | 0.0197300 | 1.32 |
| GNAQ | 720 | 6560625 | C | 0.2861 | 0.0025030 | 1.41 |
| NTRK2 | 721 | 7038866 | A | 0.2661 | 0.0426500 | 0.80 |
| DIRAS2 | 722 | 690232 | A | 0.2587 | 0.0229200 | 0.78 |
| DIRAS2 | 723 | 1360471 | C | 0.2232 | 0.0404200 | 0.79 |
| NFIL3 | 724 | 2440589 | T | 0.4654 | 0.0235300 | 1.25 |
| FKTN | 730 | 885954 | C | 0.4666 | 0.0206900 | 1.26 |
| FKTN | 731 | 17251166 | C | 0.3305 | 0.0368900 | 1.25 |
| PALM2 | 732 | 1535457 | A | 0.2804 | 0.0005222 | 1.49 |
| PALM2 | 733 | 7849021 | G | 0.3216 | 0.0000471 | 1.57 |
| PALM2 | 734 | 10980026 | G | 0.3258 | 0.0000760 | 1.54 |
| PALM2 | 735 | 10980027 | A | 0.3266 | 0.0001044 | 1.53 |
| PALM2 | 736 | 17202497 | A | 0.3333 | 0.0003072 | 1.48 |
| ZNF618 | 737 | 4978561 | T | 0.3285 | 0.0213400 | 0.79 |
| ZNF618 | 738 | 10982031 | C | 0.3174 | 0.0250200 | 0.79 |
| ZNF618 | 739 | 4979326 | A | 0.3153 | 0.0300000 | 0.80 |
| PTGS1 | 743 | 1234909 | G | 0.1830 | 0.0481300 | 1.30 |
| RALGPS1 | 744 | 665249 | G | 0.3377 | 0.0015720 | 1.40 |
| RALGPS1 | 745 | 10760473 | G | 0.3373 | 0.0020180 | 1.39 |
| RALGPS1 | 746 | 10819264 | T | 0.4688 | 0.0276700 | 0.81 |
| RALGPS1 | 747 | 13298677 | G | 0.2088 | 0.0018200 | 1.49 |
| RALGPS1 | 748 | 1028866 | G | 0.4714 | 0.0401800 | 0.82 |
| SLC25A25 | 749 | 9695803 | C | 0.1432 | 0.0342600 | 1.37 |
| TSC1 | 750 | 7875558 | A | 0.1849 | 0.0035010 | 1.49 |
| TSC1 | 751 | 7858160 | T | 0.1890 | 0.0313800 | 1.33 |
| TSC1 | 752 | 7035308 | A | 0.2644 | 0.0281600 | 1.29 |
| VAV2 | 753 | 10993826 | T | 0.1675 | 0.0055510 | 1.48 |
| VAV2 | 754 | 2073929 | G | 0.1615 | 0.0137900 | 1.42 |
| VAV2 | 755 | 3819500 | T | 0.1595 | 0.0178000 | 1.40 |
| VAV2 | 756 | 7023551 | G | 0.2206 | 0.0377700 | 1.29 |
| PITRM1 | 757 | 7924157 | C | 0.1914 | 0.0075340 | 1.42 |
| PRKCQ | 758 | 2387902 | G | 0.5012 | 0.0037310 | 1.33 |
| PRKCQ | 759 | 11254972 | A | 0.4353 | 0.0051860 | 0.76 |
| PRKCQ | 760 | 4747393 | A | 0.3819 | 0.0175000 | 0.79 |
| CACNB2 | 761 | 12779641 | G | 0.1427 | 0.0161900 | 0.73 |
| ARMC3 | 762 | 1171136 | A | 0.2645 | 0.0089570 | 1.35 |
| ARMC3 | 763 | 9665603 | G | 0.3464 | 0.0002269 | 1.49 |
| ARMC3 | 764 | 10764367 | C | 0.3477 | 0.0005803 | 1.45 |
| ARMC3 | 765 | 1054052 | T | 0.3218 | 0.0226200 | 1.28 |
| ARMC3 | 766 | 12252340 | T | 0.2215 | 0.0334200 | 1.30 |
| ARMC3 | 767 | 1382580 | G | 0.1396 | 0.0389300 | 1.36 |
| ARMC3 | 768 | 1382579 | G | 0.0470 | 0.0357500 | 1.74 |
| JMJD1C | 774 | 12355784 | A | 0.4467 | 0.0067450 | 0.77 |
| JMJD1C | 775 | 3999089 | C | 0.4450 | 0.0070740 | 0.77 |
| CDH23 | 776 | 17635709 | T | 0.2038 | 0.0493200 | 0.79 |
| KCNMA1 | 778 | 4639876 | T | 0.3353 | 0.0200300 | 1.28 |
| SORCS3 | 779 | 902300 | T | 0.2764 | 0.0218900 | 1.30 |
| VTI1A | 780 | 7088500 | C | 0.3103 | 0.0006779 | 1.45 |
| VTI1A | 781 | 7907361 | C | 0.2962 | 0.0011970 | 1.44 |
| VTI1A | 782 | 7096151 | A | 0.3055 | 0.0021240 | 1.40 |
| VTI1A | 783 | 7070850 | G | 0.3067 | 0.0451800 | 1.24 |
| VTI1A | 784 | 6585184 | A | 0.3390 | 0.0499900 | 1.23 |
| ATRNL1 | 785 | 2068463 | C | 0.4153 | 0.0452900 | 1.22 |
| HSPA12A | 786 | 3010460 | C | 0.3612 | 0.0179500 | 1.28 |
| STIM1 | 798 | 2959068 | T | 0.4258 | 0.0093460 | 0.77 |
| STIM1 | 799 | 12803023 | G | 0.4569 | 0.0028360 | 0.75 |
| TRIM21 | 800 | 1426378 | A | 0.1534 | 0.0099630 | 1.46 |
| GALNTL4 | 801 | 4910288 | A | 0.3890 | 0.0198900 | 0.79 |
| GALNTL4 | 802 | 4910289 | C | 0.2578 | 0.0137900 | 0.77 |
| GALNTL4 | 803 | 10831565 | A | 0.4672 | 0.0100100 | 1.29 |
| SPON1 | 804 | 2174146 | T | 0.2243 | 0.0357900 | 1.29 |
| SPON1 | 805 | 11238 | T | 0.2852 | 0.0035120 | 0.74 |
| IGSF22 | 815 | 4537730 | A | 0.2407 | 0.0041330 | 0.73 |
| IGSF22 | 816 | 7125943 | T | 0.2206 | 0.0059280 | 0.73 |
| IGSF22 | 817 | 10766494 | C | 0.2255 | 0.0102600 | 0.75 |
| C11ORF49 | 818 | 4548577 | T | 0.2410 | 0.0178300 | 1.33 |
| C11ORF49 | 819 | 11039112 | T | 0.3433 | 0.0037250 | 1.36 |
| C11ORF49 | 820 | 747650 | A | 0.3437 | 0.0041950 | 1.36 |
| DLG2 | 821 | 2507850 | A | 0.3353 | 0.0039160 | 0.75 |
| DLG2 | 822 | 7108874 | T | 0.2524 | 0.0106300 | 0.76 |
| DLG2 | 823 | 10898133 | A | 0.3624 | 0.0456200 | 0.82 |
| DLG2 | 827 | 2514147 | G | 0.1064 | 0.0098870 | 1.57 |
| DLG2 | 828 | 1943708 | C | 0.3527 | 0.0046480 | 1.35 |
| DLG2 | 829 | 1943701 | G | 0.5252 | 0.0095020 | 1.29 |
| DLG2 | 830 | 2187359 | A | 0.3398 | 0.0243200 | 1.27 |
| DLG2 | 831 | 11234201 | T | 0.4075 | 0.0341200 | 0.81 |
| ELMOD1 | 832 | 684901 | T | 0.3141 | 0.0002093 | 1.51 |
| OPCML | 833 | 10894536 | A | 0.0823 | 0.0049550 | 0.63 |
| TSPAN9 | 837 | 10774140 | C | 0.3126 | 0.0416600 | 0.81 |
| STYK1 | 838 | 1078437 | G | 0.4050 | 0.0082290 | 1.31 |
| STYK1 | 839 | 7350597 | A | 0.4647 | 0.0009245 | 1.39 |
| STYK1 | 840 | 10845198 | T | 0.4050 | 0.0223300 | 1.26 |
| STYK1 | 841 | 10743918 | C | 0.3533 | 0.0466500 | 0.82 |
| LOC729025 | 842 | 1624759 | G | 0.2975 | 0.0448900 | 1.25 |
| LRP1 | 843 | 1800176 | T | 0.2760 | 0.0040280 | 0.74 |
| LRP1 | 844 | 1800168 | C | 0.2816 | 0.0075630 | 0.75 |
| CNOT2 | 845 | 1595410 | C | 0.4129 | 0.0488400 | 0.82 |
| CNOT2 | 846 | 3817487 | A | 0.3969 | 0.0458300 | 0.82 |
| KCNC2 | 847 | 2136036 | G | 0.3333 | 0.0396900 | 0.81 |
| NAV3 | 848 | 7139116 | C | 0.1319 | 0.0173000 | 0.72 |
| NAV3 | 849 | 10777655 | A | 0.1313 | 0.0243500 | 0.73 |
| GAS2L3 | 850 | 17030333 | T | 0.0431 | 0.0009065 | 2.84 |
| SLC46A3 | 853 | 9579226 | T | 0.3534 | 0.0142500 | 0.78 |
| SLC46A3 | 854 | 7339280 | A | 0.4234 | 0.0027410 | 0.75 |
| SLC46A3 | 855 | 715704 | G | 0.4141 | 0.0451800 | 1.22 |
| UBL3 | 856 | 7328052 | A | 0.1039 | 0.0234600 | 1.49 |
| N4BP2L2 | 857 | 208431 | G | 0.2160 | 0.0064940 | 0.73 |
| NBEA | 858 | 7320580 | G | 0.2337 | 0.0249300 | 1.31 |
| NBEA | 859 | 7988705 | A | 0.2265 | 0.0481900 | 1.27 |
| NBEA | 860 | 17769661 | T | 0.1483 | 0.0490100 | 1.33 |
| SLAIN1 | 861 | 1343911 | A | 0.1775 | 0.0061900 | 1.46 |
| GPC5 | 862 | 10851317 | A | 0.4604 | 0.0068410 | 1.31 |
| GPC5 | 863 | 1330469 | T | 0.4486 | 0.0319200 | 0.81 |
| GPC5 | 864 | 9515990 | C | 0.3796 | 0.0288700 | 1.25 |
| GPC6 | 865 | 9561427 | G | 0.3005 | 0.0154800 | 1.31 |
| GPC6 | 866 | 9589816 | A | 0.4012 | 0.0160400 | 1.28 |
| NALCN | 867 | 16958230 | G | 0.0692 | 0.0074300 | 0.63 |
| NALCN | 868 | 625331 | C | 0.1392 | 0.0061870 | 0.69 |
| ITGBL1 | 869 | 4400915 | T | 0.0653 | 0.0362700 | 1.60 |
| WDR23 | 870 | 3825584 | T | 0.3134 | 0.0020680 | 1.40 |
| WDR23 | 871 | 11574503 | C | 0.3505 | 0.0103900 | 1.31 |
| GNG2 | 874 | 2357247 | C | 0.3589 | 0.0066230 | 0.76 |
| GNG2 | 875 | 1959518 | C | 0.3969 | 0.0066270 | 0.76 |
| SAMD4A | 876 | 709939 | C | 0.4246 | 0.0123600 | 0.78 |
| SAMD4A | 877 | 8021957 | T | 0.4224 | 0.0069810 | 0.77 |
| SAMD4A | 878 | 8021151 | G | 0.4115 | 0.0070300 | 0.77 |
| PPP2R5E | 880 | 1255741 | A | 0.3301 | 0.0386300 | 1.25 |
| RGS6 | 882 | 2239268 | C | 0.2760 | 0.0076130 | 0.75 |
| RGS6 | 883 | 12885938 | C | 0.2754 | 0.0076460 | 0.75 |
| KCNK10 | 886 | 11625905 | T | 0.2098 | 0.0181400 | 0.76 |
| KCNK13 | 887 | 11621045 | G | 0.4200 | 0.0029250 | 1.35 |
| KCNK13 | 888 | 11159957 | G | 0.4209 | 0.0043360 | 1.33 |
| PSMC1 | 889 | 7150093 | A | 0.4179 | 0.0038340 | 1.34 |
| PSMC1 | 890 | 6575120 | G | 0.4123 | 0.0050780 | 1.33 |
| PSMC1 | 891 | 2236403 | T | 0.4118 | 0.0060210 | 1.33 |
| RPS6KA5 | 892 | 1286264 | C | 0.3699 | 0.0126600 | 0.78 |
| RPS6KA5 | 893 | 941847 | T | 0.2524 | 0.0237800 | 1.31 |
| RPS6KA5 | 894 | 1286060 | C | 0.2691 | 0.0119300 | 1.33 |
| RPS6KA5 | 895 | 9944098 | T | 0.5193 | 0.0264300 | 1.25 |
| RPS6KA5 | 896 | 7156252 | T | 0.3184 | 0.0059240 | 1.35 |
| RPS6KA5 | 897 | 11159988 | C | 0.3186 | 0.0065270 | 1.34 |
| RPS6KA5 | 898 | 7492628 | G | 0.3423 | 0.0222200 | 1.28 |
| CCDC88C | 901 | 8015982 | C | 0.2566 | 0.0043250 | 1.40 |
| CCDC88C | 902 | 10131741 | G | 0.2542 | 0.0082870 | 1.36 |
| CCDC88C | 903 | 8007791 | A | 0.2536 | 0.0099850 | 1.35 |
| BCL11B | 904 | 2793321 | T | 0.3254 | 0.0009968 | 1.43 |
| BCL11B | 905 | 1257446 | C | 0.3141 | 0.0005659 | 1.46 |
| BCL11B | 906 | 1257416 | G | 0.3529 | 0.0128600 | 1.30 |
| ATP10A | 907 | 4906747 | G | 0.0719 | 0.0298000 | 0.68 |
| ATP10A | 908 | 17667563 | A | 0.1947 | 0.0350500 | 1.32 |
| C15ORF41 | 909 | 1990659 | A | 0.3301 | 0.0255100 | 0.80 |
| GLDN | 910 | 2470172 | C | 0.4440 | 0.0116100 | 1.29 |
| GLDN | 911 | 2470173 | T | 0.2074 | 0.0137300 | 1.37 |
| GLDN | 912 | 16964319 | T | 0.0385 | 0.0182600 | 2.05 |
| GLDN | 913 | 10163098 | G | 0.0349 | 0.0317900 | 1.97 |
| CLK3 | 914 | 11072496 | T | 0.1695 | 0.0067190 | 1.46 |
| CLK3 | 915 | 7161903 | G | 0.1675 | 0.0088530 | 1.44 |
| TBC1D2B | 916 | 16969397 | T | 0.0933 | 0.0019920 | 1.81 |
| TBC1D2B | 917 | 8030999 | G | 0.1295 | 0.0021610 | 1.64 |
| PCSK6 | 918 | 11247300 | A | 0.1527 | 0.0073280 | 1.48 |
| PCSK6 | 919 | 735504 | C | 0.4748 | 0.0495100 | 0.82 |
| A2BP1 | 920 | 9935875 | G | 0.4207 | 0.0444500 | 0.82 |
| A2BP1 | 922 | 1034971 | G | 0.3708 | 0.0348400 | 0.81 |
| TMC5 | 923 | 8058400 | C | 0.2434 | 0.0489700 | 1.26 |
| TMC5 | 924 | 8063463 | G | 0.1954 | 0.0000370 | 1.76 |
| TMC5 | 925 | 2856608 | G | 0.3055 | 0.0040570 | 1.37 |
| TMC5 | 926 | 11640077 | G | 0.3034 | 0.0038910 | 1.38 |
| GDE1 | 927 | 739639 | C | 0.3926 | 0.0128300 | 1.29 |
| GDE1 | 928 | 3751821 | T | 0.0962 | 0.0098070 | 1.61 |
| HYDIN | 929 | 9939485 | G | 0.4475 | 0.0004328 | 0.71 |
| HYDIN | 930 | 9939194 | T | 0.3664 | 0.0012840 | 1.40 |
| HYDIN | 931 | 184647 | C | 0.3522 | 0.0317600 | 0.80 |
| W WOX | 933 | 11648121 | A | 0.1791 | 0.0043000 | 0.71 |
| WWOX | 934 | 12924899 | C | 0.2494 | 0.0315300 | 0.79 |
| WWOX | 935 | 7204203 | A | 0.0354 | 0.0466200 | 1.86 |
| MPHOSPH6 | 936 | 11150443 | C | 0.1265 | 0.0000856 | 1.97 |
| MPHOSPH6 | 937 | 10514533 | C | 0.1492 | 0.0003630 | 1.72 |
| MPHOSPH6 | 938 | 1862820 | G | 0.3819 | 0.0188200 | 1.27 |
| MPHOSPH6 | 939 | 918763 | C | 0.4904 | 0.0009759 | 1.38 |
| MPHOSPH6 | 940 | 902542 | A | 0.1679 | 0.0000579 | 1.80 |
| MPHOSPH6 | 941 | 2967337 | C | 0.1408 | 0.0000754 | 1.88 |
| MPHOSPH6 | 942 | 2967333 | G | 0.1667 | 0.0000047 | 2.00 |
| MPHOSPH6 | 943 | 2967328 | C | 0.1663 | 0.0001142 | 1.76 |
| CDH13 | 944 | 4598906 | T | 0.3449 | 0.0296400 | 0.80 |
| USP10 | 947 | 964453 | G | 0.2739 | 0.0149200 | 1.32 |
| DNAH9 | 948 | 11650055 | T | 0.4511 | 0.0110200 | 0.78 |
| DNAH9 | 949 | 7213187 | G | 0.4463 | 0.0039930 | 1.33 |
| RAB11FIP4 | 950 | 757378 | T | 0.4111 | 0.0069170 | 1.32 |
| CA10 | 952 | 11652641 | G | 0.2041 | 0.0441000 | 1.29 |
| HRNBP3 | 953 | 7212305 | A | 0.1208 | 0.0238800 | 1.44 |
| ZFP161 | 954 | 990072 | T | 0.4275 | 0.0000163 | 0.65 |
| ZFP161 | 955 | 11665417 | T | 0.4344 | 0.0030090 | 1.35 |
| ZFP161 | 956 | 9945680 | A | 0.4354 | 0.0037980 | 1.34 |
| ZFP161 | 957 | 9965501 | T | 0.0275 | 0.0069490 | 2.91 |
| ZFP161 | 958 | 8082898 | G | 0.0191 | 0.0318600 | 2.70 |
| PTPRM | 959 | 8089695 | C | 0.0801 | 0.0492000 | 1.47 |
| PTPRM | 960 | 1866854 | G | 0.3229 | 0.0031190 | 1.38 |
| PTPRM | 961 | 10513901 | A | 0.4928 | 0.0390600 | 1.23 |
| KIAA0802 | 962 | 17407982 | C | 0.2200 | 0.0023400 | 0.71 |
| KIAA0802 | 963 | 17408213 | T | 0.2159 | 0.0026080 | 0.71 |
| OSBPL1A | 964 | 275875 | G | 0.4257 | 0.0078430 | 0.77 |
| CHST9 | 965 | 559547 | T | 0.3413 | 0.0086020 | 0.77 |
| CHST9 | 966 | 12604227 | G | 0.3828 | 0.0291900 | 0.80 |
| CHST9 | 967 | 8084423 | A | 0.4535 | 0.0261100 | 1.25 |
| CHST9 | 968 | 1426879 | C | 0.3205 | 0.0033150 | 1.38 |
| CHST9 | 969 | 4800797 | G | 0.3216 | 0.0039910 | 1.37 |
| NEDD4L | 970 | 4940385 | C | 0.4057 | 0.0265100 | 0.80 |
| NEDD4L | 971 | 7242486 | G | 0.4115 | 0.0400400 | 0.81 |
| NEDD4L | 972 | 17064977 | C | 0.2542 | 0.0401800 | 0.80 |
| NEDD4L | 973 | 11152073 | A | 0.2542 | 0.0424300 | 0.80 |
| CCBE1 | 974 | 17065761 | C | 0.0979 | 0.0433000 | 0.73 |
| CCBE1 | 975 | 12969965 | A | 0.2017 | 0.0212200 | 1.34 |
| CDH7 | 977 | 7228669 | T | 0.3065 | 0.0427800 | 1.25 |
| CDH7 | 978 | 2628210 | G | 0.2141 | 0.0092920 | 0.74 |
| CDH7 | 979 | 2587427 | A | 0.3317 | 0.0249700 | 0.80 |
| CDH7 | 980 | 2587428 | T | 0.2811 | 0.0063980 | 0.75 |
| TXNDC10 | 981 | 309242 | G | 0.1506 | 0.0000764 | 1.84 |
| FERMT1 | 983 | 6516104 | T | 0.1229 | 0.0002756 | 1.85 |
| FERM1 | 984 | 16991866 | C | 0.1193 | 0.0005018 | 1.81 |
| PLCB1 | 985 | 6055513 | G | 0.4435 | 0.0060730 | 0.76 |
| PLCB4 | 986 | 6056653 | G | 0.1411 | 0.0384900 | 0.76 |
| MACROD2 | 987 | 6079910 | C | 0.1085 | 0.0008739 | 1.83 |
| MACROD2 | 988 | 6043402 | A | 0.0627 | 0.0474900 | 1.56 |
| MACROD2 | 989 | 463020 | G | 0.4248 | 0.0060440 | 0.76 |
| MACROD2 | 990 | 6135667 | T | 0.3305 | 0.0463400 | 0.82 |
| KIF16B | 991 | 6044051 | T | 0.1803 | 0.0000708 | 1.76 |
| KIF16B | 992 | 720592 | T | 0.3138 | 0.0441900 | 1.24 |
| MAPRE1 | 993 | 410488 | A | 0.3959 | 0.0021780 | 0.74 |
| MAPRE1 | 994 | 242529 | C | 0.3353 | 0.0006392 | 1.44 |
| ZSWIM3 | 996 | 1967656 | A | 0.4640 | 0.0035740 | 1.34 |
| ZSWIM3 | 997 | 6514065 | C | 0.4104 | 0.0033630 | 1.35 |
| ZSWIM3 | 998 | 1045493 | G | 0.4315 | 0.0471300 | 1.22 |
| SNAI1 | 999 | 6020157 | A | 0.1468 | 0.0298400 | 0.75 |
| SNAI1 | 1000 | 16995010 | C | 0.1147 | 0.0150300 | 0.70 |
| SNAI1 | 1001 | 6091080 | G | 0.1727 | 0.0003309 | 0.65 |
| SNAI1 | 1002 | 6020187 | G | 0.2064 | 0.0017860 | 0.70 |
| C21ORF37 | 1005 | 12483129 | C | 0.4519 | 0.0044570 | 0.76 |
| C21ORF37 | 1006 | 2824301 | A | 0.5167 | 0.0004203 | 1.41 |
| NCAM2 | 1007 | 9979231 | G | 0.2644 | 0.0095300 | 1.35 |
| PDE9A | 1011 | 2284976 | A | 0.3846 | 0.0019000 | 1.38 |
| ARVCF | 1012 | 2239395 | G | 0.0322 | 0.0229700 | 0.57 |
| ARVCF | 1013 | 12171109 | A | 0.1993 | 0.0448300 | 0.79 |
| SGSM1 | 1014 | 5760752 | C | 0.3182 | 0.0299600 | 1.26 |
| ASPHD2 | 1015 | 8140344 | A | 0.4951 | 0.0038120 | 1.33 |
| ASPHD2 | 1016 | 6005042 | A | 0.4550 | 0.0041750 | 0.75 |
| ASPHD2 | 1017 | 6519650 | C | 0.3373 | 0.0003330 | 0.70 |
| HPS4 | 1018 | 5761552 | T | 0.4507 | 0.0000837 | 0.68 |
| ARFGAP3 | 1019 | 1018448 | A | 0.4246 | 0.0077450 | 0.77 |
| ARFGAP3 | 1020 | 6002963 | G | 0.4272 | 0.0302100 | 0.81 |
| TTLL1 | 1021 | 1052160 | T | 0.3245 | 0.0088850 | 1.33 |
| TTLL1 | 1022 | 135002 | C | 0.4725 | 0.0079110 | 1.30 |
| EFCAB6 | 1023 | 137794 | A | 0.1067 | 0.0406800 | 0.74 |
| EFCAB6 | 1024 | 5764310 | C | 0.0863 | 0.0318600 | 0.71 |
| RIBC2 | 1025 | 6007043 | C | 0.1683 | 0.0004614 | 0.65 |

| Table 2: CNV Deletions Influencing Schizophrenia Risk | | | | |
|---|---|---|---|---|
| Gene | Seq ID | NCBI RS# | P | Odds Ratio |
| BMP7 | 1003 | 2180782 | 0.004756 | >7.00 |
| CTNNA2 | 81 | 1930 | 0.020961 | 7.99 |
| CTNNA2 | 82 | 10170020 | 0.039020 | 6.85 |
| CTNNA2 | 83 | 6743980 | 0.032919 | >4.00 |
| CTNNA2 | 84 | 13423141 | 0.043911 | 2.45 |
| DNER | 114 | 6436860 | 0.032919 | >4.00 |
| ITPR1 | 120 | 3792490 | 0.021448 | <0.17 |
| ROBO1 | 155 | 1457659 | 0.031601 | 0.14 |
| ROBO1 | 156 | 3821603 | 0.031601 | 0.14 |
| KCNIP4 | 196 | 7694587 | 0.050947 | 0.58 |
| STIM2 | 197 | 4557250 | 0.033306 | 0.58 |
| STIM2 | 198 | 4532223 | 0.033306 | 0.58 |
| STIM2 | 199 | 4274829 | 0.033306 | 0.58 |
| STIM2 | 200 | 4692128 | 0.013672 | 0.43 |
| STIM2 | 201 | 7671671 | 0.021696 | 0.36 |
| INPP4B | 230 | 3102439 | 0.035939 | <0.20 |
| INPP4B | 231 | 1907119 | 0.035939 | <0.20 |
| INPP4B | 232 | 2636645 | 0.021448 | <0.17 |
| INPP4B | 235 | 881611 | 0.030163 | 2.98 |
| INPP4B | 239 | 17016175 | 0.010488 | 0.26 |
| FSTL5 | 243 | 7690342 | 0.037009 | 0.28 |
| DNAH5 | 257 | 6876673 | 0.032919 | >4.00 |
| DNAH5 | 258 | 3777093 | 0.032919 | >4.00 |
| DNAH5 | 259 | 6554808 | 0.032919 | >4.00 |
| DNAH5 | 262 | 7715811 | 0.008964 | >6.00 |
| DNAH5 | 263 | 10513153 | 0.008964 | >6.00 |
| DNAH5 | 264 | 10039621 | 0.050044 | 3.43 |
| DNAH5 | 267 | 6874349 | 0.019794 | 5.14 |
| DNAH5 | 268 | 9885366 | 0.019794 | 5.14 |
| FBXL17 | 313 | 288180 | 0.002420 | 0.44 |
| FBXL17 | 314 | 288173 | 0.000172 | 0.22 |
| FBXL17 | 317 | 288146 | 0.035939 | <0.20 |
| JARID2 | 348 | 4085876 | 0.021448 | <0.17 |
| JARID2 | 349 | 2282827 | 0.035939 | <0.20 |
| ELOVL5 | 379 | 10948744 | 0.008060 | 0.81 |
| ELOVL5 | 380 | 2562898 | 0.009014 | 0.81 |
| ELOVL5 | 381 | 735860 | 0.007927 | 0.81 |
| ELOVL5 | 382 | 2057024 | 0.007927 | 0.81 |
| ELOVL5 | 383 | 1429146 | 0.006239 | 0.80 |
| ELOVL5 | 384 | 2073040 | 0.006239 | 0.80 |
| ELOVL5 | 385 | 9463895 | 0.006239 | 0.80 |
| ELOVL5 | 386 | 2235723 | 0.007090 | 0.81 |
| ELOVL5 | 387 | 1346603 | 0.007090 | 0.81 |
| ELOVL5 | 388 | 9474476 | 0.007090 | 0.81 |
| ELOVL5 | 389 | 2294867 | 0.007090 | 0.81 |
| ELOVL5 | 390 | 9349660 | 0.007090 | 0.81 |
| ELOVL5 | 391 | 974323 | 0.007090 | 0.81 |
| ELOVL5 | 392 | 6909592 | 0.006239 | 0.80 |
| ELOVL5 | 393 | 9367520 | 0.006239 | 0.80 |
| ELOVL5 | 394 | 9395854 | 0.007090 | 0.81 |
| ELOVL5 | 395 | 209485 | 0.007090 | 0.81 |
| ELOVL5 | 396 | 9395856 | 0.009860 | 0.81 |
| ELOVL5 | 397 | 7747926 | 0.010371 | 0.81 |
| ELOVL5 | 398 | 7738788 | 0.010371 | 0.81 |
| ELOVL5 | 399 | 209500 | 0.009167 | 0.81 |
| ELOVL5 | 400 | 9357760 | 0.009167 | 0.81 |
| ELOVL5 | 401 | 9370196 | 0.005869 | 0.80 |
| ELOVL5 | 402 | 209517 | 0.003217 | 0.79 |
| ELOVL5 | 403 | 9370201 | 0.003217 | 0.79 |
| ELOVL5 | 404 | 1579454 | 0.003217 | 0.79 |
| ELOVL5 | 405 | 9474507 | 0.004235 | 0.80 |
| ELOVL5 | 406 | 9296711 | 0.004235 | 0.80 |
| ELOVL5 | 407 | 1429143 | 0.005342 | 0.80 |
| NXPH1 | 447 | 2107280 | 0.032919 | >4.00 |
| NXPH1 | 448 | 10085720 | 0.028460 | 1.78 |
| NXPH1 | 449 | 4455737 | 0.028460 | 1.78 |
| DGKB | 452 | 17168091 | 0.025056 | 1.73 |
| DGKB | 453 | 6972310 | 0.033501 | 1.69 |
| DGKB | 454 | 12699619 | 0.035939 | 0.32 |
| DGKB | 455 | 10499441 | 0.035939 | 0.32 |
| CALN1 | 498 | 11768892 | 0.034094 | 0.35 |
| CALN1 | 499 | 1914381 | 0.034094 | 0.35 |
| MAGI2 | 502 | 12670134 | 0.022848 | 0.21 |
| PPP1R9A | 513 | 10252856 | 0.008106 | 1.28 |
| PPP1R9A | 514 | 6465448 | 0.028295 | 1.38 |
| PPP1R9A | 515 | 854717 | 0.020137 | 1.29 |
| PPP1R9A | 516 | 854737 | 0.006379 | 0.70 |
| PPP1R9A | 517 | 6961415 | 0.002304 | 0.70 |
| PPP1R9A | 518 | 17305991 | 0.020961 | 1.31 |
| PPP1R9A | 520 | 11762113 | 0.008914 | 0.73 |
| PPP1R9A | 523 | 854520 | 0.001873 | 1.50 |
| NRCAM | 532 | 401573 | 0.039482 | 0.70 |
| NRCAM | 535 | 422011 | 0.014469 | 0.74 |
| NRCAM | 536 | 425013 | 0.022980 | 1.33 |
| KCND2 | 542 | 9886190 | 0.019235 | 1.41 |
| SLC13A1 | 547 | 12706494 | 0.028960 | 0.78 |
| SLC13A1 | 549 | 2222514 | 0.004704 | 1.55 |
| SLC13A1 | 550 | 2402637 | 0.044434 | 0.82 |
| GRM8 | 556 | 2299500 | 0.028131 | 1.39 |
| GRM8 | 560 | 13240418 | 0.031601 | 0.80 |
| GRM8 | 561 | 4731330 | 0.010313 | 0.70 |
| GRM8 | 562 | 2299523 | 0.048865 | 0.79 |
| GRM8 | 563 | 2188187 | 0.026857 | 1.60 |
| GRM8 | 564 | 4141415 | 0.040424 | 1.38 |
| GRM8 | 565 | 10226889 | 0.049157 | 0.72 |
| DGKI | 572 | 4732255 | 0.015055 | 1.27 |
| DGKI | 576 | 7781327 | 0.046318 | 1.27 |
| CNTNAP2 | 580 | 10261766 | 0.016489 | 0.79 |
| CNTNAP2 | 581 | 13228526 | 0.023788 | 1.57 |
| CNTNAP2 | 588 | 17417154 | 0.013828 | 1.49 |
| CNTNAP2 | 589 | 1637845 | 0.026091 | 1.96 |
| PTPRN2 | 591 | 221247 | 0.045231 | 1.91 |
| PTPRN2 | 593 | 11767654 | 0.023113 | 1.34 |
| PTPRN2 | 594 | 10231253 | 0.023113 | 0.33 |
| PTPRN2 | 595 | 7789111 | 0.021448 | 2.30 |
| CSMD1 | 597 | 341724 | 0.001131 | 0.62 |
| CSMD1 | 599 | 7001551 | 0.005054 | 1.79 |
| CSMD1 | 601 | 7819225 | 0.043394 | 0.84 |
| PSD3 | 617 | 4298523 | 0.010548 | 1.83 |
| PSD3 | 618 | 2634435 | 0.025494 | 1.62 |
| PSD3 | 619 | 6987039 | 0.002996 | 1.63 |
| PSD3 | 621 | 10106032 | 0.025791 | 0.61 |
| PSD3 | 625 | 11778310 | 0.016210 | 1.65 |
| TOX | 640 | 12115074 | 0.046871 | 1.27 |
| TOX | 641 | 16924623 | 0.017555 | 1.29 |
| CSMD3 | 662 | 1873746 | 0.042883 | 0.80 |
| CSMD3 | 665 | 12676848 | 0.000006 | 2.32 |
| CSMD3 | 666 | 2123489 | 0.020022 | 1.48 |
| CSMD3 | 667 | 10505174 | 0.026242 | 1.44 |
| CSMD3 | 669 | 17602393 | 0.022717 | 0.70 |
| CSMD3 | 671 | 12545742 | 0.048865 | 0.84 |
| CSMD3 | 673 | 6999348 | 0.045500 | 0.74 |
| CSMD3 | 675 | 1402944 | 0.033501 | 1.39 |
| SAMD12 | 676 | 4631494 | 0.032347 | 1.32 |
| SAMD12 | 678 | 9297587 | 0.002542 | 1.52 |
| SAMD12 | 679 | 10955887 | 0.001095 | 1.65 |
| SAMD12 | 681 | 2514591 | 0.005492 | 1.40 |
| SAMD12 | 682 | 2514602 | 0.044434 | 1.54 |
| MTSS1 | 686 | 3829037 | 0.009696 | 1.29 |
| MTSS1 | 687 | 4871517 | 0.047432 | 0.81 |
| MTSS1 | 688 | 919544 | 0.014970 | 0.79 |
| KCNQ3 | 697 | 2469625 | 0.009805 | 4.58 |
| KCNQ3 | 698 | 2436134 | 0.019794 | 5.14 |
| COL22A1 | 702 | 7842696 | 0.009167 | 0.17 |
| SMARCA2 | 707 | 16937123 | 0.005167 | 2.76 |
| FKTN | 725 | 1858744 | 0.041881 | 0.85 |
| FKTN | 726 | 2768294 | 0.034094 | 0.84 |
| FKTN | 727 | 17309806 | 0.031054 | 0.84 |
| FKTN | 728 | 2518106 | 0.031418 | 0.84 |
| FKTN | 729 | 1858743 | 0.025940 | 0.84 |
| FKTN | 730 | 885954 | 0.018800 | 0.83 |
| FKTN | 731 | 17251166 | 0.015141 | 0.82 |
| ASTN2 | 740 | 7032028 | 0.006102 | 10.30 |
| CDK5RAP2 | 742 | 10760103 | 0.048865 | 2.74 |
| MYO3A | 770 | 10764597 | 0.012993 | <0.14 |
| JMJD1C | 772 | 10761739 | 0.035939 | <0.20 |
| JMJD1C | 773 | 10761741 | 0.021448 | <0.17 |
| ATE1 | 788 | 11200107 | 0.032919 | >4.00 |
| ATE1 | 790 | 11818001 | 0.035106 | 4.57 |
| ATE1 | 791 | 10732824 | 0.035106 | 4.57 |
| ATE1 | 792 | 10510101 | 0.019794 | 5.14 |
| ATE1 | 793 | 10788221 | 0.019794 | 5.14 |
| ATE1 | 794 | 3750835 | 0.019794 | 5.14 |
| ATE1 | 795 | 11200257 | 0.035106 | 4.57 |
| ATE1 | 796 | 11200260 | 0.035106 | 4.57 |
| ATE1 | 797 | 11200267 | 0.035106 | 4.57 |
| USH1C | 806 | 2072230 | 0.045500 | 0.40 |
| USH1C | 808 | 1076311 | 0.039715 | 0.80 |
| USH1C | 809 | 2190453 | 0.039715 | 0.80 |
| USH1C | 810 | 2190454 | 0.037446 | 0.79 |
| USH1C | 812 | 7113935 | 0.009805 | 0.45 |
| USH1C | 813 | 710G302 | 0.009375 | 0.48 |
| USH1C | R14 | 2073582 | 0.005082 | 0.45 |
| DLG2 | 825 | G592142 | 0.037667 | 0.66 |
| DLG2 | 826 | 1914203 | 0.021448 | <0.17 |
| TSPAN9 | 834 | IOR4R7R4 | 0.020961 | 7.99 |
| NPAS3 | 872 | 8022434 | 0.017062 | >5.00 |
| NPAS3 | 873 | 1 185 667 | 0.019457 | 0.13 |
| PPP2R5E | 879 | 6573522 | 0.011095 | 5.72 |
| RGS6 | RR 1 | 12586947 | 0.011221 | 2.95 |
| RGS6 | 882 | 22392fiR | 0.011221 | 2.95 |
| RGS6 | 885 | 2139594 | 0.012632 | 0.74 |
| CCDC88C | 899 | 4900075 | 0.035106 | 4.57 |
| A2BP1 | 921 | 7404422 | 0.034094 | 0.35 |
| WWOX | 932 | 16947095 | 0.006102 | 10.30 |
| CDH13 | 945 | 2061629 | 0.012280 | 0.43 |
| CCBE1 | 976 | 1864307 | 0.035939 | <0.20 |

| **Table 3: CNV Duplications Influencing Schizophrenia Risk** | | | | |
|---|---|---|---|---|
| **Gene** | **Seq ID** | **NCBI RS#** | **P** | **Odds Ratio** |
| CTNNA2 | 73 | 10189345 | 0.032919 | >4.00 |
| CTNNA2 | 74 | 3795994 | 0.017062 | >5.00 |
| CTNNA2 | 75 | 7571658 | 0.020961 | 7.99 |
| CTNNA2 | 76 | 6741563 | 0.048865 | 2.74 |
| FH1T | 141 | 2205353 | 0.050044 | 3.43 |
| CBLB | 161 | 12497428 | 0.029298 | 3.81 |
| CBLB | 162 | 6795961 | 0.029298 | 3.81 |
| NLGN1 | 178 | 1553122 | 0.017062 | >5.00 |
| STIM2 | 202 | 725981 | 0.020961 | 7.99 |
| LIMCH1 | 210 | 13113314 | 0.013993 | <0.14 |
| INPP4B | 233 | 2635429 | 0.035939 | <0.20 |
| INPP4B | 237 | 1391099 | 0.016965 | 0.45 |
| INPP4B | 238 | 9308152 | 0.050947 | 0.52 |
| CTNND2 | 255 | 6885224 | 0.035939 | <0.20 |
| CTNND2 | 256 | 2190989 | 0.039020 | 6.85 |
| DNAH5 | 269 | 1867679 | 0.048865 | 2.74 |
| ITGA1 | 287 | 16876270 | 0.039020 | 6.85 |
| PDE4D | 289 | 35285 | 0.021448 | <0.17 |
| GPR98 | 308 | 10043564 | 0.024912 | 2.42 |
| GPR98 | 310 | 1852731 | 0.006102 | 10.30 |
| FBXL17 | 315 | 288172 | 0.030961 | 7.99 |
| FBXL17 | 317 | 288146 | 0.011095 | 5.72 |
| SERPINB6 | 345 | 7751676 | 0.042630 | 1.17 |
| SERPINB6 | 346 | 1358869 | 0.020722 | 1.20 |
| JARID2 | 350 | 9396591 | 0.032919 | >4.00 |
| ATXN1 | 351 | 2237166 | 0.032919 | >4.00 |
| ATXN1 | 352 | 720006 | 0.032919 | >4.00 |
| ATXN1 | 353 | 2237165 | 0.032919 | >4.00 |
| RNF144B | 357 | 577287 | 0.003455 | 0.37 |
| BTN3A1 | 370 | 4712986 | 0.045500 | 0.40 |
| PDE7B | 427 | 9385740 | 0.002963 | <0.10 |
| DGKB | 456 | 1367780 | 0.020961 | 7.99 |
| DGKB | 457 | 1431538 | 0.017062 | >5.00 |
| ABCA13 | 487 | 11983883 | 0.000125 | 0.28 |
| ABCA13 | 488 | 2117089 | 0.000814 | 0.33 |
| ABCA13 | 489 | 7778020 | 0.032919 | >4.00 |
| WBSCR17 | 494 | 12538186 | 0.039020 | 6.85 |
| WBSCR17 | 495 | 648415 | 0.032919 | >4.00 |
| CALN1 | 496 | 12673109 | 0.023516 | 3.43 |
| CALN1 | 497 | 10260183 | 0.023113 | 0.33 |
| CALN1 | 500 | 17503400 | 0.000270 | 0.06 |
| MAGI2 | 501 | 7810778 | 0.011095 | 5.72 |
| MAGI2 | 507 | 10255710 | 0.032919 | >4.00 |
| MAGI2 | 509 | 7778707 | 0.021448 | <0.17 |
| PPP1R9A | 515 | 854717 | 0.005869 | 1.48 |
| PPP1R9A | 519 | 7806304 | 0.024484 | 0.65 |
| PPP1R9A | 520 | 11762113 | 0.047432 | 0.74 |
| PPP1R9A | 521 | 1918482 | 0.019794 | 1.49 |
| PPP1R9A | 523 | 854520 | 0.001989 | 1.75 |
| PPP1R9A | 525 | 854537 | 0.013672 | 1.82 |
| NRCAM | 529 | 409797 | 0.048286 | 1.87 |
| NRCAM | 530 | 449514 | 0.010488 | 1.26 |
| NRCAM | 531 | 107589 | 0.026394 | 0.74 |
| NRCAM | 533 | 404287 | 0.037227 | 1.22 |
| NRCAM | 534 | 369800 | 0.002931 | 1.28 |
| NRCAM | 535 | 422011 | 0.002775 | 2.38 |
| NRCAM | 537 | 13236767 | 0.016965 | 0.70 |
| NRCAM | 538 | 2284290 | 0.003878 | 1.74 |
| KCND2 | 540 | 12111791 | 0.002656 | 1.50 |
| KCND2 | 541 | 10224891 | 0.006170 | 1.45 |
| SLC13A1 | 550 | 2402637 | 0.019907 | 1.37 |
| GRM8 | 551 | 2299449 | 0.008864 | 1.66 |
| GRM8 | 552 | 2237735 | 0.024064 | 2.20 |
| GRM8 | 553 | 9942681 | 0.024064 | 1.21 |
| GRM8 | 554 | 1074728 | 0.047151 | 1.61 |
| GRM8 | 555 | 886176 | 0.021203 | 1.54 |
| GRM8 | 557 | 10487459 | 0.013291 | 1.42 |
| GRM8 | 558 | 1419508 | 0.000007 | 1.78 |
| GRM8 | 559 | 2023735 | 0.012280 | 1.87 |
| GRM8 | 565 | 10226889 | 0.024064 | 1.75 |
| EXOC4 | 566 | 1149550 | 0.040186 | 1.38 |
| EXOC4 | 567 | 7783217 | 0.048865 | 2.74 |
| EXOC4 | 568 | 10488164 | 0.005492 | 1.40 |
| EXOC4 | 570 | 6966403 | 0.035313 | 2.10 |
| DGKI | 571 | 2278829 | 0.002979 | 2.09 |
| DGKI | 572 | 4732255 | 0.014065 | 1.32 |
| DGKI | 573 | 4728415 | 0.002996 | 1.69 |
| DGKI | 574 | 1731913 | 0.030163 | 1.28 |
| DGKI | 575 | 2113578 | 0.008060 | 2.08 |
| DGKI | 577 | 706561 | 0.018481 | 3.21 |
| CNTNAP2 | 579 | 17480133 | 0.031601 | 1.43 |
| CNTNAP2 | 582 | 1548743 | 0.032919 | 0.74 |
| CNTNAP2 | 583 | 7802708 | 0.000396 | >11.00 |
| CNTNAP2 | 584 | 2022226 | 0.002715 | 1.66 |
| CNTNAP2 | 585 | 2373133 | 0.009696 | 2.11 |
| CNTNAP2 | 586 | 10270551 | 0.000003 | 0.59 |
| CNTNAP2 | 587 | 6953679 | 0.041389 | 1.81 |
| PTPRN2 | 590 | 221242 | 0.004098 | 0.72 |
| PTPRN2 | 596 | 10081235 | 0.025940 | 0.69 |
| CSMD1 | 597 | 341724 | 0.028960 | 0.75 |
| CSMD1 | 600 | 750318 | 0.024203 | 1.86 |
| CSMD1 | 602 | 13254344 | 0.006136 | 2.62 |
| CSMD1 | 603 | 895696 | 0.007372 | 1.65 |
| CSMD1 | 607 | 17066296 | 0.001131 | 0.52 |
| PSD3 | 620 | 13259762 | 0.010141 | 1.27 |
| PSD3 | 622 | 747555 | 0.012006 | 1.55 |
| PSD3 | 623 | 7007413 | 0.013518 | 1.57 |
| PSD3 | 624 | 6993404 | 0.018693 | 1.43 |
| PSD3 | 625 | 11778310 | 0.001389 | 2.01 |
| PSD3 | 626 | 1506894 | 0.009805 | 4.58 |
| TOX | 638 | 7845800 | 0.008151 | 1.85 |
| TOX | 639 | 4738732 | 0.021203 | 1.62 |
| CSMD3 | 660 | 10505182 | 0.044171 | 0.71 |
| CSMD3 | 664 | 6994009 | 0.000010 | 2.13 |
| CSMD3 | 665 | 12676848 | 0.048865 | 2.74 |
| CSMD3 | 668 | 1021876 | 0.047715 | 1.39 |
| CSMD3 | 670 | 964764 | 0.000229 | 1.94 |
| CSMD3 | 672 | 11995656 | 0.024064 | 2.20 |
| CSMD3 | 674 | 12680523 | 0.034094 | 0.72 |
| SAMD12 | 677 | 9642841 | 0.000002 | 1.86 |
| SAMD12 | 680 | 12541916 | 0.008016 | 2.65 |
| SAMD12 | 681 | 2514591 | 0.004053 | 3.90 |
| SAMD12 | 682 | 2514602 | 0.004500 | 1.57 |
| MTSS1 | 686 | 3829037 | 0.033501 | 0.73 |
| MTSS1 | 689 | 10956192 | 0.029298 | 2.05 |
| MTSS1 | 690 | 6470263 | 0.040186 | 0.64 |
| KCNQ3 | 696 | 977939 | 0.017063 | >5.00 |
| KCNQ3 | 697 | 2469625 | 0.002542 | >8.00 |
| KCNQ3 | 699 | 7832589 | 0.035939 | <0.20 |
| KCNQ3 | 700 | 1379582 | 0.035939 | <0.20 |
| SMARCA2 | 708 | 10965113 | 0.035106 | 4.57 |
| CDK5RAP2 | 741 | 914617 | 0.034294 | 1.94 |
| MYO3A | 769 | 12773856 | 0.035939 | <0.20 |
| ANK3 | 771 | 4948254 | 0.011284 | 9.14 |
| KCNMA1 | 777 | 2569360 | 0.035939 | <0.2 |
| GRK5 | 787 | 10787948 | 0.019794 | 5.14 |
| ATE1 | 789 | 753455 | 0.029298 | 2.05 |
| USH1C | 807 | 7104083 | 0.013141 | 1.66 |
| USH1C | 808 | 1076311 | 0.006344 | 1.71 |
| USH1C | 809 | 2190453 | 0.003455 | 1.77 |
| USH1C | 810 | 2190454 | 0.016677 | 1.64 |
| USH1C | 811 | 17714673 | 0.020961 | 7.99 |
| DLG2 | 824 | 6592141 | 0.032919 | >4.00 |
| TSPAN9 | 835 | 10848789 | 0.004756 | >7.00 |
| TSPAN9 | 836 | 7969363 | 0.048865 | 2.74 |
| MTIF3 | 851 | 3759434 | 0.000181 | 6.05 |
| MTIF3 | 852 | 3759433 | 0.001194 | 4.03 |
| RGS6 | 884 | 149641 | 0.021448 | <0.17 |
| CCDC88C | 900 | 747159 | 0.035106 | 4.57 |
| CDH13 | 946 | 451434 | 0.011284 | 9.14 |
| CA10 | 951 | 9303589 | 0.035939 | <0.20 |
| ATRN | 982 | 6076524 | 0.000877 | 0.22 |
| PTPRT | 995 | 6016664 | 0.008964 | >6.00 |
| CDH4 | 1004 | 4812313 | 0.034495 | 0.55 |
| NCAM2 | 1008 | 13052286 | 0.032919 | >4.00 |
| NCAM2 | 1009 | 2826815 | 0.029298 | 3.81 |
| NCAM2 | 1010 | 232509 | 0.050044 | 3.43 |

| **TABLE A: Summary of SNPs (IVCBI Human Genome Reference Assembly Build 36.3)** | | | | | | | |
|---|---|---|---|---|---|---|---|
| **Seq ID** | **Gene** | **Chr** | **Position** | **Seq ID** | **Gene** | **Chr** | **Position** |
| 1 | PIK3CD | 1 | 9,655,808 | 2 | RP1-21O18.1 | 1 | 15,265,882 |
| 3 | AGBL4-C1ORF165 | 1 | 49,012,555 | 4 | AGBL4-C1ORF165 | 1 | 49,046,634 |
| 5 | AGBL4-C1ORF165 | 1 | 49,107,917 | 6 | AGBL4-C1ORF165 | 1 | 49,124,764 |
| 7 | AGBL4-C1ORF165 | 1 | 49,132,460 | 8 | AGBL4-C1ORF165 | 1 | 49,133,207 |
| 9 | AGBL4-C1ORF165 | 1 | 49,214,610 | 10 | SCP2 | 1 | 53,261,152 |
| 11 | LRP8 | 1 | 53,485,315 | 12 | LRP8 | 1 | 53,504,903 |
| 13 | LRP8 | 1 | 53,519,183 | 14 | PRKACB | 1 | 84,323,437 |
| 15 | PRKACB | 1 | 84,343,593 | 16 | CGN | 1 | 149,764,000 |
| 17 | OLFML2B | 1 | 160,247,126 | 18 | DPT | 1 | 166,964,218 |
| 19 | SEC16B | 1 | 176,175,475 | 20 | SEC16B | 1 | 176,178,830 |
| 21 | MR1 | 1 | 179,279,594 | 22 | LAMC1 | 1 | 181,352,319 |
| 23 | LAMC1 | 1 | 181,374,069 | 24 | LAMC1 | 1 | 181,375,234 |
| 25 | KCNK2 | 1 | 213,267,433 | 26 | KCNK2 | 1 | 213,269,771 |
| 27 | KCNK2 | 1 | 213,286,968 | 28 | KCNK2 | 1 | 213,304,166 |
| 29 | KCNK2 | 1 | 313,312,765 | 30 | KCNK2 | 1 | 213,393,108 |
| 31 | KCNK2 | 1 | 213,405,685 | 32 | KCNK2 | 1 | 213,412,561 |
| 33 | KCNK2 | 1 | 213,428,830 | 34 | ESRRG | 1 | 214,811,868 |
| 35 | ESRRG | 1 | 214,951,990 | 36 | ESRRG | 1 | 214,952,115 |
| 37 | GNG4 | 1 | 233,794,439 | 38 | RYR2 | 1 | 235,793,994 |
| 39 | RYR2 | 1 | 235,885,637 | 40 | RYR2 | 1 | 236,061,906 |
| 41 | FMN2 | 1 | 238,512,719 | 42 | RGS7 | 1 | 239,145,433 |
| 43 | PLD5 | 1 | 240,426,890 | 44 | C2ORF46 | 2 | 8,321,006 |
| 45 | C2ORF46 | 2 | 8,354,700 | 46 | C2ORF46 | 2 | 8,355,126 |
| 47 | KCNF1 | 2 | 10,948,881 | 48 | KCNF1 | 2 | 10,981,799 |
| 49 | KCNF1 | 2 | 10,999,213 | 50 | ASXL2 | 2 | 25,956,101 |
| 51 | CRIM1 | 2 | 36,467,658 | 52 | CRIM1 | 2 | 36,521,723 |
| 53 | CRIM1 | 2 | 36,532,532 | 54 | CRIM1 | 2 | 36,610,853 |
| 55 | CRIM1 | 2 | 36,611,604 | 56 | HAAO | 2 | 42,869,208 |
| 57 | PLEKHH2 | 2 | 43,822,986 | 58 | PRKCE | 2 | 45,919,740 |
| 59 | PRKCE | 2 | 45,927,156 | 60 | FBXO11 | 2 | 47,947,841 |
| 61 | PSME4 | 2 | 53,970,304 | 62 | PSME4 | 2 | 54,064,285 |
| 63 | PSME4 | 2 | 54,136,333 | 64 | ACYP2 | 2 | 54,201,406 |
| 65 | CCDC85A | 2 | 56,425,867 | 66 | AAK1 | 2 | 69,604,707 |
| 67 | AAK1 | 2 | 69,637,782 | 68 | AAK1 | 2 | 69,650,512 |
| 69 | AAK1 | 2 | 69,708,598 | 70 | AAK1 | 2 | 69,716,927 |
| 71 | AAK1 | 2 | 69,740,261 | 72 | AAK1 | 2 | 69,768,980 |
| 73 | CTNNA2 | 2 | 79,724,263 | 74 | CTNNA2 | 2 | 79,726,512 |
| 75 | CTNNA2 | 2 | 79,728,810 | 76 | CTNNA2 | 2 | 79,734,051 |
| 77 | CTNNA2 | 2 | 79,931,793 | 78 | CTNNA2 | 2 | 79,934,690 |
| 79 | CTNNA2 | 2 | 79,942,001 | 80 | CTNNA2 | 2 | 80,337,745 |
| 81 | CTNNA2 | 2 | 80,368,183 | 82 | CTNNA2 | 2 | 80,373,325 |
| 83 | CTNNA2 | 2 | 80,377,211 | 84 | CTNNA2 | 2 | 80,624,319 |
| 85 | NAP5 | 2 | 133,558,451 | 86 | LRP1B | 2 | 140,986,740 |
| 87 | LRP1B | 2 | 142,371,550 | 88 | LRP1B | 2 | 142,377,358 |
| 89 | LRP1B | 2 | 142,395,682 | 90 | LRP1B | 2 | 142,419,855 |
| 91 | LRP1B | 2 | 142,598,638 | 92 | KYNU | 2 | 143,405,309 |
| 93 | KYNU | 2 | 143,455,211 | 94 | KYNU | 2 | 143,516,377 |
| 95 | KIF5C | 2 | 149,600,091 | 96 | CACNB4 | 2 | 152,587,594 |
| 97 | CACNB4 | 2 | 152,632,727 | 98 | FMNL2 | 2 | 152,981,465 |
| 99 | FMNL2 | 2 | 153,086,705 | 100 | FMNL2 | 2 | 153,096,733 |
| 101 | FMNL2 | 2 | 153,097,194 | 102 | FMNL2 | 2 | 153,146,684 |
| 103 | FMNL2 | 2 | 153,147,664 | 104 | FMNL2 | 2 | 153,159,182 |
| 105 | PKP4 | 2 | 159,340,155 | 106 | PKP4 | 2 | 159,240,805 |
| 107 | SCN7A | 2 | 166,998,337 | 108 | CERKL | 2 | 183,130,703 |
| 109 | CERKL | 2 | 183,145,339 | 110 | PDE1A | 2 | 182,763,240 |
| 111 | PARD3B | 2 | 205,659,848 | 112 | NRP2 | 2 | 206,441,948 |
| 113 | PIP5K3 | 2 | 208,956,492 | 114 | DNER | 2 | 229,932,713 |
| 115 | CNTN6 | 3 | 1,414,974 | 116 | CNTN4 | 3 | 2,326,831 |
| 117 | CNTN4 | 3 | 2,395,095 | 118 | CNTN4 | 3 | 2,476,259 |
| 119 | CNTN4 | 3 | 2,509,772 | 120 | ITPR1 | 3 | 4,688,162 |
| 121 | ITPR1 | 3 | 4,715,592 | 122 | IRAK3 | 3 | 10,234,114 |
| 123 | ATP2B2 | 3 | 10,396,240 | 124 | ATP2B2 | 3 | 10,411,869 |
| 125 | SLC6A6 | 3 | 14,495,168 | 126 | SLC6A6 | 3 | 14,495,564 |
| 127 | DAZL | 3 | 16,602,593 | 128 | DAZL | 3 | 16,611,541 |
| 129 | ARPP-21 | 3 | 35,817,608 | 130 | ARPP-21 | 3 | 35,819,665 |
| 131 | STAC | 3 | 36,433,107 | 132 | STAC | 3 | 36,445,311 |
| 133 | ULK4 | 3 | 41,455,051 | 134 | ULK4 | 3 | 41,476,741 |
| 135 | ULK4 | 3 | 41,482,456 | 136 | ULK4 | 3 | 41,501,971 |
| 137 | ULK4 | 3 | 41,507,368 | 138 | ZNF167 | 3 | 44,582,017 |
| 139 | CACNA2D3 | 3 | 55,011,390 | 140 | FLNB | 3 | 58,093,595 |
| 141 | FHIT | 3 | 60,782,868 | 142 | FHIT | 3 | 61,135,266 |
| 143 | FHIT | 3 | 61,137,444 | 144 | FHIT | 3 | 61,146,142 |
| 145 | FHIT | 3 | 61,159,361 | 146 | FHIT | 3 | 61,182,117 |
| 147 | FHIT | 3 | 61,214,250 | 148 | PRICKLE2 | 3 | 64,140,810 |
| 149 | ADAMTS9 | 3 | 64,500,753 | 150 | ADAMTS9 | 3 | 64,507,516 |
| 151 | MAGII | 3 | 65,576,699 | 152 | MAGI1 | 3 | 65,576,717 |
| 153 | MAGI1 | 3 | 65,576,735 | 154 | FOXP1 | 3 | 71,021,299 |
| 155 | ROBO1 | 3 | 78,866,959 | 156 | ROBO1 | 3 | 78,867,259 |
| 157 | GBE1 | 3 | 81,669,129 | 158 | GBE1 | 3 | 81,687,869 |
| 159 | GBE1 | 3 | 81,859,450 | 160 | EPHA6 | 3 | 98,268,377 |
| 161 | CBLB | 3 | 107,007,179 | 162 | CBLB | 3 | 107,007,333 |
| 163 | PLCXD2 | 3 | 112,949,845 | 164 | PLCXD2 | 3 | 112,950,789 |
| 165 | PLCXD2 | 3 | 112,953,907 | 166 | PLCXD2 | 3 | 112,960,464 |
| 167 | PLCXD2 | 3 | 112,964,847 | 168 | EPHB1 | 3 | 136,129,897 |
| 169 | SPSB4 | 3 | 142,287,637 | 170 | SPSB4 | 3 | 142,294,879 |
| 171 | SPSB4 | 3 | 142,328,274 | 172 | SERPIN12 | 3 | 168,658,115 |
| 173 | SEPPINI2 | 3 | 168,672,301 | 174 | SERPINI2 | 3 | 168,681,607 |
| 175 | TNIK | 3 | 172,402,113 | 176 | PLD1 | 3 | 172,790,551 |
| 177 | PLD1 | 3 | 172,791,538 | 178 | NLGN1 | 3 | 174,870,074 |
| 179 | NLGN1 | 3 | 175,493,649 | 180 | NLGN1 | 3 | 175,515,979 |
| 181 | NLGN1 | 3 | 175,543,000 | 182 | LEPREL1 | 3 | 191,218,155 |
| 183 | LEPREL1 | 3 | 191,244,878 | 184 | LEPREL1 | 3 | 191,245,227 |
| 185 | LEPREL1 | 3 | 191,250,258 | 186 | LEPREL1 | 3 | 191,253,292 |
| 187 | LEPREL1 | 3 | 191,261,577 | 188 | UBXD7 | 3 | 197,576,308 |
| 189 | UBXD7 | 3 | 197,632,452 | 190 | UBXD7 | 3 | 197,636,526 |
| 191 | SLC2A9 | 4 | 9,480,639 | 192 | SLC2A9 | 4 | 9,502,295 |
| 193 | SLC2A9 | 4 | 9,531,228 | 194 | LDB2 | 4 | 16,363,986 |
| 195 | LDB2 | 4 | 16,365,914 | 196 | KCN1P4 | 4 | 20,459,978 |
| 197 | STIM2 | 4 | 26,458,937 | 198 | STIM2 | 4 | 26,459,024 |
| 199 | STIM2 | 4 | 26,459,062 | 200 | STIM2 | 4 | 26,462,717 |
| 201 | STIM2 | 4 | 26,462,750 | 202 | STIM2 | 4 | 26,570,337 |
| 203 | KIAA1239 | 4 | 37,005,415 | 204 | KIAA1239 | 4 | 37,007,192 |
| 205 | UBE2K | 4 | 39,464,805 | 206 | LIMCH1 | 4 | 41,213,350 |
| 207 | LIMCH1 | 4 | 41,313,408 | 208 | LIMCH1 | 4 | 41,221,506 |
| 209 | LIMCH1 | 4 | 41,223,320 | 210 | LIMCH1 | 4 | 41,317,805 |
| 211 | LOC389207 | 4 | 42,652,288 | 212 | LOC389207 | 4 | 42,691,897 |
| 213 | LOC389207 | 4 | 42,693,025 | 214 | SCD5 | 4 | 83,920,815 |
| 215 | SCD5 | 4 | 83,923,596 | 216 | HERC3 | 4 | 89,803,447 |
| 217 | HERC3 | 4 | 89,843,208 | 218 | FAM13A1 | 4 | 89,887,882 |
| 219 | FAM13A1 | 4 | 89,915,913 | 220 | COL25A1 | 4 | 109,934,307 |
| 231 | ANK2 | 4 | 114,340,609 | 222 | CAMK2D | 4 | 114,648,856 |
| 223 | NDST3 | 4 | 119,186,644 | 224 | NDST3 | 4 | 119,256,090 |
| 225 | GPR103 | 4 | 122,487,863 | 226 | MAML3 | 4 | 140,897,308 |
| 227 | IL15 | 4 | 142,764,901 | 228 | IL15 | 4 | 142,911,691 |
| 229 | IL15 | 4 | 142,924,868 | 230 | INPP4B | 4 | 143,414,201 |
| 231 | INPP4B | 4 | 143,421,104 | 232 | INPP4B | 4 | 143,436,219 |
| 233 | INPP4B | 4 | 143,463,006 | 234 | INPP4B | 4 | 143,524,042 |
| 235 | INPP4B | 4 | 143,547,226 | 236 | INPP4B | 4 | 143,566,886 |
| 237 | INPP4B | 4 | 143,593,360 | 238 | INPP4B | 4 | 143,593,371 |
| 239 | INPP4B | 4 | 143,610,749 | 240 | POU4F2 | 4 | 147,739,219 |
| 241 | POU4F2 | 4 | 147,758,283 | 242 | DCLK2 | 4 | 151,378,402 |
| 243 | FSTL5 | 4 | 162,611,763 | 244 | FSTL5 | 4 | 162,953,212 |
| 245 | FSTL5 | 4 | 163,224,500 | 246 | TLL1 | 4 | 167,149,564 |
| 247 | PALLD | 4 | 169,939,539 | 248 | PALLD | 4 | 169,971,032 |
| 249 | CASP3 | 4 | 185,783,023 | 250 | CASP3 | 4 | 185,791,294 |
| 251 | PLEKHG4B | 5 | 238,160 | 252 | PLEKHG4B | 5 | 241,719 |
| 253 | AHRR | 5 | 401,598 | 254 | AHRR | 5 | 431,413 |
| 255 | CTNND2 | 5 | 11,222,945 | 256 | CTNND2 | 5 | 11,637,342 |
| 257 | DNAH5 | 5 | 13,780,350 | 258 | DNAH5 | 5 | 13,782,714 |
| 259 | DNAH5 | 5 | 13,788,147 | 260 | DNAH5 | 5 | 13,795,858 |
| 261 | DNAH5 | 5 | 13,802,952 | 262 | DNAH5 | 5 | 13,822,974 |
| 263 | DNAH5 | 5 | 13,823,000 | 264 | DNAH5 | 5 | 13,825,457 |
| 265 | DNAH5 | 5 | 13,841,886 | 266 | DNAH5 | 5 | 13,842,420 |
| 267 | DNAH5 | 5 | 13,852,381 | 268 | DNAH5 | 5 | 13,854,786 |
| 269 | DNAH5 | 5 | 13,875,140 | 270 | DNAH5 | 5 | 13,887,824 |
| 271 | DNAH5 | 5 | 13,986,416 | 272 | DNAH5 | 5 | 13,989,500 |
| 273 | DNAH5 | 5 | 14,032,397 | 274 | DNAH5 | 5 | 14,034,968 |
| 275 | CDH10 | 5 | 24,728,325 | 276 | CDH10 | 5 | 24,738,958 |
| 277 | SLC45A2 | 5 | 33,979,974 | 278 | SLC45A2 | 5 | 33,990,637 |
| 279 | SLC1A3 | 5 | 36,670,315 | 280 | SLC1A3 | 5 | 36,675,215 |
| 281 | EGFLAM | 5 | 38,269,319 | 282 | EGFLAM | 5 | 38,296,060 |
| 283 | EGFLAM | 5 | 38,337,535 | 284 | EGFLAM | 5 | 38,357,424 |
| 285 | EGFLAM | 5 | 38,382,422 | 286 | EGFLAM | 5 | 38,485,966 |
| 287 | ITGA1 | 5 | 52,294,108 | 288 | ITGA2 | 5 | 52,425,212 |
| 289 | PDE4D | 5 | 58,692,313 | 290 | ELOVL7 | 5 | 60,152,959 |
| 291 | ELOVL7 | 5 | 60,201,944 | 292 | TNPO1 | 5 | 72,123,236 |
| 293 | TNPO1 | 5 | 72,174,208 | 294 | TNPO1 | 5 | 72,182,236 |
| 295 | TNPO1 | 5 | 72,217,160 | 296 | TNPO1 | 5 | 72,292,936 |
| 297 | FCHO2 | 5 | 72,374,865 | 298 | FCHO2 | 5 | 72,384,028 |
| 299 | FCHO2 | 5 | 72,413,771 | 300 | CMYA5 | 5 | 79,058,467 |
| 301 | CMYA5 | 5 | 79,070,418 | 302 | CMYA5 | 5 | 79,074,951 |
| 303 | THBS4 | 5 | 79,376,918 | 304 | THBS4 | 5 | 79,415,294 |
| 305 | VCAN | 5 | 82,914,275 | 306 | MEF2C | 5 | 88,138,619 |
| 307 | GPR98 | 5 | 89,979,411 | 308 | GPR98 | 5 | 89,986,028 |
| 309 | GPR98 | 5 | 90,071,845 | 310 | GPR98 | 5 | 90,428,377 |
| 311 | FBXL17 | 5 | 107,323,636 | 312 | FBXD7 | 5 | 107,224,897 |
| 313 | FBXL17 | 5 | 107,377,610 | 314 | FBXL17 | 5 | 107,380,193 |
| 315 | FBXL17 | 5 | 107,381,014 | 316 | FBXL17 | 5 | 107,400,737 |
| 317 | FBXL17 | 5 | 107,420,461 | 318 | FBXL17 | 5 | 107,556,166 |
| 319 | FBXL17 | 5 | 107,612,678 | 320 | PJA2 | 5 | 108,742,197 |
| 321 | PJA2 | 5 | 108,776,974 | 322 | SNX2 | 5 | 132,116,630 |
| 323 | SNX2 | 5 | 122,137,822 | 324 | SNX2 | 5 | 122,173,535 |
| 325 | SNX2 | 5 | 122,174,171 | 326 | SNX2 | 5 | 122,194,063 |
| 327 | SNX2 | 5 | 122,201,930 | 328 | SNX24 | 5 | 122.232,671 |
| 329 | SNX24 | 5 | 122,255,495 | 330 | SNX24 | 5 | 122,273,273 |
| 331 | SNX24 | 5 | 122,283,900 | 332 | SNX24 | 5 | 122,309,550 |
| 333 | SNX24 | 5 | 122,311,168 | 334 | SNX24 | 5 | 122,383,127 |
| 335 | ADAMTS19 | 5 | 129,039,887 | 336 | TRPC7 | 5 | 135,708,380 |
| 337 | TRPC7 | 5 | 135,713,994 | 338 | CTNNA1 | 5 | 138,167,176 |
| 339 | CTNNA1 | 5 | 138,264,654 | 340 | CTNNA1 | 5 | 138,300,046 |
| 341 | ODZ2 | 5 | 167,527,141 | 342 | ODZ2 | 5 | 167,606,948 |
| 343 | ODZ2 | 5 | 167,621,874 | 344 | ODZ2 | 5 | 167,634,311 |
| 345 | SERPINB6 | 6 | 2,876,878 | 346 | SERPINB6 | 6 | 2,926,223 |
| 347 | SLC22A23 | 6 | 3,395,265 | 348 | JARID2 | 6 | 15,537,710 |
| 349 | JARID2 | 6 | 15,545,369 | 350 | JARID2 | 6 | 15,624,264 |
| 351 | ATXN1 | 6 | 16,755,137 | 352 | ATXN1 | 6 | 16,755,970 |
| 353 | ATXN1 | 6 | 16,762,002 | 354 | RNF144B | 6 | 18,526,687 |
| 355 | RNF144B | 6 | 18,530,528 | 356 | RNF144B | 6 | 18,553,561 |
| 357 | RNF144B | 6 | 18,553,773 | 358 | RNF144B | 6 | 18,561,748 |
| 359 | RNF144B | 6 | 18,566,740 | 360 | RNF144B | 6 | 18,575,501 |
| 361 | SLC17A4 | 6 | 25,876,893 | 362 | SLC17A4 | 6 | 25,892,460 |
| 363 | SLC17A1 | 6 | 25,917,888 | 364 | SLC17A1 | 6 | 25,921,129 |
| 365 | SLC17A1 | 6 | 25,937,638 | 366 | SLC17A1 | 6 | 25,939,191 |
| 367 | SLC17A1 | 6 | 25,950,182 | 368 | SLC17A3 | 6 | 25,978,521 |
| 369 | SLC17A2 | 6 | 26,067,106 | 370 | BTN3A1 | 6 | 26,504,182 |
| 371 | BTN3A1 | 6 | 26,513,969 | 372 | BTN3A1 | 6 | 26,524,633 |
| 373 | BTN2A3 | 6 | 26,532,708 | 374 | BTN2A3 | 6 | 26,534,466 |
| 375 | BTN3A3 | 6 | 26,553,839 | 376 | BTNL2 | 6 | 32,463,841 |
| 377 | BTNL2 | 6 | 32,482,600 | 378 | LRFN2 | 6 | 40,528,210 |
| 379 | ELOVL5 | 6 | 53,213,187 | 380 | ELOVL5 | 6 | 53,221,300 |
| 381 | ELOVL5 | 6 | 53,231,077 | 382 | ELOVL5 | 6 | 53,238,040 |
| 383 | ELOVL5 | 6 | 53,238,244 | 384 | ELOVL5 | 6 | 53,242,957 |
| 385 | ELOVL5 | 6 | 53,246,219 | 386 | ELOVL5 | 6 | 53,254,362 |
| 387 | ELOVL5 | 6 | 53,257,221 | 388 | ELOVL5 | 6 | 53,257,463 |
| 389 | ELOVL5 | 6 | 53,261,913 | 390 | ELOVL5 | 6 | 53,262,169 |
| 391 | ELOVL5 | 6 | 53,267,506 | 392 | ELOVL5 | 6 | 53,271,049 |
| 393 | ELOVL5 | 6 | 53,271,883 | 394 | ELOVL5 | 6 | 53,280,718 |
| 395 | ELOVL5 | 6 | 53,282,407 | 396 | ELOVL5 | 6 | 53,284,867 |
| 397 | ELOVL5 | 6 | 53,292,467 | 398 | ELOVL5 | 6 | 53,294,567 |
| 399 | ELOVL5 | 6 | 53,296,096 | 400 | ELOVL5 | 6 | 53,298,093 |
| 401 | ELOVL5 | 6 | 53,298,209 | 402 | ELOVL5 | 6 | 53,314,948 |
| 403 | ELOVL5 | 6 | 53,318,131 | 404 | ELOVL5 | 6 | 53,341,053 |
| 405 | ELOVL5 | 6 | 53,350,763 | 406 | ELOVL5 | 6 | 53,362,070 |
| 407 | ELOVL5 | 6 | 53,372,444 | 408 | RIMS1 | 6 | 73,967,123 |
| 409 | KLHL32 | 6 | 97,480,306 | 410 | KLHL32 | 6 | 97,589,639 |
| 411 | KLHL32 | 6 | 97,595,870 | 412 | KLHL32 | 6 | 97,601,993 |
| 413 | KLHL32 | 6 | 97,619,298 | 414 | KLHL32 | 6 | 97,673,659 |
| 415 | SLC22A16 | 6 | 110,900,563 | 416 | SLC22A16 | 6 | 110,900,785 |
| 417 | SLC22A16 | 6 | 110,903,098 | 418 | HS3ST5 | 6 | 114,502,447 |
| 419 | TRDN | 6 | 123,982,387 | 420 | TRDN | 6 | 123,991,308 |
| 421 | TRDN | 6 | 124,017,012 | 422 | NKAIN2 | 6 | 124,462,813 |
| 423 | EYA4 | 6 | 133,642,955 | 424 | EYA4 | 6 | 133,643,088 |
| 425 | EYA4 | 6 | 133,651,991 | 426 | EYA4 | 6 | 133,861,896 |
| 427 | PDE7B | 6 | 136,206,096 | 428 | PDE7B | 6 | 136,318,421 |
| 429 | PDE7B | 6 | 136,346,863 | 430 | PLAGL1 | 6 | 144,354,821 |
| 431 | PLAGL1 | 6 | 144,355,380 | 432 | UTRN | 6 | 144,848,384 |
| 433 | SYNE1 | 6 | 152,510,881 | 434 | SYNE1 | 6 | 153,511,829 |
| 435 | SYNE1 | 6 | 152,553,368 | 436 | TFB1M | 6 | 155,655,424 |
| 437 | TFB1M | 6 | 155,663,514 | 438 | PARK2 | 6 | 161,941,630 |
| 439 | PARK2 | 6 | 161,952,077 | 440 | PARK2 | 6 | 162,026,087 |
| 441 | PDE10A | 6 | 165,674,966 | 442 | PDE10A | 6 | 165,679,905 |
| 443 | SDK1 | 7 | 4,152,228 | 444 | SDK1 | 7 | 4,153,102 |
| 445 | SDK1 | 7 | 4,158,337 | 446 | SDK1 | 7 | 4,162,572 |
| 447 | NXPH1 | 7 | 8,590,239 | 448 | NXPH1 | 7 | 8,633,152 |
| 449 | NXPH1 | 7 | 8,633,615 | 450 | SCIN | 7 | 12,627,051 |
| 451 | SCIN | 7 | 12,634,312 | 452 | DGKB | 7 | 14,357,487 |
| 453 | DGKB | 7 | 14,357,894 | 454 | DGKB | 7 | 14,471,463 |
| 455 | DGKB | 7 | 14,472,219 | 456 | DGKB | 7 | 14,529,482 |
| 457 | DGKB | 7 | 14,539,481 | 458 | FERD3L | 7 | 19,167,340 |
| 459 | STK31 | 7 | 23,760,554 | 460 | SKAP2 | 7 | 26,770,543 |
| 461 | SKAP2 | 7 | 36,795,178 | 462 | SKAP2 | 7 | 26,814,684 |
| 463 | SKAP2 | 7 | 26,827,213 | 464 | SKAP2 | 7 | 26,830,698 |
| 465 | SKAP2 | 7 | 26,858,965 | 466 | SKAP2 | 7 | 26,880,921 |
| 467 | CREB5 | 7 | 28,811,671 | 468 | CREB5 | 7 | 28,906,935 |
| 469 | CREB5 | 7 | 28,953,482 | 470 | CHN2 | 7 | 29,315,509 |
| 471 | PDE1C | 7 | 32,037,670 | 472 | PDE1C | 7 | 32,064,508 |
| 473 | PDE1C | 7 | 32,141,959 | 474 | BMPER | 7 | 34,112,924 |
| 475 | BMPER | 7 | 34,176,289 | 476 | BMPER | 7 | 34,184,882 |
| 477 | EEPD1 | 7 | 36,280,985 | 478 | EEPD1 | 7 | 36,391,476 |
| 479 | VPS41 | 7 | 38,798,875 | 480 | VPS41 | 7 | 38,806,116 |
| 481 | VPS41 | 7 | 38,812,386 | 482 | VPS41 | 7 | 38,859,847 |
| 483 | VPS41 | 7 | 38,908,142 | 484 | VPS41 | 7 | 38,911,555 |
| 485 | VPS41 | 7 | 38,936,691 | 486 | CDC2L5 | 7 | 40,063,896 |
| 487 | ABCA13 | 7 | 48,363,317 | 488 | ABCA13 | 7 | 48,380,185 |
| 489 | ABCA13 | 7 | 48,427,057 | 490 | ABCA13 | 7 | 48,454,811 |
| 491 | ABCA13 | 7 | 48,714,222 | 492 | GRB10 | 7 | 50,823,341 |
| 493 | GRB10 | 7 | 50,848,124 | 494 | WBSCR17 | 7 | 70,313,808 |
| 495 | WBSCR17 | 7 | 70,765,881 | 496 | CALN1 | 7 | 71,180,137 |
| 497 | CALN1 | 7 | 71,235,264 | 498 | CALN1 | 7 | 71,338,388 |
| 499 | CALN1 | 7 | 71,339,231 | 500 | CALN1 | 7 | 71,344,745 |
| 501 | MAGI2 | 7 | 77,856,639 | 502 | MAGI2 | 7 | 77,950,934 |
| 503 | MAGI2 | 7 | 78,091,049 | 504 | MAGI2 | 7 | 78,305,682 |
| 505 | MAGI2 | 7 | 78,554,879 | 506 | MAGI2 | 7 | 78,581,862 |
| 507 | MAGI2 | 7 | 78,589,166 | 508 | MAGI2 | 7 | 78,760,470 |
| 509 | MAGI2 | 7 | 78,817,962 | 510 | SEMA3E | 7 | 83,011,138 |
| 511 | ABCB4 | 7 | 86,897,339 | 512 | GTPBP10 | 7 | 89,357,529 |
| 513 | PPP1R9A | 7 | 94,401,225 | 514 | PPP1R9A | 7 | 94,411,224 |
| 515 | PPP1R9A | 7 | 94,431,076 | 516 | PPP1R9A | 7 | 94,479,997 |
| 517 | PPP1R9A | 7 | 94,534,075 | 518 | PPP1R9A | 7 | 94,589,831 |
| 519 | PPP1R9A | 7 | 94,632,721 | 520 | PPP1R9A | 7 | 94,677,380 |
| 521 | PPP1R9A | 7 | 94,681,040 | 522 | PPP1R9A | 7 | 94,717,161 |
| 523 | PPP1R9A | 7 | 94,733,892 | 524 | PPP1R9A | 7 | 94,747,598 |
| 525 | PPP1R9A | 7 | 94,748,184 | 526 | DYNCIII | 7 | 95,556,260 |
| 527 | ZNF3 | 7 | 99,480,681 | 528 | ZNF3 | 7 | 99,516,558 |
| 529 | NRCAM | 7 | 107,585,096 | 530 | NRCAM | 7 | 107,598,816 |
| 531 | NRCAM | 7 | 107,608,956 | 532 | NRCAM | 7 | 107,618,181 |
| 533 | NRCAM | 7 | 107,621,970 | 534 | NRCAM | 7 | 107,626,668 |
| 535 | NRCAM | 7 | 107,627,184 | 536 | NRCAM | 7 | 107,628,207 |
| 537 | NRCAM | 7 | 107,738,458 | 538 | NRCAM | 7 | 107,862,002 |
| 539 | KCND2 | 7 | 119,925,320 | 540 | KCND2 | 7 | 119,999,690 |
| 541 | KCND2 | 7 | 120,001,113 | 542 | KCND2 | 7 | 120,018,452 |
| 543 | CADPS2 | 7 | 122,066,038 | 544 | CADPS2 | 7 | 122,074,175 |
| 545 | CADPS2 | 7 | 122,146,928 | 546 | SLC13A1 | 7 | 122,533,104 |
| 547 | SLC13A1 | 7 | 122,560,268 | 548 | SLC13A1 | 7 | 122,596,470 |
| 549 | SLC13A1 | 7 | 122,633,763 | 550 | SLC13A1 | 7 | 122,659,570 |
| 551 | GRM8 | 7 | 125,938,463 | 552 | GRM8 | 7 | 125,940,799 |
| 553 | GRM8 | 7 | 125,974,975 | 554 | GRM8 | 7 | 126,100,080 |
| 555 | GRM8 | 7 | 126,134,129 | 556 | GRM8 | 7 | 126,159,625 |
| 557 | GRM8 | 7 | 126,165,491 | 558 | GRM8 | 7 | 126,174,101 |
| 559 | GRM8 | 7 | 126,208,986 | 560 | GRM8 | 7 | 126,225,935 |
| 561 | GRM8 | 7 | 126,234,800 | 562 | GRM8 | 7 | 126,436,133 |
| 563 | GRM8 | 7 | 126,483,848 | 564 | GRM8 | 7 | 126,674,369 |
| 565 | GRM8 | 7 | 126,692,493 | 566 | EXOC4 | 7 | 132,728,495 |
| 567 | EXOC4 | 7 | 132,754,657 | 568 | EXOC4 | 7 | 132,756,739 |
| 569 | EXOC4 | 7 | 132,889,141 | 570 | EXOC4 | 7 | 133,264,961 |
| 571 | DGKI | 7 | 136,723,471 | 572 | DGKI | 7 | 136,749,535 |
| 573 | DGKI | 7 | 136,769,973 | 574 | DGKI | 7 | 136,775,455 |
| 575 | DGKI | 7 | 136,780,737 | 576 | DGKI | 7 | 136,838,221 |
| 577 | DGKI | 7 | 136,925,970 | 578 | TBXAS1 | 7 | 139,184,991 |
| 579 | CNTNAP2 | 7 | 145,414,794 | 580 | CNTNAP2 | 7 | 145,900,444 |
| 581 | CNTNAP2 | 7 | 146,180,657 | 582 | CNTNAP2 | 7 | 146,405,829 |
| 583 | CNTNAP2 | 7 | 146,414,565 | 584 | CNTNAP2 | 7 | 146,788,354 |
| 585 | CNTNAP2 | 7 | 146,792,712 | 586 | CNTNAP2 | 7 | 146,795,212 |
| 587 | CNTNAP2 | 7 | 147,069,599 | 588 | CNTNAP2 | 7 | 147,073,223 |
| 589 | CNTNAP2 | 7 | 147,710,739 | 590 | PTPRN2 | 7 | 157,017,352 |
| 591 | PTPRN2 | 7 | 157,020,289 | 592 | PTPRN2 | 7 | 157,499,807 |
| 593 | PTPRN2 | 7 | 157,634,704 | 594 | PTPRN2 | 7 | 157,676,114 |
| 595 | PTPRN2 | 7 | 157,714,280 | 596 | PTPRN2 | 7 | 158,015,477 |
| 597 | CSMD1 | 8 | 2,793,553 | 598 | CSMD1 | 8 | 2,864,416 |
| 599 | CSMD1 | 8 | 2,943,570 | 600 | CSMD1 | 8 | 2,954,259 |
| 601 | CSMD1 | 8 | 3,328,881 | 602 | CSMD1 | 8 | 3,230,805 |
| 603 | CSMD1 | 8 | 3,232,022 | 604 | CSMD1 | 8 | 3,232,222 |
| 605 | CSMD1 | 8 | 3,245,103 | 606 | CSMD1 | 8 | 3,250,935 |
| 607 | CSMD1 | 8 | 3,338,555 | 608 | MCPH1 | 8 | 6,414,463 |
| 609 | MCPH1 | 8 | 6,488,786 | 610 | SGCZ | 8 | 14,619,388 |
| 611 | SLC7A2 | 8 | 17,436,984 | 612 | SLC7A2 | 8 | 17,444,141 |
| 613 | PSD3 | 8 | 18,428,754 | 614 | PSD3 | 8 | 18,431,654 |
| 615 | PSD3 | 8 | 18,440,935 | 616 | PSD3 | 8 | 18,457,737 |
| 617 | PSD3 | 8 | 18,465,335 | 618 | PSD3 | 8 | 18,467,749 |
| 619 | PSD3 | 8 | 18,469,732 | 620 | PSD3 | 8 | 18,514,979 |
| 621 | PSD3 | 8 | 18,558,380 | 622 | PSD3 | 8 | 18,736,236 |
| 623 | PSD3 | 8 | 18,749,712 | 624 | PSD3 | 8 | 18,750,003 |
| 625 | PSD3 | 8 | 18,819,182 | 626 | PSD3 | 8 | 18,953,685 |
| 627 | ENTPD4 | 8 | 23,357,109 | 628 | UNC5D | 8 | 35,516,694 |
| 629 | SNTG1 | 8 | 51,805,005 | 630 | SNTG1 | 8 | 51,816,608 |
| 631 | LYN | 8 | 57,039,314 | 632 | LYN | 8 | 57,053,118 |
| 633 | LYN | 8 | 57,054,784 | 634 | LYN | 8 | 57,108,529 |
| 635 | LYN | 8 | 57,110,114 | 636 | PLAG1 | 8 | 57,246,679 |
| 637 | PLAG1 | 8 | 57,263,345 | 638 | TOX | 8 | 59,966,273 |
| 639 | TOX | 8 | 59,972,497 | 640 | TOX | 8 | 59,980,027 |
| 641 | TOX | 8 | 60,198,077 | 642 | NKAIN3 | 8 | 63,649,765 |
| 643 | NKAIN3 | 8 | 63,900,353 | 644 | NKAIN3 | 8 | 63,935,531 |
| 645 | NKAIN3 | 8 | 63,936,341 | 646 | DEPDC2 | 8 | 69,208,963 |
| 647 | DEPDC2 | 8 | 69,209,296 | 648 | CRISPLD1 | 8 | 76,073,693 |
| 649 | CRISPLD1 | 8 | 76,086,655 | 650 | CRISPLD1 | 8 | 76,127,254 |
| 651 | CRISPLD1 | 8 | 76,156,200 | 652 | CRISPLD1 | 8 | 76,158,048 |
| 653 | MMP16 | 8 | 89,276,696 | 654 | BAALC | 8 | 104,271,601 |
| 655 | ZFPM2 | 8 | 106,614,970 | 656 | ZFPM2 | 8 | 106,687,838 |
| 657 | ZFPM2 | 8 | 106,688,431 | 658 | ZFPM2 | 8 | 106,847,240 |
| 659 | ZFPM2 | 8 | 106,880,874 | 660 | CSMD3 | 8 | 113,337,274 |
| 661 | CSMD3 | 8 | 113,443,580 | 662 | CSMD3 | 8 | 113,452,239 |
| 663 | CSMD3 | 8 | 113,467,267 | 664 | CSMD3 | 8 | 113,469,659 |
| 665 | CSMD3 | 8 | 113,473,493 | 666 | CSMD3 | 8 | 113,510,014 |
| 667 | CSMD3 | 8 | 113,554,821 | 668 | CSMD3 | 8 | 114,069,034 |
| 669 | CSMD3 | 8 | 114,093,751 | 670 | CSMD3 | 8 | 114,098,597 |
| 671 | CSMD3 | 8 | 114,104,594 | 672 | CSMD3 | 8 | 114,105,767 |
| 673 | CSMD3 | 8 | 114,160,908 | 674 | CSMD3 | 8 | 114,208,773 |
| 675 | CSMD3 | 8 | 114,359,208 | 676 | SAMD12 | 8 | 119,514,190 |
| 677 | SAMD12 | 8 | 119,514,541 | 678 | SAMD12 | 8 | 119,518,606 |
| 679 | SAMD12 | 8 | 119,531,636 | 680 | SAMD12 | 8 | 119,587,094 |
| 681 | SAMD12 | 8 | 119,680,657 | 682 | SAMD12 | 8 | 119,719,731 |
| 683 | FER1L6 | 8 | 125,053,616 | 684 | FER1L6 | 8 | 125,122,813 |
| 685 | FER1L6 | 8 | 125,123,546 | 686 | MTSS1 | 8 | 125,634,509 |
| 687 | MTSS1 | 8 | 125,700,325 | 688 | MTSS1 | 8 | 125,708,636 |
| 689 | MTSS1 | 8 | 125,737,780 | 690 | MTSS1 | 8 | 125,802,038 |
| 691 | DDEF1 | 8 | 131,286,874 | 692 | DDEF1 | 8 | 131,298,362 |
| 693 | DDEF1 | 8 | 131,368,653 | 694 | DDEF1 | 8 | 131,406,218 |
| 695 | DDEF1 | 8 | 131,423,273 | 696 | KCNQ3 | 8 | 133,208,937 |
| 697 | KCNQ3 | 8 | 133,211,492 | 698 | KCNQ3 | 8 | 133,212,327 |
| 699 | KCNQ3 | 8 | 133,346,913 | 700 | KCNQ3 | 8 | 133,351,663 |
| 701 | COL22A1 | 8 | 139,744,740 | 702 | COL32A1 | 8 | 139,759,058 |
| 703 | COL22A1 | 8 | 139,771,875 | 704 | COL22A1 | 8 | 139,777,590 |
| 705 | COL22A1 | 8 | 139,833,578 | 706 | ZC3H3 | 8 | 144,691,326 |
| 707 | SMARCA2 | 9 | 2,053,356 | 708 | SMARCA2 | 9 | 2,170,694 |
| 709 | PIP5K1B | 9 | 70,545,037 | 710 | PIP5K1B | 9 | 70,687,329 |
| 711 | TRPM3 | 9 | 72,616,673 | 712 | TMC1 | 9 | 74,485,413 |
| 713 | TMC1 | 9 | 74,492,549 | 714 | TMC1 | 9 | 74,493,473 |
| 715 | TMC1 | 9 | 74,507,694 | 716 | TMC1 | 9 | 74,623,740 |
| 717 | PCSK5 | 9 | 77,897,682 | 718 | GNAQ | 9 | 79,856,574 |
| 719 | GNAQ | 9 | 79,934,142 | 720 | GNAQ | 9 | 79,946,188 |
| 721 | NTRK2 | 9 | 86,577,727 | 722 | DIRAS2 | 9 | 92,422,258 |
| 723 | DIRAS2 | 9 | 92,476,677 | 724 | NFIL3 | 9 | 93,218,808 |
| 725 | FKTN | 9 | 107,389,052 | 726 | FKTN | 9 | 107,409,195 |
| 727 | FKTN | 9 | 107,420,176 | 728 | FKTN | 9 | 107,435,753 |
| 729 | FKTN | 9 | 107,445,165 | 730 | FKTN | 9 | 107,449,221 |
| 731 | FKTN | 9 | 107,449,435 | 732 | PALM2 | 9 | 111,506,598 |
| 733 | PALM2 | 9 | 111,552,543 | 734 | PALM2 | 9 | 111,554,864 |
| 735 | PALM2 | 9 | 111,555,049 | 736 | PALM2 | 9 | 111,556,167 |
| 737 | ZNF618 | 9 | 115,804,104 | 738 | ZNF618 | 9 | 115,816,110 |
| 739 | ZNF618 | 9 | 115,820,286 | 740 | ASTN2 | 9 | 119,198,404 |
| 741 | CDK5RAP2 | 9 | 122,346,776 | 742 | CDK5RAP2 | 9 | 132,377,819 |
| 743 | PTGS1 | 9 | 124,225,832 | 744 | RALGPS1 | 9 | 128,769,224 |
| 745 | RALGPS1 | 9 | 128,809,594 | 746 | RALGPS1 | 9 | 128,850,765 |
| 747 | RALGPS1 | 9 | 128,902,817 | 748 | RALGPS1 | 9 | 128,972,837 |
| 749 | SLC25A25 | 9 | 129,899,153 | 750 | TSC1 | 9 | 134,778,093 |
| 751 | TSC1 | 9 | 134,786,003 | 752 | TSC1 | 9 | 134,812,111 |
| 753 | VAV2 | 9 | 135,708,866 | 754 | VAV2 | 9 | 135,710,406 |
| 755 | VAV2 | 9 | 135,710,620 | 756 | VAV2 | 9 | 135,714,835 |
| 757 | PITRM1 | 10 | 3,179,115 | 758 | PRKCQ | 10 | 7,131,891 |
| 759 | PRKCQ | 10 | 7,136,154 | 760 | PRKCQ | 10 | 7,137,198 |
| 761 | CACNB2 | 10 | 18,861,080 | 762 | ARMC3 | 10 | 23,264,868 |
| 763 | ARMC3 | 10 | 23,270,471 | 764 | ARMC3 | 10 | 23,271,006 |
| 765 | ARMC3 | 10 | 23,343,259 | 766 | ARMC3 | 10 | 23,343,422 |
| 767 | ARMC3 | 10 | 23,372,821 | 768 | ARMC3 | 10 | 23,372,952 |
| 769 | MYO3A | 10 | 26,396,941 | 770 | MYO3A | 10 | 26,412,733 |
| 771 | ANK3 | 10 | 61,569,339 | 772 | JMJD1C | 10 | 64,732,014 |
| 773 | JMJD1C | 10 | 64,736,192 | 774 | JMJD1C | 10 | 64,791,571 |
| 775 | JMJD1C | 10 | 64,873,814 | 776 | CDH23 | 10 | 72,997,429 |
| 777 | KCNMA1 | 10 | 78,258,751 | 778 | KCNMA1 | 10 | 78,320,267 |
| 779 | SORCS3 | 10 | 106,525,169 | 780 | VTHA | 10 | 114,448,959 |
| 781 | VTI1A | 10 | 114,458,127 | 782 | VTI1A | 10 | 114,496,282 |
| 783 | VTI1A | 10 | 114,497,586 | 784 | VTHA | 10 | 114,504,041 |
| 785 | ATRNL1 | 10 | 117,429,854 | 786 | HSPA12A | 10 | 118,432,901 |
| 787 | GRK5 | 10 | 121,055,451 | 788 | ATE1 | 10 | 123,478,029 |
| 789 | ATE1 | 10 | 123,484,634 | 790 | ATE1 | 10 | 123,634,335 |
| 791 | ATE1 | 10 | 123,644,015 | 792 | ATE1 | 10 | 123,648,119 |
| 793 | ATE1 | 10 | 123,648,218 | 794 | ATE1 | 10 | 123,648,608 |
| 795 | ATE1 | 10 | 123,666,737 | 796 | ATE1 | 10 | 123,669,674 |
| 797 | ATE1 | 10 | 123,679,356 | 798 | STIM1 | 11 | 4,033,624 |
| 799 | STIM1 | 11 | 4,047,324 | 800 | TRIM21 | 11 | 4,371,449 |
| 801 | GALNTL4 | 11 | 11,222,747 | 802 | GALNTL4 | 11 | 11,228,193 |
| 803 | GALNTL4 | 11 | 11,246,740 | 804 | SPON1 | 11 | 13,883,488 |
| 805 | SPON1 | 11 | 14,244,704 | 806 | USH1C | 11 | 17,475,101 |
| 807 | USH1C | 11 | 17,487,060 | 808 | USH1C | 11 | 17,489,173 |
| 809 | USH1C | 11 | 17,489,973 | 810 | USH1C | 11 | 17,490,211 |
| 811 | USH1C | 11 | 17,491,437 | 812 | USH1C | 11 | 17,519,298 |
| 813 | USH1C | 11 | 17,520,065 | 814 | USH1C | 11 | 17,523,687 |
| 815 | IGSF22 | 11 | 18,689,422 | 816 | IGSF22 | 11 | 18,692,523 |
| 817 | IGSF22 | 11 | 18,694,857 | 818 | C11ORF49 | 11 | 46,955,088 |
| 819 | C11ORF49 | 11 | 47,130,872 | 820 | C11ORF49 | 11 | 47,132,581 |
| 821 | DLG2 | 11 | 82,843,293 | 822 | DLG2 | 11 | 82,856,041 |
| 823 | DLG2 | 11 | 83,910,180 | 824 | DLG2 | 11 | 83,186,640 |
| 825 | DLG2 | 11 | 83,187,421 | 826 | DLG2 | 11 | 83,203,872 |
| 827 | DLG2 | 11 | 83,366,230 | 828 | DLG2 | 11 | 84,153,124 |
| 829 | DLG2 | 11 | 84,175,263 | 830 | DLG2 | 11 | 84,198,828 |
| 831 | DLG2 | 11 | 84,234,326 | 832 | ELMOD1 | 11 | 107,051,899 |
| 833 | OPCML | 11 | 131,718,194 | 834 | TSPAN9 | 12 | 3,058,775 |
| 835 | TSPAN9 | 12 | 3,065,463 | 836 | TSPAN9 | 12 | 3,173,346 |
| 837 | TSPAN9 | 12 | 3,331,863 | 838 | STYK1 | 12 | 10,666,462 |
| 839 | STYK1 | 12 | 10,697,767 | 840 | STYK1 | 12 | 10,708,924 |
| 841 | STYK1 | 12 | 10,711,189 | 842 | LOC729025 | 12 | 16,250,464 |
| 843 | LRP1 | 12 | 55,847,718 | 844 | LRP1 | 12 | 55,878,824 |
| 845 | CNOT2 | 12 | 68,831,198 | 846 | CNOT2 | 12 | 69,026,461 |
| 847 | KCNC2 | 12 | 73,584,602 | 848 | NAV3 | 12 | 76,913,545 |
| 849 | NAV3 | 12 | 76,922,312 | 850 | GAS2L3 | 12 | 99,519,980 |
| 851 | MTIF3 | 13 | 26,895,700 | 852 | MTIF3 | 13 | 26,895,999 |
| 853 | SLC46A3 | 13 | 28,185,843 | 854 | SLC46A3 | 13 | 28,217,711 |
| 855 | SLC46A3 | 13 | 28,267,317 | 856 | UBL3 | 13 | 29,194,621 |
| 857 | N4BP2L2 | 13 | 32,021,516 | 858 | NBEA | 13 | 35,086,809 |
| 859 | NBEA | 13 | 35,087,894 | 860 | NBEA | 13 | 35,130,015 |
| 861 | SLAIN1 | 13 | 77,151,328 | 862 | GPC5 | 13 | 91,268,794 |
| 863 | GPC5 | 13 | 91,346,842 | 864 | GPC5 | 13 | 91,372,652 |
| 865 | GPC6 | 13 | 93,138,384 | 866 | GPC6 | 13 | 93,183,430 |
| 867 | NALCN | 13 | 100,517,778 | 868 | NALCN | 13 | 100,522,814 |
| 869 | ITGBL1 | 13 | 101,013,965 | 870 | WDR23 | 14 | 23,657,479 |
| 871 | WDR23 | 14 | 23,671,158 | 872 | NPAS3 | 14 | 32,590,670 |
| 873 | NPAS3 | 14 | 32,658,581 | 874 | GNG2 | 14 | 51,414,961 |
| 875 | GNG2 | 14 | 51,422,662 | 876 | SAMD4A | 14 | 54,319,095 |
| 877 | SAMD4A | 14 | 54,335,279 | 878 | SAMD4A | 14 | 54,335,321 |
| 879 | PPP2R5E | 14 | 63,040,607 | 880 | PPP2R5E | 14 | 63,099,534 |
| 881 | RGS6 | 14 | 71,538,177 | 882 | RGS6 | 14 | 71,539,344 |
| 883 | RGS6 | 14 | 71,540,089 | 884 | RGS6 | 14 | 71,723,690 |
| 885 | RGS6 | 14 | 71,793,717 | 886 | KCNK10 | 14 | 87,792,199 |
| 887 | KCNK13 | 14 | 89,783,756 | 888 | KCNK13 | 14 | 89,785,725 |
| 889 | PSMC1 | 14 | 89,794,414 | 890 | PSMC1 | 14 | 89,794,847 |
| 891 | PSMC1 | 14 | 89,806,078 | 892 | RPS6KA5 | 14 | 90,412,434 |
| 893 | RPS6KA5 | 14 | 90,501,524 | 894 | RPS6KA5 | 14 | 90,527,909 |
| 895 | RPS6KA5 | 14 | 90,557,351 | 896 | RPS6KA5 | 14 | 90,572,871 |
| 897 | RPS6KA5 | 14 | 90,600,927 | 898 | RPS6KA5 | 14 | 90,616,889 |
| 899 | CCDC88C | 14 | 90,821,027 | 900 | CCDC88C | 14 | 90,910,783 |
| 901 | CCDC88C | 14 | 90,916,433 | 902 | CCDC88C | 14 | 90,918,598 |
| 903 | CCDC88C | 14 | 90,920,049 | 904 | BCL11B | 14 | 98,746,376 |
| 905 | BCL11B | 14 | 98,757,526 | 906 | BCL11B | 14 | 98,765,857 |
| 907 | ATP10A | 15 | 23,493,398 | 908 | ATP10A | 15 | 23,513,966 |
| 909 | C15ORF41 | 15 | 34,833,829 | 910 | GLDN | 15 | 49,457,768 |
| 911 | GLDN | 15 | 49,457,862 | 912 | GLDN | 15 | 49,459,928 |
| 913 | GLDN | 15 | 49,460,922 | 914 | CLK3 | 15 | 72,707,585 |
| 915 | CLK3 | 15 | 72,727,323 | 916 | TBC1D2B | 15 | 76,083,890 |
| 917 | TBC1D2B | 15 | 76,098,398 | 918 | PCSK6 | 15 | 99,842,741 |
| 919 | PCSK6 | 15 | 99,858,640 | 920 | A2BP1 | 16 | 7,444,597 |
| 921 | A2BP1 | 16 | 7,547,113 | 922 | A2BP1 | 16 | 7,568,164 |
| 923 | TMC5 | 16 | 19,365,820 | 924 | TMC5 | 16 | 19,403,812 |
| 925 | TMC5 | 16 | 19,413,252 | 926 | TMC5 | 16 | 19,432,372 |
| 927 | GDE1 | 16 | 19,435,278 | 928 | GDE1 | 16 | 19,455,004 |
| 929 | HYDIN | 16 | 69,765,380 | 930 | HYDIN | 16 | 69,818,152 |
| 931 | HYDIN | 16 | 69,856,735 | 932 | WWOX | 16 | 76,673,124 |
| 933 | WWOX | 16 | 76,739,487 | 934 | WWOX | 16 | 76,793,572 |
| 935 | WWOX | 16 | 77,041,675 | 936 | MPHOSPH6 | 16 | 80,728,868 |
| 937 | MPHOSPH6 | 16 | 80,759,877 | 938 | MPHOSPH6 | 16 | 80,765,783 |
| 939 | MPHOSPH6 | 16 | 80,777,144 | 940 | MPHOSPH6 | 16 | 80,817,367 |
| 941 | MPHOSPH6 | 16 | 80,820,057 | 942 | MPHOSPH6 | 16 | 80,831,708 |
| 943 | MPHOSPH6 | 16 | 80,833,378 | 944 | CDH13 | 16 | 81,390,450 |
| 945 | CDH13 | 16 | 81,430,291 | 946 | CDH13 | 16 | 82,324,245 |
| 947 | USP10 | 16 | 83,325,736 | 948 | DNAH9 | 17 | 11,643,618 |
| 949 | DNAH9 | 17 | 11,671,648 | 950 | RAB11FIP4 | 17 | 26,746,745 |
| 951 | CA10 | 17 | 47,069,220 | 952 | CA10 | 17 | 47,599,744 |
| 953 | HRNBP3 | 17 | 74,845,144 | 954 | ZFP161 | 18 | 5,280,198 |
| 955 | ZFP161 | 18 | 5,283,943 | 956 | ZFP161 | 18 | 5,284,323 |
| 957 | ZFP161 | 18 | 5,398,814 | 958 | ZFP161 | 18 | 5,406,160 |
| 959 | PTPRM | 18 | 7,585,927 | 960 | PTPRM | 18 | 7,892,147 |
| 961 | PTPRM | 18 | 8,041,285 | 962 | KIAA0802 | 18 | 8,840,062 |
| 963 | KIAA0802 | 18 | 8,844,132 | 964 | OSBPL1A | 18 | 20,105,278 |
| 965 | CHST9 | 18 | 22,787,798 | 966 | CHST9 | 18 | 22,874,298 |
| 967 | CHST9 | 18 | 22,874,628 | 968 | CHST9 | 18 | 23,010,213 |
| 969 | CHST9 | 18 | 23,012,354 | 970 | NEDD4L | 18 | 54,173,333 |
| 971 | NEDD4L | 18 | 54,202,587 | 972 | NEDD4L | 18 | 54,258,677 |
| 973 | NEDD4L | 18 | 54,259,246 | 974 | CCBE1 | 18 | 55,220,303 |
| 975 | CCBE1 | 18 | 55,241,702 | 976 | CCBE1 | 18 | 55,483,138 |
| 977 | CDH7 | 18 | 61,594,009 | 978 | CDH7 | 18 | 61,697,973 |
| 979 | CDH7 | 18 | 61,739,825 | 980 | CDH7 | 18 | 61,759,477 |
| 981 | TXNDC10 | 18 | 64,536,945 | 982 | ATRN | 20 | 3,400,447 |
| 983 | FERMT1 | 20 | 6,037,513 | 984 | FERMT1 | 20 | 6,041,177 |
| 985 | PLCB1 | 20 | 8,000,158 | 986 | PLCB4 | 20 | 9,433,807 |
| 987 | MACROD2 | 20 | 15,551,153 | 988 | MACROD2 | 20 | 15,559,316 |
| 989 | MACROD2 | 20 | 15,586,912 | 990 | MACROD2 | 20 | 16,020,951 |
| 991 | KIF16B | 20 | 16,445,469 | 992 | KIF16B | 20 | 16,454,369 |
| 993 | MAPRE1 | 20 | 30,888,730 | 994 | MAPRE1 | 20 | 30,906,484 |
| 995 | PTPRT | 20 | 40,087,340 | 996 | ZSWIM3 | 20 | 43,921,606 |
| 997 | ZSWIM3 | 20 | 43,930,226 | 998 | ZSWIM3 | 20 | 43,939,825 |
| 999 | SNAI1 | 20 | 48,025,165 | 1000 | SNAI1 | 20 | 48,036,030 |
| 1001 | SNAI1 | 20 | 48,037,294 | 1002 | SINAI1 | 20 | 48,051,852 |
| 1003 | BMP7 | 20 | 55,238,534 | 1004 | CDH4 | 20 | 59,359,672 |
| 1005 | C21ORF37 | 21 | 17,711,037 | 1006 | C31ORP37 | 21 | 17,738,145 |
| 1007 | NCAM2 | 21 | 21,399,144 | 1008 | NCAM2 | 31 | 21,451,606 |
| 1009 | NCAM2 | 21 | 21,658,511 | 1010 | NCAM2 | 21 | 21,664,277 |
| 1011 | PDE9A | 21 | 43,061,181 | 1012 | ARVCF | 22 | 18,342,203 |
| 1013 | ARVCF | 22 | 18,371,950 | 1014 | SGSM1 | 22 | 23,675,537 |
| 1015 | ASPHD2 | 22 | 25,130,199 | 1016 | ASPHD2 | 22 | 25,131,387 |
| 1017 | ASPHD2 | 22 | 25,166,780 | 1018 | HPS4 | 22 | 25,200,502 |
| 1019 | ARFGAP3 | 22 | 41,536,894 | 1020 | ARFGAP3 | 22 | 41,576,090 |
| 1021 | TTLL1 | 22 | 41,789,790 | 1022 | TTLL1 | 22 | 41,822,906 |
| 1023 | EFCAB6 | 22 | 42,411,013 | 1024 | EFCAB6 | 22 | 42,527,300 |
| 1025 | RIBC2 | 22 | 44,217,310 | | | | |

## Claims

1. A method of determining risk of developing schizophrenia (SZ) in a human subject, the method comprising:
determining the copy number of at least one single nucleotide polymorphism (SNP), wherein the SNP is rs2180782 in BMP7 or a polymorphism that is in linkage disequilibrium (LD) with rs2180782 in BMP7; and
comparing the copy number of the SNP in the subject with a reference copy number, wherein the reference copy number is associated with a known risk of developing SZ; and
wherein the presence of a copy number of the SNP in the subject that is the same as a reference copy number that is associated with SZ indicates the risk that the subject will develop SZ.

2. The method of claim 1, wherein the at least one SNP is at position 31 of Seq ID 1003.

3. The method of claim 1, wherein determining the identity or copy number of an allele comprises obtaining a sample comprising DNA from the subject, and determining identity or copy number of the nucleotide at the polymorphic site.

4. The method of claim 3, wherein determining the identity of the nucleotide comprises contacting the sample with a probe specific for a selected allele of the polymorphism, and detecting the formation of complexes between the probe and the selected allele of the polymorphism, wherein the formation of complexes between the probe and the test marker indicates the presence of the selected allele in the sample.

5. The method of claim 4, wherein determining the identity of an allele comprises determining the identity of the nucleotide at position 31 of SEQ ID 1003.

6. The method of claim 3, wherein determining the copy number of an allele comprises contacting the sample with a probe specific for a selected allele, detecting the formation of complexes between the probe and the selected allele of the polymorphism, and quantifying the complexes, wherein the quantification of the complexes indicates the copy number of the selected allele in the sample.

7. The method of claim 6, wherein the probe comprises a fluorescent label, and quantifying the complexes comprises detecting intensity of emission from the label.

8. The method of claim 1, wherein determining the copy number of an allele comprises detecting the absence or duplication of the allele in the subject.

9. The method of claim 1, wherein the subject is a patient having or suspected of having SZ, or wherein the subject has one or more risk factors associated with SZ.

10. The method of claim 9, wherein the risk factors associated with SZ include one or more of: a relative afflicted with SZ; and a genetically based phenotypic trait associated with risk for a SZ.

11. The method of claim 1, wherein the subject has exhibited or exhibits symptoms of psychosis.

12. The method of claims 1, further comprising selecting or excluding a subject for enrollment in a clinical trial based on the identity of the allele, or stratifying a subject population for analysis of a clinical trial based on the identity of the allele in the subjects, or confirming a diagnosis of a SZ using psychometric instruments.

## Patentansprüche

1. Verfahren zur Bestimmung des Risikos der Entwicklung von Schizophrenie (SZ) bei einem menschlichen Patienten, wobei man bei dem Verfahren:
die Kopienzahl wenigstens eines Einzelnukleotid-Polymorphismus (SNP) bestimmt, wobei es sich bei dem SNP um rs2180782 in BMP7 oder einen Polymorphismus handelt, der im Verknüpfungsungleichgewicht (linkage disequilibrium, LD) mit rs2180782 in BMP7 steht; und
die Kopienzahl des SNP in dem Patienten mit einer Referenzkopienzahl vergleicht, wobei die Referenzkopienzahl mit einem bekannten Risiko der Entwicklung von SZ assoziiert ist; und
wobei das Vorliegen einer Kopienzahl des SNP in dem Patienten, die mit einer mit SZ assoziierten Referenzkopienzahl identisch ist, das Risiko anzeigt, dass sich bei dem Patienten SZ entwickelt.

2. Verfahren nach Anspruch 1, wobei sich der wenigstens eine SNP an Position 31 von Seq ID 1003 befindet.

3. Verfahren nach Anspruch 1, wobei das Bestimmen der Identität oder Kopienzahl eines Allels das Entnehmen einer DNA umfassenden Probe aus dem Patienten und Bestimmen der Identität oder Kopienzahl des Nukleotids an der polymorphen Stelle umfasst.

4. Verfahren nach Anspruch 3, wobei das Bestimmen der Identität des Nukleotids das Inkontaktbringen der Probe mit einer für ein ausgewähltes Allel des Polymorphismus spezifischen Sonde und Nachweisen der Ausbildung von Komplexen zwischen der Sonde und dem ausgewählten Allel des Polymorphismus umfasst, wobei die Ausbildung von Komplexen zwischen der Sonde und dem Testmarker das Vorliegen des ausgewählten Allels in der Probe anzeigt.

5. Verfahren nach Anspruch 4, wobei das Bestimmen der Identität eines Allels das Bestimmen der Identität des Nukleotids an Position 31 von SEQ ID 1003 umfasst.

6. Verfahren nach Anspruch 3, wobei das Bestimmen der Kopienzahl eines Allels das Inkontaktbringen der Probe mit einer für ein ausgewähltes Allel des Polymorphismus spezifischen Sonde, das Nachweisen der Ausbildung von Komplexen zwischen der Sonde und dem ausgewählten Allel des Polymorphismus und das Quantifizieren der Komplexe umfasst, wobei die Quantifizierung der Komplexe die Kopienzahl des ausgewählten Allels in der Probe anzeigt.

7. Verfahren nach Anspruch 6, wobei die Sonde eine Fluoreszenzmarkierung und das Quantifizieren der Komplexe das Nachweisen der Intensität der Emission von der Markierung umfasst.

8. Verfahren nach Anspruch 1, wobei das Bestimmen der Kopienzahl eines Allels das Nachweisen des Fehlens oder der Vervielfältigung des Allels bei dem Patienten umfasst.

9. Verfahren nach Anspruch 1, wobei es sich bei dem Patienten um einen Patienten mit SZ oder einen Patienten, bei dem SZ vermutet wird, handelt oder wobei der Patient einen oder mehrere mit SZ assoziierte Risikofaktoren aufweist.

10. Verfahren nach Anspruch 9, wobei zu den mit SZ assoziierten Risikofaktoren einer oder mehrere der Folgenden zählen: ein an SZ leidender Verwandter;
und ein mit dem Risiko einer SZ assoziiertes phänotypisches Merkmal auf genetischer Basis.

11. Verfahren nach Anspruch 1, wobei der Patient Psychosesymptome zeigte oder zeigt.

12. Verfahren nach Anspruch 1, bei dem man ferner einen Patient für die Teilnahme an einer klinischen Versuchsreihe bezogen auf die Identität des Allels auswählt oder davon ausschließt oder eine Patientenpopulation für die Analyse einer klinischen Versuchsreihe bezogen auf die Identität des Allels in den Patienten stratifiziert oder eine Diagnose einer SZ unter Verwendung psychometrischer Geräte bestätigt.

## Revendications

1. Procédé de détermination du risque de développer une schizophrénie (SZ) chez un sujet humain, le procédé comprenant :
la détermination du nombre de copies d'au moins un polymorphisme nucléotidique simple (Single Nucléotide Polymorphism - SNP), le SNP étant le rs2180782 dans le gène de la BMP7 ou d'un polymorphisme qui est dans le déséquilibre de liaison (LD) avec le rs2180782 dans le gène de la BMP7 ; et
la comparaison du nombre de copies du SNP chez le sujet à un nombre de référence de copies, le nombre de référence de copies étant associé à un risque connu de développer la SZ ; et
dans lequel la présence d'un nombre de copies du SNP chez le sujet identique au nombre de référence de copies associé à la SZ indique le risque que le sujet développe une SZ.

2. Procédé selon la revendication 1, ledit au moins un SNP étant en position 31 de la séquence Seq ID 1003.

3. Procédé selon la revendication 1, la détermination de l'identité ou du nombre de copies d'un allèle comprenant l'obtention d'un échantillon comprenant de l'ADN du sujet, et la détermination de l'identité ou du nombre de copies du nucléotide au site polymorphique.

4. Procédé selon la revendication 3, la détermination de l'identité du nucléotide comprenant la mise en contact de l'échantillon avec une sonde spécifique à un allèle sélectionné du polymorphisme et la détection de la formation de complexes entre la sonde et l'allèle sélectionné du polymorphisme, la formation de complexes entre la sonde et le marqueur test indiquant la présence de l'allèle sélectionné dans l'échantillon.

5. Procédé selon la revendication 4, la détermination de l'identité d'un allèle comprenant la détermination de l'identité du nucléotide en position 31 de la séquence SEQ ID 1003.

6. Procédé selon la revendication 3, la détermination du nombre de copies d'un allèle comprenant la mise en contact de l'échantillon avec une sonde spécifique à un allèle sélectionné, la détection de la formation de complexes entre la sonde et l'allèle sélectionné du polymorphisme et la quantification des complexes, la quantification des complexes indiquant le nombre de copies de l'allèle sélectionné dans l'échantillon.

7. Procédé selon la revendication 6, la sonde comprenant une étiquette fluorescente et la quantification des complexes comprenant la détection de l'intensité d'émission provenant de l'étiquette.

8. Procédé selon la revendication 1, la détermination du nombre de copies d'un allèle comprenant la détection de l'absence ou de la duplication de l'allèle chez le sujet.

9. Procédé selon la revendication 1, le sujet étant un patient atteint ou suspecté d'être atteint de SZ, ou le sujet présentant un ou plusieurs facteurs de risque associés à la SZ.

10. Procédé selon la revendication 9, les facteurs de risque associés à la SZ incluant un ou plusieurs parmi : un parent souffrant de SZ ; et une caractéristique phénotypique d'origine génétique associée à un risque de SZ.

11. Procédé selon la revendication 1, le sujet ayant montré ou montrant des symptômes de psychose.

12. Procédé selon la revendication 1, comprenant en outre la sélection ou l'exclusion d'un sujet pour l'enrôlement dans un essai clinique basées sur l'identité de l'allèle, ou la stratification d'une population de sujets pour l'analyse d'un essai clinique basée sur l'identité de l'allèle chez les sujets, ou la confirmation d'un diagnostic d'une SZ utilisant des instruments psychométriques.


### SEQUENCE LISTING

<110> SureGene LLC
<120> GENETIC MARKERS FOR ASSESSING RISK OF DEVELOPING SCHIZOPHRENIA
<130> 24689-0006WO1
<160> 1025
<170> PatentIn version 3.5
<210> 1
   <211> 61
   <212> DNA
   <213> Homo Sapiens
<400> 1
   ctgtcaccta ggctggaggg cagtggttca mtcacggttt tctgcagcct tgacctcctg 60
   g 61
<210> 2
   <211> 61
   <212> DNA
   <213> Homo Sapiens
<400> 2
   cattggattt caaagcttgc tttttctgtt sgttcttctc tctccttctc tctctgtctc 60
   t 61
<210> 3
   <211> 61
   <212> DNA
   <213> Homo Sapiens
<400> 3
   tgccagatga gctctctgaa gtcactgtaa kcaattctca gagccaccaa gtttaagtca 60
   g 61
<210> 4
   <211> 61
   <212> DNA
   <213> Homo Sapiens
<400> 4
   gcaagataga tattagtact cctgcttttt ytgtcaacta aactgaggca cagagatgtt 60
   a 61
<210> 5
   <211> 61
   <212> DNA
   <213> Homo Sapiens
<400> 5
   ctcaaacttg gcaactctaa aatgagcaag ygagactgaa atgccatcag tgactgggag 60
   c 61
<210> 6
   <211> 61
   <212> DNA
   <213> Homo Sapiens
<400> 6
   ctaaaacctg ctgtgggcgt cacacttctc ygggagcatc attgggcagc tcagccggcc 60
   a 61
<210> 7
   <211> 61
   <212> DNA
   <213> Homo Sapiens
<400> 7
   aatgatgaag atttagctat aaggaagttt wtcaaagcac attaagaaag acaatagtta 60
   c 61
<210> 8
   <211> 61
   <212> DNA
   <213> Homo Sapiens
<400> 8
   tatgcttcat catattagcc tttacctctc yagacaaagt tcatccacct atttggtaag 60
   t 61
<210> 9
   <211> 61
   <212> DNA
   <213> Homo Sapiens
<400> 9
   aacttggact tttcctgaat atcgaatctg ycagtagcag agaccaatac tgattctctg 60
   a 61
<210> 10
   <211> 61
   <212> DNA
   <213> Homo Sapiens
<400> 10
   atcatggagg gaattcatat gctgagtagg wtatttgctg gtttttatct tcaagtttcc 60
   t 61
<210> 11
   <211> 61
   <212> DNA
   <213> Homo Sapiens
<400> 11
   atgatgaatt ctttggtttg tctccccagc ragtggcatt aagccttgaa gatgatggac 60
   t 61
<210> 12
   <211> 61
   <212> DNA
   <213> Homo Sapiens
<400> 12
   tctctttcaa cctccctgca tgacagctgg maagagccca tccctaatct tcaccaaccg 60
   g 61
<210> 13
   <211> 61
   <212> DNA
   <213> Homo Sapiens
<400> 13
   ccagggacca gggggccatg aaagatgtta wgtagagaag ggtcagattt aggatttgaa 60
   a 61
<210> 14
   <211> 61
   <212> DNA
   <213> Homo Sapiens
<400> 14
   taaaatatta cttttataat ttaagtgtgc rtcctctaag ctattcggtt ttgtagttat 60
   a 61
<210> 15
   <211> 61
   <212> DNA
   <213> Homo Sapiens
<400> 15
   gacttttcca tgaaagagga aagtttctct scataacaag ggggtggaaa tgaattggat 60
   g 61
<210> 16
   <211> 61
   <212> DNA
   <213> Homo Sapiens
<400> 16
   tccagcagtg ccctcctctc tcagaccctg ytcttgtcac atgcctgggt tgcatcttgc 60
   a 61
<210> 17
   <211> 61
   <212> DNA
   <213> Homo Sapiens
<400> 17
   gggcagaatt ctggcagttc tctgtggtct ygtttttaca aatagaacca gggacagggg 60
   t 61
<210> 18
   <211> 61
   <212> DNA
   <213> Homo Sapiens
<400> 18
   tgtccttgtc ccctcttttc cattcacttg ytctaaatca atagttctca aaccctccgt 60
   g 61
<210> 19
   <211> 61
   <212> DNA
   <213> Homo Sapiens
<400> 19
   catcattttg ggtacctgtt gttgttcctc yggctcccga aatatctgag cgagtaggat 60
   g 61
<210> 20
   <211> 61
   <212> DNA
   <213> Homo Sapiens
<400> 20
   gaagttaatc atgcctacct cacaggattc raaacaatac acaagaagag taatgccaca 60
   a 61
<210> 21
   <211> 61
   <212> DNA
   <213> Homo Sapiens
<400> 21
   gggtaccctg gccttggggt ggtcagttct raaagataac ctgcacatta tgagttattt 60
   c 61
<210> 22
   <211> 61
   <212> DNA
   <213> Homo Sapiens
<400> 22
   agttttctcc cttctctgat tgttttatcc rtttttcatt gaataggcca tgctcttgtg 60
   a 61
<210> 23
   <211> 61
   <212> DNA
   <213> Homo Sapiens
<400> 23
   aattttgtta caaatagtac cttgcataca mattatcaca aaacgtgaaa aattaaacta 60
   g 61
<210> 24
   <211> 61
   <212> DNA
   <213> Homo Sapiens
<400> 24
   ccttattgag catccacaag cttgtctgcg mgtgtaaaag gagggagaaa ggaccctgcc 60
   a 61
<210> 25
   <211> 61
   <212> DNA
   <213> Homo Sapiens
<400> 25
   ctcatatgat cacattaata gatgttggag kgacatttga taatatttaa cagctaatcc 60
   c 61
<210> 26
   <211> 61
   <212> DNA
   <213> Homo Sapiens
<400> 26
   aaactccaga ctgtatgatt ccacagtgta yttccattca tctcatgaag gacatctttg 60
   t 61
<210> 27
   <211> 61
   <212> DNA
   <213> Homo Sapiens
<400> 27
   gttctagaaa ctatctaaga tgatcaagtc raagttaatt tcacaacttc ttagagtctg        60
   g                                                                        61
<210> 28
   <211> 61
   <212> DNA
   <213> Homo Sapiens
<400> 28
   agagaaagga gattccgtgt gaaacattta ygctaaagta ctgcttttac ctgtaacatc        60
   t                                                                        61
<210> 29
   <211> 61
   <212> DNA
   <213> Homo Sapiens
<400> 29
   gcatactaca tgtaaaacat tgtgctgagg racaagacaa caaggtccct tgtctattgg        60
   a                                                                        61
<210> 30
   <211> 61
   <212> DNA
   <213> Homo Sapiens
<400> 30
   aaaggctgct attatccaaa agtgttgctt wctacaagtt tagcctgaca tctttcagtg        60
   g                                                                        61
<210> 31
   <211> 61
   <212> DNA
   <213> Homo Sapiens
<400> 31
   gaggaaataa tttgcccagg taactaagga rtgatggttc caggaatggc tataatatac        60
   t                                                                        61
<210> 32
   <211> 61
   <212> DNA
   <213> Homo Sapiens
<400> 32
   ccagtcaaat tatattatag gcccatccta ygcaagtata acataaccac atcttaacta      60
   a                                                                      61
<210> 33
   <211> 61
   <212> DNA
   <213> Homo Sapiens
<400> 33
   tttggaaaag gagggtttgt gtgactgcta rggagatgtt ttatattcat tggtcatttg      60
   t                                                                      61
<210> 34
   <211> 61
   <212> DNA
   <213> Homo Sapiens
<400> 34
   aatcgctatc taaacttgat gagtataata ytgcatgtgg agtccctgtc aatttttact      60
   a                                                                      61
<210> 35
   <211> 61
   <212> DNA
   <213> Homo Sapiens
<400> 35
   caaaaaagtc tgatgtttca tcaaggaatt ytatgatttc aaatttgacc ttcctattcc      60
   t                                                                      61
<210> 36
   <211> 61
   <212> DNA
   <213> Homo Sapiens
<400> 36
   tattttaaga ctggaaccta ttaaagtctt rggtacatag gccctagtgg taaacatttc      60
   a                                                                      61
<210> 37
   <211> 61
   <212> DNA
   <213> Homo Sapiens
<400> 37
   gagagtataa atatggcaac ctgtcagtcc ragagtcttt attacaaata acagaagctt 60
   a 61
<210> 38
   <211> 61
   <212> DNA
   <213> Homo Sapiens
<400> 38
   aaaatttgga gattattttc agggaagaaa raatgatcag gtagttatcc ttatttttaa 60
   t 61
<210> 39
   <211> 61
   <212> DNA
   <213> Homo Sapiens
<400> 39
   aggttgcaga agaaaatctg aattaccctg sactaactga actctctgat cattcttttt 60
   c 61
<210> 40
   <211> 61
   <212> DNA
   <213> Homo Sapiens
<400> 40
   tcctcttcca ctgtgcagat tgttagtagt ytgaatttga aggatacgat attttaagag 60
   a 61
<210> 41
   <211> 61
   <212> DNA
   <213> Homo Sapiens
<400> 41
   atgaatatgg tagcaagtgt cttccttcct ktgaaatatg agttttcatc agctgttgga 60
   t 61
<210> 42
   <211> 61
   <212> DNA
   <213> Homo Sapiens
<400> 42
   atgccttatt taagaagaag tttctaggaa macccaaaga caacagcggg gaataaaaac 60
   a 61
<210> 43
   <211> 61
   <212> DNA
   <213> Homo Sapiens
<400> 43
   gcccaggagt tcgagtccag cctgggaaaa stgtgagtcc ctgtctctaa aaaataataa 60
   t 61
<210> 44
   <211> 61
   <212> DNA
   <213> Homo Sapiens
<400> 44
   tttcacctgt atgggtccat ctcataagct sttgggaaac cgggccacat ggtgggcaat 60
   t 61
<210> 45
   <211> 61
   <212> DNA
   <213> Homo Sapiens
<400> 45
   tgaactgtag aggagttcac gaccaaatct stgatgcttt ccccagatct ttcttcaggg 60
   c 61
<210> 46
   <211> 61
   <212> DNA
   <213> Homo Sapiens
<400> 46
   tgaggatgca cagggccagg gcagaattga ytgttgctca gaggatgcac cggggtgtct 60
   c 61
<210> 47
   <211> 61
   <212> DNA
   <213> Homo Sapiens
<400> 47
   cctgggtctg ggaatcaagt ccttacctgg mtagccttcc tggtgcctgc ccacctgagc 60
   g 61
<210> 48
   <211> 61
   <212> DNA
   <213> Homo Sapiens
<400> 48
   gccaggagca ggtctggggc ccaccttctg ygttgcgtag accaggcttt ggtcggctgt 60
   g 61
<210> 49
   <211> 61
   <212> DNA
   <213> Homo Sapiens
<400> 49
   acccaaaagt gactgggctg actgccaaga katggcttta aaccagaaga aatggagatg 60
   t 61
<210> 50
   <211> 61
   <212> DNA
   <213> Homo Sapiens
<400> 50
   tatttccgtt cagtcatgga tcaaacagcc yatttcagca attgatatgg atatattttc 60
   c 61
<210> 51
   <211> 61
   <212> DNA
   <213> Homo Sapiens
<400> 51
   gcatggcaga gatgaggttg aagaagtttg ytgggatcaa gtctaaactg gctttgtgtt 60
   a 61
<210> 52
   <211> 61
   <212> DNA
   <213> Homo Sapiens
<400> 52
   gaagggaatt tcaggctcat attttggtgt matgggcaga tcttgggtga ggttttcatt 60
   g 61
<210> 53
   <211> 61
   <212> DNA
   <213> Homo Sapiens
<400> 53
   aatattattt gatggcctaa aaaaacttaa mactgtctcc aagaagaaaa aaagaagtgc 60
   t 61
<210> 54
   <211> 61
   <212> DNA
   <213> Homo Sapiens
<400> 54
   gaaaagatgg aactcaactg aagtcttgct maaaacagat gcagggggcc tagacaattt 60
   c 61
<210> 55
   <211> 61
   <212> DNA
   <213> Homo Sapiens
<400> 55
   gaggaccaag gccttgaatg ccttgttcac ktctgcatcc ccaacactta ggatgacact 60
   c 61
<210> 56
   <211> 61
   <212> DNA
   <213> Homo Sapiens
<400> 56
   caccctcttc gatgtgatag tccttcctgg wgttggggcc tccgatgaac atgactttga 60
   g 61
<210> 57
   <211> 61
   <212> DNA
   <213> Homo Sapiens
<400> 57
   tttttttttt accttgttta gtggtcaagc rttaaccacc atgttcttca gaagagaatt 60
   c 61
<210> 58
   <211> 61
   <212> DNA
   <213> Homo Sapiens
<400> 58
   caaggccgct aagtttgctc ttacccacca ygtgcagagt gtctcctggg tgcttagtta 60
   a 61
<210> 59
   <211> 61
   <212> DNA
   <213> Homo Sapiens
<400> 59
   ccaaactgtt tctccaacaa aatacagagg rtacaaagga ataggaatga tttttgcaac 60
   a 61
<210> 60
   <211> 61
   <212> DNA
   <213> Homo Sapiens
<400> 60
   aggctagttt aagctgcagt aacaaatccc saaaatctcc agtctagctt aaaacaacaa 60
   a 61
<210> 61
   <211> 61
   <212> DNA
   <213> Homo Sapiens
<400> 61
   gtgagaaagc tgttgttagc taaatggatt mtcttcaatc aaagtacacg agctatggac 60
   a 61
<210> 62
   <211> 61
   <212> DNA
   <213> Homo Sapiens
<400> 62
   agtatatacg gctgaagcag agttagatca ygtagtacct tgtaaattgg gttaagaatt 60
   t 61
<210> 63
   <211> 61
   <212> DNA
   <213> Homo Sapiens
<400> 63
   aaacctgtcc tatgacaccc tccatatact rcataatagc caaagacata ttaagcctat 60
   c 61
<210> 64
   <211> 61
   <212> DNA
   <213> Homo Sapiens
<400> 64
   tggtcagccc gcagctggtc caggggtgcc rtgaccaact tctaccttgg atgctggtgt 60
   c 61
<210> 65
   <211> 61
   <212> DNA
   <213> Homo Sapiens
<400> 65
   tttcattttt ttctctctaa gaaatggcta rgaaagatca tgttttattg atgctaagat 60
   g 61
<210> 66
   <211> 61
   <212> DNA
   <213> Homo Sapiens
<400> 66
   catctgcttt ttggaagaac ctaggttgaa yagactgtga ttagctaaga cccagagagt 60
   c 61
<210> 67
   <211> 61
   <212> DNA
   <213> Homo Sapiens
<400> 67
   ttgccaccca ctcccacaag taatatctcc ragttctgag aatctaagaa tgtgttctac 60
   a 61
<210> 68
   <211> 61
   <212> DNA
   <213> Homo Sapiens
<400> 68
   ttgaataaat ctgtttgtcc tctcccttac kgatggacac gtgggttatt tcagattggg 60
   g 61
<210> 69
   <211> 61
   <212> DNA
   <213> Homo Sapiens
<400> 69
   acaccattat ctacccagtc acttaagcca stagcccaag atccaatcta gctttctgtc 60
   a 61
<210> 70
   <211> 61
   <212> DNA
   <213> Homo Sapiens
<400> 70
   tgtcatatct attcctggca tactcttcat rcatagtcac actgcaatgt agacacaatc 60
   a 61
<210> 71
   <211> 61
   <212> DNA
   <213> Homo Sapiens
<400> 71
   ttcagaatag ttaagtaaga atcatgtata raaatcagca atagagcagg gcatggtggc 60
   t 61
<210> 72
   <211> 61
   <212> DNA
   <213> Homo Sapiens
<400> 72
   gtcctgtatt tctcactagg agaatagtgg rattttgatt ttgttttata gggattgctt 60
   t 61
<210> 73
   <211> 61
   <212> DNA
   <213> Homo Sapiens
<400> 73
   ttagtatgtg gtaacttacc attgtattac yacttagttt ttgaaggtca gaagttcagg        60
   c                                                                         61
<210> 74
   <211> 61
   <212> DNA
   <213> Homo Sapiens
<400> 74
   cagaggtagg tgaattatct ccacagctct mtgataatga caggcattat cagtcattag        60
   c                                                                         61
<210> 75
   <211> 61
   <212> DNA
   <213> Homo Sapiens
<400> 75
   ttcctaattc ataaatattg aatgtacagg ygggatgaca ttctcagtta ggagtcacct        60
   t                                                                         61
<210> 76
   <211> 61
   <212> DNA
   <213> Homo Sapiens
<400> 76
   agaacattgt gattttacta tgcacatctc yaggattcac tccattagaa aaacaggctc        60
   c                                                                         61
<210> 77
   <211> 61
   <212> DNA
   <213> Homo Sapiens
<400> 77
   aaggagccaa gaggcatgat gtaatgcatc magatcttct gtggtgacct tgtaacttct        60
   g                                                                         61
<210> 78
   <211> 61
   <212> DNA
   <213> Homo Sapiens
<400> 78
   gatcttacct caagctgatg cacaattatg kattcatccc agtgctttca gggtagccta 60
   g 61
<210> 79
   <211> 61
   <212> DNA
   <213> Homo Sapiens
<400> 79
   cagaggaaca aagagagaat tacctttttc ygcagcaact tatattcatg ttttttcatc 60
   a 61
<210> 80
   <211> 61
   <212> DNA
   <213> Homo Sapiens
<400> 80
   ctggaaagtt ctgaggatcc agatgcacca ytacaagggc attttatagt cttcagtgca 60
   g 61
<210> 81
   <211> 61
   <212> DNA
   <213> Homo Sapiens
<400> 81
   cgaggatgac actgatggtg acgatactga ygatagtaat aacacttact gaatactcac 60
   t 61
<210> 82
   <211> 61
   <212> DNA
   <213> Homo Sapiens
<400> 82
   gcctatgcca gcatagtcct cttgagaatg yctcaccttc gcaggtgcaa tcatttataa 60
   g 61
<210> 83
   <211> 61
   <212> DNA
   <213> Homo Sapiens
<400> 83
   atttagattt ccagttacct ggcaagagaa mgacttaaaa ggaataagtc taggtaaatc 60
   t 61
<210> 84
   <211> 61
   <212> DNA
   <213> Homo Sapiens
<400> 84
   taaagatgcc cagtggttga acttcctgat ytccaggcct gtactcttct cactatgtca 60
   t 61
<210> 85
   <211> 61
   <212> DNA
   <213> Homo Sapiens
<400> 85
   catggatagt taacattcag acaggcttat yttcaaatct ggattctatt tactgtgtga 60
   c 61
<210> 86
   <211> 61
   <212> DNA
   <213> Homo Sapiens
<400> 86
   tgtaaacaca ttctttattg atatgcacat mgtgattctg atttggaata ttaagggtcc 60
   c 61
<210> 87
   <211> 61
   <212> DNA
   <213> Homo Sapiens
<400> 87
   attttacaga agcttaatat gagtctaaat rgaggtatac aacctaagga gttccactcc 60
   c 61
<210> 88
   <211> 61
   <212> DNA
   <213> Homo Sapiens
<400> 88
   ttataagaag gtaaatcaac ttctccatgc wgtgcaacaa gccattgtta aatctggatt 60
   g 61
<210> 89
   <211> 61
   <212> DNA
   <213> Homo Sapiens
<400> 89
   tgtttaaaaa tttaaagcat acttctaaga yatagtggtg attctctata ctagcatgaa 60
   c 61
<210> 90
   <211> 61
   <212> DNA
   <213> Homo Sapiens
<400> 90
   tcagaggcct tggcctagag ttgagccata maccatattt tagaagtaaa tacattccta 60
   a 61
<210> 91
   <211> 61
   <212> DNA
   <213> Homo Sapiens
<400> 91
   ttcagttaaa aagtaaagac aaaaagaaaa rtaataaatt tccactttca atatagtaaa 60
   t 61
<210> 92
   <211> 61
   <212> DNA
   <213> Homo Sapiens
<400> 92
   cattcccgtc tatttggttg tagataaaag rgataagggg cctctttctc aataactact 60
   t 61
<210> 93
   <211> 61
   <212> DNA
   <213> Homo Sapiens
<400> 93
   tttttcaacg tggtaccaca aatgtggcca saaggatttt cataatggga tattgtacat 60
   a 61
<210> 94
   <211> 61
   <212> DNA
   <213> Homo Sapiens
<400> 94
   aaaatctgat ataatttttc agagtctgtg rcactaagga gtccacaggg ctgcctaggt 60
   g 61
<210> 95
   <211> 61
   <212> DNA
   <213> Homo Sapiens
<400> 95
   gtggtggcct cataccatag tagctgctta sttaattatg actaatataa ggaggtatag 60
   g 61
<210> 96
   <211> 61
   <212> DNA
   <213> Homo Sapiens
<400> 96
   caactctcct acttgatcat gtacaggact yaaaactgta atcattaaaa cccagatgat 60
   c 61
<210> 97
   <211> 61
   <212> DNA
   <213> Homo Sapiens
<400> 97
   taaaattata tgtttaggtg cattaactat yaactcaaag aggattgtga aaggcctcct 60
   t 61
<210> 98
   <211> 61
   <212> DNA
   <213> Homo Sapiens
<400> 98
   aaatatagca gctacaatct aatttcattg raagtctcct ccaaatcaca gaagtgccac 60
   t 61
<210> 99
   <211> 61
   <212> DNA
   <213> Homo Sapiens
<400> 99
   cttttctctc tttatttttt ttaaacagaa ygctatgaac ctacctcctg acaaagccag 60
   g 61
<210> 100
   <211> 61
   <212> DNA
   <213> Homo Sapiens
<400> 100
   gctagtgaat taagcaaata tgaaatgaca rtgaagtcgt attaatggtt tgtggtatgg 60
   a 61
<210> 101
   <211> 61
   <212> DNA
   <213> Homo Sapiens
<400> 101
   aatccaatca acccttggta ttgtcagtcc kttaaaattc tagccatttt tatgggcata 60
   t 61
<210> 102
   <211> 61
   <212> DNA
   <213> Homo Sapiens
<400> 102
   cccgagctgg attaaaatcc aggtcacaga sggactgttt tctctcctct ttcctgaaag 60
   g 61
<210> 103
   <211> 61
   <212> DNA
   <213> Homo Sapiens
<400> 103
   ataacaggaa tctgattata ctgcaggtca rattatatcc taaagatata tttgtataga 60
   a 61
<210> 104
   <211> 61
   <212> DNA
   <213> Homo Sapiens
<400> 104
   gcaggagagc aacaatccta aaagagatca ytatgactgc agacagtatt gtgacttccg 60
   g 61
<210> 105
   <211> 61
   <212> DNA
   <213> Homo Sapiens
<400> 105
   atgtgtggcc caagataatt attactcttc ratgtggccc aaggacgcca aaagattgca 60
   c 61
<210> 106
   <211> 61
   <212> DNA
   <213> Homo Sapiens
<400> 106
   aaaagagtcc taaggataga tacggtgtta ytagatgttg gctgaagctg gggtaaaaca 60
   g 61
<210> 107
   <211> 61
   <212> DNA
   <213> Homo Sapiens
<400> 107
   caaacgatca aaggttacat agattagacc maagtctgct ttattccaaa gcttattttc 60
   t 61
<210> 108
   <211> 61
   <212> DNA
   <213> Homo Sapiens
<400> 108
   ccatggaggc tcctacatgc cacagtaagg kctctgaatt gtatttgata agcagtaggg 60
   a 61
<210> 109
   <211> 61
   <212> DNA
   <213> Homo Sapiens
<400> 109
   ctcattgtat gcttgctctg tgttcaacat wgtgcaggat gcgagatgaa taagctatca 60
   t 61
<210> 110
   <211> 61
   <212> DNA
   <213> Homo Sapiens
<400> 110
   aggtcacaga aattcttatt ccttgttttc yctgagttat gacatagtga aatgttcctg 60
   t 61
<210> 111
   <211> 61
   <212> DNA
   <213> Homo Sapiens
<400> 111
   aaattgtgca attcagggaa tatggcaatt kaaaaccaga tgcttagacc actaagccat 60
   c 61
<210> 112
   <211> 61
   <212> DNA
   <213> Homo Sapiens
<400> 112
   cacctcccat gtgacctgat gctggagaaa stgtggttta gcgggaggct gatggggaag 60
   t 61
<210> 113
   <211> 61
   <212> DNA
   <213> Homo Sapiens
<400> 113
   tgcttaaatg tttaaataag cttcttgaaa wctcatggaa cattcaatca caactccaac 60
   a 61
<210> 114
   <211> 61
   <212> DNA
   <213> Homo Sapiens
<400> 114
   tcatgtatgg agatgggaag tagaatcaag ygggaaatga gaaaacagtg cgttcttcat 60
   g 61
<210> 115
   <211> 61
   <212> DNA
   <213> Homo Sapiens
<400> 115
   ttaaggtgat ggccttctaa agcagctttc rgaagtttta tttgtcaata tcagacataa 60
   a 61
<210> 116
   <211> 61
   <212> DNA
   <213> Homo Sapiens
<400> 116
   cagatggatg gatggatgaa tttgtgttct yatgattgag acattacgat aaggggaaag 60
   a 61
<210> 117
   <211> 61
   <212> DNA
   <213> Homo Sapiens
<400> 117
   ataccttatt tccactcaca ctggttagaa stcagtccca ttgcgttttt tggccatgca 60
   c 61
<210> 118
   <211> 61
   <212> DNA
   <213> Homo Sapiens
<400> 118
   ctaggtgcag agcaactctc gtgatatggc yagttagact tttcatcttg gtcttgccct 60
   t 61
<210> 119
   <211> 61
   <212> DNA
   <213> Homo Sapiens
<400> 119
   ttagtatctg aaagttctta aattgtaggc rtaaatgttt cattttaatt gtgcatttcc 60
   a 61
<210> 120
   <211> 61
   <212> DNA
   <213> Homo Sapiens
<400> 120
   ctaatacttc caattccatt ccaacaccat rggatttatt ctagccttca ccttttccat 60
   t 61
<210> 121
   <211> 61
   <212> DNA
   <213> Homo Sapiens
<400> 121
   accctctcaa tggcaaatct tattctaggc rgcagaatta agcagagtac gggtgaaatc 60
   g 61
<210> 122
   <211> 61
   <212> DNA
   <213> Homo Sapiens
<400> 122
   caaaggtatc tcatagaact aacttcgttc rtcatggttc tggaggtgtt agccaatgca 60
   g 61
<210> 123
   <211> 61
   <212> DNA
   <213> Homo Sapiens
<400> 123
   ggtttctgct ggaatacttt tggccttgag ygtgttacag atcaaaggga agtatcagag 60
   g 61
<210> 124
   <211> 61
   <212> DNA
   <213> Homo Sapiens
<400> 124
   agaggggtca ctgctttagt ggaactctca yggaagttca tgacctagga gggtttaaaa 60
   a 61
<210> 125
   <211> 61
   <212> DNA
   <213> Homo Sapiens
<400> 125
   gagggcctac tgttcaactc aaattgaatt yctgttagta ttctctgctt ctgaagcttc 60
   t 61
<210> 126
   <211> 61
   <212> DNA
   <213> Homo Sapiens
<400> 126
   gcaagagaca gagggggaca ttttctgaca sagcgttcaa ggccagcagc agctcagggg 60
   c 61
<210> 127
   <211> 61
   <212> DNA
   <213> Homo Sapiens
<400> 127
   ctgagcttgt gctataacca aatattctgg rtaatggttg gtatatacgt aaccgtagat 60
   a 61
<210> 128
   <211> 61
   <212> DNA
   <213> Homo Sapiens
<400> 128
   attgatgaaa gtccgtctgg ctgtttcggc yatttgacag caaagatgaa aaaatacatg 60
   c 61
<210> 129
   <211> 61
   <212> DNA
   <213> Homo Sapiens
<400> 129
   ttagaaataa ttaatgggac tgaggcaagt raccaagaat gcactatgta catagagttt 60
   c 61
<210> 130
   <211> 61
   <212> DNA
   <213> Homo Sapiens
<400> 130
   ccacactgcc atattgctat acctacaaca ygggatataa aaagcatcct ttaacttatg 60
   t 61
<210> 131
   <211> 61
   <212> DNA
   <213> Homo Sapiens
<400> 131
   cttgtatttt ttatgcccat catctccagt yggttccctg tgaaagattg cttattcttg 60
   c 61
<210> 132
   <211> 61
   <212> DNA
   <213> Homo Sapiens
<400> 132
   gaatgatcag aggtcagatc acggaaagga yattagattt tattgtaact acagagaaac 60
   t 61
<210> 133
   <211> 61
   <212> DNA
   <213> Homo Sapiens
<400> 133
   acaaacatac acagcagctt gaagttcaaa macagtcaga agtagctacc aagaaatagg 60
   a 61
<210> 134
   <211> 61
   <212> DNA
   <213> Homo Sapiens
<400> 134
   ggtcctacta tcaggaggat gaggtaacaa rcttccccat accacctcag aaaggataaa 60
   c 61
<210> 135
   <211> 61
   <212> DNA
   <213> Homo Sapiens
<400> 135
   ataaccaagg tgtgaagtgg gaagggtaga sgaagataga aactaccaaa agttactaat 60
   g 61
<210> 136
   <211> 61
   <212> DNA
   <213> Homo Sapiens
<400> 136
   agggagagaa aattgttatg gcagcaagaa rttctacctt gaaaataaat ctgctttgct 60
   a 61
<210> 137
   <211> 61
   <212> DNA
   <213> Homo Sapiens
<400> 137
   acatggacta catcttctaa atcattatac mcagaaacgc tctcagtagc tgacaccaat 60
   c 61
<210> 138
   <211> 61
   <212> DNA
   <213> Homo Sapiens
<400> 138
   cagccccagc acagaaacag gaaatgcatt ytgaggagac aacagctctg ggtacaacaa 60
   a 61
<210> 139
   <211> 61
   <212> DNA
   <213> Homo Sapiens
<400> 139
   gctctggcac atgttttagg tgtagagaca rcattgggca gtgggaaaag gataagtcaa 60
   t 61
<210> 140
   <211> 61
   <212> DNA
   <213> Homo Sapiens
<400> 140
   ctgtcctcag gcttggtgga gccagtgaac rtggtggaca atggagatgg cacacacaca 60
   g 61
<210> 141
   <211> 61
   <212> DNA
   <213> Homo Sapiens
<400> 141
   tgagcattgc cacacaggta ctgttaaaag sgcagaatga aggtacatat gtgatttttg 60
   c 61
<210> 142
   <211> 61
   <212> DNA
   <213> Homo Sapiens
<400> 142
   gcggggggga cctcatgatt ccatctttat rgcccaactg ttgattaact tccaaatgaa 60
   a 61
<210> 143
   <211> 61
   <212> DNA
   <213> Homo Sapiens
<400> 143
   attcagcagc atattcaaag agacagagaa kaactgacat gatgctgatg aagcttaaac 60
   t 61
<210> 144
   <211> 61
   <212> DNA
   <213> Homo Sapiens
<400> 144
   agcagtggcc aagcagacac aagtgatcat yatcatacga acttaatttc tgaccaaatt 60
   c 61
<210> 145
   <211> 61
   <212> DNA
   <213> Homo Sapiens
<400> 145
   gactattaac tagtaattga tgacaaccaa rgaaggtagt tttcctaata cctacaaaac 60
   t 61
<210> 146
   <211> 61
   <212> DNA
   <213> Homo Sapiens
<400> 146
   ttggggagaa actgatgttt tgcagatgca wtattaaaac aatccattta taaggaaggc 60
   a 61
<210> 147
   <211> 61
   <212> DNA
   <213> Homo Sapiens
<400> 147
   cccagaagca gatctggaga caggtattca mgagcaagta ttttatttag gaggttatcg 60
   c 61
<210> 148
   <211> 61
   <212> DNA
   <213> Homo Sapiens
<400> 148
   tgaactccaa agaaggtaga aatcaactgc rttattgagt aatgtgcata ctttgaatgt 60
   t 61
<210> 149
   <211> 61
   <212> DNA
   <213> Homo Sapiens
<400> 149
   cagaggcagg tttcatcttt ctgcctcgtc wttcaggaat ggccttaatc ttcctacagt 60
   t 61
<210> 150
   <211> 61
   <212> DNA
   <213> Homo Sapiens
<400> 150
   gccaactctc caggtggccg agacagggca stccctcagg taacaggggt gaacactggg 60
   g 61
<210> 151
   <211> 61
   <212> DNA
   <213> Homo Sapiens
<400> 151
   cacagtttgt gctaaacatt atgaggagta saaaaacaaa tcaaaagcct aagaattcgt 60
   t 61
<210> 152
   <211> 61
   <212> DNA
   <213> Homo Sapiens
<400> 152
   ttatgaggag tacaaaaaca aatcaaaagc staagaattc gttgctagcc atgaaagtcc 60
   t 61
<210> 153
   <211> 61
   <212> DNA
   <213> Homo Sapiens
<400> 153
   caaatcaaaa gcctaagaat tcgttgctag mcatgaaagt cctctgttgg aacatcctct 60
   t 61
<210> 154
   <211> 61
   <212> DNA
   <213> Homo Sapiens
<400> 154
   tcctttgctg tgggtccctg tcctgggcac ygtagaattc ttagcagctt tcctggtccc 60
   t 61
<210> 155
   <211> 61
   <212> DNA
   <213> Homo Sapiens
<400> 155
   gttttacaag tgtttggttt tttgatccac raaatatctc attcaaaagg tttgctttaa 60
   t 61
<210> 156
   <211> 61
   <212> DNA
   <213> Homo Sapiens
<400> 156
   ttgttagggc agtttcaaaa atgtgctttg kacataacag acctttccca agatggtcat 60
   a 61
<210> 157
   <211> 61
   <212> DNA
   <213> Homo Sapiens
<400> 157
   aactgaagct ggaccctggg cttctgagtc yaaaggtggt acttcctgtg ctagaccgtg 60
   c 61
<210> 158
   <211> 61
   <212> DNA
   <213> Homo Sapiens
<400> 158
   gagctttgat ctggactatg taggaaggac wtttcagttg tgtgccttac tttgtacagt 60
   g 61
<210> 159
   <211> 61
   <212> DNA
   <213> Homo Sapiens
<400> 159
   tttacattat gcaatgaatg ctaattgaca rctgattcat aattagttaa ttgtaaacac 60
   t 61
<210> 160
   <211> 61
   <212> DNA
   <213> Homo Sapiens
<400> 160
   tgagcactaa atgttccttg agtatgctca ratgactgta atagcagaca ggacatatcc 60
   a 61
<210> 161
   <211> 61
   <212> DNA
   <213> Homo Sapiens
<400> 161
   aattcttata ctactatatt ttattccctc ractgccagg ttcattaaaa accctttaca 60
   t 61
<210> 162
   <211> 61
   <212> DNA
   <213> Homo Sapiens
<400> 162
   ttggaattaa tcactctatt ctctgaagcc rcataccact ttgccaccat cagtagtagg 60
   a 61
<210> 163
   <211> 61
   <212> DNA
   <213> Homo Sapiens
<400> 163
   atctctaaag gttcactgga aatttgcatt rctcagtgaa gaatcaaatc ctagccagat 60
   g 61
<210> 164
   <211> 61
   <212> DNA
   <213> Homo Sapiens
<400> 164
   tcatgaattc ttgaaagaga tcttaaacta ygatagcaca gaaatcactg aattgcttaa 60
   c 61
<210> 165
   <211> 61
   <212> DNA
   <213> Homo Sapiens

<400> 165
   ctctctactt ttttaatgga atttgtgaag ycccttaata acaagttgat ttataaagct 60
   t 61
<210> 166
   <211> 61
   <212> DNA
   <213> Homo Sapiens
<400> 166
   gatgcggcac agacagagac aacaagctat rttttgctaa tgggtcctat caatatgggg 60
   c 61
<210> 167
   <211> 61
   <212> DNA
   <213> Homo Sapiens
<400> 167
   tgacaagtta ctctaacata atagcgaact rcaaactgca cagggctttg gagcccaact 60
   g 61
<210> 168
   <211> 61
   <212> DNA
   <213> Homo Sapiens
<400> 168
   tagaaatgaa tccaattcta acacagagag rtataaactt tgggactgtg tattgtgcta 60
   t 61
<210> 169
   <211> 61
   <212> DNA
   <213> Homo Sapiens
<400> 169
   atcccctggt gagaggtttt ccaaaatgca rtaatacaga aagaatttaa gggaagttct 60
   g 61
<210> 170
   <211> 61
   <212> DNA
   <213> Homo Sapiens
<400> 170
   aagcactttt catattgtca atcttcccag saaatctgca gagtagacct tagtttcttt 60
   t 61
<210> 171
   <211> 61
   <212> DNA
   <213> Homo Sapiens
<400> 171
   ttcaagtcct atcccagcca aggtcctagc raactcagct cgtggacctg tctctgacgg 60
   t 61
<210> 172
   <211> 61
   <212> DNA
   <213> Homo Sapiens
<400> 172
   attaccacta ctcagtttaa agcaacaaat sctcattgaa tagctactat tttttatata 60
   c 61
<210> 173
   <211> 61
   <212> DNA
   <213> Homo Sapiens
<400> 173
   ttccaaaaaa cagcaataga agactccaca mgaagattgt gtccattttg acttctctgt 60
   a 61
<210> 174
   <211> 61
   <212> DNA
   <213> Homo Sapiens
<400> 174
   ctaattaatt aaaagaggaa agttttaaaa ygaacacagc atgaacacaa acttatataa 60
   g 61
<210> 175
   <211> 61
   <212> DNA
   <213> Homo Sapiens
<400> 175
   acagaaggct gctttatgtg caaaaggtag kttaagtctc aaaatataaa atgtgtgtat 60
   a 61
<210> 176
   <211> 61
   <212> DNA
   <213> Homo Sapiens
<400> 176
   ggctgttaat ggcctgtcat gtgctttttt yggcagagca gtgaggaact ctttgagagc 60
   c 61
<210> 177
   <211> 61
   <212> DNA
   <213> Homo Sapiens
<400> 177
   aggaaggtac atctcctggg aagtagagct wtcacacaat aatcaattca tgcaaaaagc 60
   a 61
<210> 178
   <211> 61
   <212> DNA
   <213> Homo Sapiens
<400> 178
   accctatttc actaagtcct catcttaatt yaactagtta cacccacagc aaccttattt 60
   c 61
<210> 179
   <211> 61
   <212> DNA
   <213> Homo Sapiens
<400> 179
   aaggacagca agatggggta acctaaaata stgttgatag atcacagagg aaataagctg 60
   t 61
<210> 180
   <211> 61
   <212> DNA
   <213> Homo Sapiens
<400> 180
   actatgcaca gacacatttt tccacattat rttgctgctt cagtaaagat gttgtacttg 60
   a 61
<210> 181
   <211> 61
   <212> DNA
   <213> Homo Sapiens
<400> 181
   caactgactg cattcccatg agtgactcca ytgagtaagg accacctagc taagctctgt 60
   c 61
<210> 182
   <211> 61
   <212> DNA
   <213> Homo Sapiens
<400> 182
   cacaatgtat tatgcatgtg ccgagttgct ragtatggct gatgctgcag cccttgttag 60
   t 61
<210> 183
   <211> 61
   <212> DNA
   <213> Homo Sapiens
<400> 183
   ggagtaatac agaaactttt agccaaggca yattctctgg gctctcaatc ctttgcagct 60
   g 61
<210> 184
   <211> 61
   <212> DNA
   <213> Homo Sapiens
<400> 184
   agtgattgat tgtacttgga aagtgaattg ygatgcctct atttattcca cgagtatgag 60
   g 61
<210> 185
   <211> 61
   <212> DNA
   <213> Homo Sapiens
<400> 185
   ctgtcaatac atatggagaa aatagtttca yggaatcaaa gttatctggt tcacctcctt 60
   t 61
<210> 186
   <211> 61
   <212> DNA
   <213> Homo Sapiens
<400> 186
   acatgtaaaa gaatataatt tgatgaattt yggcatatgc attcccccat gaatccacac 60
   a 61
<210> 187
   <211> 61
   <212> DNA
   <213> Homo Sapiens
<400> 187
   ctttgtatat tcctctatag tcagaaacaa yggaatgtcc tagagccctt tagtgaagaa 60
   t 61
<210> 188
   <211> 61
   <212> DNA
   <213> Homo Sapiens
<400> 188
   gtggccatgg cagacactgt caggctccta ytgaatttca ttctcctctg atttccttac 60
   t 61
<210> 189
   <211> 61
   <212> DNA
   <213> Homo Sapiens
<400> 189
   acagagaaat cgaattacta agttagggga yagataaggc aaacattcat tagttcacaa 60
   a 61
<210> 190
   <211> 61
   <212> DNA
   <213> Homo Sapiens
<400> 190
   attttgactc tgatttcttc ccataccttt yggagtccac ttctccgcca ctaacttttt 60
   t 61
<210> 191
   <211> 61
   <212> DNA
   <213> Homo Sapiens
<400> 191
   agtgtgcaaa tattactcaa atggcaaaca yatgattagg ttaaggatgt tgagaggagg 60
   a 61
<210> 192
   <211> 61
   <212> DNA
   <213> Homo Sapiens
<400> 192
   agactgcact caacacacag gttaggtact yggattatgt gaggtatcct cctctcatgc 60
   t 61
<210> 193
   <211> 61
   <212> DNA
   <213> Homo Sapiens
<400> 193
   aagaggtaga ggaggtcctg gctgagagcc rcgtgcagag gagcatccgc ctggtgtccg 60
   t 61
<210> 194
   <211> 61
   <212> DNA
   <213> Homo Sapiens
<400> 194
   ttattggttc gaactcaccc agctatattt ktactgtgct tgctatatac atgatttaaa 60
   a 61
<210> 195
   <211> 61
   <212> DNA
   <213> Homo Sapiens
<400> 195
   agaatagtgt ggggaacggt tctgctacat kcctaaaagg agcaaaggga aattaatatt 60
   g 61
<210> 196
   <211> 61
   <212> DNA
   <213> Homo Sapiens
<400> 196
   acgaagtcct aggtctttgc agatgatcaa rttttggaga agaaacaaat ttactctgca 60
   t 61
<210> 197
   <211> 61
   <212> DNA
   <213> Homo Sapiens
<400> 197
   caatctgtcc agggtctgaa tagaatgaaa mggtgaggaa aagaagattc actccctctc 60
   t 61
<210> 198
   <211> 61
   <212> DNA
   <213> Homo Sapiens
<400> 198
   gttttcttct gccctcagac taagccatac ractggctct cctgggtctc cagcttgtca 60
   a 61
<210> 199
   <211> 61
   <212> DNA
   <213> Homo Sapiens
<400> 199
   ctcctgggtc tccagcttgt caactacaga ytatgggttt tagctttcat aatcatgtag 60
   c 61
<210> 200
   <211> 61
   <212> DNA
   <213> Homo Sapiens
<400> 200
   atgttgtcca tatgctggtg actcctaaat mtaaatctcc agcttgggct tttcctctga 60
   a 61
<210> 201
   <211> 61
   <212> DNA
   <213> Homo Sapiens
<400> 201
   aatctccagc ttgggctttt cctctgaatt yctggcctct atccaattac ttactgatgt 60
   t 61
<210> 202
   <211> 61
   <212> DNA
   <213> Homo Sapiens
<400> 202
   aaaaggagtt taagaataga gtcctcttta wttaggatac attaataaac aaaacataca 60
   a 61
<210> 203
   <211> 61
   <212> DNA
   <213> Homo Sapiens
<400> 203
   ggatacaggc aaaggaaaac tgtactaggc raggcccata gaataaattg acaggattat 60
   t 61
<210> 204
   <211> 61
   <212> DNA
   <213> Homo Sapiens
<400> 204
   ttgcttctct gaaattcaat gcataactcc ygataatcca tccagttcat tcaagaagta 60
   t 61
<210> 205
   <211> 61
   <212> DNA
   <213> Homo Sapiens
<400> 205
   gagcctgaag agagttttac tgcactgaat rggggacaga aagcagtact ggcaacattt 60
   t 61
<210> 206
   <211> 61
   <212> DNA
   <213> Homo Sapiens
<400> 206
   cttttctagt ttccaaagtc cataatttcc rgacagcagg aatagttatg caaagaacag 60
   a 61
<210> 207
   <211> 61
   <212> DNA
   <213> Homo Sapiens
<400> 207
   agactgtaaa agatattgga aatggatcaa rgcactctga acatattcaa ataattgctg 60
   t 61
<210> 208
   <211> 61
   <212> DNA
   <213> Homo Sapiens
<400> 208
   ttcattctca aagagctaaa tttgttttgg kgtgagaggc tgataattag gttgaccctt 60
   g 61
<210> 209
   <211> 61
   <212> DNA
   <213> Homo Sapiens
<400> 209
   cgccatcccg caaccccctg ttatgttcat rtacttccac ggttctcgcg tcatgcctgt 60
   g 61
<210> 210
   <211> 61
   <212> DNA
   <213> Homo Sapiens
<400> 210
   tttctattgt ttgtggcttg ttaattttca ygctactcct aaatggctca tatcttactt 60
   t 61
<210> 211
   <211> 61
   <212> DNA
   <213> Homo Sapiens
<400> 211
   gaaagcacgt ggactttgga atggtgcaca wctgagttcc aattttctaa ttttgctgaa 60
   c 61
<210> 212
   <211> 61
   <212> DNA
   <213> Homo Sapiens
<400> 212
   tatacataaa cttgggaatt actttactat rtatcatcac tgtgacactt ttgaatttgt 60
   t 61
<210> 213
   <211> 61
   <212> DNA
   <213> Homo Sapiens
<400> 213
   cacaaggaca gatgatcgta actttattta kgttcaagat agatttctgt agcactacat 60
   a 61
<210> 214
   <211> 61
   <212> DNA
   <213> Homo Sapiens
<400> 214
   tcacaacagt gcctggagaa cttcttttgc wccttaaaat acttcatctg tctcataagc 60
   c 61
<210> 215
   <211> 61
   <212> DNA
   <213> Homo Sapiens
<400> 215
   ggaaaaaaat agctaaagag gacatggcaa rgatgcctga tgacattaga ctttccactg 60
   t 61
<210> 216
   <211> 61
   <212> DNA
   <213> Homo Sapiens
<400> 216
   agggcgtttc taatgctctc tcactcacaa mgttactagc tcaagtgcct caaagtgtag 60
   c 61
<210> 217
   <211> 61
   <212> DNA
   <213> Homo Sapiens
<400> 217
   ttgggcccac tgttacagaa gttatcgaga sagatggcaa tcaggctgcc tgcaagtctg 60
   a 61
<210> 218
   <211> 61
   <212> DNA
   <213> Homo Sapiens
<400> 218
   tcctctgaat agattccaat gtggcttcaa yactgggctt tattgcttta tccaccagct 60
   c 61
<210> 219
   <211> 61
   <212> DNA
   <213> Homo Sapiens
<400> 219
   aaaaaaaaat agaactgaaa ctaacaagta sagactgttc aacatgtgtg atggtaaaat 60
   g 61
<210> 220
   <211> 61
   <212> DNA
   <213> Homo Sapiens
<400> 220
   gacatctatt attacctcta tacttgtgct rctcaggatg cgtgtcttac ttgcaaagag 60
   c 61
<210> 221
   <211> 61
   <212> DNA
   <213> Homo Sapiens
<400> 221
   tagatggcac aagttctgga cctagaaaat rcaaaaggca atggttaaat cagccctatt 60
   t 61
<210> 222
   <211> 61
   <212> DNA
   <213> Homo Sapiens
<400> 222
   cagatagatc ctcaccatca tctgaacact ygaactggac ttccttttct gaattgacca 60
   g 61
<210> 223
   <211> 61
   <212> DNA
   <213> Homo Sapiens
<400> 223
   cggagaaaaa ccaagggttt gagtaaatca rcataatttg gacagatctc tagaaagaaa 60
   a 61
<210> 224
   <211> 61
   <212> DNA
   <213> Homo Sapiens
<400> 224
   taattgaaat gaaatgaggc tgagggaaaa kagcaggtag gagaccataa tgactgtatg 60
   a 61
<210> 225
   <211> 61
   <212> DNA
   <213> Homo Sapiens
<400> 225
   gaattatgag catgtggttt aaaatcagaa rggctacaat aaaaaagaat aggtgcctgc 60
   c 61
<210> 226
   <211> 61
   <212> DNA
   <213> Homo Sapiens
<400> 226
   ctgtcagaca ttaactcaat tcagagcaaa ragttgcaga gaatgtaggg ggaaggtgca 60
   g 61
<210> 227
   <211> 61
   <212> DNA
   <213> Homo Sapiens
<400> 227
   agagttggtc aaacccaatg aaaggagggc ygaaacaaca aaaggcaaca ataaaaacag 60
   g 61
<210> 228
   <211> 50
   <212> DNA
   <213> Homo Sapiens
<400> 228
   attaggttaa atatgtattw ggcaaaaaaa ccacacttac ttttgcacca 50
<210> 229
   <211> 61
   <212> DNA
   <213> Homo Sapiens
<400> 229
   ggcatcttta gggtgagggg cattattctg yctaacatgc tacttttgct cttgagatgg 60
   c 61
<210> 230
   <211> 61
   <212> DNA
   <213> Homo Sapiens
<400> 230
   attgaaaaag tagccaaatg ctttaagaaa sggaatagat agagctatag ttccaaagtt 60
   t 61
<210> 231
   <211> 61
   <212> DNA
   <213> Homo Sapiens
<400> 231
   atttcattct caaaaaggaa tagctatttt satggacaac agatcagtgg tggccagggg 60
   t 61
<210> 232
   <211> 61
   <212> DNA
   <213> Homo Sapiens
<400> 232
   ctgttgataa gtattccatg atgaaagctt yttgcattca aattaattta agaaaagctg 60
   c 61
<210> 233
   <211> 61
   <212> DNA
   <213> Homo Sapiens
<400> 233
   cctgtgggca tgtgagtttg taacactacc rttatgatac tgatttgtga gatagtaagc        60
   t                                                                        61
<210> 234
   <211> 61
   <212> DNA
   <213> Homo Sapiens
<400> 234
   aaggaagtag aaatttaggc agttaggttt kacccacaac ctcatagcct aggtggtaag        60
   c                                                                        61
<210> 235
   <211> 61
   <212> DNA
   <213> Homo Sapiens
<400> 235
   tgggaaaatt atttaactgc ttaacttctt rccagacttt cagatcctgc tgcttctgta        60
   t                                                                        61
<210> 236
   <211> 61
   <212> DNA
   <213> Homo Sapiens
<400> 236
   aagattagtc tcctttatac attttccaca rttgtttcca taaagaaatg ttcttagttt        60
   t                                                                        61
<210> 237
   <211> 61
   <212> DNA
   <213> Homo Sapiens
<400> 237
   acatgtcatc taaaggatag aaactttcac rggatgttct caaaaagaca aacttcatgg        60
   a                                                                        61
<210> 238
   <211> 61
   <212> DNA
   <213> Homo Sapiens
<400> 238
   aaaggataga aactttcaca ggatgttctc raaaagacaa acttcatgga aactttttct 60
   t 61
<210> 239
   <211> 61
   <212> DNA
   <213> Homo Sapiens
<400> 239
   aagtgctaga agaaattata aagaaaacgc rtagcagatt gggctacata aaaacttccc 60
   t 61
<210> 240
   <211> 61
   <212> DNA
   <213> Homo Sapiens
<400> 240
   tgtctttatt agcagtgtga gaacagacta rtgtggtaga atggtagctg gtaggggggt 60
   g 61
<210> 241
   <211> 61
   <212> DNA
   <213> Homo Sapiens
<400> 241
   attgggttgg ccaggaccag cagtacaatt sagatagggc ttgctgagtt ctagtgttac 60
   a 61
<210> 242
   <211> 61
   <212> DNA
   <213> Homo Sapiens
<400> 242
   ttaatagtgg ttgcgataaa tatctggttc rgcaaaatat gagacaaagc aaactatgct 60
   t 61
<210> 243
   <211> 61
   <212> DNA
   <213> Homo Sapiens
<400> 243
   gtgggcatcc ttgtcttttt cctgaactta saggagaagc tcaatctctc attgttgaat 60
   t 61
<210> 244
   <211> 61
   <212> DNA
   <213> Homo Sapiens
<400> 244
   aaaaatagta ttttgaggct ttaaagtagt rtttggcaca taggaagtcc ccatcaattg 60
   t 61
<210> 245
   <211> 61
   <212> DNA
   <213> Homo Sapiens
<400> 245
   tgagatgatg cttcttaatg taaaattgac yggcatgact accaatgaat atttgatgtg 60
   a 61
<210> 246
   <211> 61
   <212> DNA
   <213> Homo Sapiens
<400> 246
   agagaagggt gtgtaaatga aaccttaata yttggacaca tttattaatt acccattttt 60
   t 61
<210> 247
   <211> 61
   <212> DNA
   <213> Homo Sapiens
<400> 247
   tttcaatata atgatggcat gacttttagc ytgtacttga acttgtttgg tccagtcttg 60
   t 61
<210> 248
   <211> 61
   <212> DNA
   <213> Homo Sapiens
<400> 248
   taacatatct tctttacttc caagttagtg yttttcactc tacatcatgc ccaacacttt 60
   c 61
<210> 249
   <211> 61
   <212> DNA
   <213> Homo Sapiens
<400> 249
   attccctatt agtcccttct tttcatttct stgtctatcc agcttggagt ccatggttca 60
   t 61
<210> 250
   <211> 61
   <212> DNA
   <213> Homo Sapiens
<400> 250
   ttaacgtaca gattgactgc atgatgcact yagatttcaa aatgctaaat cttggtggga 60
   a 61
<210> 251
   <211> 61
   <212> DNA
   <213> Homo Sapiens
<400> 251
   aggtagattg tggtaagcta aagatgtact kataaaccct tgaaacaccc actaacaaag 60
   a 61
<210> 252
   <211> 61
   <212> DNA
   <213> Homo Sapiens
<400> 252
   cacatgtagc tgctacgaga cagacgctct ygttccggat cagagcagca gacagaaccg 60
   g 61
<210> 253
   <211> 61
   <212> DNA
   <213> Homo Sapiens
<400> 253
   gctatcacca aaatcgagag gaagcgtttc yatcactcca cgagcttctt tgtggcccct 60
   c 61
<210> 254
   <211> 61
   <212> DNA
   <213> Homo Sapiens
<400> 254
   agcactctga agcctgtgcc ggctcaaggc kgtttatgga tttagggtgc cctgccataa 60
   a 61
<210> 255
   <211> 61
   <212> DNA
   <213> Homo Sapiens
<400> 255
   gggccacttt aaagagcatc ttgggttcta yggctttcaa agaaaagcaa agcaaccttg 60
   a 61
<210> 256
   <211> 61
   <212> DNA
   <213> Homo Sapiens
<400> 256
   gtgtgactaa tgcgaaagct atgcaataaa yaaccatctt cagagttgct ctccttgtag 60
   g 61
<210> 257
   <211> 61
   <212> DNA
   <213> Homo Sapiens
<400> 257
   aataacgcat tacctgtatg ttatacctat wtgccaacct tagaatcatg gttttctcat 60
   t 61
<210> 258
   <211> 61
   <212> DNA
   <213> Homo Sapiens
<400> 258
   aatacaaatt ttactaaata gattgtttta yagtgaagga agggaaatct gataatttaa 60
   t 61
<210> 259
   <211> 61
   <212> DNA
   <213> Homo Sapiens
<400> 259
   catctaaatg cagtcttaca taatgcaagg maattgttat aaccaaaaaa tgtactgcaa 60
   a 61
<210> 260
   <211> 61
   <212> DNA
   <213> Homo Sapiens
<400> 260
   ttgcctatat tcccaaagga ttaaaataaa kctaagtaca tttccccaaa gtttgaaacg 60
   g 61
<210> 261
   <211> 61
   <212> DNA
   <213> Homo Sapiens
<400> 261
   cttagttttt gagtcccgag ttaaatctca rtagaggtgg tgaaggaaat ttagcattgt 60
   c 61
<210> 262
   <211> 61
   <212> DNA
   <213> Homo Sapiens
<400> 262
   ctgctagaac acacgacatg aaattaggaa ygacagtaag gctagaatct gtatcacgaa 60
   g 61
<210> 263
   <211> 61
   <212> DNA
   <213> Homo Sapiens
<400> 263
   ggaacgacag taaggctaga atctgtatca ygaaggaaac atacttaatg acattaggct 60
   a 61
<210> 264
   <211> 61
   <212> DNA
   <213> Homo Sapiens
<400> 264
   agtatcaata atggtgacct tattctttac rtagcaaatt gtccttttgt ctctccaaaa 60
   a 61
<210> 265
   <211> 61
   <212> DNA
   <213> Homo Sapiens
<400> 265
   cacaaaatat gtgtcaattc cacaatccac maagagtttt ttctcttcac caaactcctc 60
   c 61
<210> 266
   <211> 61
   <212> DNA
   <213> Homo Sapiens
<400> 266
   gaacaactct catgtataca actgaaaaat yacaaaggtg tattcactta acacaaagtg 60
   a 61
<210> 267
   <211> 61
   <212> DNA
   <213> Homo Sapiens
<400> 267
   gctaaatttg ctcttctttt caatgaattg raatgcacct ccttatggcc aactaggact 60
   t 61
<210> 268
   <211> 61
   <212> DNA
   <213> Homo Sapiens
<400> 268
   tgcatcttgt tctccactct acagccagaa ygagctttct aaagggcaat gcaatcctcc 60
   t 61
<210> 269
   <211> 61
   <212> DNA
   <213> Homo Sapiens
<400> 269
   gccaatagtc ccagtgcttt gataggccaa ygttggggga ttacttgagg ctagcctgag 60
   c 61
<210> 270
   <211> 61
   <212> DNA
   <213> Homo Sapiens
<400> 270
   aaggaagagg gtgccaggcc aagtgtataa ygagggcaag ggccctgtgg cagggctgcg 60
   c 61
<210> 271
   <211> 61
   <212> DNA
   <213> Homo Sapiens
<400> 271
   agaacctgca aaagtacaca tcaacagctc kttcaaccag caggtggtat gtgcattcaa 60
   t 61
<210> 272
   <211> 61
   <212> DNA
   <213> Homo Sapiens
<400> 272
   accccaaaaa gctcagtgac tgtctcaaaa staatctatc catctgatgt ttaaaaaata 60
   c 61
<210> 273
   <211> 61
   <212> DNA
   <213> Homo Sapiens
<400> 273
   gagaaggtaa ggagcctcaa gcgtgtatgg yggggggaat tgaaattttg aataaggtgg 60
   g 61
<210> 274
   <211> 61
   <212> DNA
   <213> Homo Sapiens
<400> 274
   aatcaggatg acttcagtgt tggccacatg bttatgggtg gatggccttg ggtacatcta 60
   a 61
<210> 275
   <211> 61
   <212> DNA
   <213> Homo Sapiens
<400> 275
   tgatatattt gaagaaagta ataaggccaa wgtgtttgaa atacagagaa tgaggcagca 60
   c 61
<210> 276
   <211> 61
   <212> DNA
   <213> Homo Sapiens
<400> 276
   ttcctccagt gcacctactc attgtcacgg yggatcatgt actggatcag cacagaaaga 60
   t 61
<210> 277
   <211> 61
   <212> DNA
   <213> Homo Sapiens
<400> 277
   ctgccaccca cacttacccc ttgccaccga kgcatagagg catagtccag gcaaacgttg 60
   c 61
<210> 278
   <211> 61
   <212> DNA
   <213> Homo Sapiens
<400> 278
   atgagaatgg tgctagcttc aggccaaggt rtaggagaca ttgagcatcc tatgtctgaa 60
   t 61
<210> 279
   <211> 61
   <212> DNA
   <213> Homo Sapiens
<400> 279
   tcactgtgat ttaatattaa ttaaggtagg rtttccacac caccagaaca cctatcccac 60
   t 61
<210> 280
   <211> 61
   <212> DNA
   <213> Homo Sapiens
<400> 280
   taaaacatcg gcactattaa tctttcacac rctttgttaa aagcttacac acaaatatac 60
   c 61
<210> 281
   <211> 61
   <212> DNA
   <213> Homo Sapiens
<400> 281
   agaatgagtt atatcctcaa ttggtgacaa wctttctctt ttttttttct acttctattg 60
   t 61
<210> 282
   <211> 61
   <212> DNA
   <213> Homo Sapiens
<400> 282
   tacatatacc ttcaaggatt tcagtgaagg rcttctttct aactttcctc cttaagataa 60
   c 61
<210> 283
   <211> 61
   <212> DNA
   <213> Homo Sapiens
<400> 283
   aagctccatg acccaagcat aattctttgg wtttctatgt taaccctagc acagaaagat 60
   g 61
<210> 284
   <211> 61
   <212> DNA
   <213> Homo Sapiens
<400> 284
   aagagttgac aacctactag agtcagtcaa ygcagttatg ttagagaagg taaaggcgca 60
   a 61
<210> 285
   <211> 61
   <212> DNA
   <213> Homo Sapiens
<400> 285
   gccttctttc cctttgttca tgaaacagag raaacaccca tcactggtaa ttaatggtag 60
   c 61
<210> 286
   <211> 61
   <212> DNA
   <213> Homo Sapiens
<400> 286
   agaattgtat ctcattggac aaaagggata ygattgtgtc tcttctgtgc tgccacatgc 60
   c 61
<210> 287
   <211> 61
   <212> DNA
   <213> Homo Sapiens
<400> 287
   agaggaagtc actggaagag aaatgaaaac mcagaggtag aggagaacac acatctattt 60
   t 61
<210> 288
   <211> 61
   <212> DNA
   <213> Homo Sapiens
<400> 288
   agtatttctc aagcactttt aagcaaaggt ragtattcat acaagaaatt tagggggaaa 60
   a 61
<210> 289
   <211> 61
   <212> DNA
   <213> Homo Sapiens
<400> 289
   cattctaaac cttatatgag tctacttgat ygaaaggtca attattccat gttctgaaat 60
   g 61
<210> 290
   <211> 61
   <212> DNA
   <213> Homo Sapiens
<400> 290
   tcgctgaacg aatgatctca accccacgtg wctctctagc ttcatctctt gcctaacagc 60
   a 61
<210> 291
   <211> 61
   <212> DNA
   <213> Homo Sapiens
<400> 291
   aagataagac aatgaaagag aagtcgaagt saggcccggc acggtggctc acgcctgtaa 60
   a 61
<210> 292
   <211> 61
   <212> DNA
   <213> Homo Sapiens
<400> 292
   atctatctgt tcaattacct cttcattttt yaaactcatt ccatatcatt tccactcatt 60
   t 61
<210> 293
   <211> 61
   <212> DNA
   <213> Homo Sapiens
<400> 293
   ataggaagac ctaactgtcc aatctgcata yaaaatggaa ttccagtttg ttaggggaag 60
   g 61
<210> 294
   <211> 61
   <212> DNA
   <213> Homo Sapiens
<400> 294
   tcttcccctc atccccaaga tgcaacattt ytagtgatac caatttgcag gtatttcatc 60
   g 61
<210> 295
   <211> 61
   <212> DNA
   <213> Homo Sapiens
<400> 295
   tctgtatgta ctgaggaaga atgaggcata ygatacacag aaagaaagca gcagagtaca 60
   t 61
<210> 296
   <211> 61
   <212> DNA
   <213> Homo Sapiens
<400> 296
   ggaaatttca agtaatgaaa agcctttatt mtgggtttca aatatggtca cctaaactaa 60
   t 61
<210> 297
   <211> 61
   <212> DNA
   <213> Homo Sapiens
<400> 297
   catactgttg gtaggaatgt aaattgatac ygtctgtctt gtggatagtt tggcagtatt 60
   g 61
<210> 298
   <211> 61
   <212> DNA
   <213> Homo Sapiens
<400> 298
   cctatgcatc caaataactc acatcacaca rtggcttctt tggatgaatt aaaagtatct 60
   a 61
<210> 299
   <211> 61
   <212> DNA
   <213> Homo Sapiens
<400> 299
   atcaaaaata aacaaaacta gttttgctct magtaccacc cagacttcaa tgattccata 60
   c 61
<210> 300
   <211> 61
   <212> DNA
   <213> Homo Sapiens
<400> 300
   agaaagatat gtgctattta agtgtcagag staggattgt ttaggccagt gatttttttt 60
   t 61
<210> 301
   <211> 61
   <212> DNA
   <213> Homo Sapiens
<400> 301
   tctggagcgt gcagatgaag caggcagtca sggtaatgaa gtcggaaatg caagtccaga 60
   g 61
<210> 302
   <211> 61
   <212> DNA
   <213> Homo Sapiens
<400> 302
   gcagtttctc tcagttggta tgctaaattt crggccttgt ggcactctga tcaccttctc 60
   a 61
<210> 303
   <211> 61
   <212> DNA
   <213> Homo Sapiens
<400> 303
   gaaacccaaa gtgtttgctg taatgaccag mgagtgtcag tgaaactcct tagcctaagg 60
   a 61
<210> 304
   <211> 61
   <212> DNA
   <213> Homo Sapiens
<400> 304
   ttatttatat atttggctcc ccctagtgga mtgtaagcat attgacaata aaggatcatg 60
   t 61
<210> 305
   <211> 61
   <212> DNA
   <213> Homo Sapiens
<400> 305
   aaatcatgca gttagtgtgt agcagaagca rgtaatccct acagcccaca cagaatttcc 60
   a 61
<210> 306
   <211> 61
   <212> DNA
   <213> Homo Sapiens
<400> 306
   tatatgtatt gttaaaacat tttgcatatg rgttgttaaa aaaatttcat gggaaaattt 60
   a 61
<210> 307
   <211> 61
   <212> DNA
   <213> Homo Sapiens
<400> 307
   ttaataagat gaaattaagc actgcagtcc rataatgagg acaagtaggg cctaactaca 60
   t 61
<210> 308
   <211> 61
   <212> DNA
   <213> Homo Sapiens
<400> 308
   atcacagtga aaagtgatta tagggttcat rctatcagca ggtaacttgg tgaattctac 60
   a 61
<210> 309
   <211> 61
   <212> DNA
   <213> Homo Sapiens
<400> 309
   ctgtgaagga gatcaccagg gcttggctag rtcacagctc tgtggctgca tctaggtggg 60
   g 61
<210> 310
   <211> 61
   <212> DNA
   <213> Homo Sapiens
<400> 310
   gtgatgaaaa agttctggaa acatggatag yggtgacagt tgcacaacat tgtcagtgta 60
   c 61
<210> 311
   <211> 61
   <212> DNA
   <213> Homo Sapiens
<400> 311
   cccatttagt tatgggaaga atactcatgg ygcttactga ctagattttc ctaaccgatt 60
   g 61
<210> 312
   <211> 61
   <212> DNA
   <213> Homo Sapiens
<400> 312
   atatgtgaga gagtgtaagt gcctgtacgt rtaacatcac ttctgctgta gtcatcatca 60
   g 61
<210> 313
   <211> 61
   <212> DNA
   <213> Homo Sapiens
<400> 313
   ctaaaggtat atatatagaa ttattattga ygattatcag tattatatca ctgatggata 60
   g 61
<210> 314
   <211> 61
   <212> DNA
   <213> Homo Sapiens
<400> 314
   tgggatggat gaataaagcg cttatctcca ytagtgtctc cacagtgaat attaccattg 60
   t 61
<210> 315
   <211> 61
   <212> DNA
   <213> Homo Sapiens
<400> 315
   ctttccctct gaagaagaat tagccttctc stcttccaaa tgtgactttt ctgtttgtgg 60
   a 61
<210> 316
   <211> 61
   <212> DNA
   <213> Homo Sapiens
<400> 316
   tctctcagta aacatatctc ctatgctagg yaagacaaaa ggattgagaa gacaccatga 60
   t 61
<210> 317
   <211> 61
   <212> DNA
   <213> Homo Sapiens
<400> 317
   tggcatttta taatgtcctg taggagttaa rtgtgtatgc agaaatggtc ttaaaattgt 60
   a 61
<210> 318
   <211> 61
   <212> DNA
   <213> Homo Sapiens
<400> 318
   taggaaacat aagcttggtg gattatcgaa mgacctcaaa tgggtcagga gttcgagacc 60
   a 61
<210> 319
   <211> 61
   <212> DNA
   <213> Homo Sapiens
<400> 319
   cttcagtttt ctaaattatg tttgacatca rgacagtcaa gtcagtccag gatgagaaaa 60
   g 61
<210> 320
   <211> 61
   <212> DNA
   <213> Homo Sapiens
<400> 320
   cctgtggtcc agggcatatt tgtagtgaac raaataccaa tgatagggaa aagaaccatg 60
   g 61
<210> 321
   <211> 61
   <212> DNA
   <213> Homo Sapiens
<400> 321
   ttttggggcc ctcttgccct gcatcagttt ygagtgtttg gttaagctgt atttggcccc 60
   a 61
<210> 322
   <211> 61
   <212> DNA
   <213> Homo Sapiens
<400> 322
   agttcctaaa catggtcctg ggaagagata yggtgaacta gcaatattat tcattctaac 60
   a 61
<210> 323
   <211> 61
   <212> DNA
   <213> Homo Sapiens
<400> 323
   ggagctttca taagaggttg aagtttttga yccatgacag actccagact ttccataagg 60
   t 61
<210> 324
   <211> 61
   <212> DNA
   <213> Homo Sapiens
<400> 324
   agaagtttag gttaggcatt tgaattgatt ytattgtagg agaggtcata gactgaatat 60
   t 61
<210> 325
   <211> 61
   <212> DNA
   <213> Homo Sapiens
<400> 325
   ggtagaatca aattatatgt ccttggtaca rtaaatcttc attaagttcc ttatcaagca 60
   t 61
<210> 326
   <211> 61
   <212> DNA
   <213> Homo Sapiens
<400> 326
   tgttcatctt ctcgttatct tcagaatctc rtactttgca tatatttaat ttattcttgt 60
   c 61
<210> 327
   <211> 61
   <212> DNA
   <213> Homo Sapiens
<400> 327
   aggcatatat tgctatcaat catcaatttt rggcattacc tacttatctt ttttatggta 60
   a 61
<210> 328
   <211> 61
   <212> DNA
   <213> Homo Sapiens
<400> 328
   aaacttttat cctgcactat attcacattt waaaaatttg gttgcacact ttccacaggt 60
   a 61
<210> 329
   <211> 61
   <212> DNA
   <213> Homo Sapiens
<400> 329
   atgtgtgtaa gttggagact gttgagtgac yaaaagtttg cttctctcat tgaaatagga 60
   a 61
<210> 330
   <211> 61
   <212> DNA
   <213> Homo Sapiens
<400> 330
   actgacttaa atgggagcta caaagaaaca yagacagtgc caagaagtca tcttaatagg 60
   t 61
<210> 331
   <211> 61
   <212> DNA
   <213> Homo Sapiens
<400> 331
   aaaaccctat cctctacctt acttatgcta ycttacttct ctatcctttg tgtaatttct 60
   g 61
<210> 332
   <211> 61
   <212> DNA
   <213> Homo Sapiens
<400> 332
   ctatttttta ttgcagatag cagtagttca ytttctttta ttggcctttt ttccccaaat 60
   a 61
<210> 333
   <211> 61
   <212> DNA
   <213> Homo Sapiens
<400> 333
   tgatttgtaa taatccccat ttcctttaag mtctcacccc cagagagaga cagtgaagga 60
   t 61
<210> 334
   <211> 61
   <212> DNA
   <213> Homo Sapiens
<400> 334
   ataaagtaat tgataattcc aaaagtgtca ktccaactaa ttgacccaaa agtaaatttt 60
   a 61
<210> 335
   <211> 61
   <212> DNA
   <213> Homo Sapiens

<400> 335
   gacagttaac ttacttgcag atcagcttaa sttcttgagg ctttttaaaa gctttgtcag 60
   t 61
<210> 336
   <211> 61
   <212> DNA
   <213> Homo Sapiens
<400> 336
   tcttagtgat agacatccag gctgcctctc rcttgttgtt accacaaagg aggctgcaaa 60
   g 61
<210> 337
   <211> 61
   <212> DNA
   <213> Homo Sapiens
<400> 337
   tatctgcctc caaggtacag ggagactgta wgctctgggt gacagttatt tgagtgtgat 60
   a 61
<210> 338
   <211> 61
   <212> DNA
   <213> Homo Sapiens
<400> 338
   ccaagtcaaa gatctaaaaa gattatggaa rgccaaggaa gccaatgtgg ctagcacaag 60
   a 61
<210> 339
   <211> 61
   <212> DNA
   <213> Homo Sapiens
<400> 339
   atccagagat acttctcact gttctacgaa rtcttggctc tgctgggttc tctgtggaag 60
   t 61
<210> 340
   <211> 61
   <212> DNA
   <213> Homo Sapiens
<400> 340
   gaagccatgg tgttccctta aaacctgata yggagggtga ttccccatca tgtctgtatt 60
   c 61
<210> 341
   <211> 61
   <212> DNA
   <213> Homo Sapiens
<400> 341
   cgggacaggc tcagcctaaa aaggcttcct yctaccctac ctccaggtga tgtggttatg 60
   a 61
<210> 342
   <211> 61
   <212> DNA
   <213> Homo Sapiens
<400> 342
   tggagtcatc tattatgaca tcaaccagat yatcaccact gccgtgatga ccctcagcaa 60
   a 61
<210> 343
   <211> 61
   <212> DNA
   <213> Homo Sapiens
<400> 343
   tagccgcaag gtggcatctg tgctgaacaa ygcctactac ctggacaaga tgcactacag 60
   c 61
<210> 344
   <211> 61
   <212> DNA
   <213> Homo Sapiens
<400> 344
   tgtcaggttc actagaggtg aatgcaaggg yttagagagg tgaggttgca gagggaaaag 60
   g 61
<210> 345
   <211> 61
   <212> DNA
   <213> Homo Sapiens
<400> 345
   atatggcatg tcagcaggat gaccatacag yatggcttgt ctgggacagt cccagttata 60
   c 61
<210> 346
   <211> 61
   <212> DNA
   <213> Homo Sapiens
<400> 346
   ccccgggaaa gcttctggaa caacacttta sgggctactg acaggctgct ctgcttctca 60
   c 61
<210> 347
   <211> 61
   <212> DNA
   <213> Homo Sapiens
<400> 347
   tccagcataa gtgaaaaaac agtgtttcgg rtacgctagt gtttctgaga caaaatatgt 60
   a 61
<210> 348
   <211> 61
   <212> DNA
   <213> Homo Sapiens
<400> 348
   tgcctgggct ggttgcgaac tcctggtggc ytcaattgat ctttgcactt cagcctccca 60
   a 61
<210> 349
   <211> 61
   <212> DNA
   <213> Homo Sapiens
<400> 349
   cctgaaacaa cctccctctg ggacagaaat yggcttggga actcaatcat tcaccaaaag 60
   g 61
<210> 350
   <211> 61
   <212> DNA
   <213> Homo Sapiens
<400> 350
   cataggcaaa aattggggtt tgacttatac rttgacagtt tgcattgctt ggattactaa 60
   t 61
<210> 351
   <211> 61
   <212> DNA
   <213> Homo Sapiens
<400> 351
   tttcaaaaaa tggtagctgt tattatcata ycactttcat ggaatttcgt tagttactca 60
   g 61
<210> 352
   <211> 61
   <212> DNA
   <213> Homo Sapiens
<400> 352
   aaagcatcgt gttgtacaca aattttgtca rtcataactc aataatgctg gaaaaataaa 60
   a 61
<210> 353
   <211> 61
   <212> DNA
   <213> Homo Sapiens
<400> 353
   catgagggtt gcattccctc ttcagttgcc magctttttc aggatgtgag gtcagcatat 60
   t 61
<210> 354
   <211> 61
   <212> DNA
   <213> Homo Sapiens
<400> 354
   ctacatttca tataaatgga atgatacaat rtatggtcct ttgtcaatgg cttcacttag 60
   c 61
<210> 355
   <211> 61
   <212> DNA
   <213> Homo Sapiens
<400> 355
   agtgtgttct aaataacatc tcacactgag racaacattc tgcttattcc acttcagact 60
   a 61
<210> 356
   <211> 61
   <212> DNA
   <213> Homo Sapiens
<400> 356
   gggttttaaa ttgagatttt gaaatctgaa rgcaatcatt aaggtcagat gtggtcttta 60
   g 61
<210> 357
   <211> 61
   <212> DNA
   <213> Homo Sapiens
<400> 357
   ttcagtaatc aattctactt tttgtaagta rattgcagtt taagtctttg aatttgtcta 60
   t 61
<210> 358
   <211> 61
   <212> DNA
   <213> Homo Sapiens
<400> 358
   agcaaataat atacattcat acagttgaac kccacatagc ttttttaaaa aaaataaatc 60
   a 61
<210> 359
   <211> 61
   <212> DNA
   <213> Homo Sapiens
<400> 359
   gcatgtgccc tcttagccgg attcaaacca stgttgccta cattttcgtg gccatagcag 60
   g 61
<210> 360
   <211> 61
   <212> DNA
   <213> Homo Sapiens
<400> 360
   acctaaaaga ctgtgttatc agaggaagag rtcccaaatt tggagtaaag atgggagaaa 60
   a 61
<210> 361
   <211> 61
   <212> DNA
   <213> Homo Sapiens
<400> 361
   tgctcccagc caattctttc tctaaatcca yttcttccca ttcctattct aatcttctca 60
   t 61
<210> 362
   <211> 61
   <212> DNA
   <213> Homo Sapiens
<400> 362
   gaacatatgt attctcttca cataggcaat matgatacat cttatcctag ggagacaata 60
   t 61
<210> 363
   <211> 61
   <212> DNA
   <213> Homo Sapiens
<400> 363
   gaatctttag attgctttgg atcgtacaga yattttaaca acattaattc ttcctatcca 60
   t 61
<210> 364
   <211> 61
   <212> DNA
   <213> Homo Sapiens
<400> 364
   ttaagtcgct tccagtctgg gctatttcca ytggtagttt tacgtttttc tggtcacata 60
   a 61
<210> 365
   <211> 61
   <212> DNA
   <213> Homo Sapiens
<400> 365
   attcacttcc ctatgatact gagtttaggc wgcaaatcaa aggaagggct ggctgatgat 60
   t 61
<210> 366
   <211> 61
   <212> DNA
   <213> Homo Sapiens
<400> 366
   tctgacattc ttcctttctt ttcatactgc ygagtttctg acctatagca atctccttct 60
   g 61
<210> 367
   <211> 61
   <212> DNA
   <213> Homo Sapiens
<400> 367
   aattcacact aaaaatgact agatgaaaga ygtggtacta gaggctttgt atatgcctag 60
   a 61
<210> 368
   <211> 61
   <212> DNA
   <213> Homo Sapiens
<400> 368
   tagcacaaaa caatgcaggt tgacccagct wtgtcatttg gtaaaatatc tcccaggcaa 60
   t 61
<210> 369
   <211> 61
   <212> DNA
   <213> Homo Sapiens
<400> 369
   cagtggatga ttcctttcct taacacatgc rcagttcagg aaattaatca atatggagta 60
   g 61
<210> 370
   <211> 61
   <212> DNA
   <213> Homo Sapiens
<400> 370
   acctctcaga ggggagccac aggaaggtta ragcagaaaa aggactggac ttacttacat 60
   t 61
<210> 371
   <211> 61
   <212> DNA
   <213> Homo Sapiens
<400> 371
   tgaagtccat cccgatctga aggttcaccc rtcactaaga gacaaatggt gcttctgtta 60
   a 61
<210> 372
   <211> 61
   <212> DNA
   <213> Homo Sapiens
<400> 372
   ttctaagggc tctgcttctg agaatataat saatgccttt caaggtccct ctgccctccc 60
   t 61
<210> 373
   <211> 61
   <212> DNA
   <213> Homo Sapiens
<400> 373
   gtttcttgat cttgtgtcat ggctgtgttt rtagcagcac tgcagggttg gccagttatg 60
   g 61
<210> 374
   <211> 61
   <212> DNA
   <213> Homo Sapiens
<400> 374
   tatctctccc caggactgga ctctgagccc rtcattgaaa tgagagacca cgaggacggg 60
   g 61
<210> 375
   <211> 61
   <212> DNA
   <213> Homo Sapiens
<400> 375
   atcttccttg gattatcaaa tgtgagtctg ycattgattc ccattgagac cctccctgat 60
   a 61
<210> 376
   <211> 61
   <212> DNA
   <213> Homo Sapiens
<400> 376
   ctgagtatct tactacatat tttacaaaag rctcattgtt tcacagtgtg ttattagaat 60
   t 61
<210> 377
   <211> 61
   <212> DNA
   <213> Homo Sapiens
<400> 377
   aagatgaaag aactaaggta tgaggtgctc wagtaacttt ccaaaggtga ctcagctagg 60
   a 61
<210> 378
   <211> 61
   <212> DNA
   <213> Homo Sapiens
<400> 378
   tgcattagtg agtcctgagc actgctggat yattacttca gagcatcctg acatgaagga 60
   a 61
<210> 379
   <211> 61
   <212> DNA
   <213> Homo Sapiens
<400> 379
   tcttactgtt tgagtcagaa gcccaaggta kcttgctatt tttcacactg gcacactgga 60
   g 61
<210> 380
   <211> 61
   <212> DNA
   <213> Homo Sapiens
<400> 380
   tgtttttgca aactttcagt catataaaaa mcgtaaattt cacttttccc ccattttctt 60
   g 61
<210> 381
   <211> 61
   <212> DNA
   <213> Homo Sapiens
<400> 381
   acctcaaggg ggccttggtc ctgctccatc ygaagagctg attcattaga cttttgcagt 60
   c 61
<210> 382
   <211> 61
   <212> DNA
   <213> Homo Sapiens
<400> 382
   aaactctggc aaaatcagga ataatataga yggtgaacat ttgactccta acaattgctg 60
   a 61
<210> 383
   <211> 61
   <212> DNA
   <213> Homo Sapiens
<400> 383
   ttgagattac aatcctgaag ttatcactta yactttactc tgagctagat ggggttccaa 60
   a 61
<210> 384
   <211> 61
   <212> DNA
   <213> Homo Sapiens
<400> 384
   gaatgtataa gaaagctggg acaaaaaagc rgatctccat atctgaggac tgtgataaaa 60
   a 61
<210> 385
   <211> 61
   <212> DNA
   <213> Homo Sapiens
<400> 385
   tttgaatcag aaagagagat gacttccaac rgagctgaca aacactgcaa cagcaagcaa 60
   t 61
<210> 386
   <211> 61
   <212> DNA
   <213> Homo Sapiens
<400> 386
   tgaagaattc tgtggaaaag cagaccttta rctgcatctt tttcttccgc agatgtttgg 60
   t 61
<210> 387
   <211> 61
   <212> DNA
   <213> Homo Sapiens
<400> 387
   ctgtcgtaag ttaaaaatgc atggtaaaca yctatgggta accactcaac agaatgaata 60
   a 61
<210> 388
   <211> 61
   <212> DNA
   <213> Homo Sapiens
<400> 388
   actctgcatt aagcaactct attggtacca yttttacaac agcacgtgtt cactatgtgt 60
   t 61
<210> 389
   <211> 61
   <212> DNA
   <213> Homo Sapiens
<400> 389
   acttgctaaa gcagctgtga atttatgcca magaattaaa taaacaaaaa taccaaagac 60
   t 61
<210> 390
   <211> 61
   <212> DNA
   <213> Homo Sapiens
<400> 390
   tcttctgtgt cttagcattc aatggagtag waatgctaag tataaaaaga aaaatggccg 60
   g 61
<210> 391
   <211> 61
   <212> DNA
   <213> Homo Sapiens
<400> 391
   tgggccatac actaatttac agaacctctc ycctactgac aggcatccag atgattcctt 60
   g 61
<210> 392
   <211> 61
   <212> DNA
   <213> Homo Sapiens
<400> 392
   cataaaggct atgactaatg gattgaataa stgatggaca agctgctcac tggggacaga 60
   c 61
<210> 393
   <211> 61
   <212> DNA
   <213> Homo Sapiens
<400> 393
   acattcataa tggggaaaag gatccagata yagtaaagca aaaacaaaat ccagtttgca 60
   a 61
<210> 394
   <211> 61
   <212> DNA
   <213> Homo Sapiens
<400> 394
   agaatttgaa ccacatctcc aactctattg mctatcattc ttaaaatacc ccatgcagtc 60
   t 61
<210> 395
   <211> 61
   <212> DNA
   <213> Homo Sapiens
<400> 395
   catctgttat cacctaatcc cagaagcgac rtaccatccc ttttgcttta ttctgttggt 60
   c 61
<210> 396
   <211> 61
   <212> DNA
   <213> Homo Sapiens
<400> 396
   tctagaagta accttctaga tgaaaccttc ygtaacctag aagtaacctt ctgctacaga 60
   a 61
<210> 397
   <211> 61
   <212> DNA
   <213> Homo Sapiens
<400> 397
   ccttttcttt ccacaacatg cctgttcatt yggttcatct ggagttctca tttagttcac 60
   t 61
<210> 398
   <211> 61
   <212> DNA
   <213> Homo Sapiens
<400> 398
   ctaaatatat acactgaaaa tttgtgtaaa sagccatttc ggctaacact ggtaagagaa 60
   a 61
<210> 399
   <211> 61
   <212> DNA
   <213> Homo Sapiens
<400> 399
   aagaaaaacc aaattaagtg tcacataaac kttgtaatta atgtaaccat tgactcactg 60
   a 61
<210> 400
   <211> 61
   <212> DNA
   <213> Homo Sapiens
<400> 400
   ttctaggctt aagataccca ttactgtgtc rtaagccaaa tatctgatac ttatgtccaa 60
   a 61
<210> 401
   <211> 61
   <212> DNA
   <213> Homo Sapiens
<400> 401
   aggtgatgtg ataccgcacc ctgtagatat ytatgcctgt caagtattca taagcactct 60
   t 61
<210> 402
   <211> 61
   <212> DNA
   <213> Homo Sapiens
<400> 402
   aatacagctg atggaggcgt caacgacttc wgtaaatatt gaagtattat ttcttctgcc 60
   a 61
<210> 403
   <211> 61
   <212> DNA
   <213> Homo Sapiens
<400> 403
   tgcaccaaaa gcatcctgac caattcagga rtgctaggcc cttaacatgg atagctctgc 60
   c 61
<210> 404
   <211> 61
   <212> DNA
   <213> Homo Sapiens
<400> 404
   gatcacatat tgtgattcca cttacatgaa raacctagat aagcaaattt atagaaccag 60
   a 61
<210> 405
   <211> 61
   <212> DNA
   <213> Homo Sapiens
<400> 405
   tggaactttg ctcatgtctg cgtcagtgga rcctagacct ggaatctcaa ttgtgtgatt 60
   t 61
<210> 406
   <211> 61
   <212> DNA
   <213> Homo Sapiens
<400> 406
   tatgaagaaa acctgaaaat tctgccaaag rtatccaaac gcgcaccctc tgtcctttgc 60
   a 61
<210> 407
   <211> 61
   <212> DNA
   <213> Homo Sapiens
<400> 407
   tctacatcta ggctctattc tgtcctattg ratgtactgt ttatcccttt gccaataata      60
   t                                                                      61
<210> 408
   <211> 61
   <212> DNA
   <213> Homo Sapiens
<400> 408
   gaaggataaa caattctccc tcttaactac ygaaaaggag acatatgact tggggcatta      60
   g                                                                      61
<210> 409
   <211> 61
   <212> DNA
   <213> Homo Sapiens
<400> 409
   tctattgtaa attgaccaga acaggaatac rtttgtgaat tcgtaacgta agaaggtagc      60
   a                                                                      61
<210> 410
   <211> 61
   <212> DNA
   <213> Homo Sapiens
<400> 410
   taggacttcc agtactaagt tgaataaaag yggtctctcg tcttctcttg ccatcttcct      60
   t                                                                      61
<210> 411
   <211> 61
   <212> DNA
   <213> Homo Sapiens
<400> 411
   ctgtgtatta catatagaag tactgtatat rgtgaggaat gccatcctgt gtgtgttgtt      60
   a                                                                      61
<210> 412
   <211> 61
   <212> DNA
   <213> Homo Sapiens
<400> 412
   tgcatggagg ccattattac agcagaacca rtggtggagg gcatagaggc caggctgctc 60
   t 61
<210> 413
   <211> 61
   <212> DNA
   <213> Homo Sapiens
<400> 413
   gcagtcacct acagctgttg gagcttctca rtttatgctc ccactatctc atccaggtat 60
   g 61
<210> 414
   <211> 61
   <212> DNA
   <213> Homo Sapiens
<400> 414
   tcctccctgt cctcacactt gaacttcatg rtccaatcac ttcaataatt atcttgtcct 60
   c 61
<210> 415
   <211> 61
   <212> DNA
   <213> Homo Sapiens
<400> 415
   gaaaaaaaac caagccccat ttccttaggc rtctagcatt ctaacacatc actctctctt 60
   t 61
<210> 416
   <211> 61
   <212> DNA
   <213> Homo Sapiens
<400> 416
   ccagggaatg gacccatgca atctgagtga yggatgaaga aaactgcttt cagtcctaga 60
   t 61
<210> 417
   <211> 61
   <212> DNA
   <213> Homo Sapiens
<400> 417
   tgagatcata cataaagagc ttggcccagc ygaggagccg tcacctaacc gtgctcagga 60
   g 61
<210> 418
   <211> 61
   <212> DNA
   <213> Homo Sapiens
<400> 418
   agaaagaaaa ggatcacagg ggtctcagaa wtgaaaacag gcctctgtaa aaaggcagaa 60
   t 61
<210> 419
   <211> 61
   <212> DNA
   <213> Homo Sapiens
<400> 419
   acagcttcaa caaagaacat acagtgaaaa ygacttccaa ggttagatca taaaaatacc 60
   a 61
<210> 420
   <211> 61
   <212> DNA
   <213> Homo Sapiens
<400> 420
   aacaattaca atattgatat caaagaacaa yggtcacaga tcaccgaaac agacatacca 60
   a 61
<210> 421
   <211> 61
   <212> DNA
   <213> Homo Sapiens
<400> 421
   cccttacatt ttacaccaga ggtaagtgtc ytgctccttt catactaatc cttgccctat 60
   t 61
<210> 422
   <211> 61
   <212> DNA
   <213> Homo Sapiens
<400> 422
   ttgttagaag acaaccagta tcctacaagg magtaatgct ggtagcaacc gatctttaaa 60
   t 61
<210> 423
   <211> 61
   <212> DNA
   <213> Homo Sapiens
<400> 423
   ttctttttca ggatccaatt ctgtatcgaa raacttgacc tttaaactag ttaaaagaat 60
   c 61
<210> 424
   <211> 61
   <212> DNA
   <213> Homo Sapiens
<400> 424
   aagcccagtt tcagaattac tgttaactcc raattgccaa ttacagcttg actttgcagt 60
   t 61
<210> 425
   <211> 61
   <212> DNA
   <213> Homo Sapiens
<400> 425
   acttcaagtt gttgcagaag ggatgatgag rttaatggag aacagaaaga aggtgaacta 60
   g 61
<210> 426
   <211> 61
   <212> DNA
   <213> Homo Sapiens
<400> 426
   ctaatatacc agagagataa tggtaactaa kctcttaaaa acacatagag caaccttctg 60
   t 61
<210> 427
   <211> 61
   <212> DNA
   <213> Homo Sapiens
<400> 427
   gcagatagct cctgaagcct gcaggctagc rgggagctca gttacacaag atgaaggggg 60
   t 61
<210> 428
   <211> 61
   <212> DNA
   <213> Homo Sapiens
<400> 428
   ttggaaatca tgtatttgca atggtgttct yaaaaattag aatgttaaag agagccaaaa 60
   t 61
<210> 429
   <211> 61
   <212> DNA
   <213> Homo Sapiens
<400> 429
   aggaataaaa atgaagcaaa taaatgtggt ycatctttct tcaggacctg atgctcctgc 60
   a 61
<210> 430
   <211> 61
   <212> DNA
   <213> Homo Sapiens
<400> 430
   agtgccatgt tctagaggta caggctccaa raagtccaca ttcaaacatg gttttccagg 60
   a 61
<210> 431
   <211> 61
   <212> DNA
   <213> Homo Sapiens
<400> 431
   atgaccttgg tttagcggtc agcaggtagc raggaaagtg atctcaaggg ccagaaagat 60
   a 61
<210> 432
   <211> 61
   <212> DNA
   <213> Homo Sapiens
<400> 432
   aacccgtgat ctccacggca tttctctttg ytgtgatgag gccacacgtg tattacaatg 60
   a 61
<210> 433
   <211> 61
   <212> DNA
   <213> Homo Sapiens
<400> 433
   aactggagag gctctttcct acctgataag scaacagctc cccggaggta gaaatctttg 60
   t 61
<210> 434
   <211> 61
   <212> DNA
   <213> Homo Sapiens
<400> 434
   ggttaggttt aggtgaattt tcattcttcc rttttcctaa ttgctaccat aatcacatct 60
   t 61
<210> 435
   <211> 61
   <212> DNA
   <213> Homo Sapiens
<400> 435
   atattttaat agatgatagc aaagtaaata yggtattcat cacctgaagc atttatcctt 60
   t 61
<210> 436
   <211> 61
   <212> DNA
   <213> Homo Sapiens
<400> 436
   caccagaaca ataaaagcct caagcaatct yatctatgtg tcacaacaaa cccaacactc 60
   a 61
<210> 437
   <211> 61
   <212> DNA
   <213> Homo Sapiens
<400> 437
   aacagaacaa tgtaaatttg gctgtttgac rttaacttaa tttgaagtgt taacttttcc 60
   c 61
<210> 438
   <211> 61
   <212> DNA
   <213> Homo Sapiens
<400> 438
   tatactctag aaaattacgg ttctgttgaa ytggcatgct tttgttaatg cagattgatg 60
   c 61
<210> 439
   <211> 61
   <212> DNA
   <213> Homo Sapiens
<400> 439
   ctgaaacagg agcttgaagc cttgttctca ygtctaaagc catccaaata taggggccac 60
   t 61
<210> 440
   <211> 61
   <212> DNA
   <213> Homo Sapiens
<400> 440
   aaaaactctt ttttttccta ttagaacatc raatacttct acagaatatt tctattctat 60
   g 61
<210> 441
   <211> 61
   <212> DNA
   <213> Homo Sapiens
<400> 441
   tgaaatatct ttttatctca tactaaaaat rcacgttttg ttcaggtaca ggattgctat 60
   g 61
<210> 442
   <211> 61
   <212> DNA
   <213> Homo Sapiens
<400> 442
   gggataatta atacatggca gtcggggaga raggagggaa aaggaaacga agacgggaga 60
   g 61
<210> 443
   <211> 61
   <212> DNA
   <213> Homo Sapiens
<400> 443
   gctgtttgat agtgtgtcca gctccatgtc rggtgcaatg ggtgctttgt aaatgctcac 60
   a 61
<210> 444
   <211> 61
   <212> DNA
   <213> Homo Sapiens
<400> 444
   gcgcccacta tagactttca tgggaagagt yggcctcgtg gtgtcagagg ggagtccaaa 60
   t 61
<210> 445
   <211> 61
   <212> DNA
   <213> Homo Sapiens
<400> 445
   tgctgaccgc cacggaactg gcagccaaga mataaggcaa tcggaagagt cagactgtcc 60
   g 61
<210> 446
   <211> 61
   <212> DNA
   <213> Homo Sapiens
<400> 446
   tttttatggt cggacttcag aagcatctcc rttaaagctg ggaccaaggc cagctgtctc 60
   c 61
<210> 447
   <211> 61
   <212> DNA
   <213> Homo Sapiens
<400> 447
   attggatgct gttctgtgta ctttacagat rttaactcaa ttaatattca caacacattg 60
   g 61
<210> 448
   <211> 61
   <212> DNA
   <213> Homo Sapiens
<400> 448
   tctctgggtg ttctttgaat atcctgtgaa ygcctctgac tttatgcccc caccttgttt 60
   a 61
<210> 449
   <211> 61
   <212> DNA
   <213> Homo Sapiens
<400> 449
   aaacaaagaa aagacttcca agccttcaat rttctaagtt gccatggcag cagcaccatc 60
   a 61
<210> 450
   <211> 61
   <212> DNA
   <213> Homo Sapiens
<400> 450
   tgcaagatac atcttggcta gaacaactga ygaaattttt atgtaacacc ttcaaatgag 60
   g 61
<210> 451
   <211> 61
   <212> DNA
   <213> Homo Sapiens
<400> 451
   tacaattctc atctctccaa gttggagggt stttaatctg tgctgaagat gaggaggcag 60
   a 61
<210> 452
   <211> 61
   <212> DNA
   <213> Homo Sapiens
<400> 452
   gtgggacctg attcaccagt gaatgaggag ygtctgaaag ctgcagttca gggagagaaa 60
   a 61
<210> 453
   <211> 61
   <212> DNA
   <213> Homo Sapiens
<400> 453
   aaatttaagt gtataaaaag ttaggtattt scttgtagaa acttgaaact acaattttgt 60
   g 61
<210> 454
   <211> 61
   <212> DNA
   <213> Homo Sapiens
<400> 454
   atagaactat aataaatatt tctcaagcta yacaaaataa cgaaaagaaa acataagagt 60
   c 61
<210> 455
   <211> 61
   <212> DNA
   <213> Homo Sapiens
<400> 455
   ttatcatata aatcctaaac atttcaagtg yacatctact tccagggatc ataaacataa 60
   a 61
<210> 456
   <211> 61
   <212> DNA
   <213> Homo Sapiens
<400> 456
   aaaagagact accttttcct tgttcatgaa mgctgtttca aaatgaaatg actacattat 60
   t 61
<210> 457
   <211> 61
   <212> DNA
   <213> Homo Sapiens
<400> 457
   ggtgaatgaa tgaacttgaa aacaggtcat ytgaaattct ccgatcataa aagcaaaaag 60
   a 61
<210> 458
   <211> 61
   <212> DNA
   <213> Homo Sapiens
<400> 458
   ttaatttgtt taacgttatg gagaattagg ytcataatgt gacagtgata gtcctgaaga 60
   a 61
<210> 459
   <211> 61
   <212> DNA
   <213> Homo Sapiens
<400> 459
   ggtgctttac tactccagct tctttgaatg rattagagat aatatgggca gaatacagtc 60
   t 61
<210> 460
   <211> 61
   <212> DNA
   <213> Homo Sapiens
<400> 460
   gttgaaccgt aaacttcgtg tgcgttccta sacgattcaa tctttctaga cttcacttat 60
   a 61
<210> 461
   <211> 61
   <212> DNA
   <213> Homo Sapiens
<400> 461
   ttggattttg ctagtgatac ttaactaatg rttcatactg tatttgaagt gtctgctgcc 60
   a 61
<210> 462
   <211> 61
   <212> DNA
   <213> Homo Sapiens
<400> 462
   cgtttacaat gatgaaaaca ttatgaaaac rgtgattggt ggttactcaa cattgtgaat 60
   a 61
<210> 463
   <211> 61
   <212> DNA
   <213> Homo Sapiens
<400> 463
   ggtatatagg atacaataat ctgtgcattt racgacaaca gtactattta ctcatgacat 60
   g 61
<210> 464
   <211> 61
   <212> DNA
   <213> Homo Sapiens
<400> 464
   atttgtaact tggcctatca gtttgagacc yttgttctag acactttgta tactgtctca 60
   a 61
<210> 465
   <211> 61
   <212> DNA
   <213> Homo Sapiens
<400> 465
   taattaagcc acaaagcaca atgcaagttc rataatcact ggaaataata tgccttttaa 60
   g 61
<210> 466
   <211> 61
   <212> DNA
   <213> Homo Sapiens
<400> 466
   agggggctaa gaatcctcct ataattcagc ycataaaggt gtttaactat agaatgaaca 60
   g 61
<210> 467
   <211> 61
   <212> DNA
   <213> Homo Sapiens
<400> 467
   tgtctcctct gtcatctgcc tctggagacg ygactttata cccggcagct ttttgaactc 60
   t 61
<210> 468
   <211> 61
   <212> DNA
   <213> Homo Sapiens
<400> 468
   cctttgtgaa ttaatattaa cagataaagt rgagatccga ggcacttaca tgagtaaata 60
   a 61
<210> 469
   <211> 61
   <212> DNA
   <213> Homo Sapiens
<400> 469
   ggactattcc cactgtgaca gaaagctcta ygggacagtg ttgcctgcaa tataaaacca 60
   a 61
<210> 470
   <211> 61
   <212> DNA
   <213> Homo Sapiens
<400> 470
   tcaaaaagct tattatagtc ttagttgaga rccaagcaat tagtgcaact attcacttag 60
   t 61
<210> 471
   <211> 61
   <212> DNA
   <213> Homo Sapiens
<400> 471
   cagggtattg tgaaatcaca gaagtgttaa sagccagttc cttttttgat catatgaggg 60
   a 61
<210> 472
   <211> 61
   <212> DNA
   <213> Homo Sapiens
<400> 472
   gataaaaaca aaatgcacag caaggctcgt rtagttaact acaggttctc atgagtacag 60
   a 61
<210> 473
   <211> 61
   <212> DNA
   <213> Homo Sapiens
<400> 473
   ctcaaaccag tccagcaagc agggactata ragtgaggta gcaggaaata aggctggcaa 60
   g 61
<210> 474
   <211> 61
   <212> DNA
   <213> Homo Sapiens
<400> 474
   ctctaccttg cctacctgtt caaataaact mttcctttct caaacctctg gtccagtcag 60
   g 61
<210> 475
   <211> 61
   <212> DNA
   <213> Homo Sapiens
<400> 475
   acccttctga atctctccat ctacctctgc ytatttataa ttcacaagtc tcttggaccc 60
   c 61
<210> 476
   <211> 61
   <212> DNA
   <213> Homo Sapiens
<400> 476
   agacatgtcc acactcccca gcacacattc ygtaatgtca ccacactggc atgaggttta 60
   g 61
<210> 477
   <211> 61
   <212> DNA
   <213> Homo Sapiens
<400> 477
   agctcctggc acacggacac ccttgcttac sgttcacatt tctaaaaatg ttgcctctgt 60
   c 61
<210> 478
   <211> 61
   <212> DNA
   <213> Homo Sapiens
<400> 478
   tggatcttga gaatctgacc ctaaaattca ygtggaagaa caatgaggca aaataatcca 60
   a 61
<210> 479
   <211> 61
   <212> DNA
   <213> Homo Sapiens
<400> 479
   ctcaaagccc tgtgctggct tcctacctca stcaaattaa aagccaaaga cttctcaagg 60
   g 61
<210> 480
   <211> 61
   <212> DNA
   <213> Homo Sapiens
<400> 480
   tcaaactact gaactgcggt cttggacacc rtctttctgg aggatcattt ggcatcatgg 60
   a 61
<210> 481
   <211> 61
   <212> DNA
   <213> Homo Sapiens
<400> 481
   attatcataa cctttgattc agtaattcca rtcctaagaa tcaatggtag gaaaacattc 60
   a 61
<210> 482
   <211> 61
   <212> DNA
   <213> Homo Sapiens
<400> 482
   aagtaatttt aactttgtgg aacatgactc kgaattgggt agtagactgg ggcttagttg 60
   t 61
<210> 483
   <211> 61
   <212> DNA
   <213> Homo Sapiens
<400> 483
   tcagctagct ccttaaagtt cctagaatgt yagaagagta aaggttcctt gaagatagat 60
   a 61
<210> 484
   <211> 61
   <212> DNA
   <213> Homo Sapiens
<400> 484
   gttatctttg taatattgaa tcttttaatc ygcaaacatg gaacatttct ccatttattt 60
   a 61
<210> 485
   <211> 61
   <212> DNA
   <213> Homo Sapiens
<400> 485
   cctgttttca ctcatctctt cctttaaccc rgattccaag aaagcttgac aactgtctac 60
   a 61
<210> 486
   <211> 61
   <212> DNA
   <213> Homo Sapiens
<400> 486
   taagacagct gatcttagat gacagcatca gktgggaaaa aattggtttt gtttctctat 60
   a 61
<210> 487
   <211> 61
   <212> DNA
   <213> Homo Sapiens
<400> 487
   cctgagggtg agcttggaca ccttggcagg ktcagacaat gaacagactt caaagctcac 60
   c 61
<210> 488
   <211> 61
   <212> DNA
   <213> Homo Sapiens
<400> 488
   ccatccgtga gcatggcatg tgtttccttt rtttgcgtca tctgtgattt cttttaacag 60
   t 61
<210> 489
   <211> 61
   <212> DNA
   <213> Homo Sapiens
<400> 489
   ttccatgctc agaattctaa accagtcttg raatctcact gcatcactgc atataccttt 60
   t 61
<210> 490
   <211> 61
   <212> DNA
   <213> Homo Sapiens
<400> 490
   gaggggtcat tttgtcccta actgaggttt mcttgttccc atactgctgc tcatacatct 60
   g 61
<210> 491
   <211> 61
   <212> DNA
   <213> Homo Sapiens
<400> 491
   atactccctg agtttttaac ttaagtcaaa rtgacttcgc tacgaaaagg gtctacttca 60
   g 61
<210> 492
   <211> 61
   <212> DNA
   <213> Homo Sapiens
<400> 492
   aagcagggaa tccaaaaatt attctatgag rtatgtggta ttataaaggt ttttatacta 60
   a 61
<210> 493
   <211> 61
   <212> DNA
   <213> Homo Sapiens
<400> 493
   aatgaaaatc cagtcccaaa gggccaactt scagcaagct taccagaccc aatttgcctg 60
   t 61
<210> 494
   <211> 61
   <212> DNA
   <213> Homo Sapiens
<400> 494
   cattgtcaac agacatgagc tcttattgtc rctatttttt aagagtcaaa taaaccccac 60
   c 61
<210> 495
   <211> 61
   <212> DNA
   <213> Homo Sapiens
<400> 495
   tgttccagca caatttgttg aagatattat yccttagccc actgaattgt cttgacaccc 60
   t 61
<210> 496
   <211> 61
   <212> DNA
   <213> Homo Sapiens
<400> 496
   tgtatccata aaaaataaaa ttggaattca yagttctgaa atcaaacagg aggacactag 60
   c 61
<210> 497
   <211> 61
   <212> DNA
   <213> Homo Sapiens
<400> 497
   ataaagtgtt gagtatctca tgtaatttct scaataccgt actgaaagtg aaaaacagaa 60
   t 61
<210> 498
   <211> 61
   <212> DNA
   <213> Homo Sapiens
<400> 498
   ttgaaggcaa ctcctaaaga aatagctcca yagtgtaaaa atcatttaac gctagcacag 60
   a 61
<210> 499
   <211> 61
   <212> DNA
   <213> Homo Sapiens
<400> 499
   gaatttcgac tttgagtatt ttatatgttc wctttcagcc ttacaagcca agtcaactct 60
   t 61
<210> 500
   <211> 61
   <212> DNA
   <213> Homo Sapiens

<400> 500
   agtttgtctc caaacaaaag cagcccatgc yatctaaaaa gtaaaggcca ctaggatgaa 60
   a 61
<210> 501
   <211> 61
   <212> DNA
   <213> Homo Sapiens
<400> 501
   ctattgagtt ctgaagaata tattatgacc rtctgttttc tgtcttcttc gtggtgagaa 60
   a 61
<210> 502
   <211> 61
   <212> DNA
   <213> Homo Sapiens
<400> 502
   atattcctaa aatgttggta tttctgagta sttctgaccc gaaaagactg gaaagggcac 60
   t 61
<210> 503
   <211> 61
   <212> DNA
   <213> Homo Sapiens
<400> 503
   gttatttatt tttggagttc tgttaggtta ygaaaatggg ctaggcatgg tggctcacac 60
   t 61
<210> 504
   <211> 61
   <212> DNA
   <213> Homo Sapiens
<400> 504
   tttatattac cctgttccct aataactttc mgtggcttct tgttaccctc aaggtacaac 60
   g 61
<210> 505
   <211> 61
   <212> DNA
   <213> Homo Sapiens
<400> 505
   tcattgtatt ttcttccacc tgcccaatct ycagttctgc caggaattta taagattctt 60
   g 61
<210> 506
   <211> 61
   <212> DNA
   <213> Homo Sapiens
<400> 506
   agtgaatgtc atagtatcaa tcaaatctac saacattctg taagcatgct tgactgtcac 60
   t 61
<210> 507
   <211> 61
   <212> DNA
   <213> Homo Sapiens
<400> 507
   aaacttcaat tgttttctaa catctttcat sagtatttca cattcgataa tataaagtca 60
   t 61
<210> 508
   <211> 61
   <212> DNA
   <213> Homo Sapiens
<400> 508
   gcttaagtaa gatcataact aagaagtgaa ygataaaaag gagtcaggct tacaaagatt 60
   t 61
<210> 509
   <211> 61
   <212> DNA
   <213> Homo Sapiens
<400> 509
   tgacggtaga attaagtttg aggaagattc raggatatgg attaaacagt cattcttctt 60
   a 61
<210> 510
   <211> 61
   <212> DNA
   <213> Homo Sapiens
<400> 510
   cctcaatatc aaggtaatgt ttatagcact rtattttcac acttcttgat cagtttacct 60
   t 61
<210> 511
   <211> 61
   <212> DNA
   <213> Homo Sapiens
<400> 511
   taggtttggt attccaaatg tagaaacgaa sgctcagaag agttacttgt cagaaagcaa 60
   g 61
<210> 512
   <211> 61
   <212> DNA
   <213> Homo Sapiens
<400> 512
   tttcactaat gaagaaaagt attgcacatt kgaaatacac taactggatt gaatatgtaa 60
   t 61
<210> 513
   <211> 61
   <212> DNA
   <213> Homo Sapiens
<400> 513
   tgccattgaa taacgtgatc aaagtaaaga ygtatgttct caatttggga caaataaaac 60
   t 61
<210> 514
   <211> 61
   <212> DNA
   <213> Homo Sapiens
<400> 514
   ccctttgttt gtaaagtact tacctcatgc yatctgtgtg agtgtcctac caaacttcca 60
   a 61
<210> 515
   <211> 61
   <212> DNA
   <213> Homo Sapiens
<400> 515
   atagtagaga ataagcagaa aaaccacaat ragtgatgca cataggtctt ggcctcatgt 60
   g 61
<210> 516
   <211> 61
   <212> DNA
   <213> Homo Sapiens
<400> 516
   tatatactca gtgatcctag tcctgccttt yataagaagg tggaataatt ttatgctcat 60
   t 61
<210> 517
   <211> 61
   <212> DNA
   <213> Homo Sapiens
<400> 517
   taatactgag attgtcctag agctagacgt ragtgaatat tgagatagaa agccttatta 60
   a 61
<210> 518
   <211> 61
   <212> DNA
   <213> Homo Sapiens
<400> 518
   tttccatgag gttagggata acatcttagt yggtagcctt gtttgtattc atatagcact 60
   t 61
<210> 519
   <211> 61
   <212> DNA
   <213> Homo Sapiens
<400> 519
   ggaggcacct ttgaagatct tccagcctta staaagcatt cagatacctg cagcttcagc 60
   t 61
<210> 520
   <211> 61
   <212> DNA
   <213> Homo Sapiens
<400> 520
   catatagaat atatttatca gggacttaaa kacaatagca tttctctctt tataagtagg 60
   t 61
<210> 521
   <211> 61
   <212> DNA
   <213> Homo Sapiens
<400> 521
   agaattcctt tctgtttaat agtactctca rctgaactct attcttttta cattttacta 60
   c 61
<210> 522
   <211> 61
   <212> DNA
   <213> Homo Sapiens
<400> 522
   aataatacct gcttttctca tctcaagtgg wttcagtgat tgtttatgat aatagaactt 60
   g 61
<210> 523
   <211> 61
   <212> DNA
   <213> Homo Sapiens
<400> 523
   tcaggagaga aaaaaaatac ttgattggta yctcatgggg ctgttgtaag gaacaaatga 60
   g 61
<210> 524
   <211> 61
   <212> DNA
   <213> Homo Sapiens
<400> 524
   gttctggcag tttctgaact cttaatgata ytttgatgac atccagttag atgctgaata 60
   c 61
<210> 525
   <211> 61
   <212> DNA
   <213> Homo Sapiens
<400> 525
   aaagtgaaat agactataag agtggtcaac rtggaataac tgctgccaaa ccctttctgc 60
   t 61
<210> 526
   <211> 61
   <212> DNA
   <213> Homo Sapiens
<400> 526
   atttaatctg aattgagaaa ctattggttg rctattagga gaaagagctt agaaatgggc 60
   c 61
<210> 527
   <211> 61
   <212> DNA
   <213> Homo Sapiens
<400> 527
   cactggaatt atttttcttt tgtcaagcca rtaatctccc aggcatggtg gtgtgcatct 60
   g 61
<210> 528
   <211> 61
   <212> DNA
   <213> Homo Sapiens
<400> 528
   tcactctgct atcttctcct ggagtttaca maagcccttc agagtgtaaa caccgatgtg 60
   g 61
<210> 529
   <211> 61
   <212> DNA
   <213> Homo Sapiens
<400> 529
   aacaacttta atagaatttt gtgtctggag yagtacgtgg caagtatttg gttactggcc 60
   t 61
<210> 530
   <211> 61
   <212> DNA
   <213> Homo Sapiens
<400> 530
   gggacactac agctcccttg tatggtacaa mataggtgct ctgtgagtga gaaggtggat 60
   g 61
<210> 531
   <211> 61
   <212> DNA
   <213> Homo Sapiens
<400> 531
   tttatatttt ttactgatag gcagccaagc rtacagtcac tactttgatt tgttaagcct 60
   g 61
<210> 532
   <211> 61
   <212> DNA
   <213> Homo Sapiens
<400> 532
   taaaacctgt gctttgtaga catttactgc yttatgactg ataagaaaaa aaagcacata 60
   t 61
<210> 533
   <211> 61
   <212> DNA
   <213> Homo Sapiens
<400> 533
   acctttgatt tctaagtgaa cttcattctt ygccatccct aatttattcc ttgcaacaca 60
   c 61
<210> 534
   <211> 61
   <212> DNA
   <213> Homo Sapiens
<400> 534
   gcaatataag ctcaaagcct aaagtgtatc rttcacagaa aggaattcat ctccaatctc 60
   a 61
<210> 535
   <211> 61
   <212> DNA
   <213> Homo Sapiens
<400> 535
   atggattctc ccctagaacc ttcagaaagc racgcagctc tgctcacgca ttggttttag 60
   a 61
<210> 536
   <211> 61
   <212> DNA
   <213> Homo Sapiens
<400> 536
   tgatgaggca gtaggtgagc atcactgaga ytgtgtgagt caggccccag tcatacaaga 60
   c 61
<210> 537
   <211> 61
   <212> DNA
   <213> Homo Sapiens
<400> 537
   atttagtttt gacaagattc aaccattcac raaaccaaaa aatgagacat tttagtggct 60
   c 61
<210> 538
   <211> 61
   <212> DNA
   <213> Homo Sapiens
<400> 538
   tcacagttta agtttttact ggaaagcaaa yaagaacacc agcagtttag atagactgaa 60
   a 61
<210> 539
   <211> 61
   <212> DNA
   <213> Homo Sapiens
<400> 539
   acaagatctt aatgattttt gcaatttgta rtaccagatt tatttactgt cacttaaaag 60
   a 61
<210> 540
   <211> 61
   <212> DNA
   <213> Homo Sapiens
<400> 540
   aatgcagagg tgtatggttt ctacacagat kattttttat ggtttattac ttataaaaat 60
   g 61
<210> 541
   <211> 61
   <212> DNA
   <213> Homo Sapiens
<400> 541
   agcaagatac cagacattga tcaaagcacc rattctataa tagcccactt taataaatta 60
   c 61
<210> 542
   <211> 61
   <212> DNA
   <213> Homo Sapiens
<400> 542
   tcaatgtctt tcagtaaagt aatgcttcaa mtttcaacct acataatttg aattagaaat 60
   g 61
<210> 543
   <211> 61
   <212> DNA
   <213> Homo Sapiens
<400> 543
   gaagacagga gaagctcctc aacacaggga wagaattagt gagtaagagt cctcagggga 60
   t 61
<210> 544
   <211> 61
   <212> DNA
   <213> Homo Sapiens
<400> 544
   ttatctgcta ctcttgtctt atttcatagc mattgccata gctggagact tcattatctc 60
   a 61
<210> 545
   <211> 61
   <212> DNA
   <213> Homo Sapiens
<400> 545
   caatcactga cttaataact tctgtccatg ytacaccatc aaatgtgcac ataaatttat 60
   t 61
<210> 546
   <211> 61
   <212> DNA
   <213> Homo Sapiens
<400> 546
   ttaatgcaca ggaatatgat gccgtaacaa ygactttcct ttctatggtg ggaagagata 60
   g 61
<210> 547
   <211> 61
   <212> DNA
   <213> Homo Sapiens
<400> 547
   gctttgatcc cagccaagat aatatgttga rgttgtcttt gaatgcatgt atttgacatt 60
   t 61
<210> 548
   <211> 61
   <212> DNA
   <213> Homo Sapiens
<400> 548
   tcaatttcta gtccgtggtt ggttgatccg ytgaagtaag tcatctgagt ggcctcgacc 60
   t 61
<210> 549
   <211> 61
   <212> DNA
   <213> Homo Sapiens
<400> 549
   acctccttgg ttctggacat gattcttcta rcaatgcagg caaaattgca tgagcttttt 60
   t 61
<210> 550
   <211> 61
   <212> DNA
   <213> Homo Sapiens
<400> 550
   tgtgctttga gcacccactg gccatgcatt sctcattaag gttttgcaaa actattctct 60
   c 61
<210> 551
   <211> 61
   <212> DNA
   <213> Homo Sapiens
<400> 551
   tgccagtagc agctatccca tggtcttcac rtagttggga gactcactaa gacgaagctc 60
   a 61
<210> 552
   <211> 61
   <212> DNA
   <213> Homo Sapiens
<400> 552
   gaagttcagt gggcactagc aaatctgcac watgcaactg gaagaaactt gtatacatag 60
   c 61
<210> 553
   <211> 61
   <212> DNA
   <213> Homo Sapiens
<400> 553
   cagcatgccc aataattata agaagaattc rtggaaaagc aaatagggag ttggaatcat 60
   t 61
<210> 554
   <211> 61
   <212> DNA
   <213> Homo Sapiens
<400> 554
   tggagtttga tgcttgaaag taagaacaga yacaccacgt tattagaaaa catgtatcaa 60
   a 61
<210> 555
   <211> 61
   <212> DNA
   <213> Homo Sapiens
<400> 555
   agcattatgt gaattcttca catgttattt ycaatggtcc tccttattca atagttttaa 60
   t 61
<210> 556
   <211> 61
   <212> DNA
   <213> Homo Sapiens
<400> 556
   attatgtaat taagtcttct atatccattc rgagcaacag caaatatctc taagtcattt 60
   a 61
<210> 557
   <211> 61
   <212> DNA
   <213> Homo Sapiens
<400> 557
   gaaccttttc aggacaggta tgatgtgtta rgccaaggag ttgaggcagg atgctgaaag 60
   c 61
<210> 558
   <211> 61
   <212> DNA
   <213> Homo Sapiens
<400> 558
   atttaaatat ctaaaattgc tcaataaagc rcgaaatgtc ttgataacaa tgatctttca 60
   t 61
<210> 559
   <211> 61
   <212> DNA
   <213> Homo Sapiens
<400> 559
   cttatgttaa aataatctca tcctatctgc yttttctaaa aaattttatt tatttatttg 60
   t 61
<210> 560
   <211> 61
   <212> DNA
   <213> Homo Sapiens
<400> 560
   cagccttggc tcttgcctag tagcgggcct ktgtagaaaa tcaaaaggga aaatgggaca 60
   g 61
<210> 561
   <211> 61
   <212> DNA
   <213> Homo Sapiens
<400> 561
   gacaaactta aaaatcaagc gagatgcatt yccctagtaa atccctgttt tcctagtcct 60
   t 61
<210> 562
   <211> 61
   <212> DNA
   <213> Homo Sapiens
<400> 562
   tgaaagtaaa atatatatcc gttgctcatc raagatgtca actgctttga cttttccgct 60
   t 61
<210> 563
   <211> 61
   <212> DNA
   <213> Homo Sapiens
<400> 563
   cacagattat gcaggtggtc ctggagactg scacataatg gtgactgaga tatgaaaagc 60
   a 61
<210> 564
   <211> 61
   <212> DNA
   <213> Homo Sapiens
<400> 564
   atatgttagt gttcttccag agctaagcat ygaagatgtg cacctttctg aaagattgca 60
   g 61
<210> 565
   <211> 61
   <212> DNA
   <213> Homo Sapiens
<400> 565
   ctcatatatt cctaaaatga ctgaaactcc rtcgtcgttt ttcttcattc cttcattcaa 60
   g 61
<210> 566
   <211> 61
   <212> DNA
   <213> Homo Sapiens
<400> 566
   aagaactcat tgcatttctt gtttttacca ytcatttgac atatctaata gttatatttt 60
   t 61
<210> 567
   <211> 61
   <212> DNA
   <213> Homo Sapiens
<400> 567
   aaggacttat ttacagatgc agtgaaggaa sacgtttgaa atgctccaat tcttcaagtt 60
   g 61
<210> 568
   <211> 61
   <212> DNA
   <213> Homo Sapiens
<400> 568
   tgctgaccta acgtaaagag attagaatcc raggaaattg tggtacagct gacttctact 60
   g 61
<210> 569
   <211> 61
   <212> DNA
   <213> Homo Sapiens
<400> 569
   ttaaaattgt ggtaagtttt tatcgctcac ktaacatgtc accagtttct cccgcctcca 60
   c 61
<210> 570
   <211> 61
   <212> DNA
   <213> Homo Sapiens
<400> 570
   tggggattta gcctacagaa tattttgtca mccagttaag aaacatagta cccgatgggt 60
   a 61
<210> 571
   <211> 61
   <212> DNA
   <213> Homo Sapiens
<400> 571
   tcatggtatt tattccattc ttggcttact matcttctct cttattcttt atgaagtcct 60
   t 61
<210> 572
   <211> 61
   <212> DNA
   <213> Homo Sapiens
<400> 572
   ccctgggctg agtgccttcc ctttgaccaa ygctgaagcc ttggaaattt atagaaatga 60
   t 61
<210> 573
   <211> 61
   <212> DNA
   <213> Homo Sapiens
<400> 573
   actagataat tatcaccaac aaagcattcc rgaaaaacca gaacaatcaa aaggattaca 60
   t 61
<210> 574
   <211> 61
   <212> DNA
   <213> Homo Sapiens
<400> 574
   cgaagtgata ggcattcaat acagaggaaa ygcagaggac agtgcaatca atcactgcca 60
   a 61
<210> 575
   <211> 61
   <212> DNA
   <213> Homo Sapiens
<400> 575
   gcggaaacct gccacaatcc ctagtcaaca ytgcctgaga agcaccattt ggcctccctg 60
   g 61
<210> 576
   <211> 61
   <212> DNA
   <213> Homo Sapiens
<400> 576
   cagctgagga tggactcacg agtcccatat rtagagcttc acaagcacta gggaaaggct 60
   g 61
<210> 577
   <211> 61
   <212> DNA
   <213> Homo Sapiens
<400> 577
   aaaaagacaa aaaaaacaac aacacagcat sgattcatag aagttgtggg atacgtcacg 60
   t 61
<210> 578
   <211> 61
   <212> DNA
   <213> Homo Sapiens
<400> 578
   ctaggcacag tacactttga tcgttttccc raaaccgcgt tggacaccat ttttccatga 60
   t 61
<210> 579
   <211> 61
   <212> DNA
   <213> Homo Sapiens
<400> 579
   gaaattgcta atgggatcta ttgcggattc stcacctaag ggaaagggaa atatagctag 60
   g 61
<210> 580
   <211> 61
   <212> DNA
   <213> Homo Sapiens
<400> 580
   tctcagactt agggatgtct ttctttggat raactattta cttccttcat tttctttact 60
   t 61
<210> 581
   <211> 61
   <212> DNA
   <213> Homo Sapiens
<400> 581
   attatttatt aaaaactgga ttccagcaaa matgcattct aaacaatgat ggataatttt 60
   t 61
<210> 582
   <211> 61
   <212> DNA
   <213> Homo Sapiens
<400> 582
   tcataatagg gtgattatga ggattgcatc mggtaacttt gtaaaggttc tggaacatat 60
   a 61
<210> 583
   <211> 61
   <212> DNA
   <213> Homo Sapiens
<400> 583
   catggcagcc tcagtttcct ggctcagtca rtaatcccac ctcagcctcc taagtagctg 60
   a 61
<210> 584
   <211> 61
   <212> DNA
   <213> Homo Sapiens
<400> 584
   aaatcatccc tgccattttt cctattcaaa yatgttctgg tatctactaa cacaactgta 60
   t 61
<210> 585
   <211> 61
   <212> DNA
   <213> Homo Sapiens
<400> 585
   ttcagtggtt tgaattttct ttgaacccag sagaagtgaa cagatgtgga ttttacaatt 60
   t 61
<210> 586
   <211> 61
   <212> DNA
   <213> Homo Sapiens
<400> 586
   aaggggatgt ttcagtttga tttcagggct staaaaaatg gtgaagccat taaaagagac 60
   a 61
<210> 587
   <211> 61
   <212> DNA
   <213> Homo Sapiens
<400> 587
   acaactcttt cttcaggaaa cctttgactc rtggtttata catacttttg tattaagttg 60
   t 61
<210> 588
   <211> 61
   <212> DNA
   <213> Homo Sapiens
<400> 588
   aagtcattct gcctctctgt gactgaatca ratgaagcct aatgatgctt ataaagttgt 60
   t 61
<210> 589
   <211> 61
   <212> DNA
   <213> Homo Sapiens
<400> 589
   ccagcggcag cactgcgatt gctctgagca rgtgccgcat caacccctca tgtccctgcc 60
   g 61
<210> 590
   <211> 61
   <212> DNA
   <213> Homo Sapiens
<400> 590
   ttttcttcat tcagtctacg gtcaatgggc mtttaggtct ctgctgctgt gaatagttct 60
   g 61
<210> 591
   <211> 61
   <212> DNA
   <213> Homo Sapiens
<400> 591
   aatagaaacc aacttaattg ccctttagct yatgtgactt gagtaattct ttaaaaataa 60
   a 61
<210> 592
   <211> 61
   <212> DNA
   <213> Homo Sapiens
<400> 592
   ctgaacacaa agcctcagga gtgataaagc ygtcacggag taaaacgcac catcctctcc 60
   t 61
<210> 593
   <211> 61
   <212> DNA
   <213> Homo Sapiens
<400> 593
   gctctccaag ctggcaccaa ttatagtaca kggaacccaa tttccaacca aaagacctcc 60
   a 61
<210> 594
   <211> 61
   <212> DNA
   <213> Homo Sapiens
<400> 594
   ccaccctcca ccatgagggg tgatttcaac maaccaggaa acacagtcca tgatcaggaa 60
   a 61
<210> 595
   <211> 61
   <212> DNA
   <213> Homo Sapiens
<400> 595
   caacctcttg ggggccctgg ctggagacct sttcctgctt caacccatgg gaagcgctgc 60
   a 61
<210> 596
   <211> 61
   <212> DNA
   <213> Homo Sapiens
<400> 596
   acgcatgcca catgcacacc cctaacttaa rgcctagctc caagtttctg tcacaaccaa 60
   g 61
<210> 597
   <211> 61
   <212> DNA
   <213> Homo Sapiens
<400> 597
   acataaaaat actcattgaa gcaattcttt yagcatagat gggcaacatg cggaggttat 60
   t 61
<210> 598
   <211> 61
   <212> DNA
   <213> Homo Sapiens
<400> 598
   gtagtgtggt attaaaatct aggacttcgg ratgcaatag cagtgctcag tttatttgtt 60
   t 61
<210> 599
   <211> 61
   <212> DNA
   <213> Homo Sapiens
<400> 599
   agttaaaggt ctaattttct tctgtgatgg ygacctcatc gctttgtaaa atacacgatg 60
   c 61
<210> 600
   <211> 61
   <212> DNA
   <213> Homo Sapiens
<400> 600
   caacacctgt gagattttcc attctaaata yggagcttaa tctatggggc atggccaacc 60
   a 61
<210> 601
   <211> 61
   <212> DNA
   <213> Homo Sapiens
<400> 601
   atatggatgg accccacaga cttaaggttg saggaaagaa gctggacaaa caggaccttc 60
   c 61
<210> 602
   <211> 61
   <212> DNA
   <213> Homo Sapiens
<400> 602
   tcctctgtgg gaaaatgcca gggctgggta ygcatagtcc atgttgcaga ttcacatatg 60
   a 61
<210> 603
   <211> 61
   <212> DNA
   <213> Homo Sapiens
<400> 603
   tcctctcagt ctacagtccg tgtctttgtt yccgtggact gaatgtctag cagagccttc 60
   t 61
<210> 604
   <211> 61
   <212> DNA
   <213> Homo Sapiens
<400> 604
   acctaccacc gtgtatgtac tagatagaac ractgtcctt caactttatc cccgaatgaa 60
   c 61
<210> 605
   <211> 61
   <212> DNA
   <213> Homo Sapiens
<400> 605
   gggtatataa ttactgctca gtgaaagtga ytatgcatac ctgtatcatc taaaggaatt 60
   t 61
<210> 606
   <211> 61
   <212> DNA
   <213> Homo Sapiens
<400> 606
   cgctgtgaag gtgcagtccc tgccaccccc ygctcttctc ctttgaaatc acacatctat 60
   c 61
<210> 607
   <211> 61
   <212> DNA
   <213> Homo Sapiens
<400> 607
   agaagaagaa gctcaaggac atactcacac rtacaagacc gatctggtgt ctcccacctt 60
   c 61
<210> 608
   <211> 61
   <212> DNA
   <213> Homo Sapiens
<400> 608
   taccaaagga aagccccttc aagttaccgt waagacagaa gaaaaggaag aaaaatataa 60
   a 61
<210> 609
   <211> 61
   <212> DNA
   <213> Homo Sapiens
<400> 609
   gagtgtctta cttctgcaag cctgctttat rgtgagcaaa gcatcaccag caagtgatca 60
   c 61
<210> 610
   <211> 61
   <212> DNA
   <213> Homo Sapiens
<400> 610
   agaagaacag tctggaaggt actggtaaga rtgactcaag gagaggagag ttctgtccat 60
   t 61
<210> 611
   <211> 61
   <212> DNA
   <213> Homo Sapiens
<400> 611
   tacagtcaga atgatgtttc tcaaattcta ygtctggctt aaagttctca gtgatttcct 60
   a 61
<210> 612
   <211> 61
   <212> DNA
   <213> Homo Sapiens
<400> 612
   gctgtagaat acgtaatgat taagttttta ygtgctttta gatgatagct aagtcctgta 60
   a 61
<210> 613
   <211> 61
   <212> DNA
   <213> Homo Sapiens
<400> 613
   gagatcagca tttgcatttt aggacaataa mtagctgcat ggtctctttg ccttgatgag 60
   a 61
<210> 614
   <211> 61
   <212> DNA
   <213> Homo Sapiens
<400> 614
   tgcagatcac aataattctt agaaggaaat rctctcgcaa aactaagacc ctgccaatca 60
   t 61
<210> 615
   <211> 61
   <212> DNA
   <213> Homo Sapiens
<400> 615
   tcaaaacatt ttaggaaaac gtaactttaa yttcaggact aaccagcagg tattagtttt 60
   a 61
<210> 616
   <211> 61
   <212> DNA
   <213> Homo Sapiens
<400> 616
   ctgcctggtt ggaatagtgt gcgcccgctg ygaggggaag aaaaagaaca gtagagggac 60
   a 61
<210> 617
   <211> 61
   <212> DNA
   <213> Homo Sapiens
<400> 617
   gatgattttc agggtaatca tgtatctagg ratccgcagt gcaacttaga agtcaaattg 60
   c 61
<210> 618
   <211> 61
   <212> DNA
   <213> Homo Sapiens
<400> 618
   ttcacagagg ccctcacgac aaagaattat ycacaccaaa atgttaatgc tgctgcgctc 60
   g 61
<210> 619
   <211> 61
   <212> DNA
   <213> Homo Sapiens
<400> 619
   ggtagatccc tcatgaatgg cttgatgcca ygcttatgat aatgaggtag ttcttgcttt 60
   a 61
<210> 620
   <211> 61
   <212> DNA
   <213> Homo Sapiens
<400> 620
   acccctcctg cccccaacac ctgacgaaat sccgccttca agcctaaaac agcatgaggg 60
   a 61
<210> 621
   <211> 61
   <212> DNA
   <213> Homo Sapiens
<400> 621
   tgtgtgtggt tgtggcagat tggataaaaa rcttctttag acccttggta acaataatgg 60
   t 61
<210> 622
   <211> 61
   <212> DNA
   <213> Homo Sapiens
<400> 622
   gaattgactt ctctagccct gacacaagga ygcagtgatt agaatgctca gtacaatgta 60
   t 61
<210> 623
   <211> 61
   <212> DNA
   <213> Homo Sapiens
<400> 623
   gcaagatagg atgtgggctg cagaaacaat yggggagaca tgagcctggt taaatattca 60
   g 61
<210> 624
   <211> 61
   <212> DNA
   <213> Homo Sapiens
<400> 624
   ctctaaaaga aggcattcac tgtttaacca ygttggtgta cagttacatc agacagtctt 60
   c 61
<210> 625
   <211> 61
   <212> DNA
   <213> Homo Sapiens
<400> 625
   tcagcctttc tcttacagct aaaagtgatc rtggcacgac acaggggcaa tgagctctaa 60
   c 61
<210> 626
   <211> 61
   <212> DNA
   <213> Homo Sapiens
<400> 626
   tctcttagct caggcgtgaa gaaattcaga scacaggtag attaattaac tccaggaagg 60
   a 61
<210> 627
   <211> 61
   <212> DNA
   <213> Homo Sapiens
<400> 627
   cactacatgt ggtgaagaca aacggttatc yttcagtggc ttaatgttag ggcaacaaag 60
   a 61
<210> 628
   <211> 61
   <212> DNA
   <213> Homo Sapiens
<400> 628
   gcagaagatc accttagaga aaatccgaac ygactctcct atcttagaag tcatccaggc 60
   t 61
<210> 629
   <211> 61
   <212> DNA
   <213> Homo Sapiens
<400> 629
   ttctatatca cctaggactt caactgtgac yaaggaattc attatgatgg gtaaaaaggc 60
   a 61
<210> 630
   <211> 61
   <212> DNA
   <213> Homo Sapiens
<400> 630
   ataatttaac agtttctcac ataacaaaac rtactgttac catccaaatc ttattccatg 60
   g 61
<210> 631
   <211> 61
   <212> DNA
   <213> Homo Sapiens
<400> 631
   aaagaaaaga aaaagaaaaa tgcagtacaa rataagtgtt agaacatatg gagaagatag 60
   t 61
<210> 632
   <211> 61
   <212> DNA
   <213> Homo Sapiens
<400> 632
   cggacaggac ttcttctgag ctttttcaat wcagagctag attctcaggc cctctcccca 60
   a 61
<210> 633
   <211> 61
   <212> DNA
   <213> Homo Sapiens
<400> 633
   tttggtgcct ggaatgcatt ccccaggttt ygctgaggtt cccatctcag gcctccaccc 60
   a 61
<210> 634
   <211> 61
   <212> DNA
   <213> Homo Sapiens
<400> 634
   atcagcacaa tagggtgact aagtattacc rcagtacaag aatttggaca aagttgattt 60
   g 61
<210> 635
   <211> 61
   <212> DNA
   <213> Homo Sapiens
<400> 635
   tttatctcca ctttagagat ggggtgtaac maacaaaatg cagtaacaac aaatgtcacc 60
   t 61
<210> 636
   <211> 61
   <212> DNA
   <213> Homo Sapiens
<400> 636
   taagtttatt aatattatgg aagtgacaat yttaggcccc tttcggaggg tacatctgga 60
   g 61
<210> 637
   <211> 61
   <212> DNA
   <213> Homo Sapiens
<400> 637
   gaaatgaagg caagtttggc aagaggttgc rtggaactga atcccatctt gcagagccat 60
   t 61
<210> 638
   <211> 61
   <212> DNA
   <213> Homo Sapiens
<400> 638
   aaagatggag caaatacgtg agacactagc ratctttagg aacagcgctg agttgtcttt 60
   t 61
<210> 639
   <211> 61
   <212> DNA
   <213> Homo Sapiens
<400> 639
   tacaaaataa attaacgtgt cagaaaatca raaacctgtg aagatagtgt ttttaggaca 60
   c 61
<210> 640
   <211> 61
   <212> DNA
   <213> Homo Sapiens
<400> 640
   atgcatcttg ttctcattct tgtttgtcca ygaattggct gtataaccat ttaactttcc 60
   a 61
<210> 641
   <211> 61
   <212> DNA
   <213> Homo Sapiens
<400> 641
   aaaccatggt acttcctggc aggcattctt ygctagattt tggagttagt ttttcttcaa 60
   g 61
<210> 642
   <211> 61
   <212> DNA
   <213> Homo Sapiens
<400> 642
   accgttctcc atagtggttg taatgattta ygtttccacc aacagtgtac agggtttccc 60
   t 61
<210> 643
   <211> 61
   <212> DNA
   <213> Homo Sapiens
<400> 643
   ttttcaccaa agtaatagac aaactttatc magctgagtg atcttattta taactcccat 60
   g 61
<210> 644
   <211> 61
   <212> DNA
   <213> Homo Sapiens
<400> 644
   cactcaaggg gctgttgaaa agattaatga sattgtgtat acaaaattcc agccaccata 60
   g 61
<210> 645
   <211> 61
   <212> DNA
   <213> Homo Sapiens
<400> 645
   cactttagtt caataaggat actggccatt rttcaagaac tggtcttagt cacacacaca 60
   a 61
<210> 646
   <211> 61
   <212> DNA
   <213> Homo Sapiens
<400> 646
   ggccttgaac cagatgtttg acaacagcaa rgaaaatgag atggaaactt gggaagccag 60
   c 61
<210> 647
   <211> 61
   <212> DNA
   <213> Homo Sapiens
<400> 647
   tgtatctctt ctctgtggct gctaaaagct ragaaagcct ccatctgtta ctaattcttg 60
   t 61
<210> 648
   <211> 61
   <212> DNA
   <213> Homo Sapiens
<400> 648
   atttgactag gcaacatctg gagactagaa mgagataatc aaagacatgt tagtaacgga 60
   t 61
<210> 649
   <211> 61
   <212> DNA
   <213> Homo Sapiens
<400> 649
   ttagcactta ttacattgta atatcagtat ytgcttacat atctatcttt ttcatgagac 60
   t 61
<210> 650
   <211> 61
   <212> DNA
   <213> Homo Sapiens
<400> 650
   tgtagatatc agaatggcta agtattttag yagcaatagt cagcacagaa gtaactggat 60
   c 61
<210> 651
   <211> 61
   <212> DNA
   <213> Homo Sapiens
<400> 651
   ttattgttct cccgtcctca ttattctctg kctaggctca acctaggtca ccctgctata 60
   c 61
<210> 652
   <211> 61
   <212> DNA
   <213> Homo Sapiens
<400> 652
   atgacatttt cacattggtt gctggttata yatgtgcctc caaaatcagt aatctccaat 60
   t 61
<210> 653
   <211> 61
   <212> DNA
   <213> Homo Sapiens
<400> 653
   tacttcctta ttattctggc actagtcaag wtagaccttc aaacaatgtg aactaatttg 60
   t 61
<210> 654
   <211> 61
   <212> DNA
   <213> Homo Sapiens
<400> 654
   aactggcttg aaggtaacat ggtaaatgca rtttggatgt attgagtata aagtgactca 60
   a 61
<210> 655
   <211> 61
   <212> DNA
   <213> Homo Sapiens
<400> 655
   aacaccaggt taccatccca gtttgtctta yaagattaac acaaaagaga accctatata 60
   c 61
<210> 656
   <211> 61
   <212> DNA
   <213> Homo Sapiens
<400> 656
   cgggcactgg agttgaaatg cttggtctta ratctgaatt ctgtcactgc tgagcttgtg 60
   g 61
<210> 657
   <211> 61
   <212> DNA
   <213> Homo Sapiens
<400> 657
   ataagccact ctcttcataa gaccatacca rttttctaat aaagctcaca cagatttcgt 60
   g 61
<210> 658
   <211> 61
   <212> DNA
   <213> Homo Sapiens
<400> 658
   gttttacaat aggaaaagaa aatggtatgc yaagtggcct cttgtttctg aaatttttgg 60
   c 61
<210> 659
   <211> 61
   <212> DNA
   <213> Homo Sapiens
<400> 659
   tctgacttcc aaaagatgca tcataacagc ktaggtggtt tttatgacat taaactactt 60
   t 61
<210> 660
   <211> 61
   <212> DNA
   <213> Homo Sapiens
<400> 660
   tacagggtac aataaaacaa ataaccagag mtttttatat acatgtaaaa gggcactgca 60
   a 61
<210> 661
   <211> 61
   <212> DNA
   <213> Homo Sapiens
<400> 661
   ctttggtaaa catgcagata tttttctggc wtaaggtacc tcagaatcaa ttgattaatc 60
   t 61
<210> 662
   <211> 61
   <212> DNA
   <213> Homo Sapiens
<400> 662
   gtcaaccaaa gaggcaatat acttttgccc ycttggattt tacattctat caggtgaaga 60
   c 61
<210> 663
   <211> 61
   <212> DNA
   <213> Homo Sapiens
<400> 663
   tgggagaaag aaagtcagtt tacttctgga wacatgtgtc tagtgtatat agctgaataa 60
   t 61
<210> 664
   <211> 61
   <212> DNA
   <213> Homo Sapiens
<400> 664
   acaacggcat aatcgacagt gtcaaaagag wtaaggagat tggagacaac tgaggtatta 60
   g 61
<210> 665
   <211> 61
   <212> DNA
   <213> Homo Sapiens

<400> 665
   tacaaagaag ctagaatgta cataagaaac wagcacacac cttggaaaac agttagaaat 60
   c 61
<210> 666
   <211> 61
   <212> DNA
   <213> Homo Sapiens
<400> 666
   catacagggt ggtaagtatt tgctaatgta ytggactcct ccactagcct gtgaaatttt 60
   t 61
<210> 667
   <211> 61
   <212> DNA
   <213> Homo Sapiens
<400> 667
   tattcgctta aagaatacca gttaccatcc rtatgctttc ggtactaaat ctccatttat 60
   c 61
<210> 668
   <211> 61
   <212> DNA
   <213> Homo Sapiens
<400> 668
   aagatggacc aaaattgcac agtctcaaaa ygatcttaac ttctgtgaaa tcagtgcctt 60
   t 61
<210> 669
   <211> 61
   <212> DNA
   <213> Homo Sapiens
<400> 669
   tttattgaat atttctggaa ttacactaac raagttccat ctacaatctt taccataaaa 60
   g 61
<210> 670
   <211> 61
   <212> DNA
   <213> Homo Sapiens
<400> 670
   tgcgttttcg gcactgaaaa gtagcatcac ygtagtaaaa tggaaaagat gtgttttatg 60
   a 61
<210> 671
   <211> 61
   <212> DNA
   <213> Homo Sapiens
<400> 671
   tggttttcaa actttctcaa attttgtagt wtcatcaaca gctatcattt ttgattgctc 60
   t 61
<210> 672
   <211> 61
   <212> DNA
   <213> Homo Sapiens
<400> 672
   gatcacactt tcttaatccg ttcatacatc wttggacact tagagtcgat tacttgacta 60
   t 61
<210> 673
   <211> 61
   <212> DNA
   <213> Homo Sapiens
<400> 673
   taaagagaat attatatata gctgctatca raatttaaca aagactgcat ctgaggttgc 60
   a 61
<210> 674
   <211> 61
   <212> DNA
   <213> Homo Sapiens
<400> 674
   cttccacatt cacctccttt tattatgcat yccttcattt aatcagccat ccttgcatgt 60
   a 61
<210> 675
   <211> 61
   <212> DNA
   <213> Homo Sapiens
<400> 675
   tttattgtca aatcacatag ataagaaatg rtaagtccat gatttctatc tgaaaccttc 60
   a 61
<210> 676
   <211> 61
   <212> DNA
   <213> Homo Sapiens
<400> 676
   tgtcaagaag gtagagagta tgacatttga saacccttga aagtagttca gtgggccttc 60
   a 61
<210> 677
   <211> 61
   <212> DNA
   <213> Homo Sapiens
<400> 677
   attcccttgt ggagggactt ccctgactaa ygctcttggc ctctctcctg ccctccattt 60
   g 61
<210> 678
   <211> 61
   <212> DNA
   <213> Homo Sapiens
<400> 678
   gggctggtaa atgcacactg gctgaagtcc ygaaagctat tttctggcca cttggcactt 60
   g 61
<210> 679
   <211> 61
   <212> DNA
   <213> Homo Sapiens
<400> 679
   ggcccccaaa cacatgctgc tgggcatcag sgtgtctaca ataaaaactc atagagggaa 60
   t 61
<210> 680
   <211> 61
   <212> DNA
   <213> Homo Sapiens
<400> 680
   gatactgtac atttgtgagt cagtcacgca ytacatactc tgggcttcat atctcgtaag 60
   a 61
<210> 681
   <211> 61
   <212> DNA
   <213> Homo Sapiens
<400> 681
   ggtgtgcaca tcttgcagca gagatgcaac rtgtccaggt ttccactgcc agaatacaga 60
   t 61
<210> 682
   <211> 61
   <212> DNA
   <213> Homo Sapiens
<400> 682
   acctgtcaca ggccatttac cctggaggta mtggccacct gagcgtgtgt gtgtgtgttt 60
   g 61
<210> 683
   <211> 61
   <212> DNA
   <213> Homo Sapiens
<400> 683
   ttcaccttgg ttctctgaca cccatggaaa rtttaggtta gggagagaga gtggaaagag 60
   a 61
<210> 684
   <211> 61
   <212> DNA
   <213> Homo Sapiens
<400> 684
   cattgttctc cagggcaaag cccatgacct sgatgataga gtagactgca ttttgattta 60
   t 61
<210> 685
   <211> 61
   <212> DNA
   <213> Homo Sapiens
<400> 685
   acaacactgt ggagttccag ggccttagtg magtacagga tttggactaa atccttggtc 60
   t 61
<210> 686
   <211> 61
   <212> DNA
   <213> Homo Sapiens
<400> 686
   agtgaccctg cagacctgag cccaagggat rctccacaag gagaagacat gctgaacgcc 60
   a 61
<210> 687
   <211> 61
   <212> DNA
   <213> Homo Sapiens
<400> 687
   gcacatcctc agtgtcattt tacactaaca yaacagtgtc atggctggaa gatctaagga 60
   g 61
<210> 688
   <211> 61
   <212> DNA
   <213> Homo Sapiens
<400> 688
   aggtttttaa acactgtaag atgattccag wtttttagat agctcagagg gccgtgggga        60
   c 61
<210> 689
   <211> 61
   <212> DNA
   <213> Homo Sapiens
<400> 689
   ataaagttct taagagagct aaatgagcct raatccattc ataggaggaa aacaagcaca 60
   c 61
<210> 690
   <211> 61
   <212> DNA
   <213> Homo Sapiens
<400> 690
   caaataggtc ctgctgggcc agctgacctt ygtaatgaac tcaggggcga tttacttagc 60
   a 61
<210> 691
   <211> 61
   <212> DNA
   <213> Homo Sapiens
<400> 691
   aattcagcct gaggtatata tacactagga yacctgttta ttggatgtat ttaagacaag 60
   g 61
<210> 692
   <211> 61
   <212> DNA
   <213> Homo Sapiens
<400> 692
   ttgctacttt tgcctcccag cagtctggta ycaacacagc atccaatgca acactgttat 60
   a 61
<210> 693
   <211> 61
   <212> DNA
   <213> Homo Sapiens
<400> 693
   attgcctgtc caaagcacag tgcctggaac wagcaaataa tagttagatg gatgaaagta 60
   t 61
<210> 694
   <211> 61
   <212> DNA
   <213> Homo Sapiens
<400> 694
   aggttcttgt aatgaacatt cacatggccc rcatcttaac catgcactca ttttttacag 60
   c 61
<210> 695
   <211> 61
   <212> DNA
   <213> Homo Sapiens
<400> 695
   tttatataat tatttattaa caaataagag rtaaaagagc agcaagaaac gagagtcaaa 60
   c 61
<210> 696
   <211> 61
   <212> DNA
   <213> Homo Sapiens
<400> 696
   ttcctctctc caacatagtg ccagactctc rtattaggaa atgatgaaac catcaaccaa 60
   a 61
<210> 697
   <211> 61
   <212> DNA
   <213> Homo Sapiens
<400> 697
   aaaaaaaaac aaaaaaaaag aatatttcct sccaaccaat agcaacgatc cccaccctca 60
   g 61
<210> 698
   <211> 61
   <212> DNA
   <213> Homo Sapiens
<400> 698
   gtgtgtgatg ttcttgcttt gacaattcac ractcaccag gaaacactgt cgtcaggtgt 60
   a 61
<210> 699
   <211> 61
   <212> DNA
   <213> Homo Sapiens
<400> 699
   gcattattca cagaacccac tgcttctaca rggtttgttt cctgatgttt ctagctgctc 60
   c 61
<210> 700
   <211> 61
   <212> DNA
   <213> Homo Sapiens
<400> 700
   gcgattaaag ctttggcaat gggcgttgac rtcgccagat tgaaactagt ggtggggaaa 60
   a 61
<210> 701
   <211> 61
   <212> DNA
   <213> Homo Sapiens
<400> 701
   tccacagtgg ctcccgggtc ttgctctgcc yatgttacag attaagagca atccccatgt 60
   t 61
<210> 702
   <211> 61
   <212> DNA
   <213> Homo Sapiens
<400> 702
   cagtctttgg ttattccatt gtgtgttgaa matttacata tttttgtcgc ctaaggatta 60
   a 61
<210> 703
   <211> 61
   <212> DNA
   <213> Homo Sapiens
<400> 703
   acctcaagga ggggcttgcc aagatggtca ygtcaattaa taaaagagct ggattcgaat 60
   c 61
<210> 704
   <211> 61
   <212> DNA
   <213> Homo Sapiens
<400> 704
   aacagcacag gtttgcagag ttctccagga yggcagataa caaccagagc acgtgtaccc 60
   a 61
<210> 705
   <211> 61
   <212> DNA
   <213> Homo Sapiens
<400> 705
   gggatgatac aaccacctac tgagcagtta ygaatgggtt ctaaagctgg ggcacagcac        60
   c                                                                         61
<210> 706
   <211> 61
   <212> DNA
   <213> Homo Sapiens
<400> 706
   cagccggccg ggatgcccac cttgtgctgc ygcgtctccg gatgatcttg gtgcggctct        60
   t                                                                         61
<210> 707
   <211> 61
   <212> DNA
   <213> Homo Sapiens
<400> 707
   ttaagaagct gatttgacac tttggattat stgtagttct tcttccctcc attgcagtga        60
   a                                                                         61
<210> 708
   <211> 61
   <212> DNA
   <213> Homo Sapiens
<400> 708
   caaatcagat attaggatct tgaacctaaa ytgtgaatct ctttgtgcct ttaaccacat        60
   c                                                                         61
<210> 709
   <211> 61
   <212> DNA
   <213> Homo Sapiens
<400> 709
   tcagataaac taaaaacact ttctgagagt rtccatgtcc taaaggcttt aagtacttct        60
   a                                                                         61
<210> 710
   <211> 61
   <212> DNA
   <213> Homo Sapiens
<400> 710
   ggagcagatg ttcaggtctg aatccagttt yttctagaca cctctttccg gatcaccccc 60
   t 61
<210> 711
   <211> 61
   <212> DNA
   <213> Homo Sapiens
<400> 711
   agatataaat atatctacat ctatgactat rtctacatat cttccaaaaa agtcatcaaa 60
   a 61
<210> 712
   <211> 61
   <212> DNA
   <213> Homo Sapiens
<400> 712
   tttctctttg tctctcttct gttgtagccc ytccctcacc gctaagcctt caccctgact 60
   c 61
<210> 713
   <211> 61
   <212> DNA
   <213> Homo Sapiens
<400> 713
   ttttatcata tttttattta aggtggaatg rcatttttcc aaaaaagcaa agatgacaaa 60
   c 61
<210> 714
   <211> 61
   <212> DNA
   <213> Homo Sapiens
<400> 714
   tcaccactta cttgagcttt cctcagtctc rtcttctttt tcctccactt tgatttgtac 60
   t 61
<210> 715
   <211> 61
   <212> DNA
   <213> Homo Sapiens
<400> 715
   aaaaaaacct atgtctagat tagagtcagg kgcggtctcc ttattgtcag tgtctgcttt 60
   c 61
<210> 716
   <211> 61
   <212> DNA
   <213> Homo Sapiens
<400> 716
   tacaaatata tgtggtttta ggcaagtctc sattttgaat tacgtgcaaa attatatttt 60
   a 61
<210> 717
   <211> 61
   <212> DNA
   <213> Homo Sapiens
<400> 717
   gaaaagcttt ttattttctt ttttatgtca rtcttctatg accatgtctt acttttataa 60
   a 61
<210> 718
   <211> 56
   <212> DNA
   <213> Homo Sapiens
<400> 718
   agatcagtag atactcaaat ctttatccta yagggagtgc tgacattggc atgaag 56
<210> 719
   <211> 61
   <212> DNA
   <213> Homo Sapiens
<400> 719
   tataatatac aaaatatact aaaggcacct raaattatca tgaaaagata taaaatataa 60
   a 61
<210> 720
   <211> 61
   <212> DNA
   <213> Homo Sapiens
<400> 720
   aaagagtaaa ctttccacgt ggagtctttt ytgttgactt atgacctaag tagctgggta 60
   g 61
<210> 721
   <211> 61
   <212> DNA
   <213> Homo Sapiens
<400> 721
   gcagagccac tgaatttagt gattggtaac raaatagtta aattgagagt gctagaatgt 60
   c 61
<210> 722
   <211> 61
   <212> DNA
   <213> Homo Sapiens
<400> 722
   ccgtaaaacc ctgcgaatgt actaaaaacc rctgaaatgt acactttaat gcgggaatta 60
   t 61
<210> 723
   <211> 61
   <212> DNA
   <213> Homo Sapiens
<400> 723
   aaagccaatt caagtgattc ctgcaaaagg yatattgtgc ttccaggcat catgcactca 60
   a 61
<210> 724
   <211> 61
   <212> DNA
   <213> Homo Sapiens
<400> 724
   accagttgac atatttttcc atacatactc ygaaggtgcc atcactaatt aatgcttttg 60
   t 61
<210> 725
   <211> 61
   <212> DNA
   <213> Homo Sapiens
<400> 725
   taagtttctt aagttcaaga ttgccaatca sgtcctaaaa cttgtattta ttcatacagc 60
   t 61
<210> 726
   <211> 61
   <212> DNA
   <213> Homo Sapiens
<400> 726
   atttaataac ttagtaaagt tcaaaattgt sagttactta ttaaacataa acatgtgaca 60
   a 61
<210> 727
   <211> 61
   <212> DNA
   <213> Homo Sapiens
<400> 727
   tttagcattt caggatgcag gacttccgct maaacacaaa tttgggaagg tcagtaacaa 60
   a 61
<210> 728
   <211> 61
   <212> DNA
   <213> Homo Sapiens
<400> 728
   tatttctcag ctatgagatt ttccgtggcc rccttttcac agtagccagc tgggattgtc 60
   c 61
<210> 729
   <211> 61
   <212> DNA
   <213> Homo Sapiens
<400> 729
   accttagcct tggacttcta gtttcctgaa ytgtgagaaa atagatttct attgttgaaa 60
   c 61
<210> 730
   <211> 61
   <212> DNA
   <213> Homo Sapiens
<400> 730
   attaaaccag tctgcatcag ctacagtgta mgtctcagaa acagatctga aaccccagct 60
   g 61
<210> 731
   <211> 61
   <212> DNA
   <213> Homo Sapiens
<400> 731
   ttggttgcat ttgaggtgac atcctctaat yttcctaagc acagtggggc ctcctggaaa 60
   c 61
<210> 732
   <211> 61
   <212> DNA
   <213> Homo Sapiens
<400> 732
   tccttcattg tagggagaaa aagttttaga rggaggatac tgcagaatgg agtttctgat 60
   g 61
<210> 733
   <211> 61
   <212> DNA
   <213> Homo Sapiens
<400> 733
   gtaatataat gtaaaggaaa aagaggaaaa raagttctgc taccatgtta aaaaaatcat 60
   g 61
<210> 734
   <211> 61
   <212> DNA
   <213> Homo Sapiens
<400> 734
   aaaggaatga taagaaccac ttcattttga raggattact ctgattgcga tgcaacagat 60
   g 61
<210> 735
   <211> 61
   <212> DNA
   <213> Homo Sapiens
<400> 735
   tttggaatat tacagaggtt aaatgatgag ragtactagc tggtcaagaa gaggagaggc 60
   a 61
<210> 736
   <211> 61
   <212> DNA
   <213> Homo Sapiens
<400> 736
   ttgttactgt actctgcaca ttgcttcctc rcttactgta cattaaaagt tttcccttga 60
   t 61
<210> 737
   <211> 61
   <212> DNA
   <213> Homo Sapiens
<400> 737
   tcctttcctc ttctgttttc cagggtctta ygaatgcgga atctgtggca agaagtacaa 60
   g 61
<210> 738
   <211> 61
   <212> DNA
   <213> Homo Sapiens
<400> 738
   ctttctccat ggtgatagac agaaaaatgc yatttcccac ttgactatta acaattagag 60
   a 61
<210> 739
   <211> 61
   <212> DNA
   <213> Homo Sapiens
<400> 739
   agcctgggct atataggtca ttcagtaccc rccatagagc cagatcttgt gtggagagag 60
   a 61
<210> 740
   <211> 61
   <212> DNA
   <213> Homo Sapiens
<400> 740
   ggccttcttt ccactgactg ccagatgagc ytgcgtttca tgttatctct gtagaacatt 60
   t 61
<210> 741
   <211> 61
   <212> DNA
   <213> Homo Sapiens
<400> 741
   gaactctatt gccaggacaa atgaagcatt yccacataag cttctggcta ggatcctcgg 60
   t 61
<210> 742
   <211> 61
   <212> DNA
   <213> Homo Sapiens
<400> 742
   cctaaagttc tccttggctc ctcctcgctc scatctagaa cccgcctagg aggtaatcac 60
   t 61
<210> 743
   <211> 61
   <212> DNA
   <213> Homo Sapiens
<400> 743
   aggaatgata ttgcaataac ctacaaaaac rtagaggtgt gaagaagtgt taagtggtct 60
   g 61
<210> 744
   <211> 61
   <212> DNA
   <213> Homo Sapiens
<400> 744
   gtcattgctc tgccatcatt ggcttcttcc rtgtttactt tgtgccaggc catactgagc 60
   c 61
<210> 745
   <211> 61
   <212> DNA
   <213> Homo Sapiens
<400> 745
   gtaggggctg tcgggggaat tacacgtgcc rtagaagtac atagaaggag catccagctt 60
   t 61
<210> 746
   <211> 61
   <212> DNA
   <213> Homo Sapiens
<400> 746
   acgtgtgatt ctttgacatg caggaatcta ytaaattgta catgtaaact atcacacttg 60
   a 61
<210> 747
   <211> 61
   <212> DNA
   <213> Homo Sapiens
<400> 747
   gagactgtgg tgctgtcctg caggcacatc rtggaagaac ttatcagacc catgactaca 60
   a 61
<210> 748
   <211> 61
   <212> DNA
   <213> Homo Sapiens
<400> 748
   agtagaagga gtaattactc atgatttgca ktttgtccaa agattatctc atggtcagaa 60
   g 61
<210> 749
   <211> 61
   <212> DNA
   <213> Homo Sapiens
<400> 749
   aagtatcaac atcttgtaac accctcttta ygagttaaca acaagcgttt tagtgcatta 60
   a 61
<210> 750
   <211> 61
   <212> DNA
   <213> Homo Sapiens
<400> 750
   atcaaggcct caggtgtcaa atccatccct rgaccagaaa cagagtataa aaagacaaac 60
   t 61
<210> 751
   <211> 61
   <212> DNA
   <213> Homo Sapiens
<400> 751
   cagctcagtc tgtaaatttc ctttatctcc ytcgctcagt cttaccctca gttccactct 60
   c 61
<210> 752
   <211> 61
   <212> DNA
   <213> Homo Sapiens
<400> 752
   ctgtttctac actgtctgga agaagaatgg rcattgttac ctcaatatgg gaccaccagg 60
   g 61
<210> 753
   <211> 61
   <212> DNA
   <213> Homo Sapiens
<400> 753
   ggcatgatcc ctacattcag actcaactgt kggagatact gggatacaga atgagcaaga 60
   t 61
<210> 754
   <211> 61
   <212> DNA
   <213> Homo Sapiens
<400> 754
   ctcattatca gggccagcca gcaaggcacg sggttccagt gctctcccgg ccactgcagg 60
   a 61
<210> 755
   <211> 61
   <212> DNA
   <213> Homo Sapiens
<400> 755
   ccccgcacat gggaagacca aaaggtgggt ycttgaagcc ataaggaaat gcgaggtcac 60
   t 61
<210> 756
   <211> 61
   <212> DNA
   <213> Homo Sapiens
<400> 756
   tggcggaggg actggtgggg cctcagaggc rttagttatc ctctagaaag tactcagggt 60
   g 61
<210> 757
   <211> 61
   <212> DNA
   <213> Homo Sapiens
<400> 757
   tttggaagca atataactac ttgccctcaa yctatttgca cttctagcac aaaattagcc 60
   t 61
<210> 758
   <211> 61
   <212> DNA
   <213> Homo Sapiens
<400> 758
   cagttcttgc aatgttggga aaacttacaa raatatcttt gaaaatcctc ttaatacaca 60
   t 61
<210> 759
   <211> 61
   <212> DNA
   <213> Homo Sapiens
<400> 759
   gaaaacttct acaattactt ctgcatttac rtggtaagac agagacaata agggcagaat 60
   t 61
<210> 760
   <211> 61
   <212> DNA
   <213> Homo Sapiens
<400> 760
   acccacatct attgcgcatt gaacctacca ygcactatgc tagctgcttt acaaacattg 60
   t 61
<210> 761
   <211> 61
   <212> DNA
   <213> Homo Sapiens
<400> 761
   ttgaagtcaa aagtattatc agaatttgtc rtcgattctt aaggttgtat ctactaatac 60
   a 61
<210> 762
   <211> 61
   <212> DNA
   <213> Homo Sapiens
<400> 762
   agtggtccca gatcatgagg tgctaattgt rtaggaaaag acagagatag ataaacagga 60
   g 61
<210> 763
   <211> 61
   <212> DNA
   <213> Homo Sapiens
<400> 763
   gaataggaga ttgagttatc taatgtgcta ktcttagcgt ctggcactta atagggattc 60
   a 61
<210> 764
   <211> 61
   <212> DNA
   <213> Homo Sapiens
<400> 764
   gatgaaaaga cagagagaca aggaggttta ygctttagca gaagtgttat tcagataata 60
   g 61
<210> 765
   <211> 61
   <212> DNA
   <213> Homo Sapiens
<400> 765
   cacctagcac acttaggggc tcaatgtgcg wcccttctct cttcccttct tagcctttgg 60
   t 61
<210> 766
   <211> 61
   <212> DNA
   <213> Homo Sapiens
<400> 766
   gaataaaata ttttaactgc attcgtctga waagccattg ggaatatttt atgttaaaaa 60
   g 61
<210> 767
   <211> 61
   <212> DNA
   <213> Homo Sapiens
<400> 767
   aatattcctt tggggttgcc agaaaagaaa rttggccttg atgacttatg aggcccatct 60
   a 61
<210> 768
   <211> 61
   <212> DNA
   <213> Homo Sapiens
<400> 768
   gagcataagg catcagggga agcttaggga rgaaagtgag gcttgatctg aatttggact 60
   t 61
<210> 769
   <211> 61
   <212> DNA
   <213> Homo Sapiens
<400> 769
   tcaagaacat caaacatctt gaaatcaaat yatgttggca agtcagagat tgcagcacct 60
   t 61
<210> 770
   <211> 61
   <212> DNA
   <213> Homo Sapiens
<400> 770
   tgaaaaatat taaaaaatgt gtcttctcaa sgtaaagaaa cttattacca ctttgcttaa 60
   t 61
<210> 771
   <211> 61
   <212> DNA
   <213> Homo Sapiens
<400> 771
   ctcacacaca ggatttcagt ttggggctta wgtaagaaac tagcaagtta tgtgaactat 60
   a 61
<210> 772
   <211> 61
   <212> DNA
   <213> Homo Sapiens
<400> 772
   aatacataca agaagaagcc ctgtactcaa stcatctccc acaaatgaat aaatgcatgt 60
   a 61
<210> 773
   <211> 61
   <212> DNA
   <213> Homo Sapiens
<400> 773
   gtcttgacat gacttttaga ttaatttttg katcctatgg tctcatcaat acagagcctc 60
   a 61
<210> 774
   <211> 61
   <212> DNA
   <213> Homo Sapiens
<400> 774
   catacactta agaaatatgg aaagcaaagt mcaaaattat agagcagaaa aaatttaaat 60
   c 61
<210> 775
   <211> 61
   <212> DNA
   <213> Homo Sapiens
<400> 775
   aagtattgat tatgtatgtg gtcagtgttg yaggctaaca tagcatttaa gaagtaaacc 60
   c 61
<210> 776
   <211> 61
   <212> DNA
   <213> Homo Sapiens
<400> 776
   tggctagaca tctccaggtc tcaagaacat ygcagcatgc cctttgtgcc agctgtttgg 60
   g 61
<210> 777
   <211> 61
   <212> DNA
   <213> Homo Sapiens
<400> 777
   agaaatagaa agaaaaggga ttagttagga sgataaggta tgaagtatga tgtgctgaga 60
   a 61
<210> 778
   <211> 61
   <212> DNA
   <213> Homo Sapiens
<400> 778
   agtaaatgat aggtataaag agccactagc yatgaaaatt ccctttcaaa aaaagcctag 60
   t 61
<210> 779
   <211> 61
   <212> DNA
   <213> Homo Sapiens
<400> 779
   gtcaacagat gtcttgcaag caggaagccc ytgacttata tacatctttg cattcctaac 60
   a 61
<210> 780
   <211> 61
   <212> DNA
   <213> Homo Sapiens
<400> 780
   taggcaagct aactacctcc tcctcactct rcctagacct gtcattggtg gtatcttagc 60
   t 61
<210> 781
   <211> 61
   <212> DNA
   <213> Homo Sapiens
<400> 781
   tatataaaca gggttacagg tctgtgtaaa saagttacat tgattacaag agctaatata 60
   a 61
<210> 782
   <211> 61
   <212> DNA
   <213> Homo Sapiens
<400> 782
   aagtgtgttt gccaaaacag ggagtgaagt rtcttgcaag gccacttgtt ccagaagaaa 60
   g 61
<210> 783
   <211> 61
   <212> DNA
   <213> Homo Sapiens
<400> 783
   gctggaacaa ggttccccat gtgtttctca sttgtaccta gcaatttccc taggaagttt 60
   t 61
<210> 784
   <211> 61
   <212> DNA
   <213> Homo Sapiens
<400> 784
   cttagcccaa gccagctgtg atcctcaggc ygccccgctc attaaaatcc ccccctttcg 60
   a 61
<210> 785
   <211> 61
   <212> DNA
   <213> Homo Sapiens
<400> 785
   acatctagat cctcactggt caccaaagca satgtagaga gagtacctgg agagttgaca 60
   t 61
<210> 786
   <211> 61
   <212> DNA
   <213> Homo Sapiens
<400> 786
   catcgcacat gaagcagacc caatgagtga stcagcaata aatcaacagt cttaatggag 60
   a 61
<210> 787
   <211> 61
   <212> DNA
   <213> Homo Sapiens
<400> 787
   gaaaataact cccagtgagg aagcaggtga yggcctgacc actgtacttc cggtatctct 60
   g 61
<210> 788
   <211> 61
   <212> DNA
   <213> Homo Sapiens
<400> 788
   caaacagaat attgagtaaa gggatcctga ygtgaaagac agcctgctgt atgttccatt 60
   t 61
<210> 789
   <211> 61
   <212> DNA
   <213> Homo Sapiens
<400> 789
   agccacggac acaacagaat tcacttttaa ygaggcttca gaaaacaacg tttaactata 60
   t 61
<210> 790
   <211> 61
   <212> DNA
   <213> Homo Sapiens
<400> 790
   tactactatt agtaataaat gtcttgtcac rgaccttgac acagactaca atacacaagt 60
   g 61
<210> 791
   <211> 61
   <212> DNA
   <213> Homo Sapiens
<400> 791
   aaatgttcct agggatatag ataattcaca raatgaaaga gtctaatcac atacataaga 60
   g 61
<210> 792
   <211> 61
   <212> DNA
   <213> Homo Sapiens
<400> 792
   tgttttctct aggaacctga aagtagtggt wtataaggtc agtcatccac tctaggtcaa 60
   c 61
<210> 793
   <211> 61
   <212> DNA
   <213> Homo Sapiens
<400> 793
   ggatttaaaa tgctcacatg tatgagtcat rtgagtgttc acagaagttc agtaatacac 60
   a 61
<210> 794
   <211> 61
   <212> DNA
   <213> Homo Sapiens
<400> 794
   ttcggtctct gcacccgtct gcctcgtcta rggtttcttt tcatgacaaa ttcgagttcg 60
   a 61
<210> 795
   <211> 61
   <212> DNA
   <213> Homo Sapiens
<400> 795
   gtgcaggggg taacctgttc agatcaagcc rgtcagaagg ctccacaagg gacttttctc 60
   a 61
<210> 796
   <211> 61
   <212> DNA
   <213> Homo Sapiens
<400> 796
   tgggggcaaa tacatgcttc aaaatgttaa ygcacttttc acattttcta tcaaaaaaaa 60
   a 61
<210> 797
   <211> 61
   <212> DNA
   <213> Homo Sapiens
<400> 797
   actcagagaa atagaaataa atttttgagg wgagttttga cattcttgac cccaagggcc 60
   t 61
<210> 798
   <211> 61
   <212> DNA
   <213> Homo Sapiens
<400> 798
   gactgacagg aatattaaag aagagagtgg wtacaaagcg cccatcatac tttatagcaa 60
   g 61
<210> 799
   <211> 61
   <212> DNA
   <213> Homo Sapiens
<400> 799
   acttctggac ctttccctga tctgtgccaa raatggcctg gtagtccagg atacctagtt 60
   c 61
<210> 800
   <211> 61
   <212> DNA
   <213> Homo Sapiens
<400> 800
   atctagtggg gttcactcac ctttacaggg ractcaacgc tggtagccag ctccctattt 60
   c 61
<210> 801
   <211> 61
   <212> DNA
   <213> Homo Sapiens
<400> 801
   gttttgggtc cctggtgaag caggtcaggg rcgtgagaaa agaggaaata atgggtttcc 60
   a 61
<210> 802
   <211> 61
   <212> DNA
   <213> Homo Sapiens
<400> 802
   tcaacactag agggtagata actttattct yaagcatgac accccaggta cttcctcacc 60
   t 61
<210> 803
   <211> 61
   <212> DNA
   <213> Homo Sapiens
<400> 803
   ggaagatttc agagctgttt cggaggcatc rctgccataa tatactgagg gcaagaaggg 60
   g 61
<210> 804
   <211> 61
   <212> DNA
   <213> Homo Sapiens
<400> 804
   aagcagccct catccattag ttcaactgac ytttgaataa cccagaccag aaaagaatag 60
   g 61
<210> 805
   <211> 61
   <212> DNA
   <213> Homo Sapiens
<400> 805
   attttaaaat cttcctacac acatctagac kttcaagttt gcaaatcagt ttttagcaag 60
   a 61
<210> 806
   <211> 61
   <212> DNA
   <213> Homo Sapiens
<400> 806
   ggcccactgt gacttccatg gtctgaggac ktttctagtc tctgaggctt cgggaccata 60
   g 61
<210> 807
   <211> 61
   <212> DNA
   <213> Homo Sapiens
<400> 807
   ctgcttcctc ctcccctccc tttttttccc rttaaatcag gagccatttc ccatattcaa 60
   g 61
<210> 808
   <211> 61
   <212> DNA
   <213> Homo Sapiens
<400> 808
   ggaggtatca ttgtttctgc ttctctttaa ygcatccatg aatcagaatg ccaatttgat 60
   t 61
<210> 809
   <211> 61
   <212> DNA
   <213> Homo Sapiens
<400> 809
   cagctggtcc tctgggagca catggagaac raccacattg tgtcccaggg ttgcttgcct 60
   g 61
<210> 810
   <211> 61
   <212> DNA
   <213> Homo Sapiens
<400> 810
   agtagatgtg gcattgttta tatgacaaag rttagaaata gtgtcgacag cacagcacac 60
   g 61
<210> 811
   <211> 61
   <212> DNA
   <213> Homo Sapiens
<400> 811
   gagacagccc acaccctggg caatttctgg sgcaaggagg aaaccccctg ttcactgagc 60
   t 61
<210> 812
   <211> 61
   <212> DNA
   <213> Homo Sapiens
<400> 812
   cagagaggcc ttcgtgactg cctaccttgt yacttactat ctctctctcc ccatcttagt 60
   t 61
<210> 813
   <211> 61
   <212> DNA
   <213> Homo Sapiens
<400> 813
   cagggagagt gggtggcttg gtcaaggtct ycctgacagt gacagggcta agatttggtc 60
   c 61
<210> 814
   <211> 61
   <212> DNA
   <213> Homo Sapiens
<400> 814
   cccctctcct agcccctctt tcctttcatc yctttccagg cctggttcat cattgtcttt 60
   g 61
<210> 815
   <211> 61
   <212> DNA
   <213> Homo Sapiens
<400> 815
   attgagaatc agtgacttaa actttcctac rgtgtgaccc ccaatcccat cattctcaat 60
   c 61
<210> 816
   <211> 61
   <212> DNA
   <213> Homo Sapiens
<400> 816
   tgtgcactgc accctgcttc acaatctgca ygcctggcaa gtccgtgatc tgggggtcag 60
   g 61
<210> 817
   <211> 61
   <212> DNA
   <213> Homo Sapiens
<400> 817
   ttaggctctt gcccaaccac tcacggtcaa yagtgagctg ggccttttgt accaggttcc 60
   c 61
<210> 818
   <211> 61
   <212> DNA
   <213> Homo Sapiens
<400> 818
   ctattgaaat ccgtattgtt caagggtcaa ycgtatttca ctttgaagga ctggtcctca 60
   g 61
<210> 819
   <211> 61
   <212> DNA
   <213> Homo Sapiens
<400> 819
   gtgtcattat atcacaatgt aaataaatag yaagtggaag ttgctgctat gggagtgctg 60
   t 61
<210> 820
   <211> 61
   <212> DNA
   <213> Homo Sapiens
<400> 820
   actgaggacc cacctcacag gaagaaaacc rtctgactca taaagggatc cgaccaaggc 60
   t 61
<210> 821
   <211> 61
   <212> DNA
   <213> Homo Sapiens
<400> 821
   gatatggtca aatctttgga gctccagggt rtataagaca atgatacctc ctccccagtt 60
   t 61
<210> 822
   <211> 61
   <212> DNA
   <213> Homo Sapiens
<400> 822
   acacctcatt gtctcaccct gcccaacatt yggtgtggaa gattgagaat aatgtgaatt 60
   t 61
<210> 823
   <211> 61
   <212> DNA
   <213> Homo Sapiens
<400> 823
   gtataaactg ccattgattt ctgataacac rtagcaatta cttaatgctg cagacttgat 60
   t 61
<210> 824
   <211> 61
   <212> DNA
   <213> Homo Sapiens
<400> 824
   cagcttaaaa ttgagaaata ttattttaac rtcattcttg tgccccagga gtgcttacag 60
   g 61
<210> 825
   <211> 61
   <212> DNA
   <213> Homo Sapiens
<400> 825
   gggaagaaat tggtgtagga tccaaactat ytctacttta ttcagagaga ccatggatag 60
   g 61
<210> 826
   <211> 61
   <212> DNA
   <213> Homo Sapiens
<400> 826
   gcacctgatg tgataatcac taggttggag wttgcagaga tgagataatc tttgctggaa 60
   g 61
<210> 827
   <211> 61
   <212> DNA
   <213> Homo Sapiens
<400> 827
   aaaattgaat atgtttttcc tcctgcctaa racaaaacta cttaagggca aaaatttaaa 60
   t 61
<210> 828
   <211> 61
   <212> DNA
   <213> Homo Sapiens
<400> 828
   ttctagtcaa tattttgacg cacctaacaa ygatagttaa catttacata gtgctcattt 60
   t 61
<210> 829
   <211> 61
   <212> DNA
   <213> Homo Sapiens
<400> 829
   tgagctccct atttctctgt tccgccttcc rtccactcat gtaaacactg gcttccccca 60
   a 61
<210> 830
   <211> 61
   <212> DNA
   <213> Homo Sapiens

<400> 830
   ccatcttctt aatatgattt ttccagtgca rtgttttcaa ttttgattaa atccagttta 60
   t 61
<210> 831
   <211> 61
   <212> DNA
   <213> Homo Sapiens
<400> 831
   tattggcaga attttaatca gataacccat ycttccctat tgaaacactt ccttcatttg 60
   a 61
<210> 832
   <211> 61
   <212> DNA
   <213> Homo Sapiens
<400> 832
   tctaggttct tcttgctgct ataaactgga ygtgacattt gccttcttgg accataaata 60
   c 61
<210> 833
   <211> 61
   <212> DNA
   <213> Homo Sapiens
<400> 833
   acgaggttgc tgccacttct cacatgcacc wcaacactgc tgttttacaa aaacttcaga 60
   t 61
<210> 834
   <211> 61
   <212> DNA
   <213> Homo Sapiens
<400> 834
   aatgatactc agagagaata agtatcttat ytaaggtcac aacactcatt aagtgaaaga 60
   a 61
<210> 835
   <211> 61
   <212> DNA
   <213> Homo Sapiens
<400> 835
   gatttggttt aaactctctt cttcgtgccc rtgcctgagg cagtgaggag taagcctcca 60
   t 61
<210> 836
   <211> 61
   <212> DNA
   <213> Homo Sapiens
<400> 836
   gtgctgggga tatggctgtg agtgagacag ytgaccgcat ggcatttgca ctcttatctg 60
   t 61
<210> 837
   <211> 61
   <212> DNA
   <213> Homo Sapiens
<400> 837
   cctccagcaa tctgatgact caaaaccctg stgagctgca ggctccctcc catggctgca 60
   g 61
<210> 838
   <211> 61
   <212> DNA
   <213> Homo Sapiens
<400> 838
   ccccaaaata gtgtgtacct gtatgcaaat rtgcaagcgt gtttatttaa ggggtggatg 60
   a 61
<210> 839
   <211> 61
   <212> DNA
   <213> Homo Sapiens
<400> 839
   tgaacagggg cttggtaaaa caaggttgag rtctactagg ttgcattccc aggaggttag 60
   g 61
<210> 840
   <211> 61
   <212> DNA
   <213> Homo Sapiens
<400> 840
   ccattaatta gactgtccct aagtatgaaa yatgggtgag gaaaagagac agagagtgaa 60
   a 61
<210> 841
   <211> 61
   <212> DNA
   <213> Homo Sapiens
<400> 841
   gatttgctct gaccatgtca attgttaaga mttagctgtc agctgacctc cgtggctgac 60
   t 61
<210> 842
   <211> 61
   <212> DNA
   <213> Homo Sapiens
<400> 842
   cattttcctc ttacatatga tggggctaca raaatctacc tccaaagaat tgttgtgaag 60
   a 61
<210> 843
   <211> 61
   <212> DNA
   <213> Homo Sapiens
<400> 843
   tgaatccctg ctgggttgcc tgggagaaaa yaggagaagt cagaaatgcc tggagggagc 60
   c 61
<210> 844
   <211> 61
   <212> DNA
   <213> Homo Sapiens
<400> 844
   agttttgggg gcaagagtgg acataaaaag yggaggatat ggcctgagga caccactcac 60
   t 61
<210> 845
   <211> 61
   <212> DNA
   <213> Homo Sapiens
<400> 845
   ttctaggatg gtagagagta gtcttcagcc ygagagccat ccctagggaa gtagctgaat 60
   t 61
<210> 846
   <211> 61
   <212> DNA
   <213> Homo Sapiens
<400> 846
   tttatctgtt tataagtacc aatacatcca rttccagtaa tgtgccacat atttgccaac 60
   t 61
<210> 847
   <211> 61
   <212> DNA
   <213> Homo Sapiens
<400> 847
   aaagggcaac aacaaaaagc agttagatct sattccacta tttgtattat atctcatatc 60
   c 61
<210> 848
   <211> 61
   <212> DNA
   <213> Homo Sapiens
<400> 848
   attactgttc cattgatgaa tcatattatg magtgtttga ccacaaagac tgcgtctgat 60
   g 61
<210> 849
   <211> 61
   <212> DNA
   <213> Homo Sapiens
<400> 849
   aagcaaattg ctatttgggg accatagaca wgaggtaagg ctgtaagcag agatagccca 60
   t 61
<210> 850
   <211> 61
   <212> DNA
   <213> Homo Sapiens
<400> 850
   gtgaatgtat agatagtgtc taacatagtg yttgatgcat agtagatcca gctcttcttg 60
   a 61
<210> 851
   <211> 61
   <212> DNA
   <213> Homo Sapiens
<400> 851
   taaacactcc tttgcctcag tctgctcatc ktgaaagtgg gaaagttgat aacgcctgtg 60
   t 61
<210> 852
   <211> 61
   <212> DNA
   <213> Homo Sapiens
<400> 852
   aaacaaatct ccaggtgatt cagtgcatat yaaaacctga gaagcacttt tgagaggcct 60
   g 61
<210> 853
   <211> 61
   <212> DNA
   <213> Homo Sapiens
<400> 853
   gggcgggact aatagagctt acattttcat ycagtttata aagcaatgca gactgagtat 60
   c 61
<210> 854
   <211> 61
   <212> DNA
   <213> Homo Sapiens
<400> 854
   ataattaaca catacatcat gatgacattt mccatagcac aataaaactt aactttcaaa 60
   t 61
<210> 855
   <211> 61
   <212> DNA
   <213> Homo Sapiens
<400> 855
   aatctgcaaa caagtaaaga gttctttcct rtaccagcca ctcagtgact tacttactta 60
   a 61
<210> 856
   <211> 61
   <212> DNA
   <213> Homo Sapiens
<400> 856
   gaattcaggc tgaagcttcc tgtgtacgtg rgcttggcca tgcctggctg cctggagttc 60
   a 61
<210> 857
   <211> 61
   <212> DNA
   <213> Homo Sapiens
<400> 857
   ttatcccctt atccaggaaa gttaacagaa ragacacgtt aagagaagaa aatattctct 60
   a 61
<210> 858
   <211> 61
   <212> DNA
   <213> Homo Sapiens
<400> 858
   ttgtgtatca acagatgaag tgacaaaacc rtccttagta aattaatgta actacaggca 60
   g 61
<210> 859
   <211> 61
   <212> DNA
   <213> Homo Sapiens
<400> 859
   gtggtggtgg caggcaagag gagccgtgga rgagaggaga aaggagaaga gaaggaaatc 60
   c 61
<210> 860
   <211> 61
   <212> DNA
   <213> Homo Sapiens
<400> 860
   aaaggggatt tatttcttaa aagctgccat ytggtagctg agataagatg ttttatataa 60
   t 61
<210> 861
   <211> 61
   <212> DNA
   <213> Homo Sapiens
<400> 861
   tgcctctcag gcaaatctcc ctctgctctc rtcccacgaa aagggggtgc ccaattccag 60
   t 61
<210> 862
   <211> 61
   <212> DNA
   <213> Homo Sapiens
<400> 862
   tagagaaatc atgtttattt gcacagtttt rgtattcaat ggagaaaaat atgatataga 60
   a 61
<210> 863
   <211> 61
   <212> DNA
   <213> Homo Sapiens
<400> 863
   agaaatcatg aaaggactga gaaaggagaa ygggggaagt atgaaggatg aaggggagat 60
   t 61
<210> 864
   <211> 61
   <212> DNA
   <213> Homo Sapiens
<400> 864
   ttccatcttt acagcctcat cttctaattg ytccccttgt ctgcaccaac ctggtatata 60
   c 61
<210> 865
   <211> 61
   <212> DNA
   <213> Homo Sapiens
<400> 865
   agccattatt tatttgctgt caacgtttca rtttatcaac caattgggat tatgagataa 60
   c 61
<210> 866
   <211> 61
   <212> DNA
   <213> Homo Sapiens
<400> 866
   tttgctaatg gaggaaattt atccttctac rctcatctta ctgtgactca aaactctcaa 60
   a 61
<210> 867
   <211> 61
   <212> DNA
   <213> Homo Sapiens
<400> 867
   aatgacaaga agaggaagag cagactgcag stactttggc taattttaag aaaccttggg 60
   t 61
<210> 868
   <211> 61
   <212> DNA
   <213> Homo Sapiens
<400> 868
   actctctcat tgtttacagg ctctataaaa ygccgacacc tctgagagaa ctttctggtt 60
   t 61
<210> 869
   <211> 61
   <212> DNA
   <213> Homo Sapiens
<400> 869
   gccaaaaatc aattaaaaag aaatagatga yttgaatagc caatattggt caagcaaagt 60
   g 61
<210> 870
   <211> 61
   <212> DNA
   <213> Homo Sapiens
<400> 870
   gactctatga ctgccgatat ggccgtttcc rtaaattcaa gagcatcaag gcccgcgacg 60
   t 61
<210> 871
   <211> 61
   <212> DNA
   <213> Homo Sapiens
<400> 871
   gggctgtaaa gggcacggca aggagagaac sagtgatgat gtgtagggtt ggggagagtg 60
   c 61
<210> 872
   <211> 61
   <212> DNA
   <213> Homo Sapiens
<400> 872
   ccctcaaaga ggtgaaaact aaaagttata ygtgaaatgg ttatttagaa taccaattta 60
   g 61
<210> 873
   <211> 61
   <212> DNA
   <213> Homo Sapiens
<400> 873
   gaaaagggga actggtgctt ttagatggag ygcataaaat tacctcacac catctgtgtc 60
   a 61
<210> 874
   <211> 61
   <212> DNA
   <213> Homo Sapiens
<400> 874
   atgggtgctt gctgaattgt tgggctttta rttgtttatt tacataactg tttaggggat 60
   a 61
<210> 875
   <211> 61
   <212> DNA
   <213> Homo Sapiens
<400> 875
   tgaggaaagc aaaacatttt caggttgata ygtcatgctt cttcatgctt ataaattata 60
   t 61
<210> 876
   <211> 61
   <212> DNA
   <213> Homo Sapiens
<400> 876
   cgtattggca cggttgtctt ccaaacccat ygatgccgga acatgggtca ggaagaacac 60
   a 61
<210> 877
   <211> 61
   <212> DNA
   <213> Homo Sapiens
<400> 877
   tgagggctgg cgagtttgaa atttgtgcaa yaggctggaa actcagccag gatttgatgt 60
   t 61
<210> 878
   <211> 61
   <212> DNA
   <213> Homo Sapiens
<400> 878
   tcagccagga tttgatgttt cagccttgag rtagaacttc tttcagaaac gtcagttctt 60
   g 61
<210> 879
   <211> 61
   <212> DNA
   <213> Homo Sapiens
<400> 879
   aatcaggcac aggtatagat tatttccacc rtaacacctg agtagccact tgcccaagac 60
   c 61
<210> 880
   <211> 61
   <212> DNA
   <213> Homo Sapiens
<400> 880
   gaagggtaaa gttaactttc taagacatac ragaggagtt gaaattagat gtgaatctag 60
   a 61
<210> 881
   <211> 61
   <212> DNA
   <213> Homo Sapiens
<400> 881
   ttgtatggtt tctgaaatgg atgctagaag mgataacttg aatgagtact tttcccctgg 60
   t 61
<210> 882
   <211> 61
   <212> DNA
   <213> Homo Sapiens
<400> 882
   cagtagtacg agtgttcagt gtaactgaag yataagctca cacatacaga aaaccaccat 60
   g 61
<210> 883
   <211> 61
   <212> DNA
   <213> Homo Sapiens
<400> 883
   aaagtttatt ttttaagggg cagttccata yagtaggtga cacaggttag gttcctccag 60
   a 61
<210> 884
   <211> 61
   <212> DNA
   <213> Homo Sapiens
<400> 884
   tcaacttatt caagacatat tcagcaatat yctataaggc agtgctcatt tactgaggct 60
   c 61
<210> 885
   <211> 61
   <212> DNA
   <213> Homo Sapiens
<400> 885
   ataatgagcc tgctttaatg tgatccaaat rttcatggga aactctaaag cagttttgaa 60
   g 61
<210> 886
   <211> 61
   <212> DNA
   <213> Homo Sapiens
<400> 886
   cggatgcatc ttttgtgtcc tctatgtgct kaactgctga gccccagctc gggttaagcg 60
   g 61
<210> 887
   <211> 61
   <212> DNA
   <213> Homo Sapiens
<400> 887
   tacaaatacc ttcactaaca ttcttttaca rtgtcaacaa gcatctaccc gtcaagagaa 60
   a 61
<210> 888
   <211> 61
   <212> DNA
   <213> Homo Sapiens
<400> 888
   atatggggca atacaaaagt ggtgagaaaa rttgatttgt ccactgttaa caagtcctat 60
   g 61
<210> 889
   <211> 61
   <212> DNA
   <213> Homo Sapiens
<400> 889
   gcctggcata tagtaggggt tcagtaattg rtgtacttgt tatttaagac tgagaaacat 60
   g 61
<210> 890
   <211> 61
   <212> DNA
   <213> Homo Sapiens
<400> 890
   ggtaaacgat ttttgagtgt gtaaaatgac rtagctttca tagcctgatt ttatggaata 60
   c 61
<210> 891
   <211> 61
   <212> DNA
   <213> Homo Sapiens
<400> 891
   aattgctcct ttcatttatt tctgaaggta ycaaagcaga gagagtttct tttaaatgaa 60
   g 61
<210> 892
   <211> 61
   <212> DNA
   <213> Homo Sapiens
<400> 892
   tgtaactatg gttaaggact tgaaaaactt yatataggcc ttcctatttg atctgtaatt 60
   a 61
<210> 893
   <211> 61
   <212> DNA
   <213> Homo Sapiens
<400> 893
   caccttaaaa tcctctgtat cttggaaatg yatgtcagat tggcaaaaat ttaaggtatt 60
   t 61
<210> 894
   <211> 61
   <212> DNA
   <213> Homo Sapiens
<400> 894
   actatctggt ttcctttggc ctatcactaa ycttgatcag ttttctgagc tagaaacttt 60
   t 61
<210> 895
   <211> 61
   <212> DNA
   <213> Homo Sapiens
<220>
   <221> misc_feature
   <222> (31)..(31)
   <223> n is a, c, g, or t
<400> 895
   ttccacttct tccaagttta tcagtcagca ntgcaccaca gagaagagac ctcccttatt 60
   c 61
<210> 896
   <211> 61
   <212> DNA
   <213> Homo Sapiens
<400> 896
   tgtctgtgca aggacaagga aaagaatttc yctactggct cctgccccct ctagttcagg 60
   g 61
<210> 897
   <211> 61
   <212> DNA
   <213> Homo Sapiens
<400> 897
   cactgaattt gtcaacttgg gtaccactga ygacaatatc cagagcagtt ttggcaaggc 60
   a 61
<210> 898
   <211> 61
   <212> DNA
   <213> Homo Sapiens
<400> 898
   gaaaagtgga gtcctagtaa gagtgcttta sactttagga aaatcttcaa caaatatgag 60
   t 61
<210> 899
   <211> 61
   <212> DNA
   <213> Homo Sapiens
<400> 899
   cagggatggc tagagacagg aaatgctcca yaggtaatgg cctagctggc cggctggcat 60
   t 61
<210> 900
   <211> 61
   <212> DNA
   <213> Homo Sapiens
<400> 900
   agcctggggt ggcccaaccg acctttgagg mcattccaac agtctggcag catctgcagt 60
   g 61
<210> 901
   <211> 61
   <212> DNA
   <213> Homo Sapiens
<400> 901
   ggaggcggca gcagcaaagt cccaagaaca magtgctggt gtaggagtca ggcccgcatc 60
   t 61
<210> 902
   <211> 61
   <212> DNA
   <213> Homo Sapiens
<400> 902
   ctgaaaggtt cttcaaagtc aggtccctaa saaagctgtt ccatctccag ctcagcgtgt 60
   t 61
<210> 903
   <211> 61
   <212> DNA
   <213> Homo Sapiens
<400> 903
   aagcagcctc cctctgttac tactataaac rcccattctg caggcagtgt gagggcacag 60
   c 61
<210> 904
   <211> 61
   <212> DNA
   <213> Homo Sapiens
<400> 904
   attccttgca gaagaaacaa acaaaagaga yagaataagc actgcagaaa atagaaaggt 60
   t 61
<210> 905
   <211> 61
   <212> DNA
   <213> Homo Sapiens
<400> 905
   ttcacctccc tggagaagtc ctgagcgcca ygatcccttc cttgagaatc atacaatact 60
   g 61
<210> 906
   <211> 61
   <212> DNA
   <213> Homo Sapiens
<400> 906
   ctcggcaacc actggggggg ccatgctatc raagatcagc ctgagtgttt attactggac 60
   g 61
<210> 907
   <211> 61
   <212> DNA
   <213> Homo Sapiens
<400> 907
   aatgaattcc tgggctcacc cataacaaag sctttgagag gcaaactctg caacagactg 60
   c 61
<210> 908
   <211> 61
   <212> DNA
   <213> Homo Sapiens
<400> 908
   acatttcaga agtgaaaggc tctttcagag rttattgctc acttctcgcc tctccgtgct 60
   g 61
<210> 909
   <211> 61
   <212> DNA
   <213> Homo Sapiens
<400> 909
   atgtggttct tgagtatgtc cagaattata wggctgttaa tgtgtcttga gccctgacct 60
   a 61
<210> 910
   <211> 61
   <212> DNA
   <213> Homo Sapiens
<400> 910
   tattttggcc tcagagagaa aaactataat yctgtgactt cttgggaaat gctaattgaa 60
   t 61
<210> 911
   <211> 61
   <212> DNA
   <213> Homo Sapiens
<400> 911
   taaagatctt cagaacagct tggtccttta ytctgaaact cttgtagctt aagcatcttt 60
   c 61
<210> 912
   <211> 61
   <212> DNA
   <213> Homo Sapiens
<400> 912
   gggtcaggag ctgagaaaca gttttctaaa yacccagttg gcacacatca tggcatttga 60
   c 61
<210> 913
   <211> 61
   <212> DNA
   <213> Homo Sapiens
<400> 913
   agtacgtgtt cgccatctgg tggtaaagtc rtggagacac aggctgtact gaccctagag 60
   a 61
<210> 914
   <211> 61
   <212> DNA
   <213> Homo Sapiens
<400> 914
   ctcccactaa tcatcatgtg agattcagac yctttaagga tgtagatgct aaagggaagg 60
   c 61
<210> 915
   <211> 61
   <212> DNA
   <213> Homo Sapiens
<400> 915
   cagaatttat ttcctgatgt ctcccagaat racatccttg tggcacctga tctaatccat 60
   a 61
<210> 916
   <211> 61
   <212> DNA
   <213> Homo Sapiens
<400> 916
   ctgcgtcaca ctgaggagtc aagacgctat yctgtcagcc aggggctttc ctagccaggg 60
   g 61
<210> 917
   <211> 61
   <212> DNA
   <213> Homo Sapiens
<400> 917
   aggctggtgt gtgaggttcc taatctccca rtcacagaag aaattatgtt caagacagct 60
   c 61
<210> 918
   <211> 61
   <212> DNA
   <213> Homo Sapiens
<400> 918
   ggactagcaa gtccagcctc accgtgtatc rccaaattgc tctccaaacg ataccaatct 60
   c 61
<210> 919
   <211> 61
   <212> DNA
   <213> Homo Sapiens
<400> 919
   aggctgtgac taagaaggtt ccagacttcc ygtctatcaa aggtgaggac tgaattagac 60
   t 61
<210> 920
   <211> 61
   <212> DNA
   <213> Homo Sapiens
<400> 920
   gttatgtccc ctaccttggt gcatgaagga raactacaca aagatgtgtg gtatcaactc 60
   a 61
<210> 921
   <211> 61
   <212> DNA
   <213> Homo Sapiens
<400> 921
   ttttgctgtg agtccacaga gcgagtatta yaaacccatt ttacagatga gggaactgag 60
   g 61
<210> 922
   <211> 47
   <212> DNA
   <213> Homo Sapiens
<400> 922
   atttcgtcac tttccaktta ttgaatacat accaggaact tggccaa 47
<210> 923
   <211> 61
   <212> DNA
   <213> Homo Sapiens
<400> 923
   gtgtcacagt gcctggccca cagtaggtgc ytgaaaaata ttaactgagt taataacaac 60
   a 61
<210> 924
   <211> 61
   <212> DNA
   <213> Homo Sapiens
<400> 924
   tgacagaaaa ggatatatgt tagggacagt rgtagagatg aagtgaggaa catgtttccc 60
   a 61
<210> 925
   <211> 61
   <212> DNA
   <213> Homo Sapiens
<400> 925
   gacacgagct tgcctccatg taaaaagcgc rtcagagaac tacagaccaa gctcttggga 60
   t 61
<210> 926
   <211> 61
   <212> DNA
   <213> Homo Sapiens
<400> 926
   aggtttttct acatacaaaa tcatgttgtc rgcaaagagg tacaatctga cttcctcttt 60
   t 61
<210> 927
   <211> 61
   <212> DNA
   <213> Homo Sapiens
<400> 927
   atttacattt aaattaaaaa ttcacttcct ygtgtcacta gccacatatc aagtgctcaa 60
   t 61
<210> 928
   <211> 61
   <212> DNA
   <213> Homo Sapiens
<400> 928
   tagctagaga ttcagaagga tttaattctc ygaagcaatg tgatagttcc aatattagtc 60
   a 61
<210> 929
   <211> 61
   <212> DNA
   <213> Homo Sapiens
<400> 929
   aggattataa tcagcttcct acaaaaatga sggtgtagga atctcacacc cacagtaaat 60
   c 61
<210> 930
   <211> 61
   <212> DNA
   <213> Homo Sapiens
<400> 930
   cttatctgag ttcctttctt agaaaactga ygctcgggcc tcccagagag tataaaggaa 60
   c 61
<210> 931
   <211> 61
   <212> DNA
   <213> Homo Sapiens
<400> 931
   tttatccagg ttctcattgt cgttttatta yctcctgctc ataatatgta gagacaaatg 60
   c 61
<210> 932
   <211> 61
   <212> DNA
   <213> Homo Sapiens
<400> 932
   agaggtcaac tggctctatc tggtggttca rggaagactt tctagaaggg gtgatgcctg 60
   a 61
<210> 933
   <211> 61
   <212> DNA
   <213> Homo Sapiens
<400> 933
   cctgtaccag ctgtgaaaac aatgagacac rttcagtaat agaatatagt gtgcttacaa 60
   a 61
<210> 934
   <211> 61
   <212> DNA
   <213> Homo Sapiens
<400> 934
   gctcctgtac tccccacaaa cctagttgat yccagaccag taatgtcttg gggacagtgt 60
   t 61
<210> 935
   <211> 61
   <212> DNA
   <213> Homo Sapiens
<400> 935
   attgcatcct tgagttgcat atgatgggat mtcttgaatc atttacattt tgaaaaaaga 60
   a 61
<210> 936
   <211> 61
   <212> DNA
   <213> Homo Sapiens
<400> 936
   gtttgaaaag cagtgacctc tctgtgcaaa ygattttttt ttccaaacag agctgaacaa 60
   g 61
<210> 937
   <211> 61
   <212> DNA
   <213> Homo Sapiens
<400> 937
   ggactacagg catgagccac tgcgaatggc statctttca aaacattatg aatgcttcct 60
   t 61
<210> 938
   <211> 61
   <212> DNA
   <213> Homo Sapiens
<400> 938
   gtggatatgt ccttggcatt gctgttgtgg stagtatagt gactatctct gaggaacagc 60
   a 61
<210> 939
   <211> 61
   <212> DNA
   <213> Homo Sapiens
<400> 939
   gtctcttgtg ttttcacttg acagttccca statttcaat atttgtaaat atttaaactt 60
   a 61
<210> 940
   <211> 61
   <212> DNA
   <213> Homo Sapiens
<400> 940
   cagtgttttg taatatatag gtacacagca raactggatc ttcaaagcct tcaaataggc 60
   t 61
<210> 941
   <211> 61
   <212> DNA
   <213> Homo Sapiens
<400> 941
   ttgacactaa acgacatggc tcgcctttta yattaattgc aaacacctct caactgtctc 60
   a 61
<210> 942
   <211> 61
   <212> DNA
   <213> Homo Sapiens
<400> 942
   agaaaagaaa taaacagaac agtaaactta sctgcaaaag aagtcatcca gaatattgga 60
   g 61
<210> 943
   <211> 61
   <212> DNA
   <213> Homo Sapiens
<400> 943
   aggcaggaga gaaataataa ccttttcata ytctttgccc agacttgagg aagcccaggg 60
   t 61
<210> 944
   <211> 61
   <212> DNA
   <213> Homo Sapiens
<400> 944
   acagcccaaa ttaagagctt ggcacccaat ycccacttgg actacaaatc acttcctctt 60
   g 61
<210> 945
   <211> 61
   <212> DNA
   <213> Homo Sapiens
<400> 945
   agagaggaga ataaatcttt gcaggctgct stgtccttcc attgggggtt acagttcttc 60
   t 61
<210> 946
   <211> 61
   <212> DNA
   <213> Homo Sapiens
<400> 946
   agtactggga agatagaagg tggtcaaagc rtatgtatgg aatgaacact ggaaatcagt 60
   a 61
<210> 947
   <211> 61
   <212> DNA
   <213> Homo Sapiens
<400> 947
   aaaaaatcaa ttcagttgac attaaagttg raagtgtact cctcttatcc aacaacaaca 60
   a 61
<210> 948
   <211> 61
   <212> DNA
   <213> Homo Sapiens
<400> 948
   gtatccacaa aatctcactc gggctagacc kcactgtctt tttttgttgt tgttgttttg 60
   a 61
<210> 949
   <211> 61
   <212> DNA
   <213> Homo Sapiens
<400> 949
   cccgccaggc cccacctcca gcatttgaca rttcgacctg aaatttctca gggatgtata 60
   t 61
<210> 950
   <211> 61
   <212> DNA
   <213> Homo Sapiens
<400> 950
   agtcctgaga aaaatgccag acaaatccca wccgagaggc attctacaaa atgcctgatt 60
   a 61
<210> 951
   <211> 61
   <212> DNA
   <213> Homo Sapiens
<400> 951
   gtctatgcag atagcctttt ctcttacacc matttgaggt attcataccc atatatcttc 60
   t 61
<210> 952
   <211> 61
   <212> DNA
   <213> Homo Sapiens
<400> 952
   ggctgtcttc cagaaatgat tttactctac kttctgcttt catcctcacc tcttctcctc 60
   t 61
<210> 953
   <211> 61
   <212> DNA
   <213> Homo Sapiens
<400> 953
   ggcacatgcg tgtacaagaa gagcccattc rcaaatcgga tcaagaaaat gcagttgcgc 60
   c 61
<210> 954
   <211> 61
   <212> DNA
   <213> Homo Sapiens
<400> 954
   catcccggtg ggtttgcttg gcctttagga ygtgtgacta cagcttttgg gtaggtacag 60
   g 61
<210> 955
   <211> 61
   <212> DNA
   <213> Homo Sapiens
<400> 955
   tctatcccta caataagggt gaaagaattt yagctttttt gtgagtttta actcttaccg 60
   g 61
<210> 956
   <211> 61
   <212> DNA
   <213> Homo Sapiens
<400> 956
   agaagcaccc aggcctgtaa tcatccagag rctaggcagc ccagcaaaaa ccttacactg 60
   t 61
<210> 957
   <211> 61
   <212> DNA
   <213> Homo Sapiens
<400> 957
   ttcaaggagt ggtctaatcc ctggactgct ktctgagtat cagtaattag taagcaggcc 60
   a 61
<210> 958
   <211> 61
   <212> DNA
   <213> Homo Sapiens
<400> 958
   agggtggtcc ccttgccagt ttgctgtctg yagctaaaag ccacatcttg atcccctagg 60
   t 61
<210> 959
   <211> 61
   <212> DNA
   <213> Homo Sapiens
<400> 959
   agaaacattg tacccattta tatccacacc yctgaaagat gcacctgaat catttgaatc 60
   t 61
<210> 960
   <211> 61
   <212> DNA
   <213> Homo Sapiens
<400> 960
   aaactttaaa aagtaaactt ctcaaacttg rcaggttcca acactgactt aaagctaatt 60
   g 61
<210> 961
   <211> 61
   <212> DNA
   <213> Homo Sapiens
<400> 961
   atcaaatatt acataacttc agaaaacatt rttataggaa aggcatgaat atcctagaaa 60
   g 61
<210> 962
   <211> 61
   <212> DNA
   <213> Homo Sapiens
<400> 962
   gtctccaaag ttgcagcctc atctctcaaa mctgagtgct ctgcttggtg tcactgctcc 60
   a 61
<210> 963
   <211> 61
   <212> DNA
   <213> Homo Sapiens
<400> 963
   aatacttaat atgttttagt ataacttatc wgcccaaata actgcataag atattagata 60
   a 61
<210> 964
   <211> 61
   <212> DNA
   <213> Homo Sapiens
<400> 964
   gtttctcagt tgaatattgg tattaggtca rgaagggctg gagtgggagg agtaagacag 60
   t 61
<210> 965
   <211> 61
   <212> DNA
   <213> Homo Sapiens
<400> 965
   aagaactggt tggctctgtt ctcccctttt ycatgacatt gtctctttct gtactatcac 60
   c 61
<210> 966
   <211> 61
   <212> DNA
   <213> Homo Sapiens
<400> 966
   aatggaagcc aagatactgg atcacgtcta rgaaaattta aatatgctag ctacactcaa 60
   t 61
<210> 967
   <211> 61
   <212> DNA
   <213> Homo Sapiens
<400> 967
   tcttatctgg ggattttcag ttaaataatc rgacaatgta tatgattgat ttctatactg 60
   c 61
<210> 968
   <211> 61
   <212> DNA
   <213> Homo Sapiens
<400> 968
   tcaaaatgca aatatgaaaa ctatcacctt scaaccacgg ataacactta aatgaattac 60
   c 61
<210> 969
   <211> 61
   <212> DNA
   <213> Homo Sapiens
<400> 969
   tatatacaat ggcctaaaaa tatgtaacgt rgaccctgac tgggaagaaa tctaagaaaa 60
   a 61
<210> 970
   <211> 61
   <212> DNA
   <213> Homo Sapiens
<400> 970
   ccagagggga gggatggggt cgaaatcgtt mtttagagag atccttttgg cagagctgta 60
   g 61
<210> 971
   <211> 61
   <212> DNA
   <213> Homo Sapiens
<400> 971
   tgaatacata gactagaaat aaggtacctc rgttcttacc tggccacatt aattggcact 60
   c 61
<210> 972
   <211> 61
   <212> DNA
   <213> Homo Sapiens
<400> 972
   aacatggaca agttggaaga ggagcgactc yaaagtttgg caatggagcc cggagttttg 60
   a 61
<210> 973
   <211> 61
   <212> DNA
   <213> Homo Sapiens
<400> 973
   tttcaaacaa ataattttcc ccacgtttta wcatctctat aatctgcata cgggttttca 60
   c 61
<210> 974
   <211> 61
   <212> DNA
   <213> Homo Sapiens
<400> 974
   tcacttgttt gtcgttggtc cagtgctaac scagcttctt ggaggaggaa accctggaaa 60
   g 61
<210> 975
   <211> 61
   <212> DNA
   <213> Homo Sapiens
<400> 975
   agatgggttg gggcagatcc agcagattac rttactgtga gggggtggtg gggaacacag 60
   c 61
<210> 976
   <211> 61
   <212> DNA
   <213> Homo Sapiens
<400> 976
   ggatcctcct ctgcctgttg atcagtcttt mactttactt ctccctcagc tcacctcttc 60
   a 61
<210> 977
   <211> 61
   <212> DNA
   <213> Homo Sapiens
<400> 977
   gacaagtatg gtctaaggaa aaaacaagga wgaactcaag caagagttat cacattggcc 60
   a 61
<210> 978
   <211> 61
   <212> DNA
   <213> Homo Sapiens
<400> 978
   gggtgattca tttcttaaat ttatctttga sagttgctga aaatctcaca gcttgtattc 60
   t 61
<210> 979
   <211> 61
   <212> DNA
   <213> Homo Sapiens
<400> 979
   tttgtaggcg ttcaaagacc ttctgaatcc racctctgac acttctttgc aatccgaaat 60
   c 61
<210> 980
   <211> 61
   <212> DNA
   <213> Homo Sapiens
<400> 980
   actgatctta ttccttcttc attaaatgcg ytcaagtcag gaaaaggggt gatgacgatg 60
   a 61
<210> 981
   <211> 61
   <212> DNA
   <213> Homo Sapiens
<400> 981
   cagattctct agggactgat cagaatctgc rttaaaccaa gctctccagg caactgatgc 60
   t 61
<210> 982
   <211> 61
   <212> DNA
   <213> Homo Sapiens
<400> 982
   agatgggaag aatgctgagg gacccaaaga rcatcaaagc cgatcgctct aaaactcggc 60
   g 61
<210> 983
   <211> 61
   <212> DNA
   <213> Homo Sapiens
<400> 983
   ttggttatgg ggccagcttg agaatccata ygtcctgact acctgcacca cttctccttt 60
   t 61
<210> 984
   <211> 61
   <212> DNA
   <213> Homo Sapiens
<400> 984
   gaactctcca ggtttagaat atcttcaatt rtgggttcct tattattttt gtcttttttc 60
   t 61
<210> 985
   <211> 61
   <212> DNA
   <213> Homo Sapiens
<400> 985
   cttccatggg tggaatcatt ggaggtccca rattaagagg tcatgtattc aatattcaat 60
   a 61
<210> 986
   <211> 61
   <212> DNA
   <213> Homo Sapiens
<400> 986
   gtaatcaagc ccttctacca tttagttgaa ratgagagat tgggggattt gacctcataa 60
   a 61
<210> 987
   <211> 61
   <212> DNA
   <213> Homo Sapiens
<400> 987
   ccatggcagt ggctctgata accaagacag stcctgaatg actaacaggg ctggtagcgt 60
   c 61
<210> 988
   <211> 61
   <212> DNA
   <213> Homo Sapiens
<400> 988
   ataaattctg caagtgagtt taatgttcac raaaatcatc aacagcaaat gtagatgcat 60
   g 61
<210> 989
   <211> 61
   <212> DNA
   <213> Homo Sapiens
<400> 989
   ttttaacttg aatatcaagc tcgctatgca rtttgactta tggagtgctg cgggaaacct 60
   g 61
<210> 990
   <211> 61
   <212> DNA
   <213> Homo Sapiens
<400> 990
   cccaatttcc tgatttttat tcagcctgaa wcactgctca ctttgccagg gcagccctgt 60
   g 61
<210> 991
   <211> 61
   <212> DNA
   <213> Homo Sapiens
<400> 991
   ccaatggcca ggccagtaac ccagcccagt kaaatatgaa tctaaggggg tgtgaaccta 60
   g 61
<210> 992
   <211> 61
   <212> DNA
   <213> Homo Sapiens
<400> 992
   gggtttgaag tttcctccta aatctccgca ytgaatagga ctcaaggtta agtggcttct 60
   t 61
<210> 993
   <211> 61
   <212> DNA
   <213> Homo Sapiens
<400> 993
   agttatacca gagctgaact ccttgaagga rgtgtgtagg tctctcaggg actgctcttg 60
   g 61
<210> 994
   <211> 61
   <212> DNA
   <213> Homo Sapiens
<400> 994
   atccagcttt ttaagattta ccctgacaga ytgactctca cccaacaaaa gcttccgaca 60
   t 61
<210> 995
   <211> 61
   <212> DNA
   <213> Homo Sapiens
<400> 995
   tggtgcaaaa ggaagttaca ggactaggga sttctgaatt atttatcttt ctccttccat 60
   t 61
<210> 996
   <211> 61
   <212> DNA
   <213> Homo Sapiens
<400> 996
   gtcaaatgcc tccatggtac tcagtaatta rccacagatc tactctagca ctctacattc 60
   a 61
<210> 997
   <211> 61
   <212> DNA
   <213> Homo Sapiens
<400> 997
   ctgtagactg aaatggcaca tctcccaaaa sttgtaggac taggcgaaat ccctgcaagt 60
   g 61
<210> 998
   <211> 61
   <212> DNA
   <213> Homo Sapiens
<400> 998
   tgtcaccagc aaggtgtcaa gcctctttcg rgaacagcag tcgctgctgg actgcatcct 60
   c 61
<210> 999
   <211> 61
   <212> DNA
   <213> Homo Sapiens
<400> 999
   gcctggccag gcgtgaacat tttaacagtc rtgaaagatt ccattgatgc gcctgtttta 60
   c 61
<210> 1000
   <211> 61
   <212> DNA
   <213> Homo Sapiens
<400> 1000
   cagccctggg aagggaaggg aggtgctgtc staactctgg tcttacaaat ggactccagc 60
   c 61
<210> 1001
   <211> 61
   <212> DNA
   <213> Homo Sapiens
<400> 1001
   agtaaatcaa ggaatgactc caggactgat sgtaaggaca ccagtcacgt cctcccttga 60
   c 61
<210> 1002
   <211> 61
   <212> DNA
   <213> Homo Sapiens
<400> 1002
   catggccacc ctgtcaatct tgagggccat rgagtgacat ttgggtgccc tgtggtgccc 60
   a 61
<210> 1003
   <211> 61
   <212> DNA
   <213> Homo Sapiens
<400> 1003
   gtctttggag agacctcggg aagatgacct ytgaaccaca gctgctagaa tcctgggcca 60
   t 61
<210> 1004
   <211> 61
   <212> DNA
   <213> Homo Sapiens
<400> 1004
   agatcagaaa tattcggagg aagaattgtg sctgtactga acatggacag attttttctt 60
   g 61
<210> 1005
   <211> 61
   <212> DNA
   <213> Homo Sapiens
<400> 1005
   accacctcga atttggaaat actttcttca yttaacagag aggttgagta aattgcctca 60
   g 61
<210> 1006
   <211> 61
   <212> DNA
   <213> Homo Sapiens
<400> 1006
   gaccgaaagc agtctgaaaa tgtctgaaaa ragtcggaaa atgtgtgtgt gtgtgtgtgt 60
   g 61
<210> 1007
   <211> 61
   <212> DNA
   <213> Homo Sapiens
<400> 1007
   gtggggaggt ttacaaggag aaaaactgaa rtctctgcca acaaccctgt gtgtgagctt 60
   a 61
<210> 1008
   <211> 61
   <212> DNA
   <213> Homo Sapiens
<400> 1008
   gattacagtc actctatgca ttaatgaaaa ytcggctttt gtatttttac agattatttt 60
   g 61
<210> 1009
   <211> 61
   <212> DNA
   <213> Homo Sapiens
<400> 1009
   tacatgagtt aacaagacag cctgaggatc wttcaaagct cacaatatag atatatttta 60
   c 61
<210> 1010
   <211> 61
   <212> DNA
   <213> Homo Sapiens
<400> 1010
   aactcccaca ttaaattttg caattgcccc ytaaaattac atagccagta cagttcctga 60
   c 61
<210> 1011
   <211> 61
   <212> DNA
   <213> Homo Sapiens
<400> 1011
   cagaaaggct ggaaggaggg gacccaaaca rgaccttgaa ggttcattct ccattgagaa 60
   g 61
<210> 1012
   <211> 61
   <212> DNA
   <213> Homo Sapiens
<400> 1012
   aaccaaggcc acacccctag caagaggacc kgctttcacc agtaactgaa gctgtgcaga 60
   g 61
<210> 1013
   <211> 61
   <212> DNA
   <213> Homo Sapiens
<400> 1013
   agaactgctt gaacccaggg gttcaaggcc rcagtaagct aggatcacac cattgcattc 60
   c 61
<210> 1014
   <211> 61
   <212> DNA
   <213> Homo Sapiens
<400> 1014
   tattgggtga caactctgtt tcaggtacta ygctagtctc ttcccacaag gacctcacag 60
   c 61
<210> 1015
   <211> 61
   <212> DNA
   <213> Homo Sapiens
<400> 1015
   cctttgttgc cactcaagtc tagcagtctt maatatttgt cgaatgagtg aatgaagtca 60
   a 61
<210> 1016
   <211> 61
   <212> DNA
   <213> Homo Sapiens
<400> 1016
   tgcaatgtgt ttggcattgt gataagctcc rtaaagatga tctcatttca tcttccctgc 60
   a 61
<210> 1017
   <211> 61
   <212> DNA
   <213> Homo Sapiens
<400> 1017
   aggaggagaa cactgtgtga gcctttgatt ytttaatcgc aactgagata gtcctagagg 60
   g 61
<210> 1018
   <211> 61
   <212> DNA
   <213> Homo Sapiens
<400> 1018
   atctcctcca atggagtagg cagtggtttt yttcaggcaa gacacagaaa gcaataacca 60
   t 61
<210> 1019
   <211> 61
   <212> DNA
   <213> Homo Sapiens
<400> 1019
   gctccttaac tccactggct cgtcaaagta mctgtaggag caagagtaca acagtgactt 60
   t 61
<210> 1020
   <211> 61
   <212> DNA
   <213> Homo Sapiens
<400> 1020
   aatacacagt gctaactagc agagtcacgc rtcggaagtg tgctgggccc ataaaataca 60
   c 61
<210> 1021
   <211> 61
   <212> DNA
   <213> Homo Sapiens
<400> 1021
   cagtgagctg gacaacatgt tcgttcatct yaccaacgtc gccatccaga aacacggggt 60
   g 61
<210> 1022
   <211> 61
   <212> DNA
   <213> Homo Sapiens
<400> 1022
   ccagtgagac gcagaaacgg atcctttgtc ygacttcctc cagattgcct ggggttggac        60
   c                                                                        61
<210> 1023
   <211> 61
   <212> DNA
   <213> Homo Sapiens
<400> 1023
   tcttcagtga cgcagagtga tcttcagtgt rtctaaataa ctttgtgatg atgttttcct        60
   t                                                                        61
<210> 1024
   <211> 61
   <212> DNA
   <213> Homo Sapiens
<400> 1024
   taccatctcc agctcttgaa aaactgctct mtaactcaac aaaaggagac accaaatcag        60
   a                                                                        61
<210> 1025
   <211> 61
   <212> DNA
   <213> Homo Sapiens
<400> 1025
   cccagctcaa attcgtgacc agggggccta ygggacctgg tcgtcttttc agatggacag        60
   t                                                                        61